# EUROPEAN PATENT APPLICATION

(11) **EP 2 818 466 A1**
(43) Date of publication of application: **31.12.2014**
(21) Application number: 14173599.3
(22) Date of filing: 24.06.2014
(51) Int. Cl.: C07D 333/54, C07D 405/12, C07D 307/79, H01L 51/50

(54) **Amine-based compound and organic light-emitting device including the same**

(30) Priority: 24.06.2013 KR 20130072718
(71) Applicant: Samsung Display Co., Ltd., Gyeonggi-Do (KR)
(72) Inventor: Kim, Myeong-Suk, Gyeonggi-Do (KR); Kim, Soung-Wook, Gyeonggi-Do (KR); Chu, Chang-Woong, Gyeonggi-Do (KR)
(74) Representative: Gulde & Partner

(57) **Abstract**

An amine-based compound is represented by Formula 1 below: 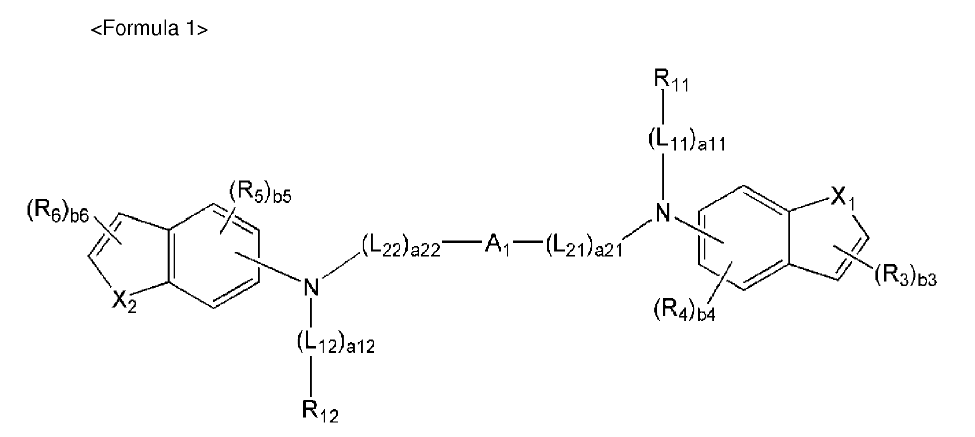 wherein A₁, X₁, X₂, R₃-R₆, R₁₁-R₁₂, L₁₁, L₁₂, L₂₁, L₂₂, a11, a12, a21, a22, and b3-b6 are as defined in the specification. An organic light-emitting device includes the amine-based compound.

## Description

One or more embodiments relate to an amine-based compound and an organic light-emitting device including the amine-based compound.

### DESCRIPTION OF THE RELATED ART

Organic light-emitting devices (OLEDs), which are self-emitting devices, have advantages such as wide viewing angles, excellent contrast, quick response, high brightness, and excellent driving voltage characteristics and can provide multicolored images.

An organic light-emitting device may include an anode, a cathode, and an emission layer disposed between the anode and the cathode. The organic light-emitting device may include a hole transport region between the anode and the emission layer, and an electron transport region between the emission layer and the cathode. Holes injected from the anode move to the emission layer via the hole transport region, while electrons injected from the cathode move to the emission layer via the electron transport region. Carriers such as holes and electrons recombine in the emission layer to generate excitons. When the excitons drop from an excited state to a ground state, light is emitted.

### SUMMARY

According to the present invention there is provided an amine-based compound represented by Formula 1 below: 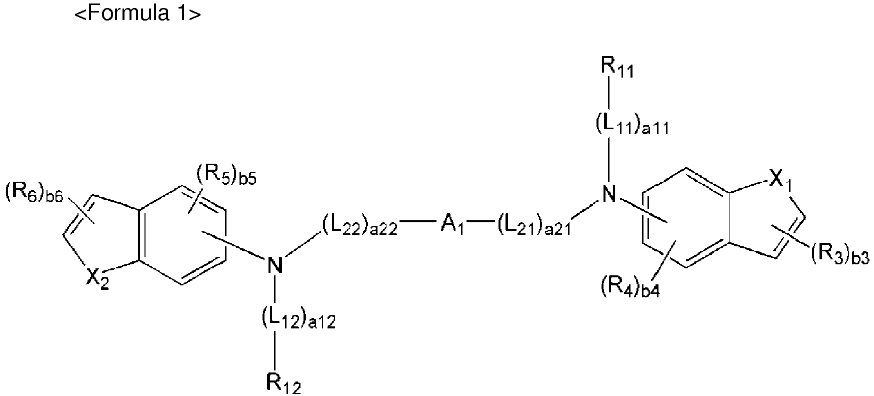 wherein, in Formula 1, A₁ is selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and - Si(Q₁₃)(Q₁₄)(Q₁₅);
X₁ is selected from N-[(L₁)ₐ₁-(R₁)_{b1}], S, and O;
X₂ is selected from N-[(L₂)ₐ₂-R₂], S, and O;
L₁, L₂, L₁₁, L₁₂, L₂₁, and L₂₂ are each independently selected from
a C₃-C₁₀ cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, and
a C₃-C₁₀ cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-_{C60} alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and -Si(Q₁₃)(Q₁₄)(Q₁₅);
a1, a2, a11, a12, a21, and a22 are each independently an integer from 0 to 5;
R₁, R₂, R₁₁, and R₁₂ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, and a C₂-C₆₀ alkynyl group,
a C₁-C₆₀ alkyl group, a C₂-C₆₀ alkenyl group, and a C₂-C₆₀ alkynyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and -N(Q₁)(Q₂), -Si(Q₃)(Q₄)(Q₅), and -B(Q₆)(Q₇);
Q₁₁ to Q₁₅, and Q₁ to Q₇ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
b3 and b6 are each independently 1 or 2; and
b1, b4 and b5 are each independently 1, 2, or 3.
A₁ may be selected from a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, and
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.
X₁ and X₂ may both be S, or X₁ and X₂ may both be O.
L₁, L₂, L₁₁, L₁₂, L₂₁, and L₂₂ may each independently be selected from a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substitute with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.
L₁, L₂, L₁₁, L₁₂, L₂₁, and L₂₂ may each independently be selected from the groups represented by Formulae 2-1 to 2-30 below: 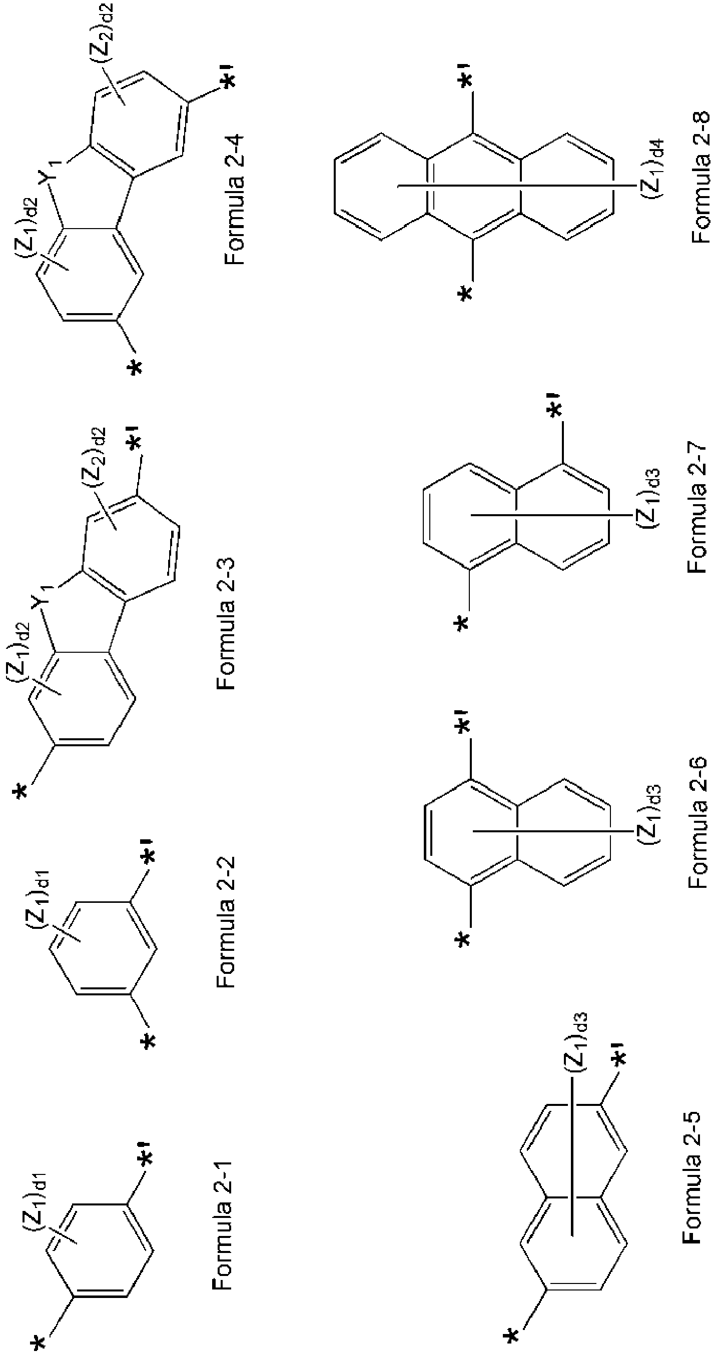 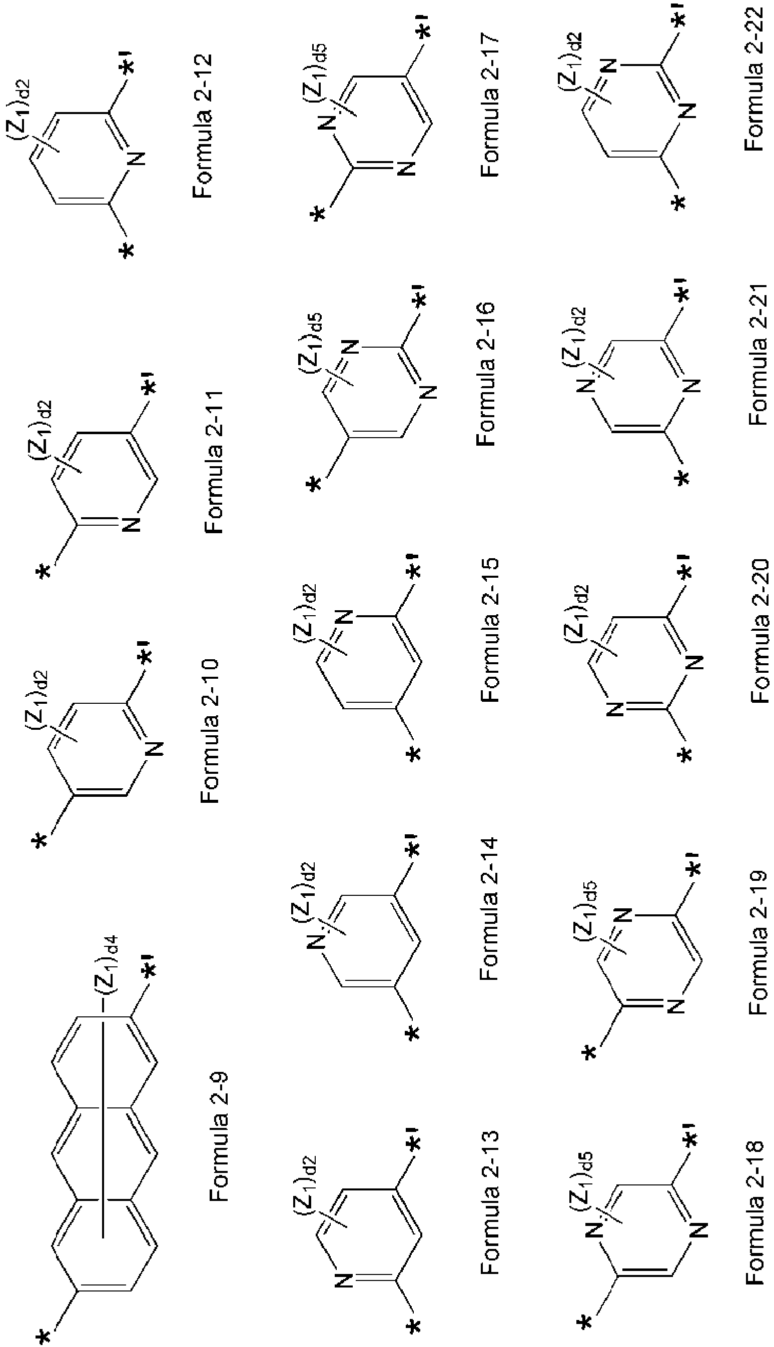 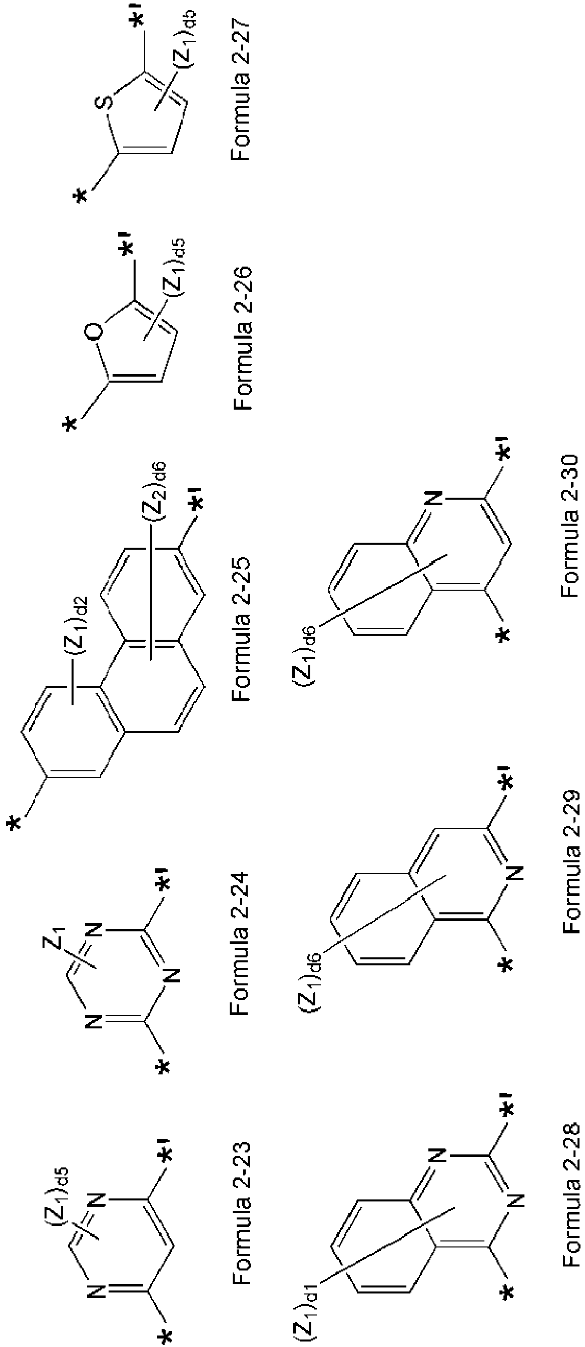
wherein, in Formulae 2-1 to 2-30,
Y₁ is O, S, S(=O), S(=O)₂, C(Z₃)(Z₄), N(Z₅), or Si(Z₆)(Z₇);
Z₁ to Z₇ are each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
d1 is an integer from 1 to 4;
d2 is an integer from 1 to 3;
d3 is an integer from 1 to 6;
d4 is an integer from 1 to 8;
d5 is 1 or 2;
d6 is an integer from 1 to 5; and
* and *' indicate binding sites of adjacent atoms.

L₁, L₂, L₁₁, L₁₂, L₂₁, and L₂₂ are each independently selected from the groups represented by Formulae 3-1 to 3-19 below: 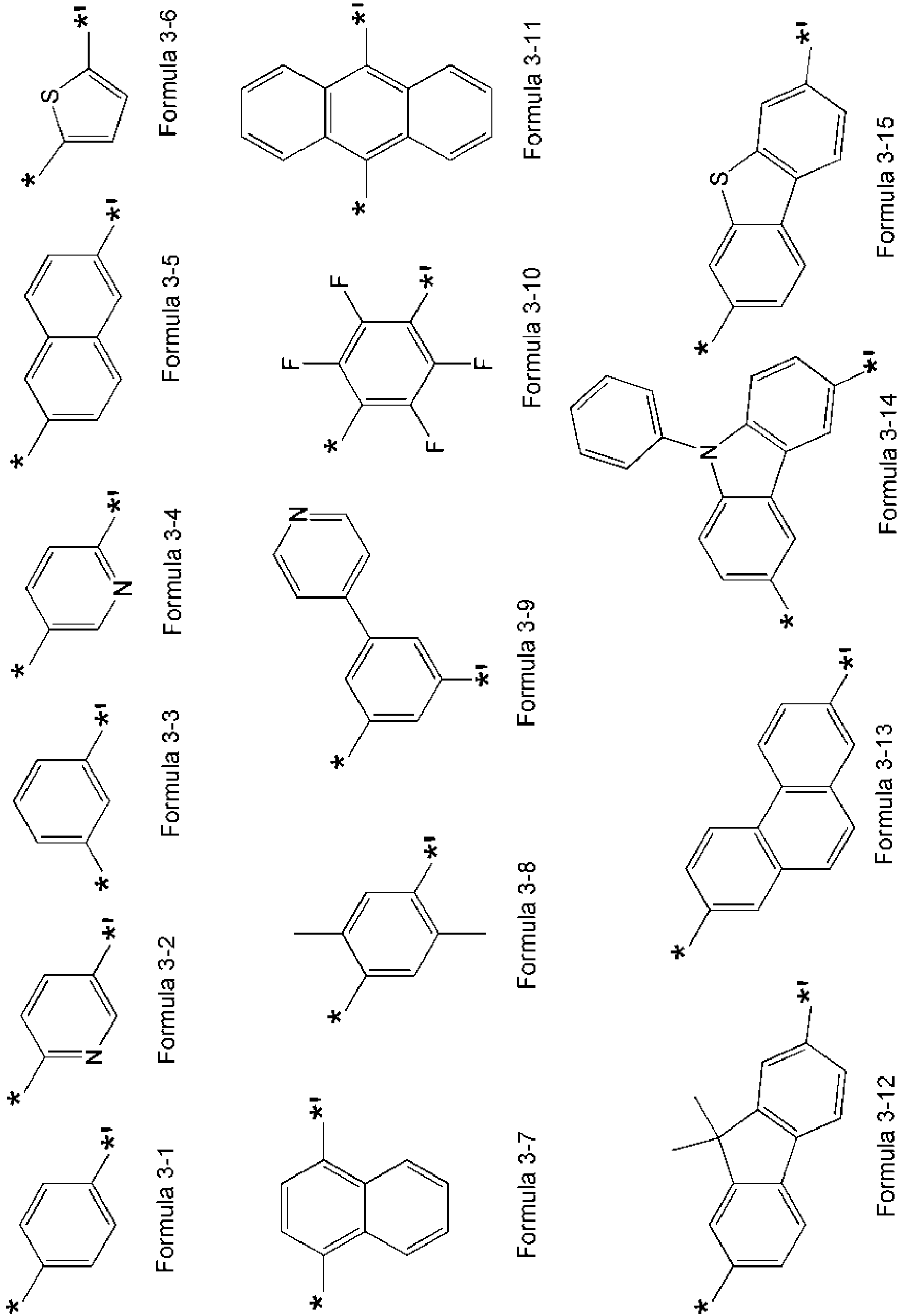 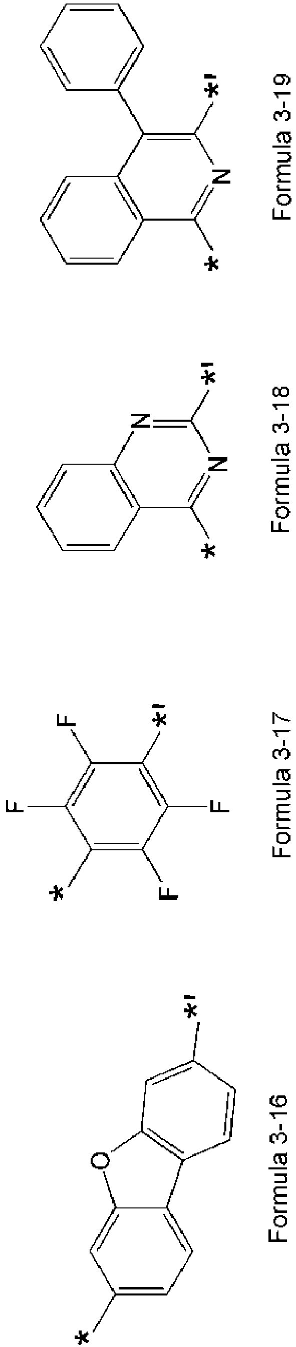
a1, a2, a11, a12, a21, and a22 may each independently be 0, 1, or 2.
R₁, R₂, R₁₁, and R₁₂ may each independently be selected from
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group, and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.
R₃ to R₆ may each independently be selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group ; and
-N(Q₁)(Q₂), and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group.

R₁, R₂, R₁₁, and R₁₂ may each independently be selected from the groups represented by Formulae 4-1 to 4-29 below; and
R₃ to R₆ may each independently be selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group,
a C₁-C₂₀ alkyl group and a C₁-C₂₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group,
the groups represented by Formulae 4-1 to 4-29 below, and
-N(Q₁)(Q₂) and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group: 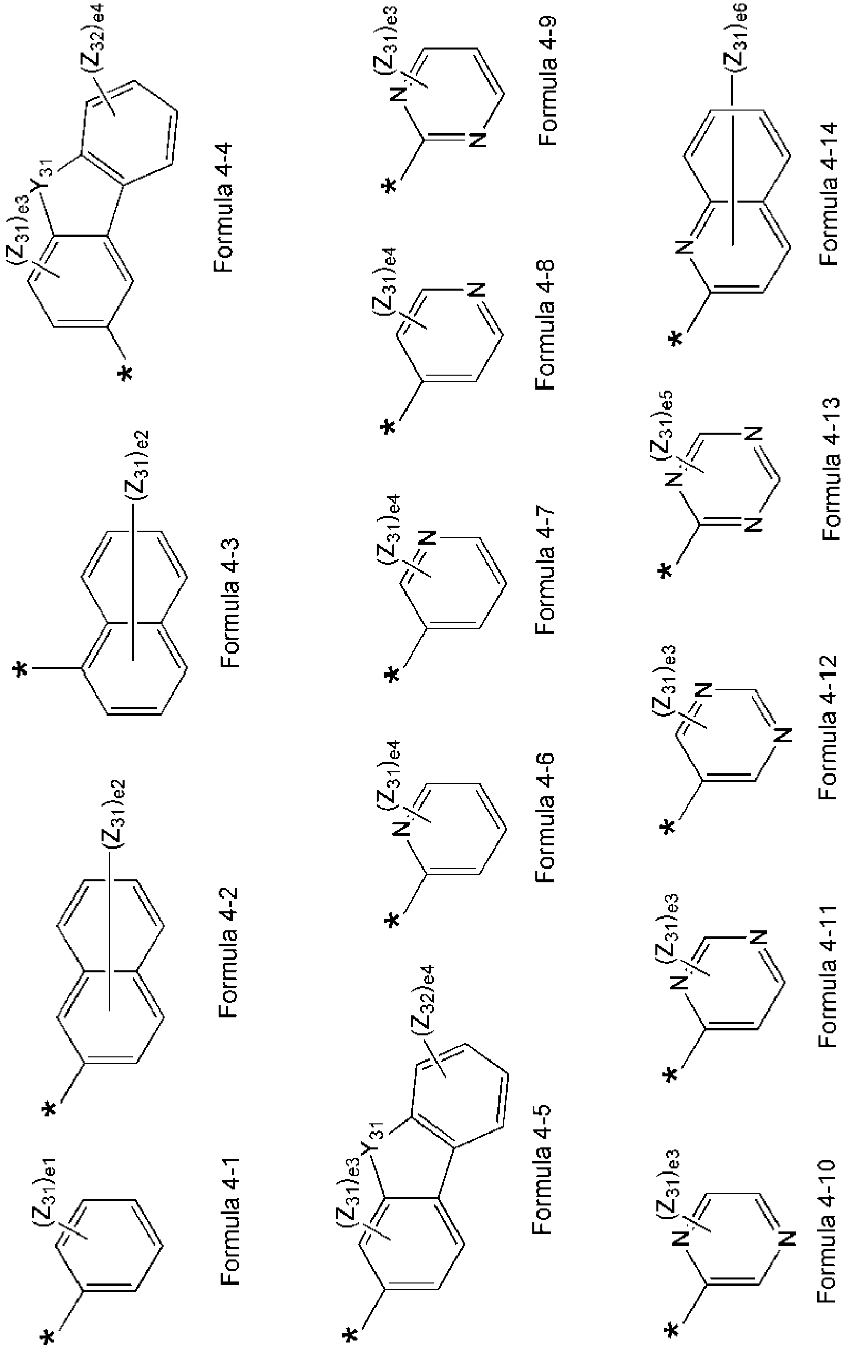 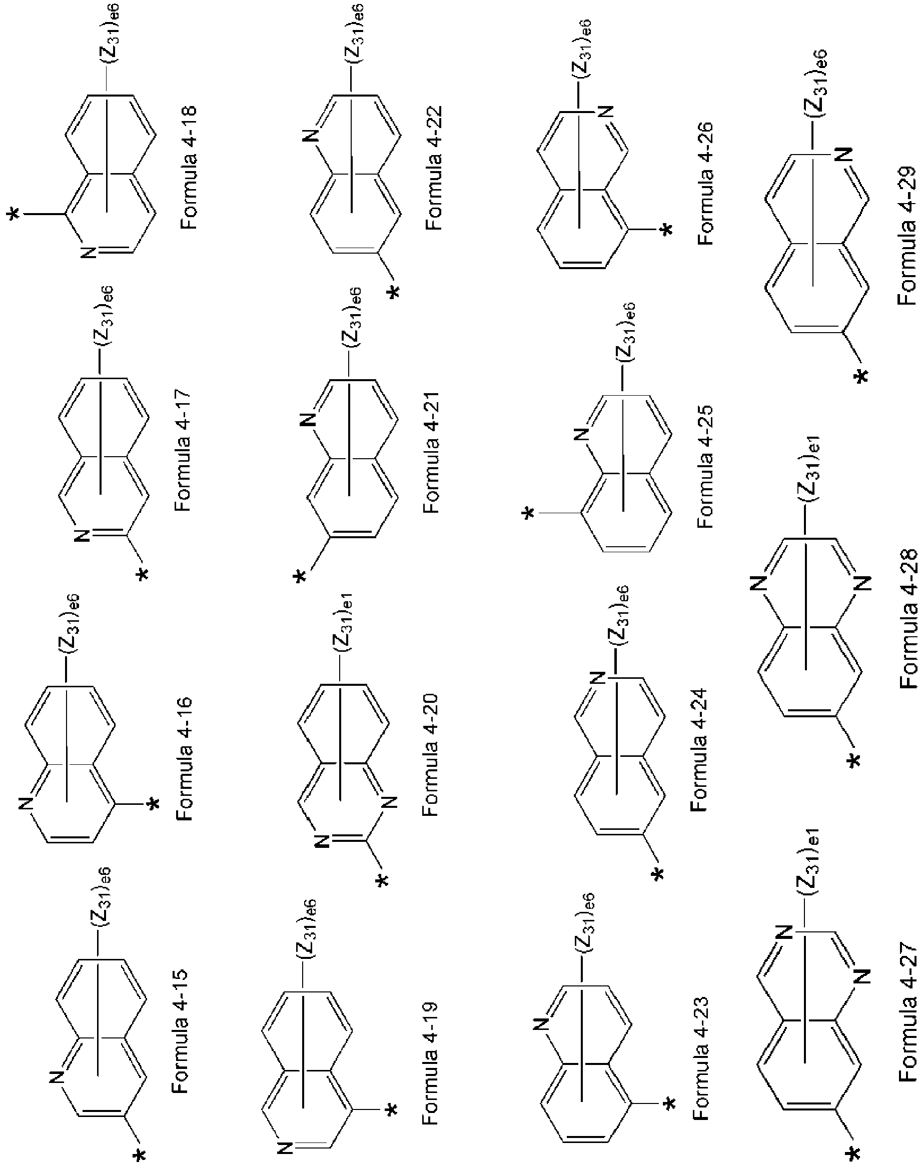
wherein, in Formulae 4-1 to 4-29,
Y₃₁ is O, S, C(Z₃₃)(Z₃₄), N(Z₃₅), or Si(Z₃₆)(Z₃₇);
Z₃₁ to Z₃₇ are each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
e1 is an integer from 1 to 5;
e2 is an integer from 1 to 7;
e3 is an integer from 1 to 3;
e4 is an integer from 1 to 4;
e5 is 1 or 2;
e6 is an integer from 1 to 6; and
* indicates a binding site of an adjacent atom.

R₁, R₂, R₁₁, and R₁₂ may each independently be selected from the groups represented by Formulae 5-1 to 5-36 below; and
R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group,
a C₁-C₂₀ alkyl group and a C₁-C₂₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group,
the groups represented by Formulae 5-1 to 5-36 below, and
-N(Q₁)(Q₂), and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group:

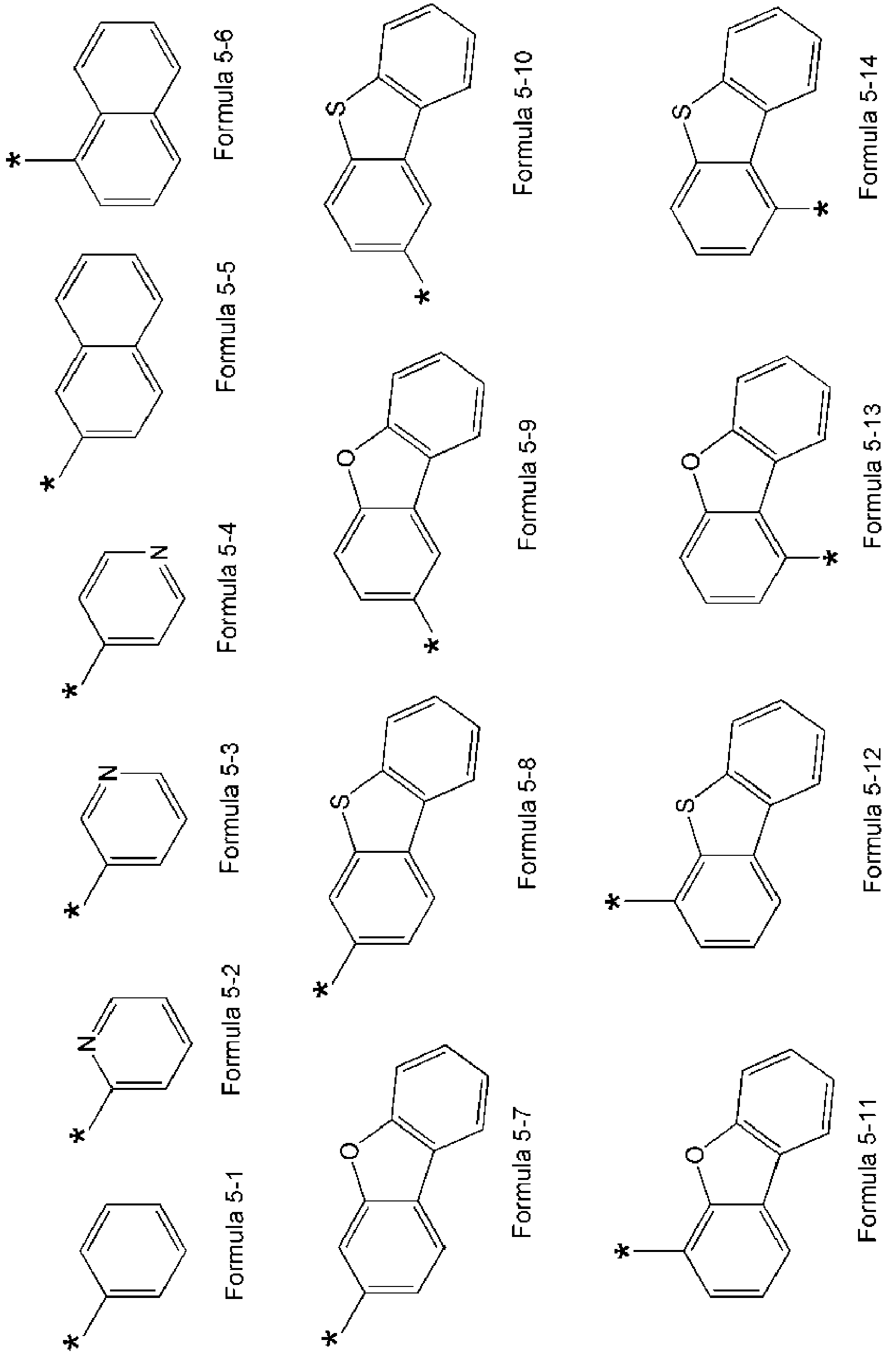

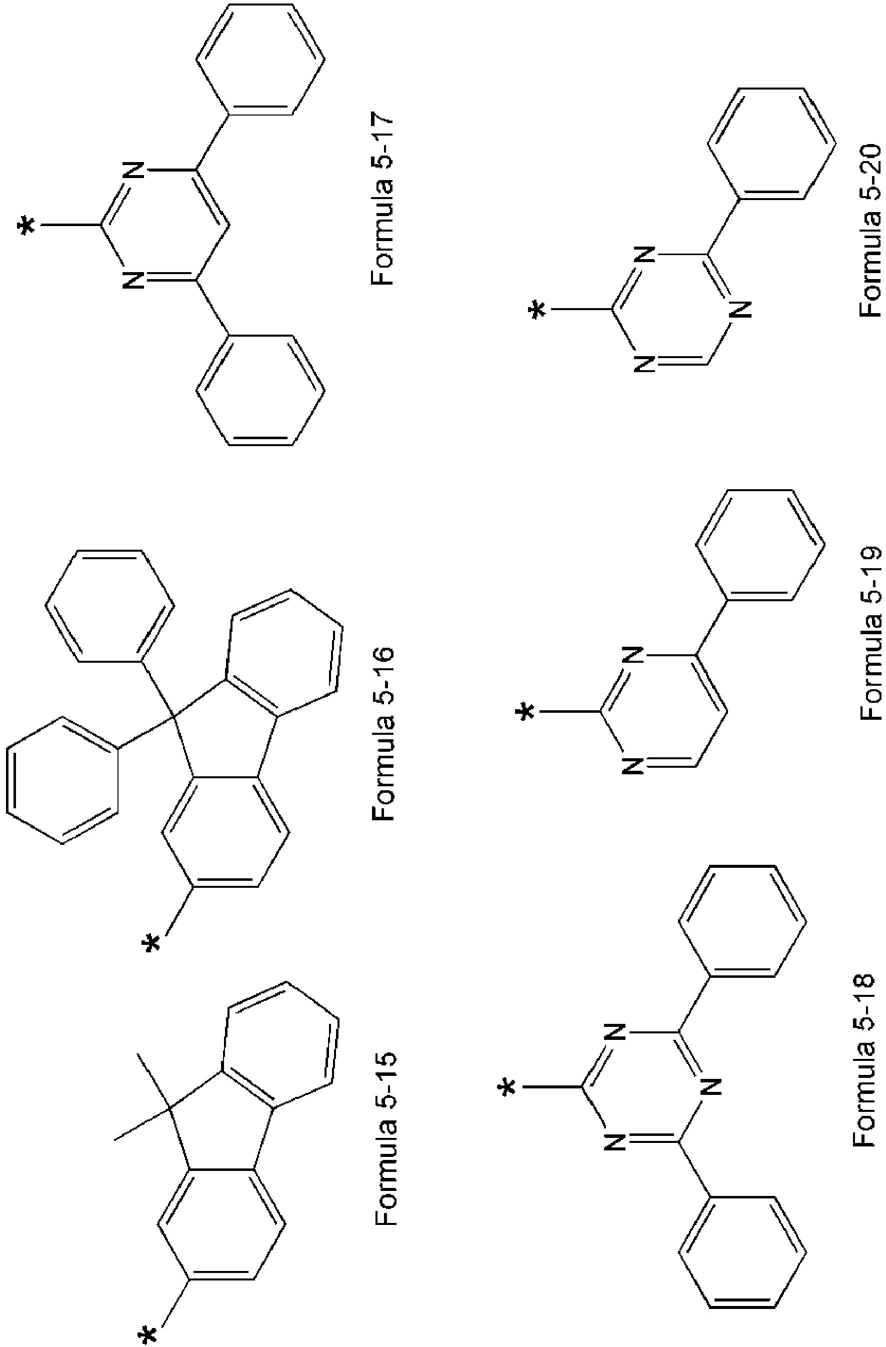

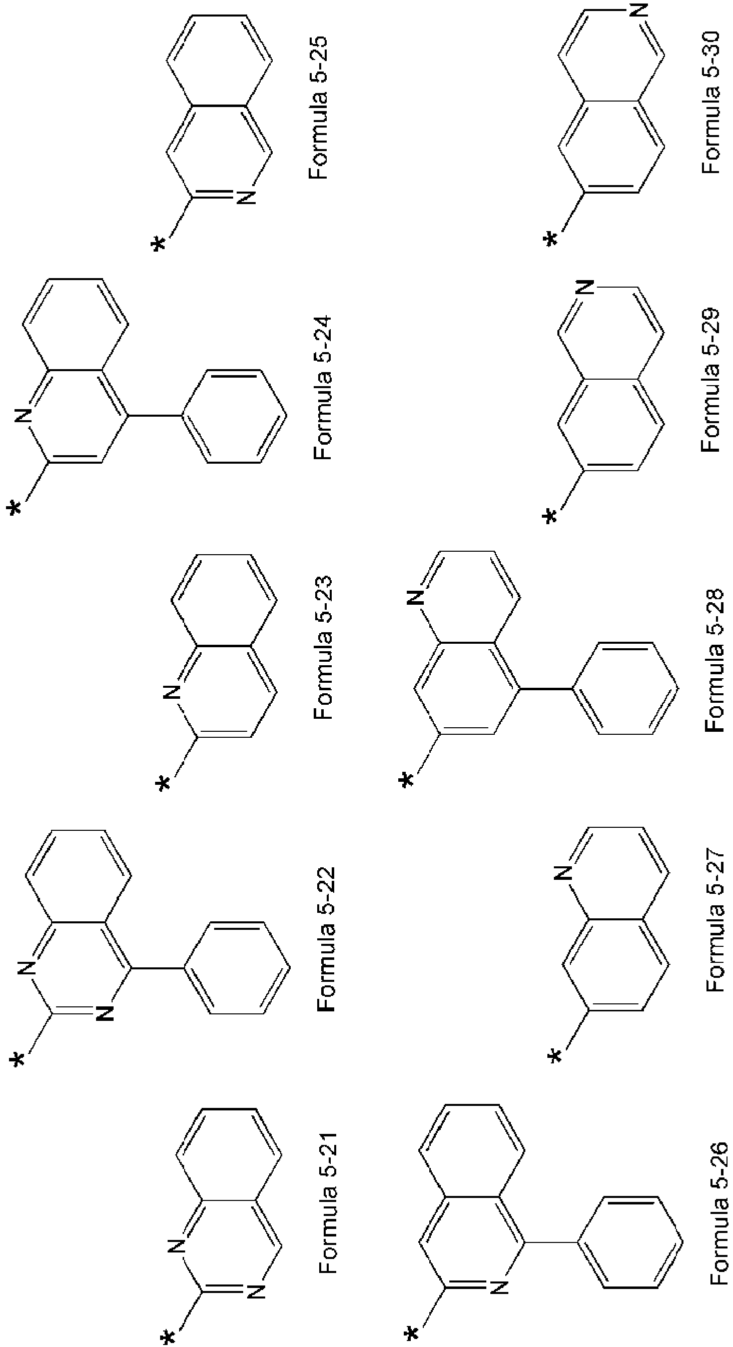

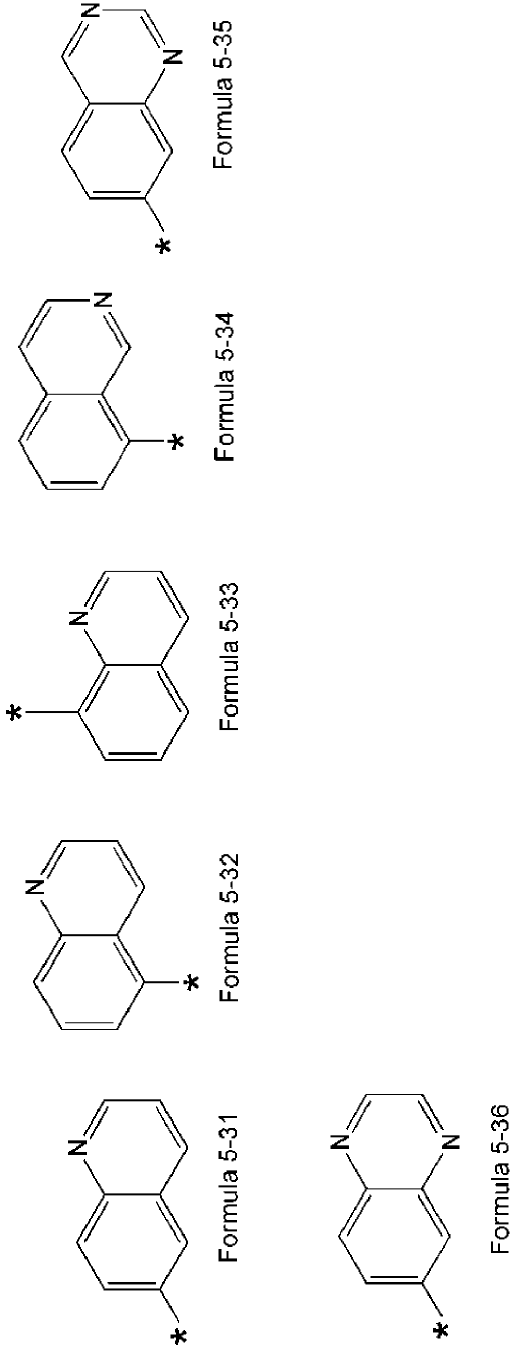

According to a preferred embodiment, R₄ and R₅ are hydrogen atoms. According to another preferred embodiment, indices b3 and b6 are 2 and R₃ and R₆ are phenyl.

The amine-based compound of Formula 1 may be a compound represented by Formula 1A below: 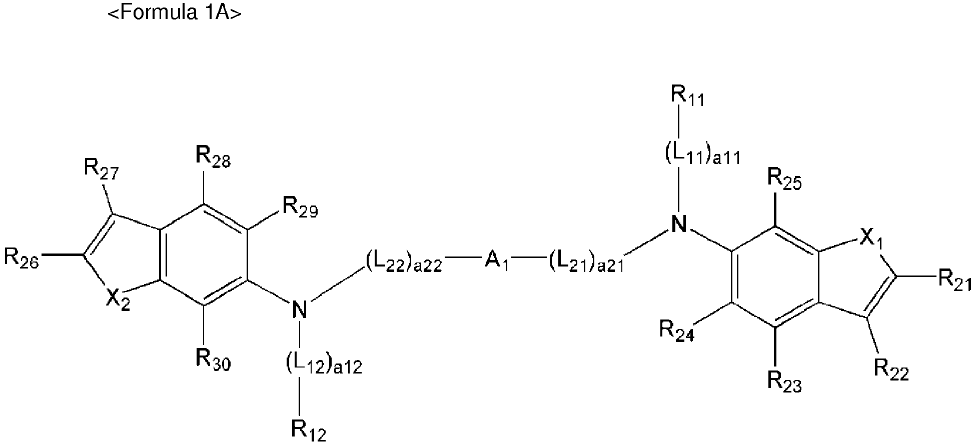 wherein, in Formula 1A, A₁, X₁, X₂, L₁₁, L₁₂, L₂₁, L₂₂, a11, a12, a21, a22, R₁₁, and R₁₂ are the same as those defined in claim 1, and R₂₁ to R₃₀ are the same as R₃ defined in claim 1.

The amine-based compound of Formula 1 may be a compound represented by one of Formulae 1A(1)to 1 A(4) below: 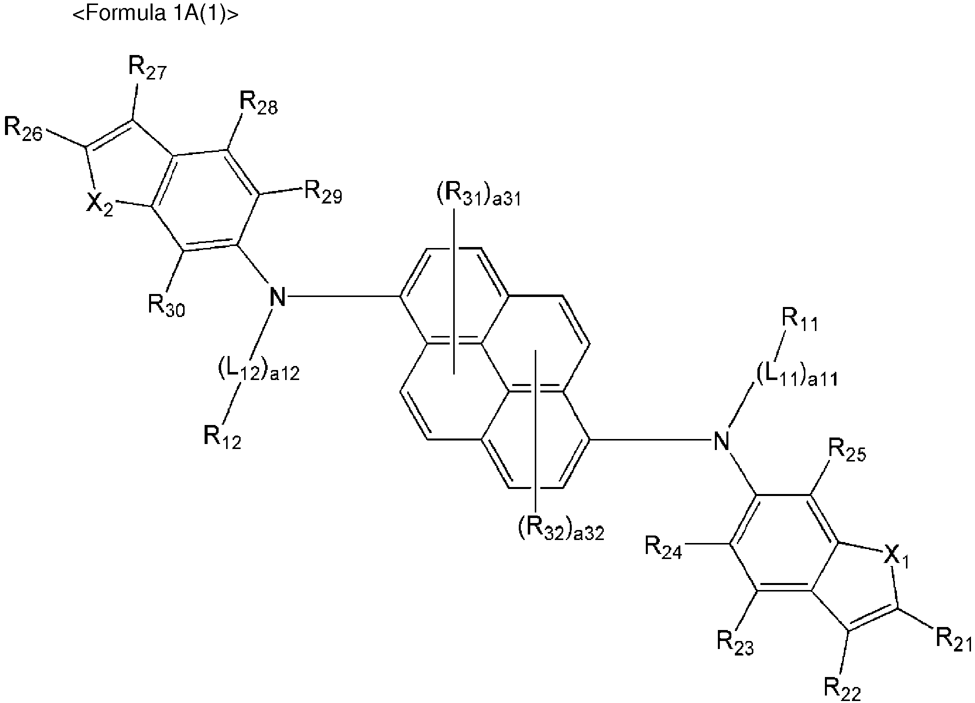 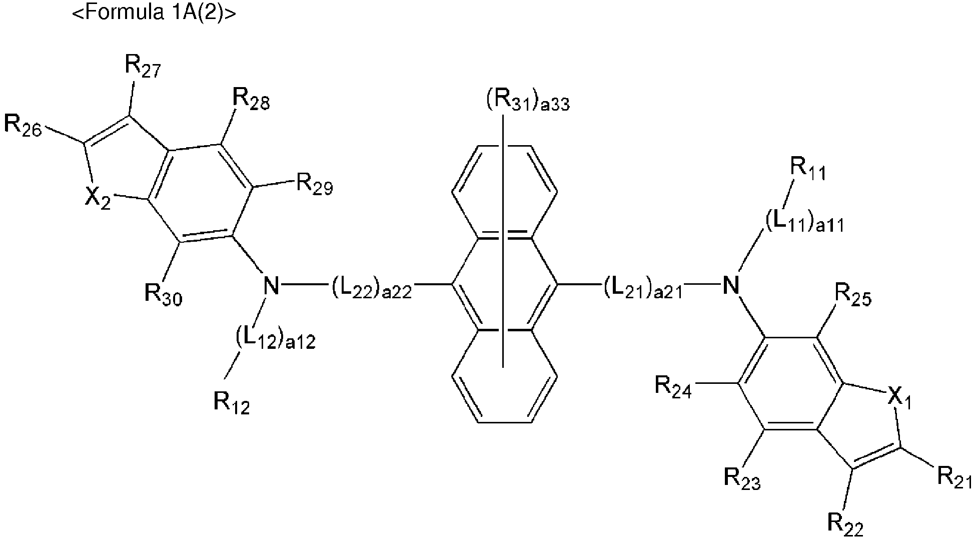 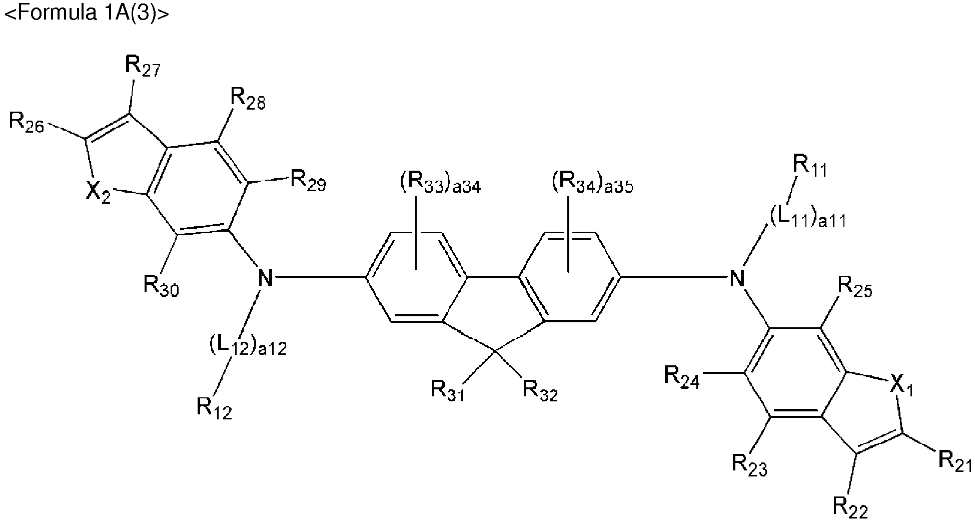 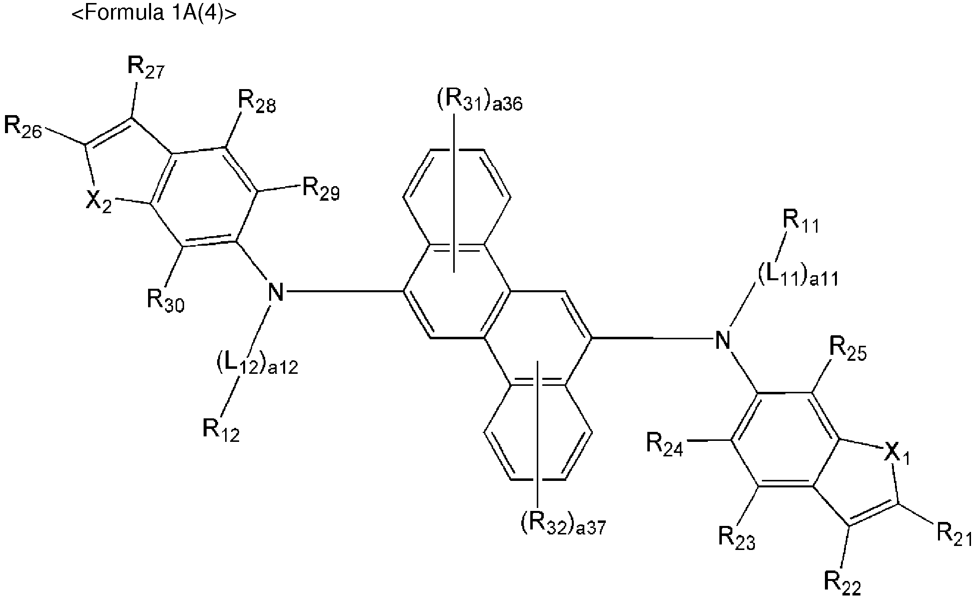 wherein, in Formulae 1A(1) to 1A(4),
X₁ X₂, L₁₁, L₁₂, L₂₁, L₂₂, a11, a12, a21, a22, R₁₁, and R₁₂ are the same as those defined in claim 1;
R₂₁ to R₃₄ are the same as R₃ defined in claim 1;
a31 and a32 are each independently an integer from 1 to 4;
a33 is an integer from 1 to 8;
a34 and a35 are each independently an integer from 1 to 3;
a36 and a37 are each independently an integer from 1 to 5.

According to a preferred embodiment, R₂₃ to R₂₅ and R₂₈ to R₃₀ are hydrogen atoms. According to another preferred embodiment, R₂₁, R₂₂, R₂₆, and R₂₇ are phenyl.

The amine-based compound of Formula 1 may be one of Compounds 1 to 108 below:

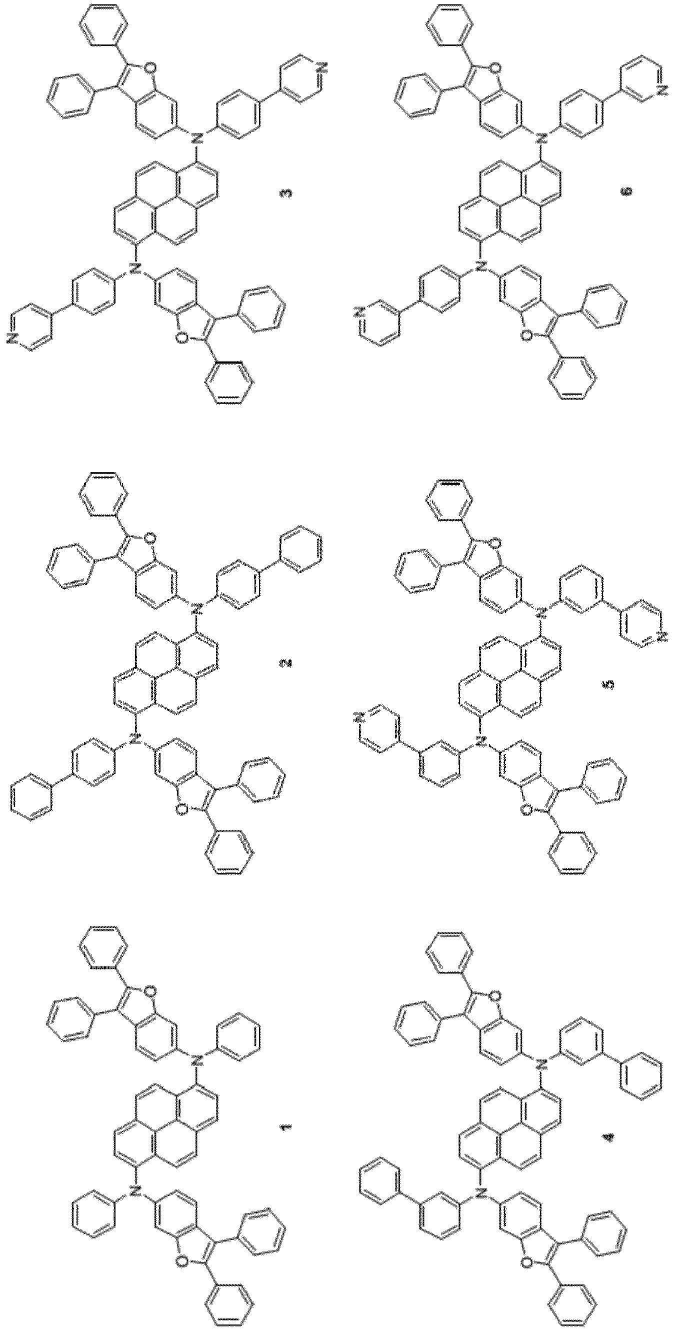

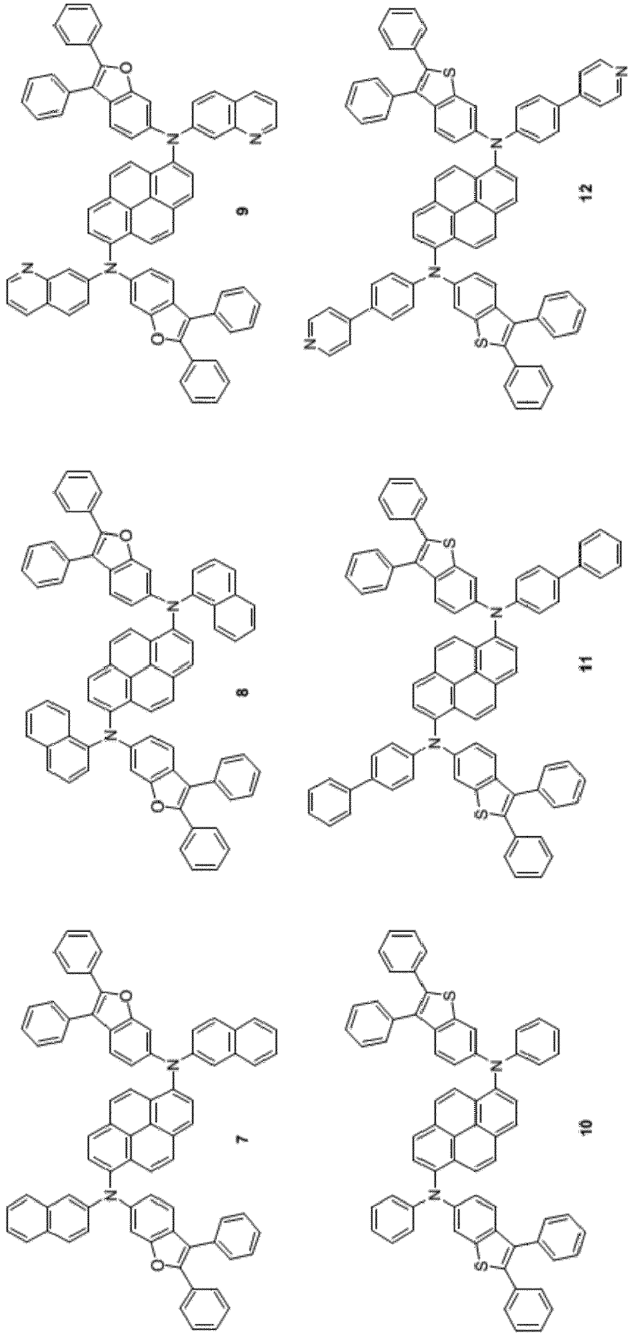

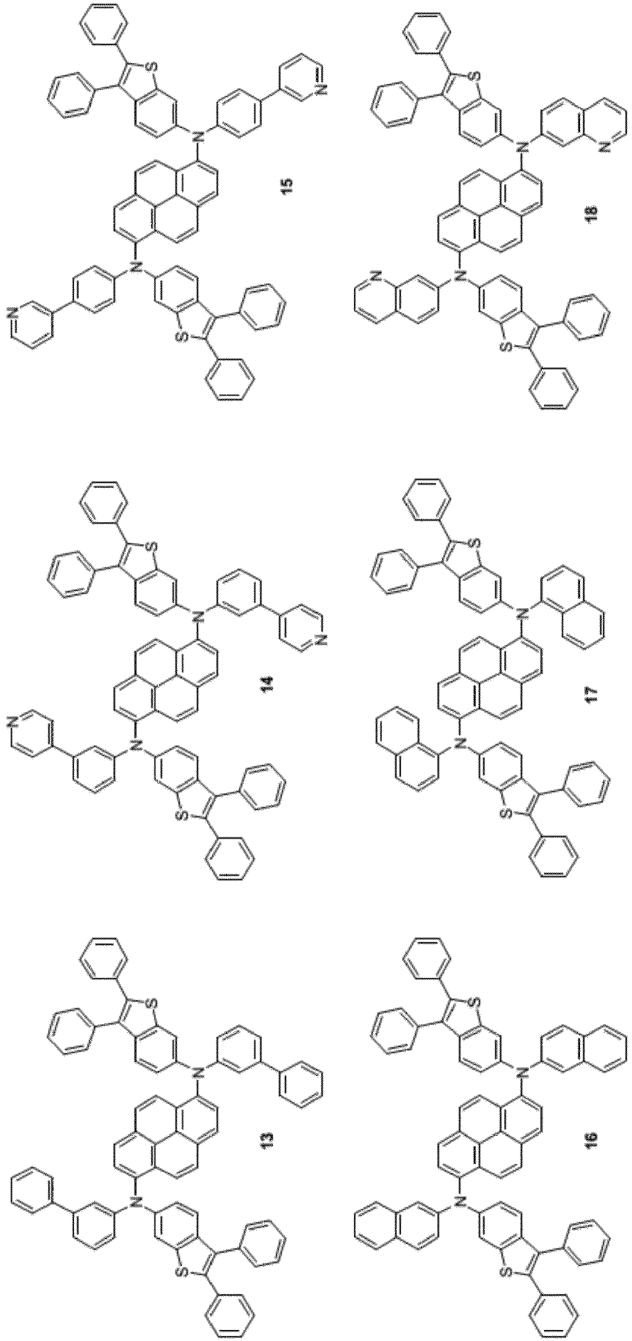

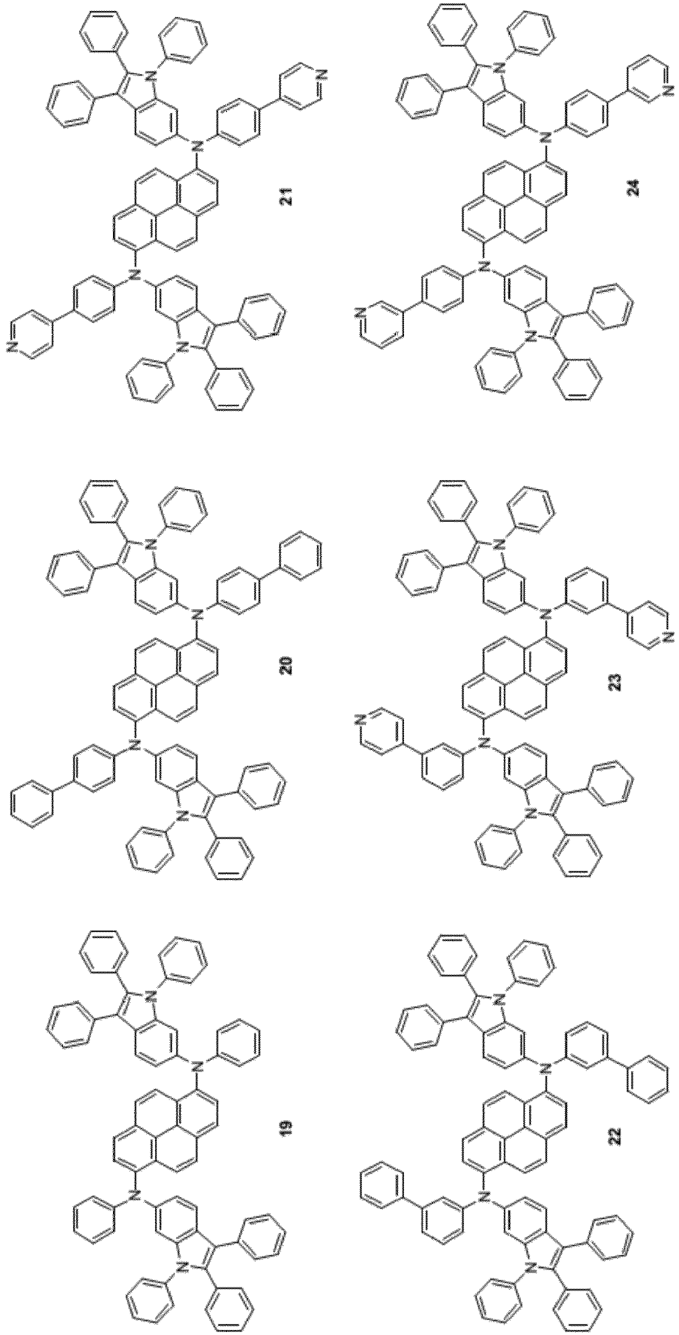

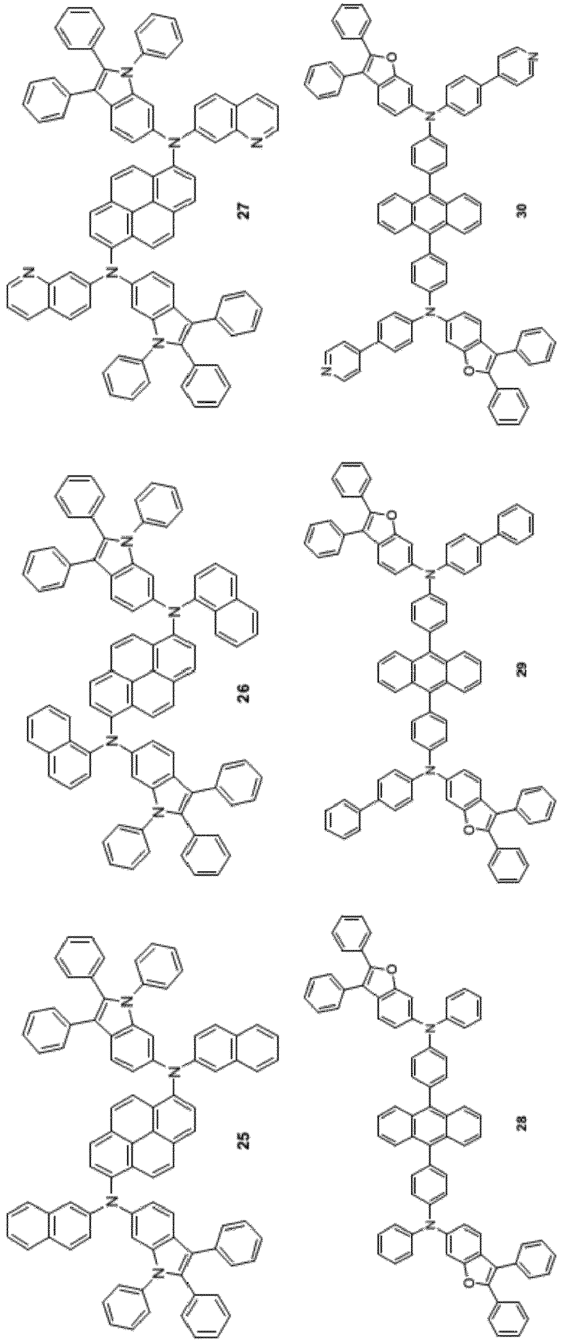

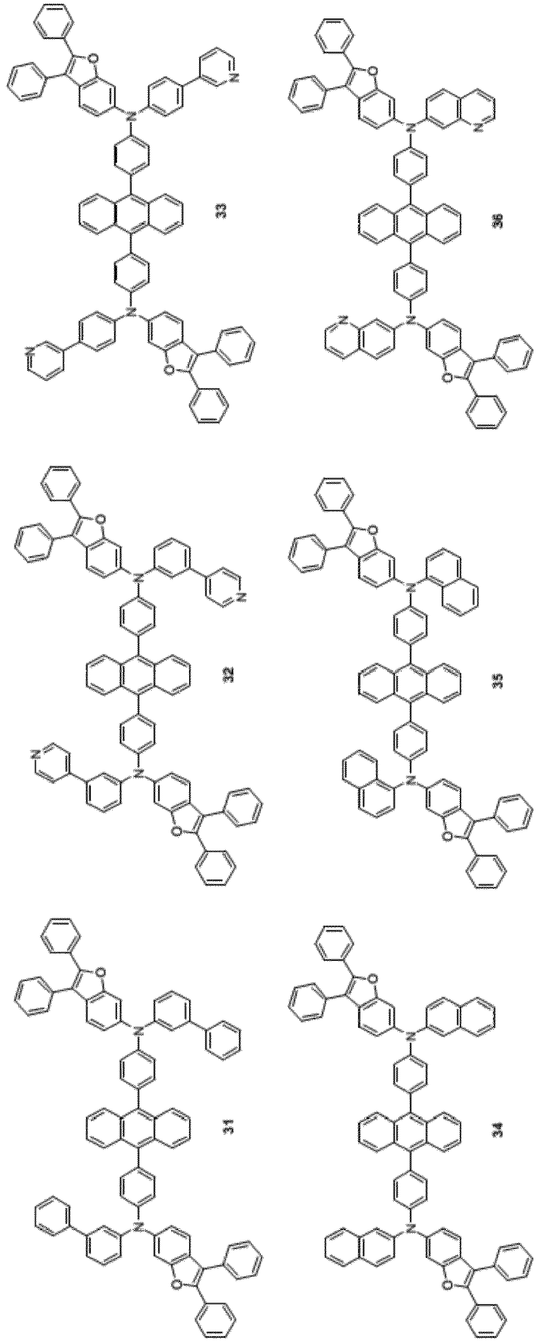

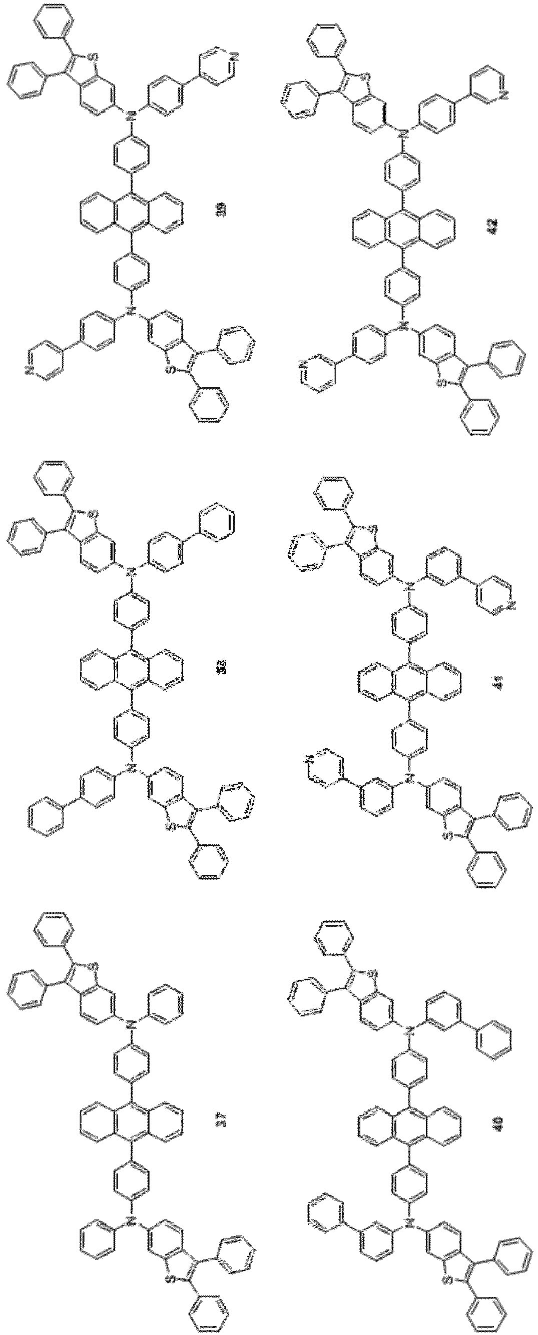

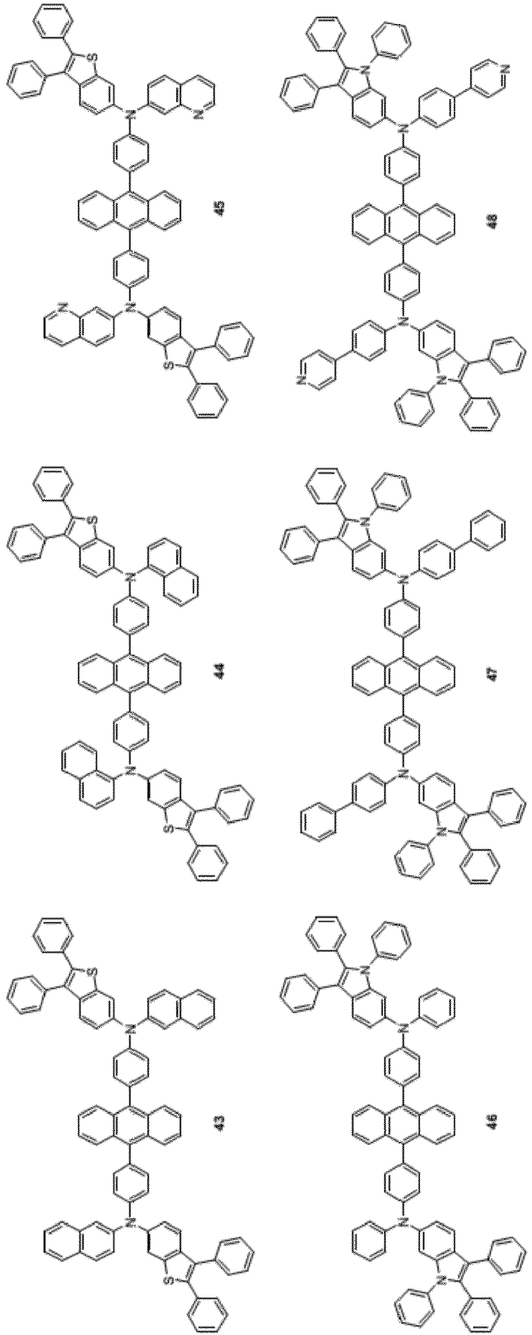

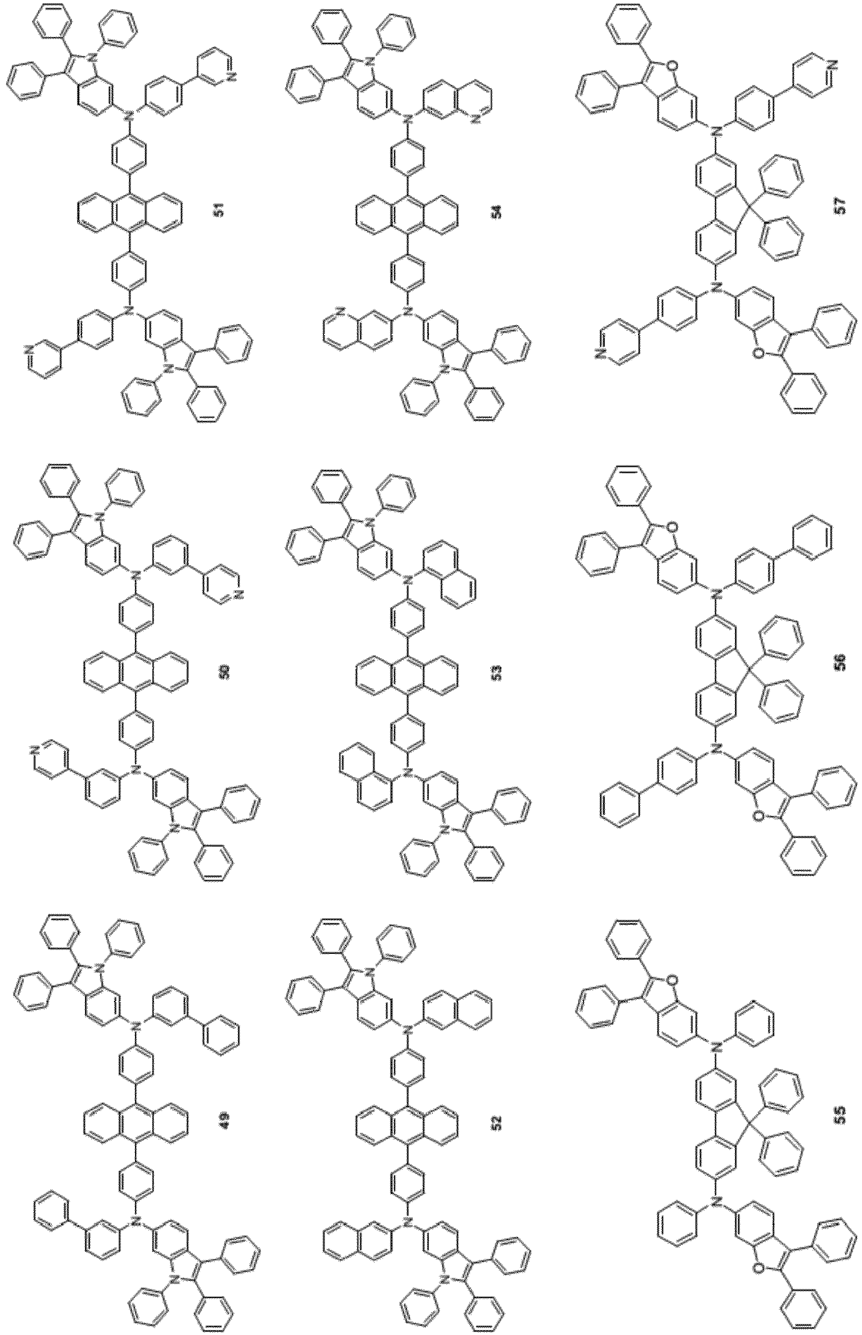

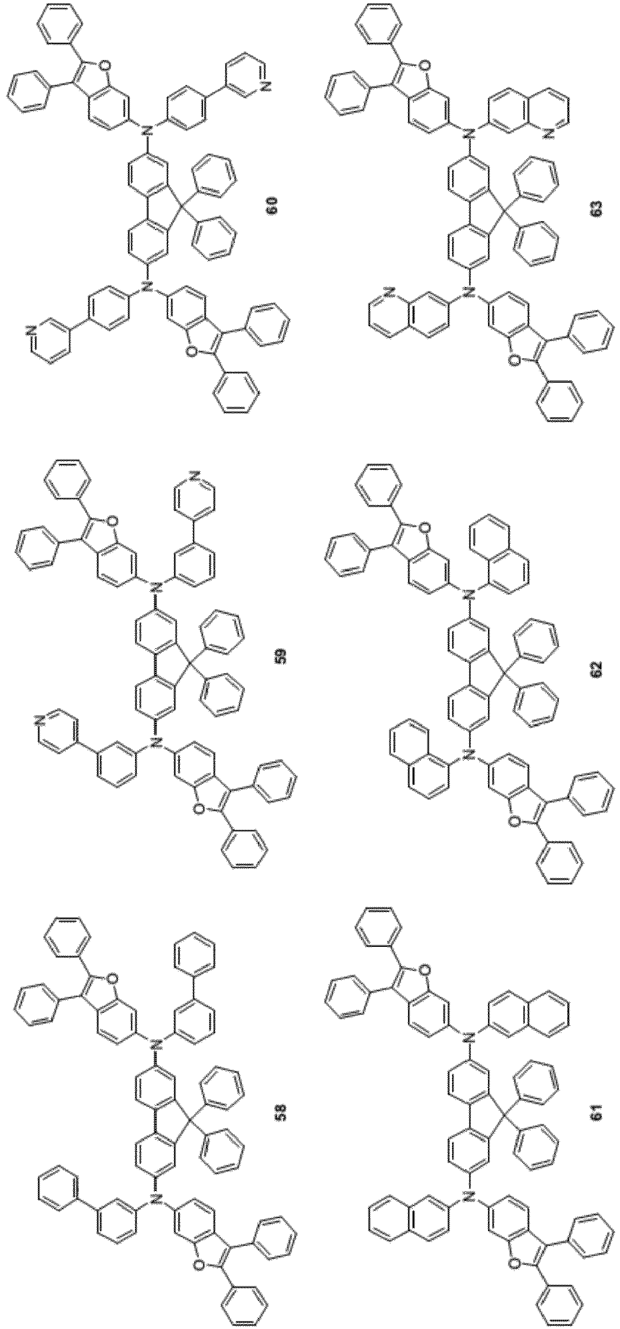

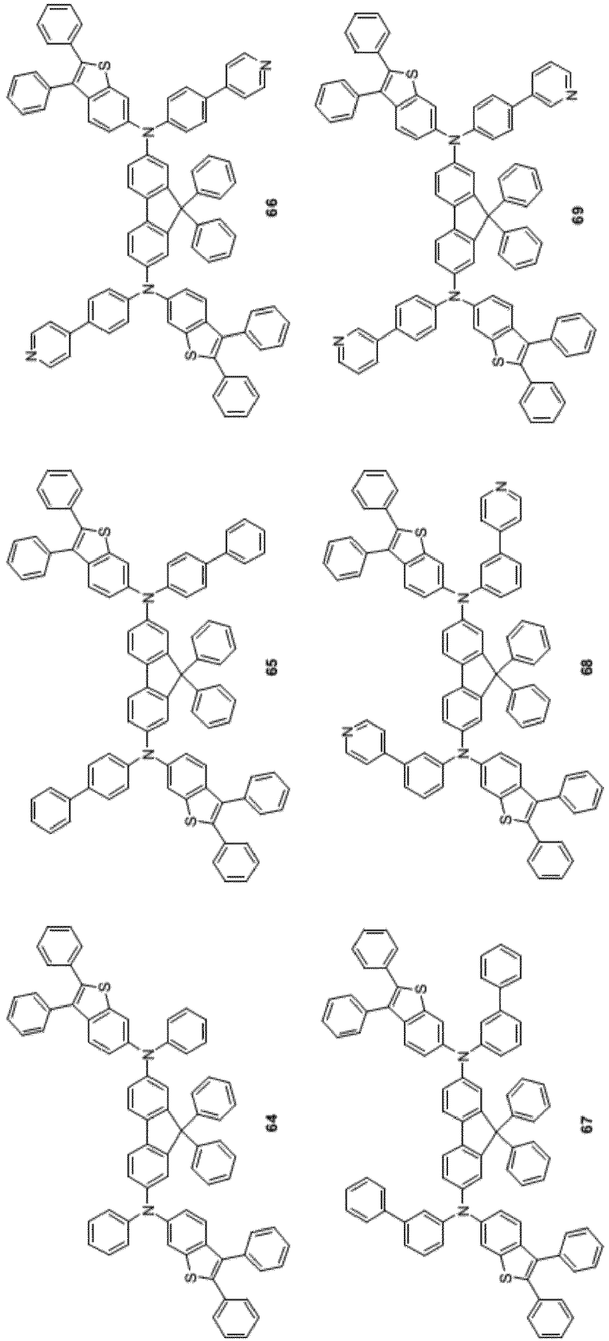

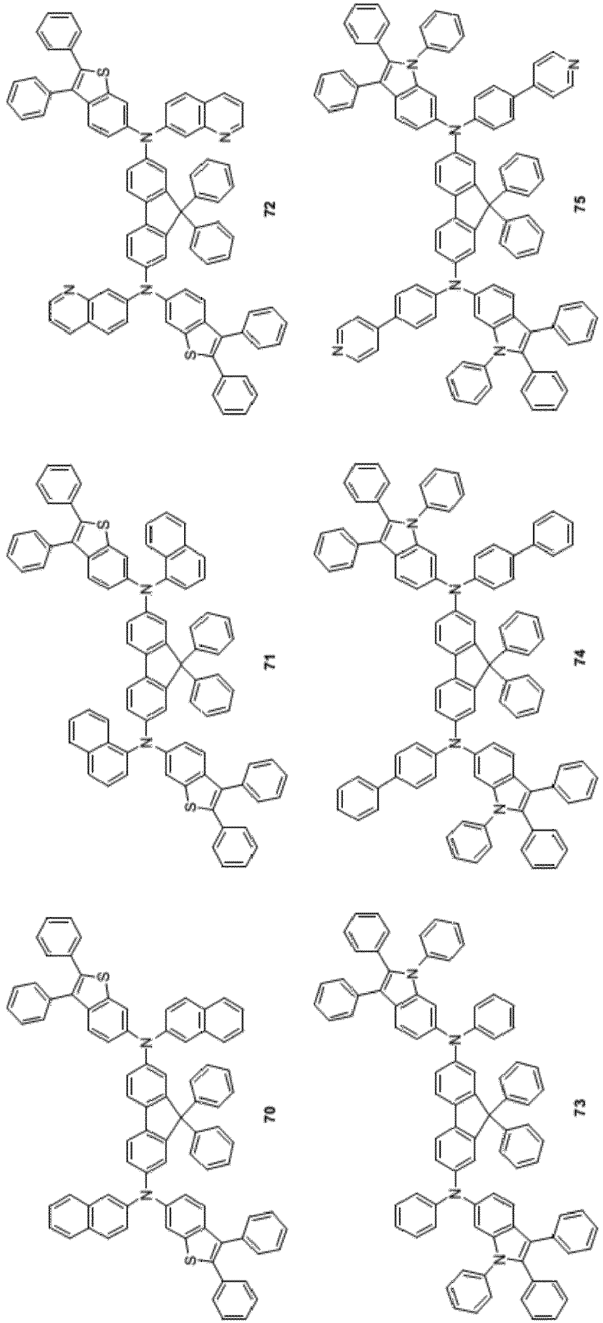

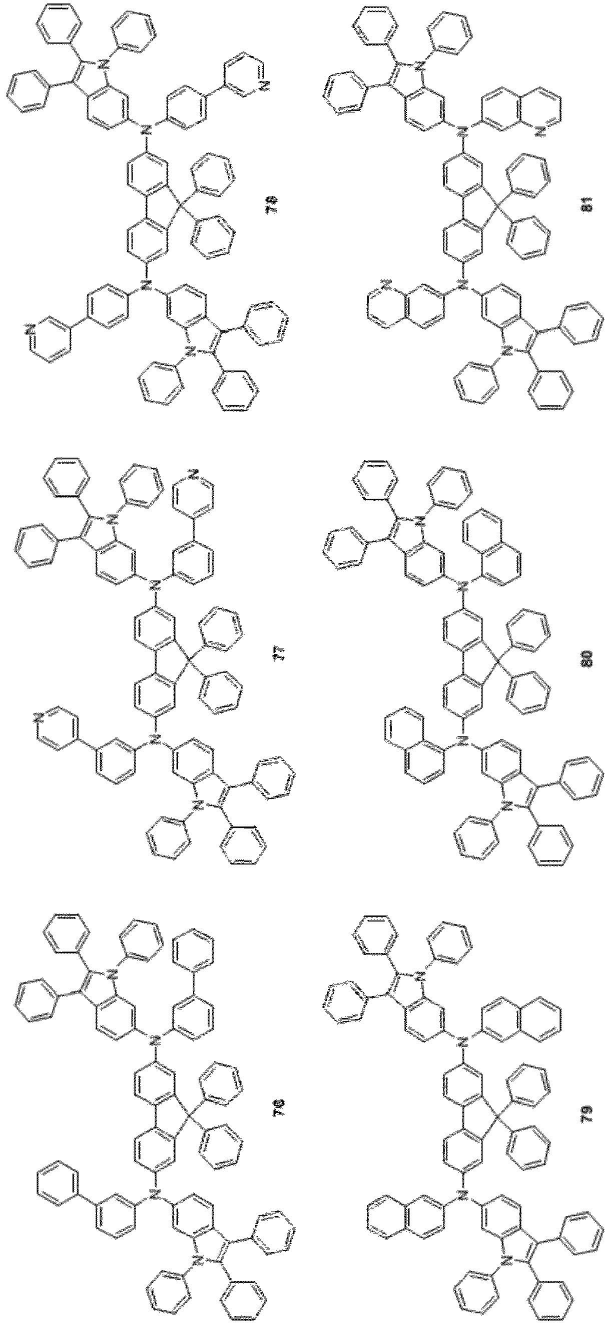

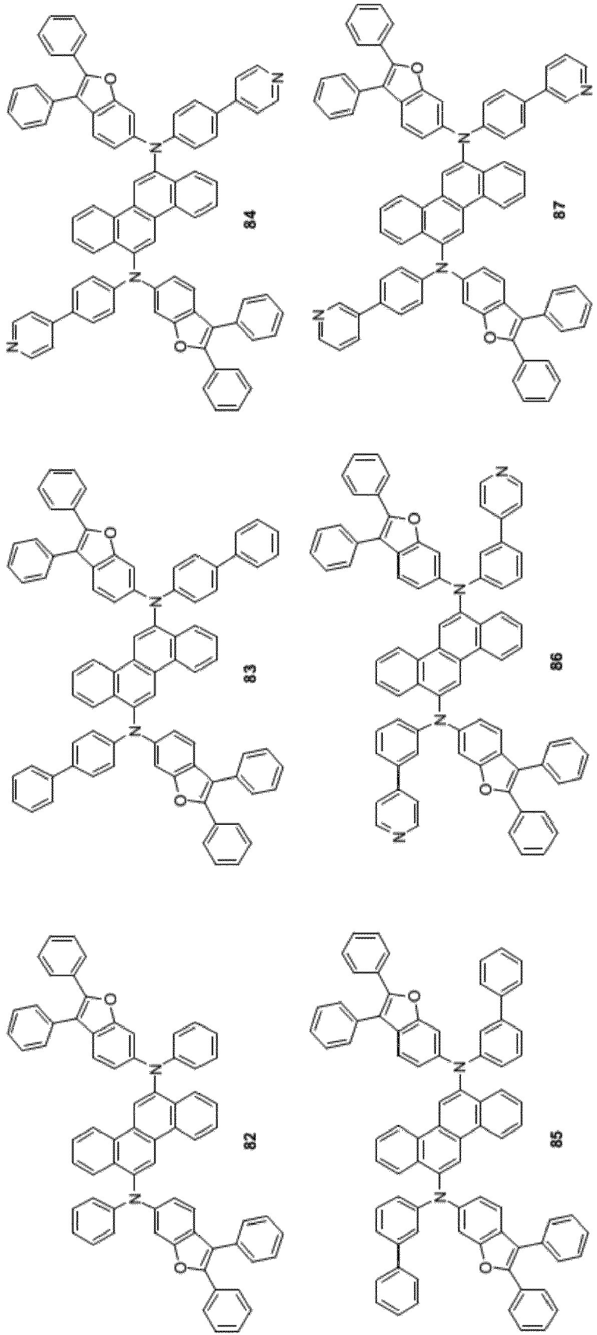

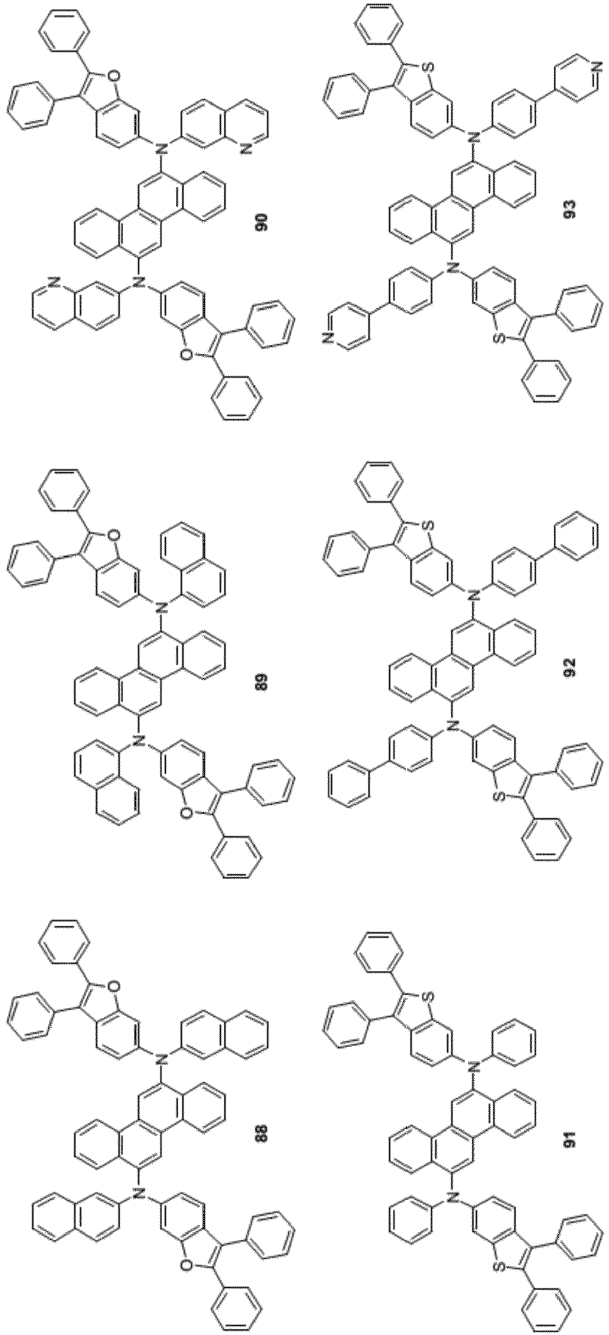

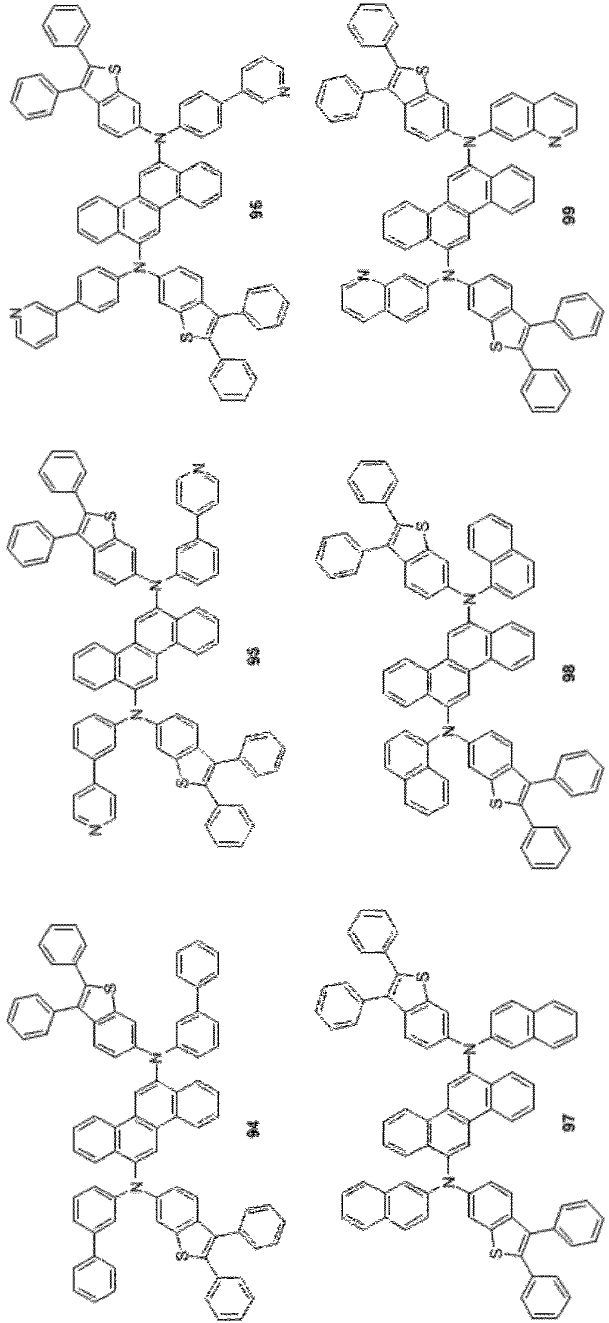

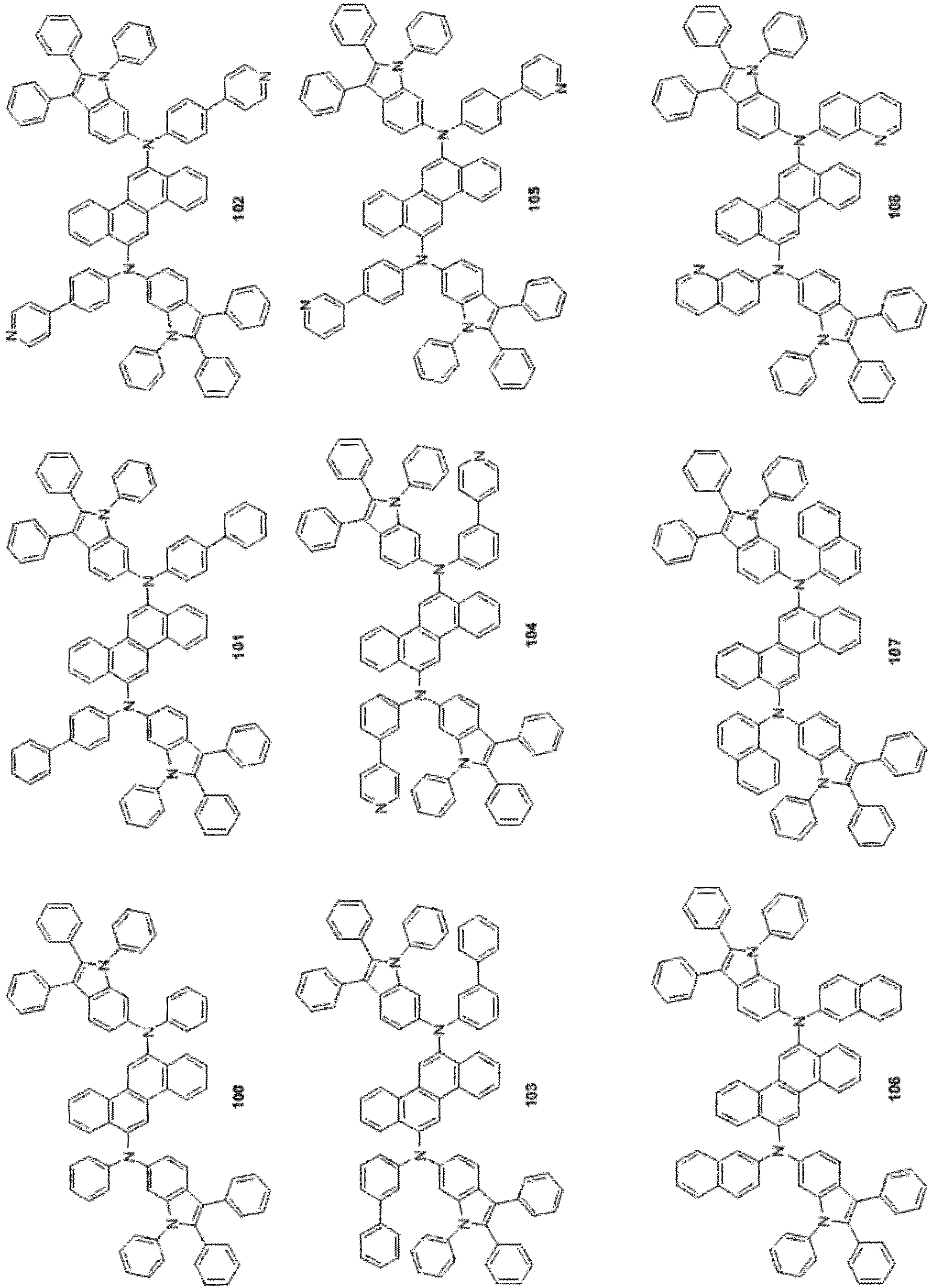

Embodiments are also directed to an organic light-emitting device including a first electrode, a second electrode, and an organic layer disposed between the first electrode and the second electrode, and including an emission layer and at least one of the above mentioned amine-based compounds.

The first electrode may be an anode, the second electrode may be a cathode, and the organic layer may include i) a hole transport region between the first electrode and the emission layer and including at least one of a hole injection layer, a hole transport layer, and an electron blocking layer, and ii) an electron transport region between the emission layer and the second electrode and including at least one of a hole blocking layer, an electron transport layer, and an electron injection layer.

The organic layer may further include a compound represented by Formula 1000 below:

<Formula 1000> Y₁-A₂-Y₂

wherein, in Formula 1000, A₂ is selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-_{C60} alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, - N(Q₁₁)(Q₁₂), and -Si(Q₁₃)(Q₁₄)(Q₁₅);
Y₁ and Y₂ are each independently selected from
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆ₒ aryloxy group, a C₆-C₆₀ arylthio group, a C₂-C₆₀ heteroaryl group, and -N(Q₁₀₁)(Q₁₀₂); and
Q₁₀₁ and Q₁₀₂ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group, and
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.

The amine-based compound may be in the emission layer.

The amine-based compound and the compound of Formula 1000 may both be in the emission layer, and an amount of the amine-based compound in the emission layer may less than an amount of the compound of Formula 1000.

The hole transport region may include at least one of a compound represented by Formula 300 below and a compound represented by Formula 301 below: 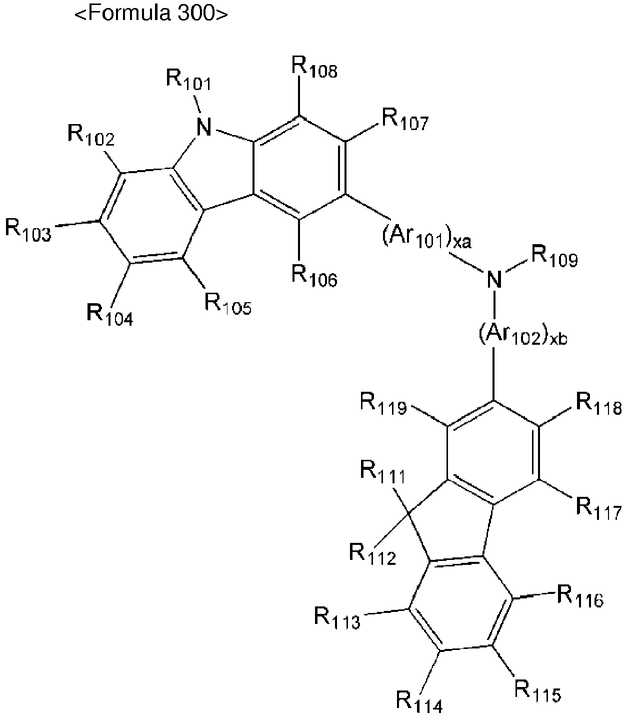 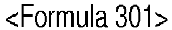 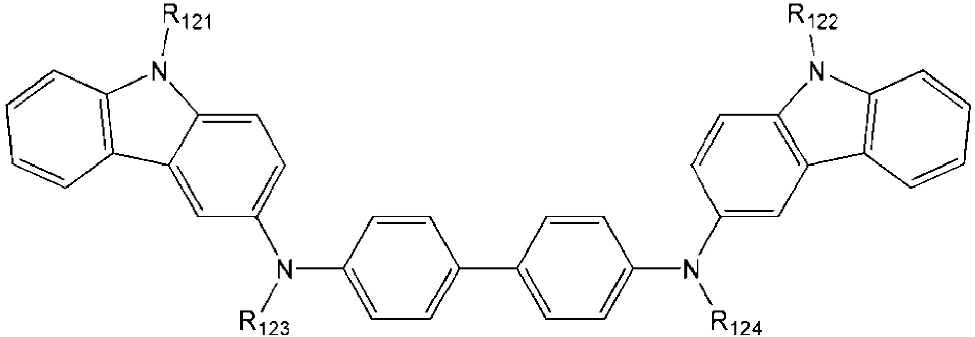
wherein, in Formula 300, Ar₁₀₁ and Ar₁₀₂ are each independently selected from
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group, and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₃-C₁₀ heterocycloalkyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
xa and xb in Formula 300 are each independently 0, 1, or 2;
R₁₀₁ to R₁₀₈, R₁₁₁ to R₁₁₉, and R₁₂₁ to R₁₂₄ in Formulae 300 and 301 are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₁₀ alkyl group, and a C₁-C₁₀ alkoxy group,
a C₁-C₁₀ alkyl group and a C₁-C₁₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, and a phosphoric acid group or a salt thereof,
a phenyl group, a naphthyl group, an anthracenylene group, a fluorenyl group, and a pyrenyl group, and
a phenyl group, a naphthyl group, an anthracenyl group, a fluorenyl group, and a pyrenyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₁₀ alkyl group, and a C₁-C₁₀ alkoxy group; and
R₁₀₉ in Formula 300 is selected from a phenyl group, a naphthyl group, an anthracenyl group, a biphenyl group, and a pyridyl group; and a phenyl group, a naphthyl group, an anthracenyl group, a biphenyl group, and a pyridyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group.

### BRIEF DESCRIPTION OF THE DRAWING

Features will become apparent to those of skill in the art by describing in detail exemplary embodiments with reference to the attached drawings in which:

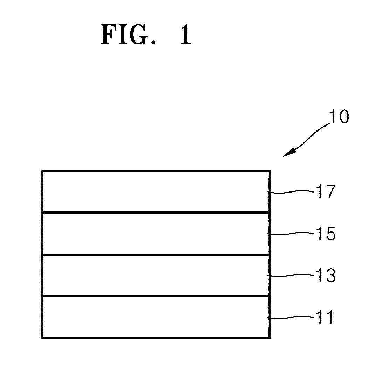

FIG. 1 illustrates a schematic view of an organic light-emitting device according to an embodiment.

### DETAILED DESCRIPTION

Example embodiments will now be described more fully hereinafter with reference to the accompanying drawing.

In the drawing figure, the dimensions of layers and regions may be exaggerated for clarity of illustration. It will also be understood that when a layer or element is referred to as being "on" another layer or substrate, it can be directly on the other layer or substrate, or intervening layers may also be present. Further, it will be understood that when a layer is referred to as being "under" another layer, it can be directly under, and one or more intervening layers may also be present. In addition, it will also be understood that when a layer is referred to as being "between" two layers, it can be the only layer between the two layers, or one or more intervening layers may also be present. As used herein, the term "and/or" includes any and all combinations of one or more of the associated listed items. Expressions such as "at least one of," when preceding a list of elements, modify the entire list of elements and do not modify the individual elements of the list.

According to the present invention, there is provided an amine-based compound represented by Formula 1: 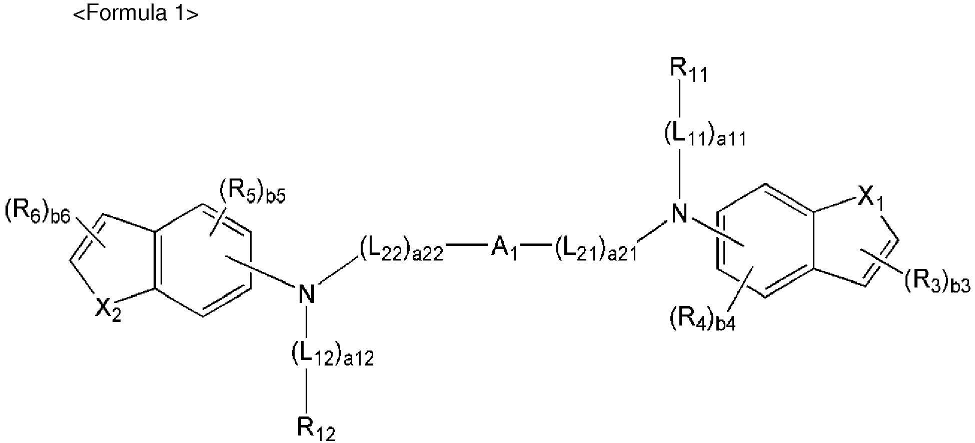 In Formula 1, A₁ is selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and -Si(Q₁₃)(Q₁₄)(Q₁₅), wherein Q₁₁ to Q₁₅ will be defined below.

For example, A₁ in Formula 1 above may be selected from
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, and
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, but is not limited thereto.

In some embodiments, A₁ in Formula 1 above may be selected from a naphthylene group, a dimethylfluorenylene group, a diphenylfluorenyl group, a spiro-fluorenylene group, a phenanthrenylene group, an anthracenylene group, a triphenylenylene group, a pyrenylene group, a chrysenylene group, and a benzofluoranthenylene group, but is not limited thereto.

In Formula 1, X₁ is selected from N-[(L₁)ₐ₁-(R₁)_{b1}], S, and O, and X₂ is selected from N-[(L₂)ₐ₂-R₂], S, and O. L₁, L₂, R₁, R₂, a1, a2, and b1 will be defined below.

In Formula 1 above, X₁ and X₂ may be identical to or different from each other. Preferably, X₁ and X₂ in Formula 1 may be identical to each other.

Preferably, X₁ and X₂ in Formula 1 may be both S, or X₁ and X₂ in Formula 1 may be both O.

In Formula 1, L₁, L₂, L₁₁, L₁₂, L₂₁, and L₂₂ are each independently selected from
a C₃-C₁₀ cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, and
a C₃-C₁₀ cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and -Si(Q₁₃)(Q₁₄)(Q₁₅).

In some embodiments, L₁, L₂, L₁₁, L₁₂, L₂₁, and L₂₂ in Formula 1 may be each independently selected from
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.

In some other embodiments, L₁, L₂, L₁₁, L₁₂, L₂₁ and L₂₂ in Formula 1 above may be each independently selected from the groups represented by Formulae 2-1 to 2-30 below: 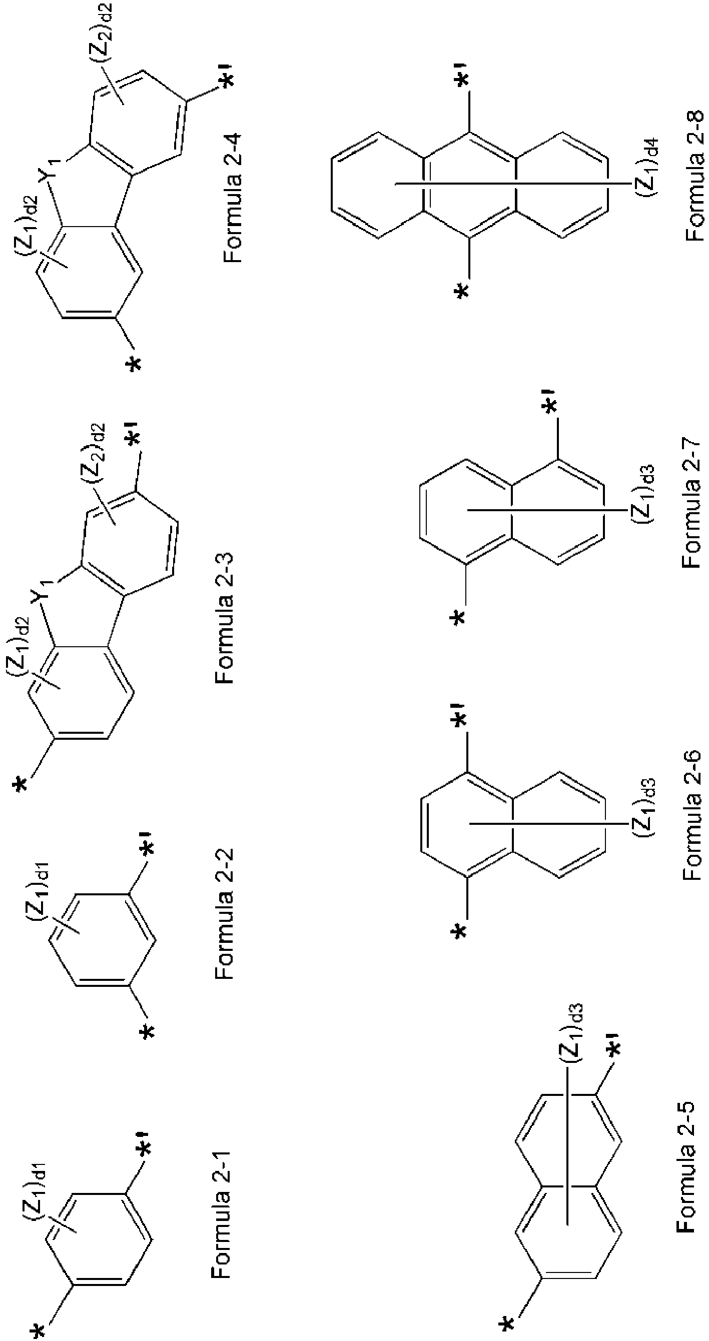 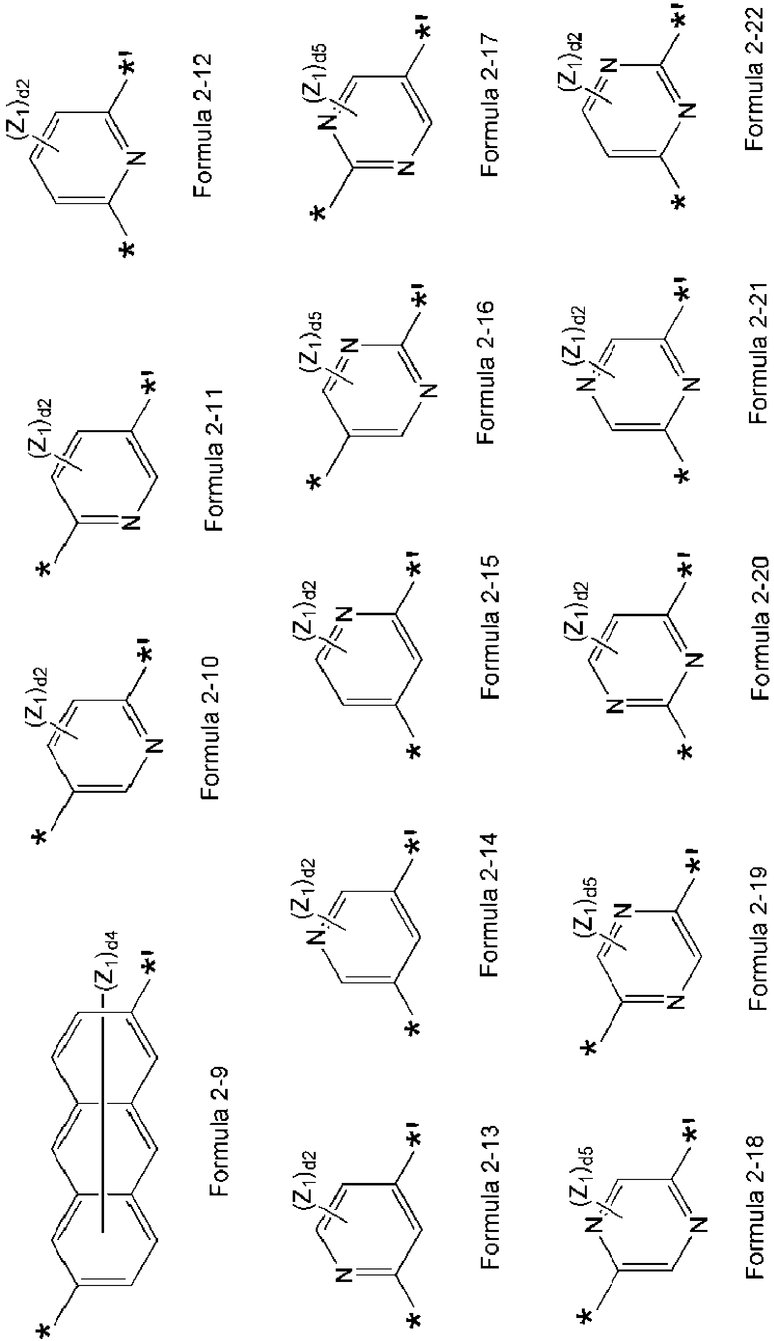 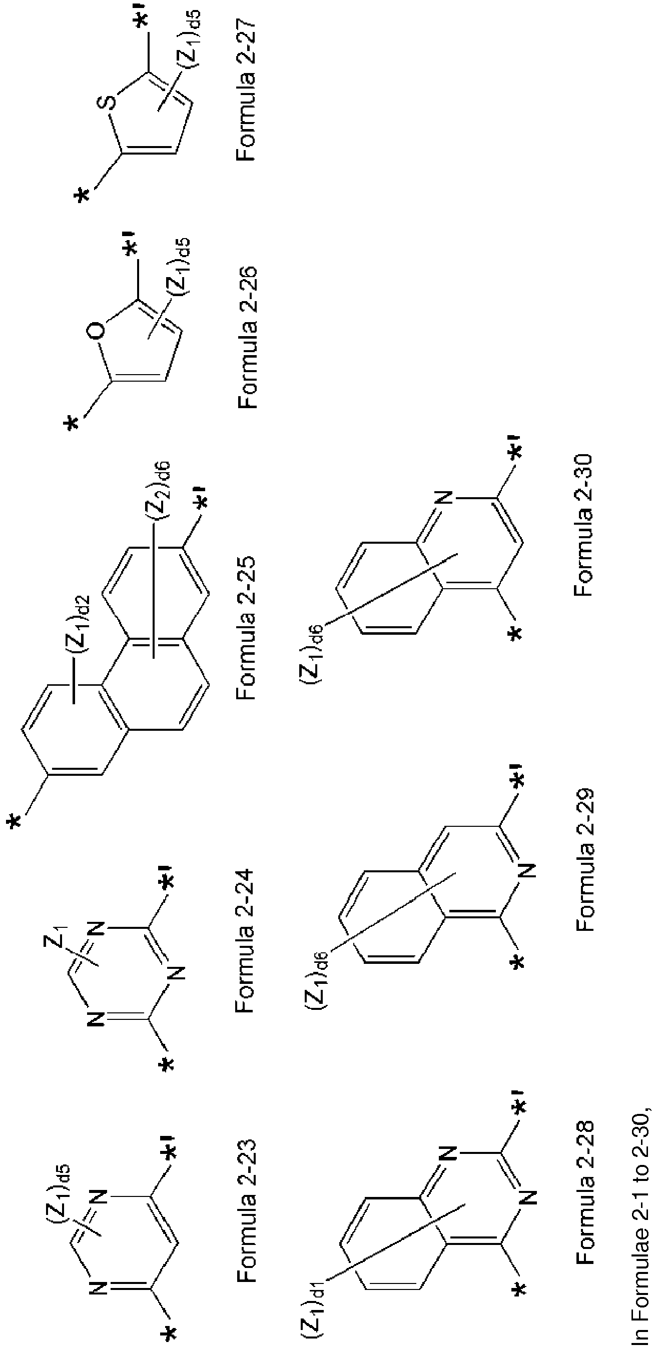
Y₁ may be O, S, S(=O), S(=O)₂, C(Z₃)(Z₄), N(Z₅), or Si(Z₆)(Z₇);
Z₁ to Z₇ may be each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and -Si(Q₁₃)(Q₁₄)(Q₁₅) (where Q₁₁ to Q₁₅ are the same as defined in the specification);
d1 may be an integer from 1 to 4;
d2 may be an integer from 1 to 3;
d3 may be an integer from 1 to 6;
d4 may be an integer from 1 to 8;
d5 may be 1 or 2;
d6 may be an integer from 1 to 5; and
* and *' indicate binding sites to adjacent atoms.

For example, in Formulae 2-1 to 2-30, * indicates a binding site of A₁ in Formula 1 or an adjacent group of L₁, L₂, L₁₁, L₁₂, L₂₁, and L₂₂; and *' indicates a binding site of an adjacent group of L₁, L₂, L₁₁, L₁₂, L₂₁, and L₂₂, or a binding site of R₁, R₂, R₁₁, R₁₂, R₂₁, and R₂₂.

For example, in Formulae 2-1 to 2-30, Z₁ to Z₇ may be each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, but are not limited thereto.

In some other embodiments, L₁, L₂, L₁₁, L₁₂, L₂₁, and L₂₂ in Formula 1 may be each independently selected from the groups represented by Formulae 3-1 to 3-19:

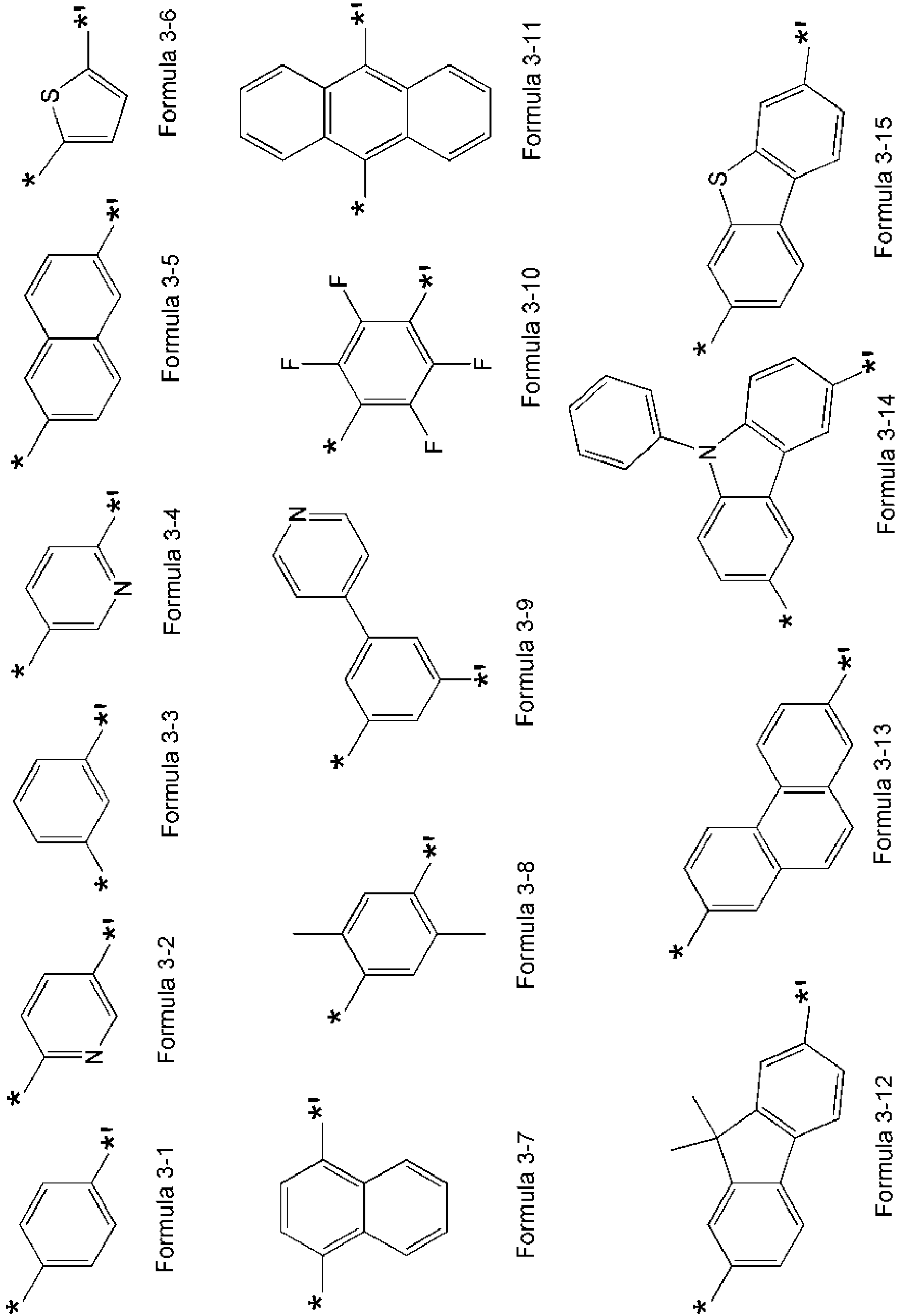

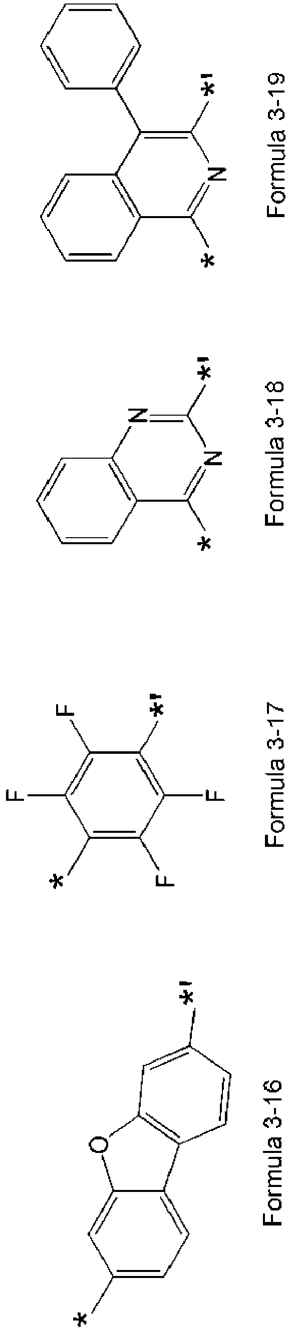

In Formula 1, a1 indicates the number of L₁s, which may be 0, 1, 2, 3, 4, or 5. For example, a1 may be 0, 1, or 2, and in some embodiments, may be 0 or 1. When a1 is 0, R₁ is bound directly to N. When a1 is 2 or greater, at least two L₁s may be identical to or different from each other.

In Formula 1, a2 indicates the number of L₂s, which may be 0, 1, 2, 3, 4, or 5. For example, a2 may be 0, 1, or 2, and in some embodiments, may be 0 or 1. When a2 is 0, R₂ is bound directly to N. When a2 is 2 or greater, at least two L₂s may be identical to or different from each other.

In Formula 1, a11 indicates the number of L₁₁s, which may be 0, 1, 2, 3, 4, or 5. For example, a11 may be 0, 1, or 2, and in some embodiments, may be 0 or 1. When a11 is 0, R₁₁ is bound directly to N. When a11 is 2 or greater, at least two L₁₁s may be identical to or different from each other.

In Formula 1, a12 indicates the number of L₁₂s, which may be 0, 1, 2, 3, 4, or 5. For example, a1 may be 0, 1, or 2, and in some embodiments, may be 0 or 1. When a'2 is 0, R₁₂ is bound directly to N. When a12 is 2 or greater, at least two L₁₂s may be identical to or different from each other.

In Formula 1, a21 indicates the number of L₂₁s, which may be 0, 1, 2, 3, 4, or 5. For example, a1 may be 0, 1, or 2, and in some embodiments, may be 0 or 1. When a21 is 0, N in Formula 1 is bound directly to A₁. When a21 is 2 or greater, at least two L₂₁s may be identical to or different from each other.

In Formula 1, a22 indicates the number of L₂₂s, which may be 0, 1, 2, 3, 4, or 5. For example, a1 may be 0, 1, or 2, and in some embodiments, may be 0 or 1. When a22 is 0, R₂₂ is bound directly to N in Formula 1. When a22 is 2 or greater, at least two L₂₂s may be identical to or different from each other.

In Formula 1, R₁, R₂, R₁₁, and R₁₂ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₁-C₂₀ alkenyl group, and a C₂-C₆₀ alkynyl group,
a C₁-C₆₀ alkyl group, a C₂-C₆₀ alkenyl group, and a C₂-C₆₀ alkynyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.

In Formula 1, R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group; and
   -N(Q₁)(Q₂), -Si(Q₃)(Q₄)(Q₅), and -B(Q₆)(Q₇).

The Q₁₁ to Q₁₅, and Q₁ to Q₇ in this specification are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group; and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.
in some embodiments, R₁, R₂, R₁₁, and R₁₂ may be each independently selected from
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group; and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, but are not limited thereto.

In some other embodiments, R₃ to R₆ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
-N(Q₁)(Q₂) and -Si(Q₃)(Q₄)(Q₅), wherein Q₁ to Q₅ may be each independently selected from a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group.

In some other embodiments, R₁, R₂, R₁₁, and R₁₂ may be each independently selected from the groups represented by Formulae 4-1 to 4-29; and R₃ to R₆ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group;
the groups represented by Formulae 4-1 to 4-29; and
-N(Q₁)(Q₂) and -Si(Q₃)(Q₄)(Q₅), wherein Q₁ to Q₅ may be each independently selected from a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group.

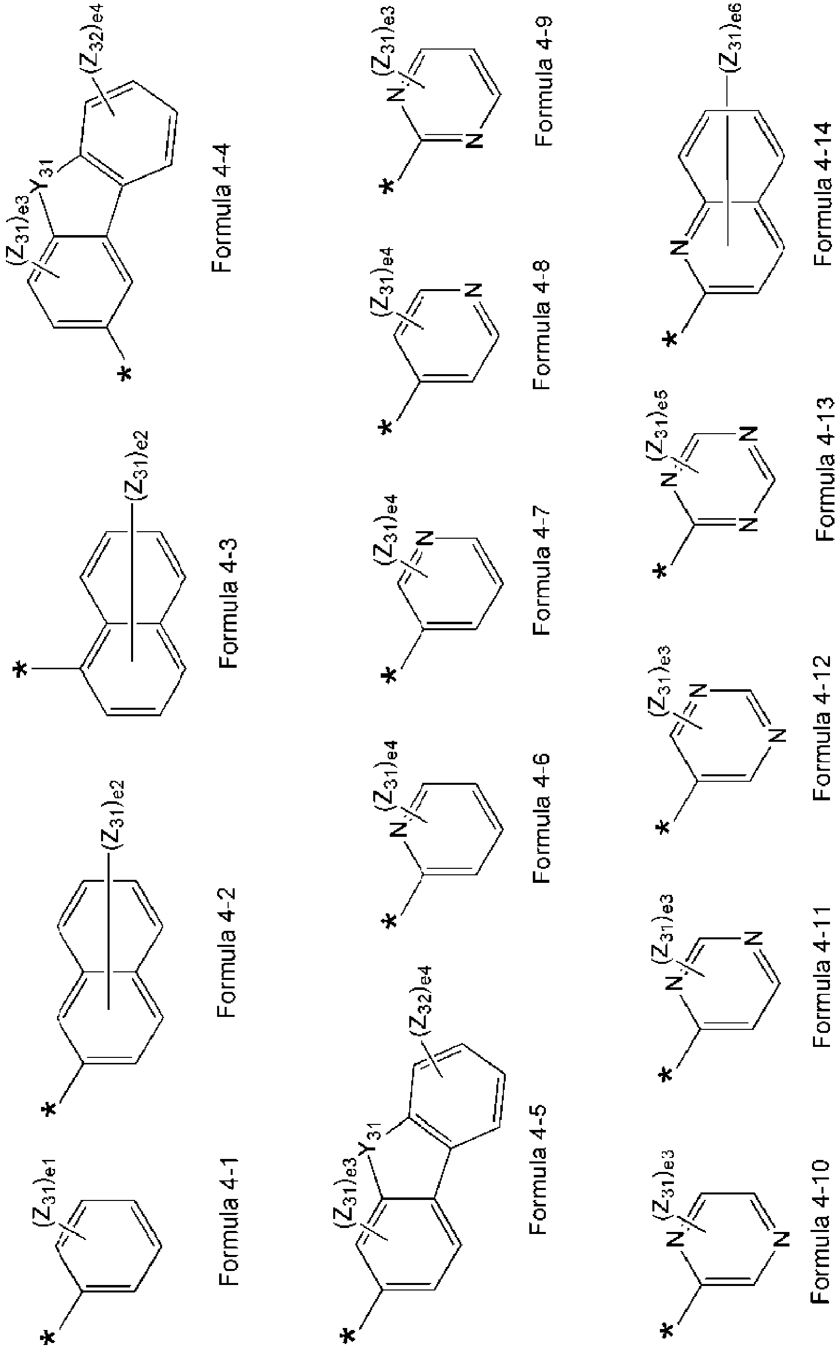

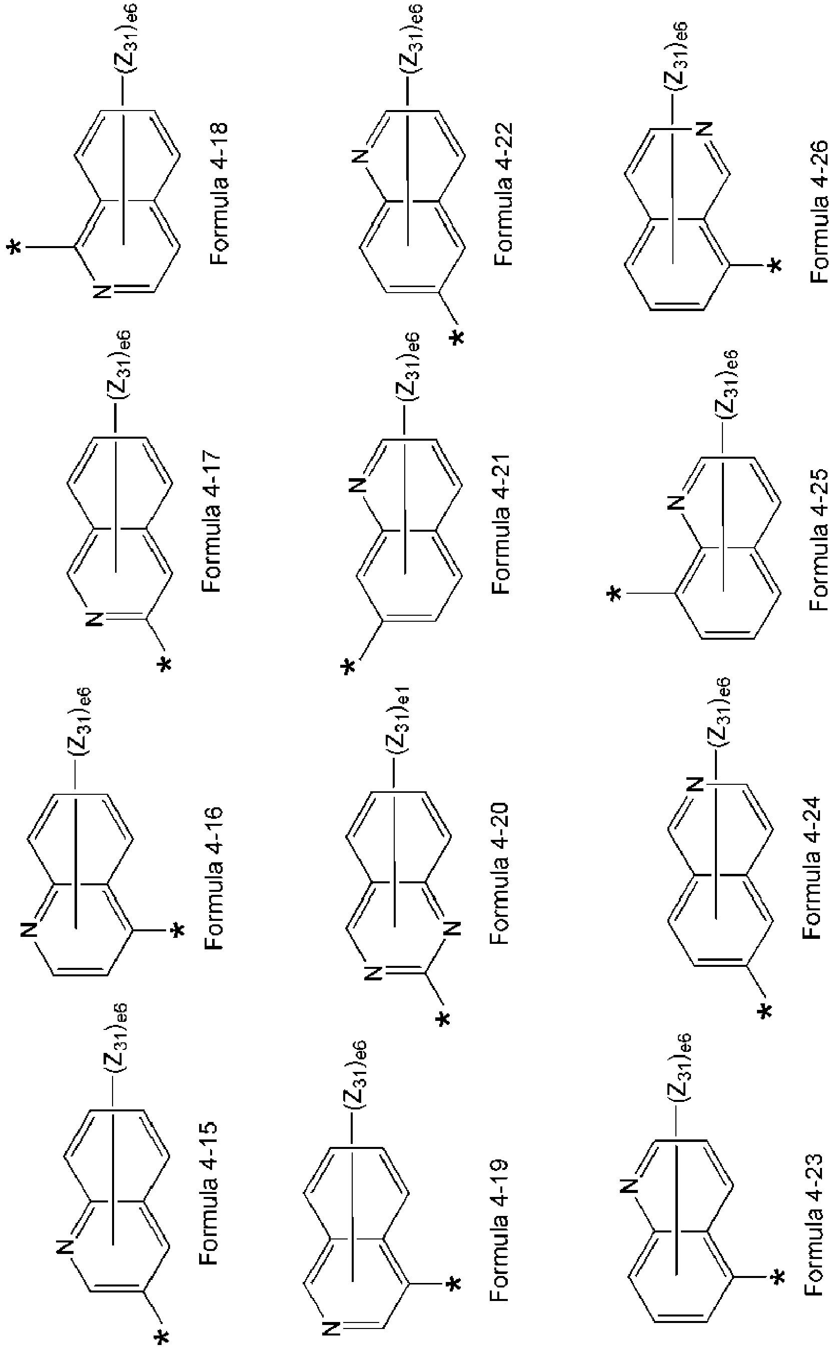

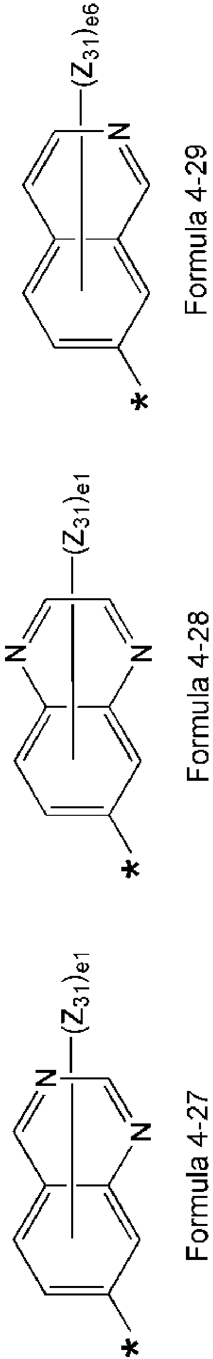

In Formulae 4-1 to 4-29,
Y₃₁ may be O, S, C(Z₃₃)(Z₃₄), N(Z₃₅), or Si(Z₃₆)(Z₃₇);
Z₃₁ to Z₃₇ may be each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀heterocycloalkenyl group, a C₆-C₆₀ aryl group, a C₆-C₆₀ aryloxy group, aC₆-C₆₀ arylthio group, and a C₂-C₆₀ heteroaryl group;
e1 may be an integer from 1 to 5;
e2 may be an integer from 1 to 7;
e3 may be an integer from 1 to 3;
e4 may be an integer from 1 to 4;
e5 may be 1 or 2;
e6 may be an integer from 1 to 6; and
* indicates a binding site of an adjacent atom.

For example, in Formulae 4-1 to 4-29, Z₃₁ to Z₃₇ may be each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.

For example, in Formulae 4-1 to 4-29, e1, e2, e3, e4, e5, and e6 may be each independently 1 or 2.

In some embodiments, in Formula 1, R₁, R₂, R₁₁, and R₁₂ may be each independently selected from the groups represented by Formulae 5-1 to 5-36; and
R₃ to R₆ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group;
the groups represented by Formulae 5-1 to 5-36 below; and
-N(Q₁)(Q₂), and -Si(Q₃)(Q₄)(Q₅), wherein Q₁ to Q₅ may be each independently selected from a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group.

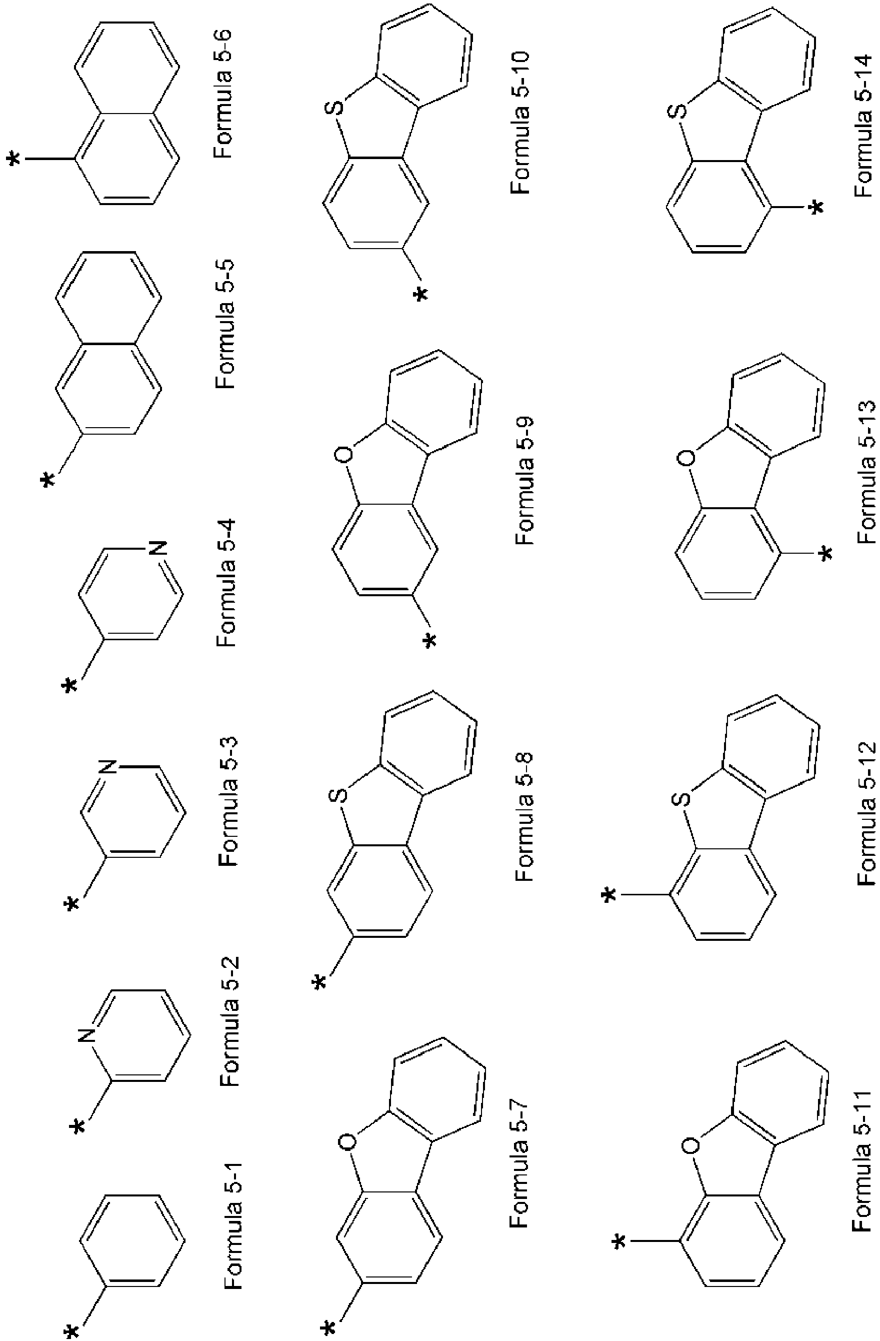

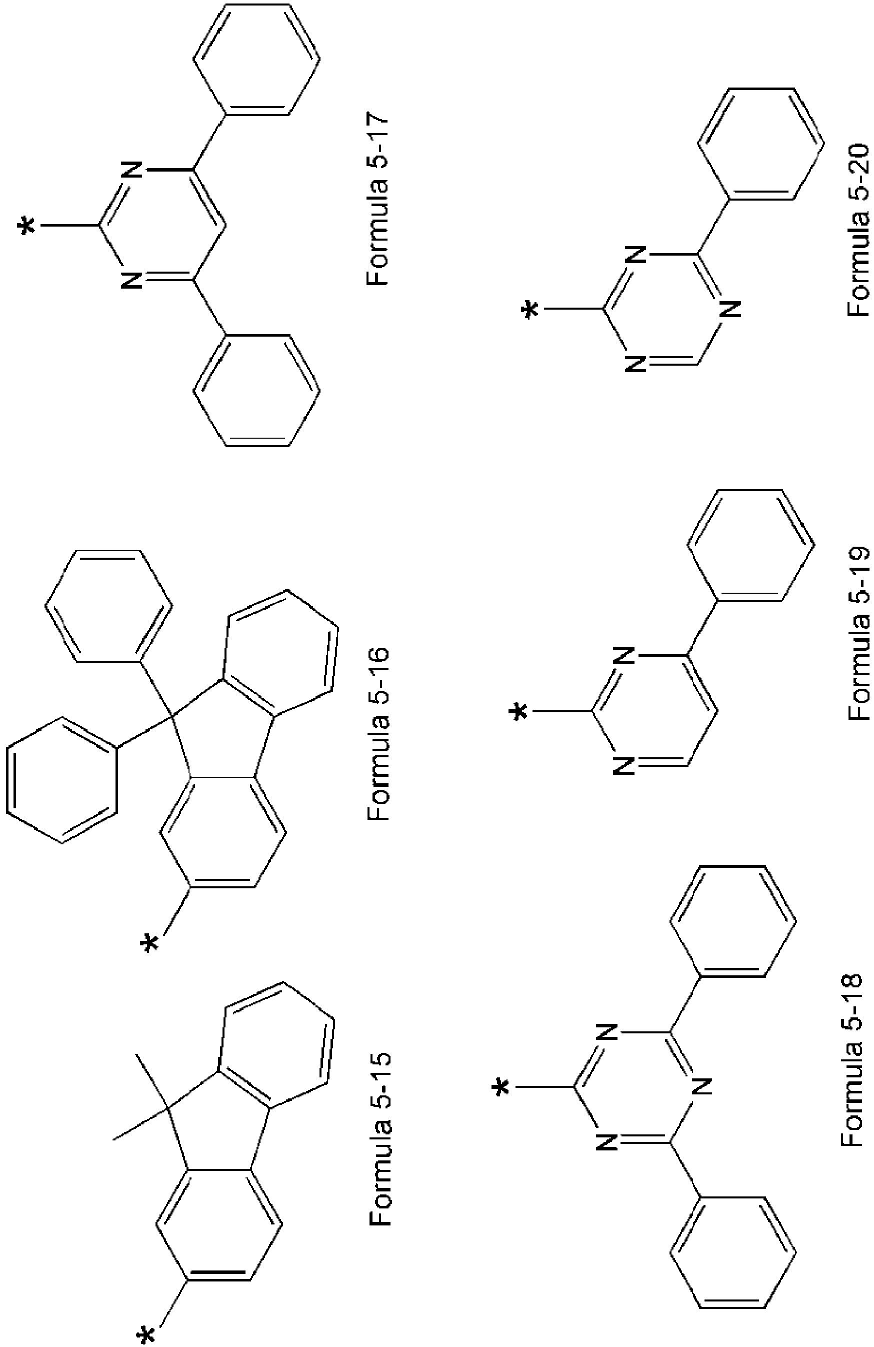

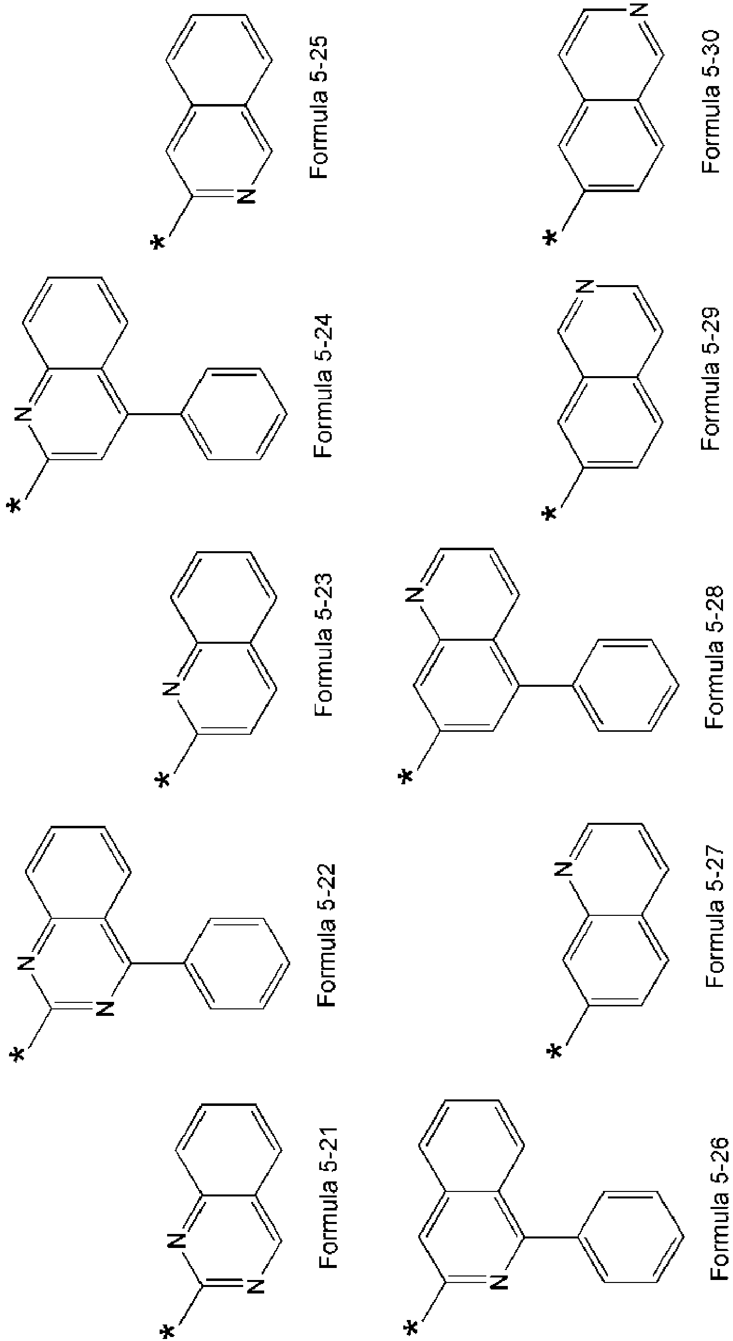

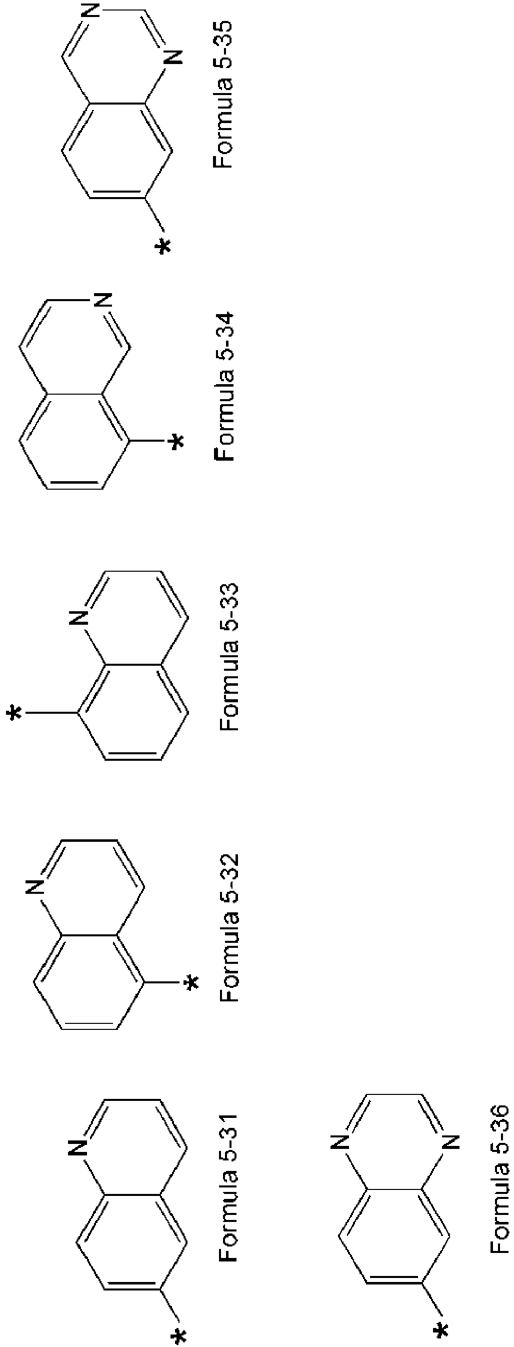

In Formula 1, b1 indicates the number of Rᵢs, and may be an integer from 1 to 3. For example, b1 may be 1 or 2, and in some embodiments, may be 1. When b1 is 2 or greater, at least two R₁s may be identical to or different from each other.

In Formula 1, b3 indicates the number of R₃s, and may be 1 or 2. When b3 is 2 two R₃s may be identical to or different from each other.

In Formula 1, b4 indicates the number of R₄s, and may be 1, 2, or 3. When b4 is 2 or greater, at least two R₄s may be identical to or different from each other.

In Formula 1, b5 indicates the number of R₅s, and may be 1, 2 or 3. When b5 is 2 or greater, at least two R₅s may be identical to or different from each other.

In Formula 1, b6 indicates the number of R₆s, and may be 1 or, 2. When b6 is 2 two R₆s may be identical to or different from each other.

In Formula 1, R₄ and R₅ may be both hydrogen atoms.

The amine-based compound of Formula 1 above may be a compound represented by Formula 1A below.

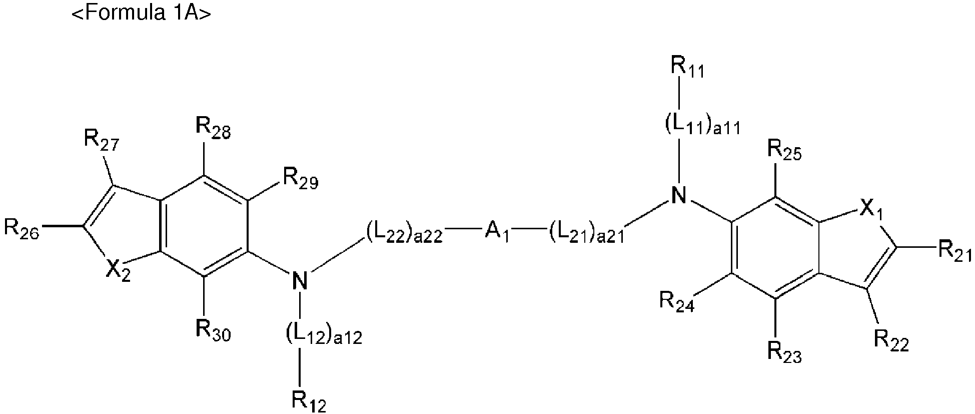

In Formula 1A, A₁, X₁, X₂, L₁₁, L₁₂, L₂₁, L₂₂, a11, a12, a21, a22, R₁₁, and R₁₂ are the same as those defined above in conduction with Formula 1. R₂₁ to R₃₀ in Formula 1A are the same as R₃ defined above in connection with Formula 1.

For example, in Formula 1A, A₁ may be selected from
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, and
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
X₁ and X₂ may be identical to each other;
L₁, L₂, L₁₁, L₁₂, L₂₁, and L₂₂ may be each independently selected from the groups represented by Formulae 2-1 to 2-30;
a1, a2, a11, a12, a21, and a22 may be each independently 0 or 1;
R₁, R₂, R₁₁, and R₁₂ may be each independently selected from the groups represented by Formulae 4-1 to 4-29;
R₂₁ to R₃₀ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group, and
the groups represented by Formulae 4-1 to 4-29 above.

In some other embodiments, in Formula 1A, A₁ may be selected from a naphthylene group, a dimethylfluorenylene group, a diphenylfluorenylene group, a spiro-fluorenylene group, a phenanthrenylene group, an anthracenylene group, a triphenylenylene group, a pyrenylene group, a chrysenylene group, and a benzofluoranthenylene group;
X₁ and X₂ may be both S, or X₁ and X₂ may be both O;
L₁₁, L₁₂, L₂₁, and L₂₂ may be each independently selected from the groups represented by Formulae 3-1 to 3-19 above;
a11, a12, a21, and a22 may be each independently 0 or 1;
R₁₁ and R₁₂ may be each independently selected from the groups represented by Formulae 5-1 to 5-36 above;
R₂₁ to R₃₀ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group, and
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group; and
the groups represented by Formulae 5-1 to 5-36 above.

For example, in Formula 1 A, R₂₃ to R₂₅, and R₂₈ to R₃₀ may be all hydrogen atoms.

In some embodiments, the amine-based compound of Formula 1 above may be a compound represented by one of Formulae 1A(1) to 1A(4) below:

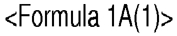

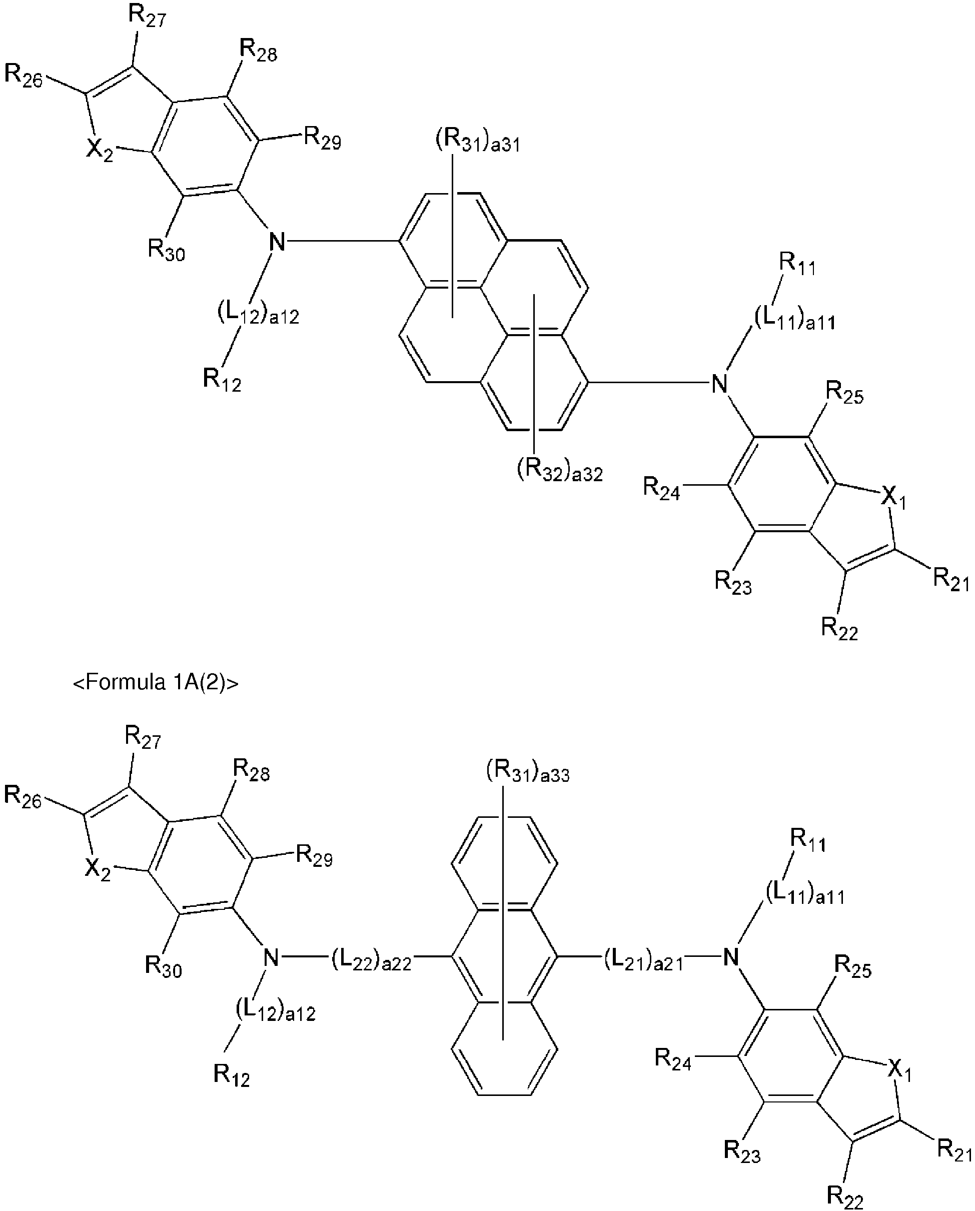

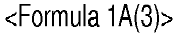

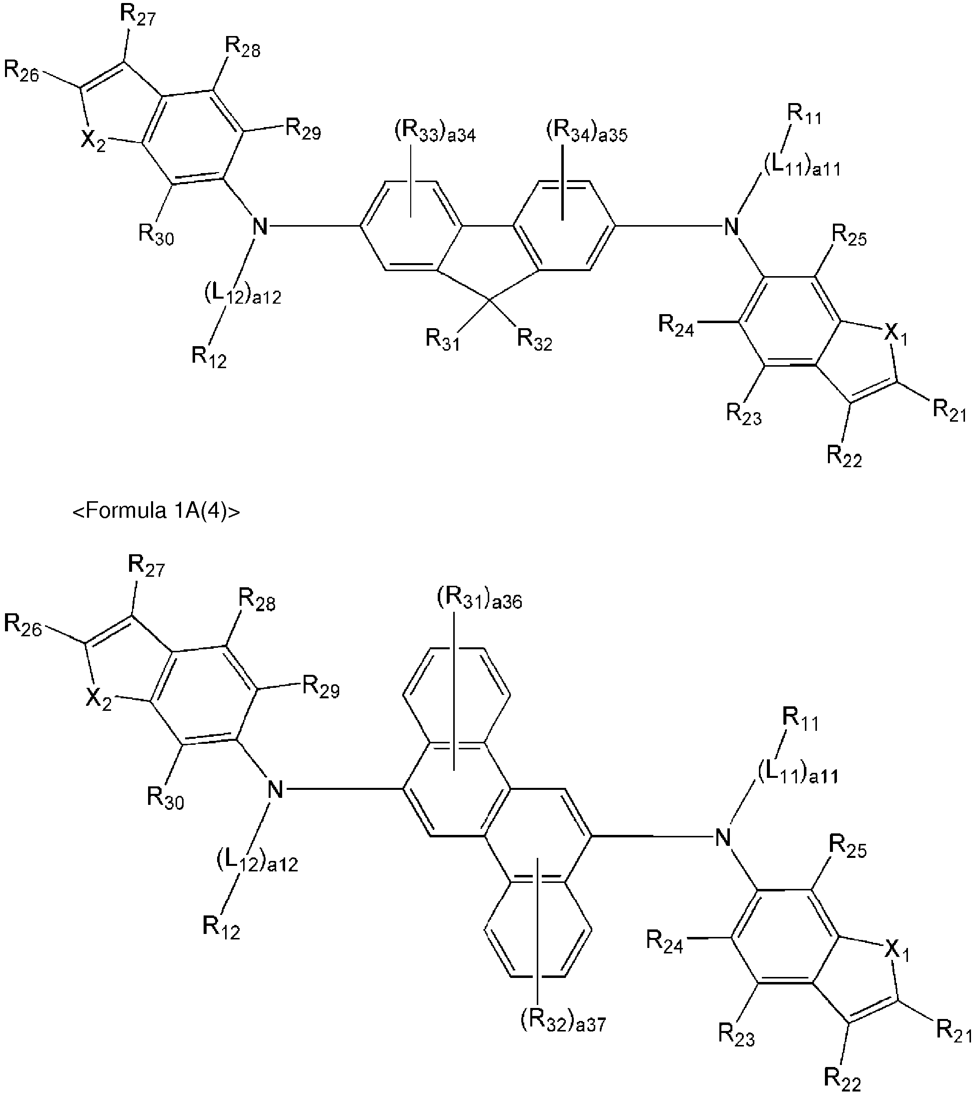

In Formulae 1A(1) to 1A(4), X₁, X₂, L₁₁, L₁₂, L₂₁, L₂₂, a11, a12, a21, a22, R₁₁, and R₁₂ are the same as those defined above in conjunction with Formula 1, and R₂₁ to R₃₄ are the same as R₃ defined above in conjunction with Formula 1.

In Formulae 1A(1) to 1A(4), a31 and a32 may be each independently an integer from 1 to 4, a33 may be an integer from 1 to 8, a34 and a35 may be each independently an integer from 1 to 3, and a36 and a37 may be each independently an integer from 1 to 5.

In some other embodiments, in Formulae 1A(1) to 1A(4), X₁ and X₂ may be identical to each other;
L₁, L₂, L₁₁, L₁₂, L₂₁, and L₂₂ may be each independently selected from the groups represented by Formulae 2-1 to 2-30;
a1, a2, a11, a12, a21, and a22 may be each independently 0 or 1;
R₁, R₂, R₁₁, and R₁₂ may be each independently selected from the groups represented by Formulae 4-1 to 4-29;
R₂₁ to R₃₄ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group, and
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group, and
the groups represented by Formulae 4-1 to 4-29 above; and
b1 may be 1 or 2. However, embodiments are not limited thereto.

In some other embodiments, in Formulae 1A(1) to 1A(4), X₁ and X₂ may be both S, or X₁ and X₂ may be both O;
L₁₁, L₁₂, L₂₁, and L₂₂ may be each independently selected from the groups represented by Formulae 3-1 to 3-19 above;
a11, a12, a21, and a22 may be each independently 0 or 1;
R₁₁ and R₁₂ may be each independently selected from the groups represented by Formulae 5-1 to 5-36 above;
R₂₁ to R₃₄ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group,
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group, and
the groups represented by Formulae 5-1 to 5-36 above.

For example, in Formulae 1A(1) to 1A(4), R₂₃ to R₂₅, and R₂₈ to R₃₀ may be all hydrogen atoms.

In Formula 1A(3), R₃, and R₃₂ may be each independently selected from a C₁-C₁₀ alkyl group, a C₁-C₁₀ alkoxy group, a phenyl group, a naphthyl group, and an anthracenyl group.

In Formula 1A(2), a21 and a22 may be both an integer of 1.

In Formulae 1A(1), 1A(2), and 1A(4), R₃, and R₃₂ may be all hydrogen atoms.

In Formulae 1A(3), R₃₃ and R₃₄ may be all hydrogen atoms.

In some embodiments, the amine-based compound of Formula 1 above may be a compound represented by one of the Compounds 1 to 108 below:

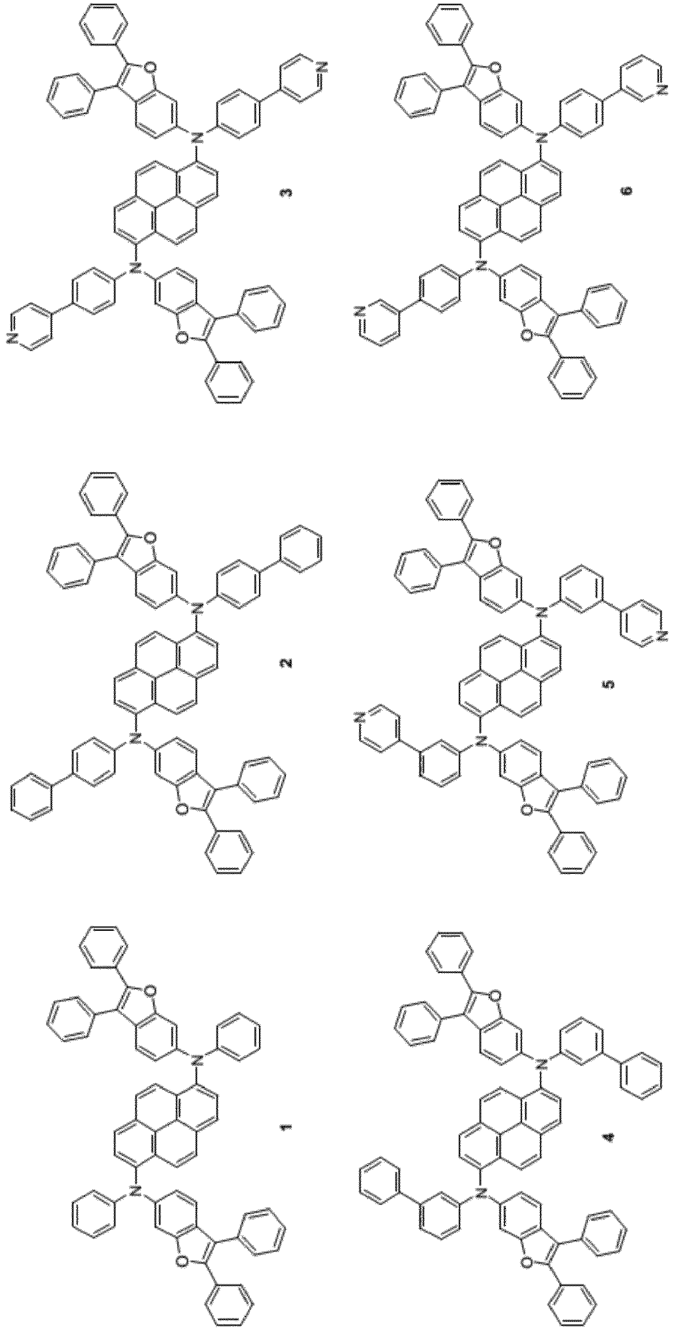

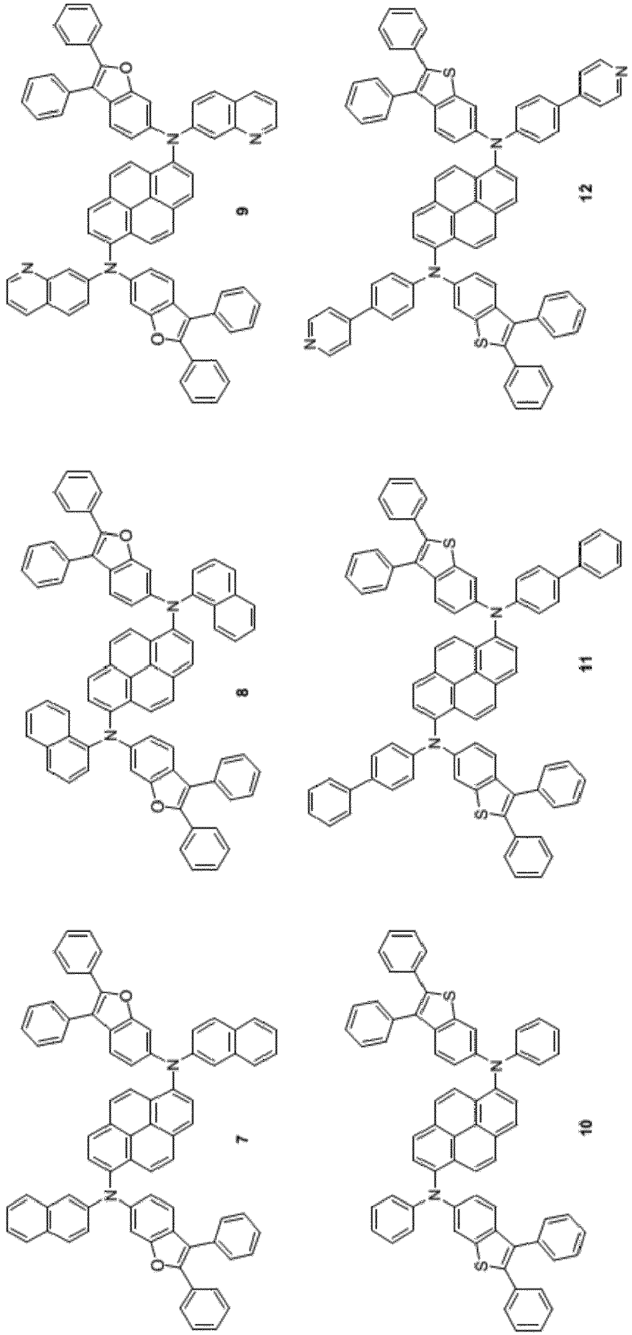

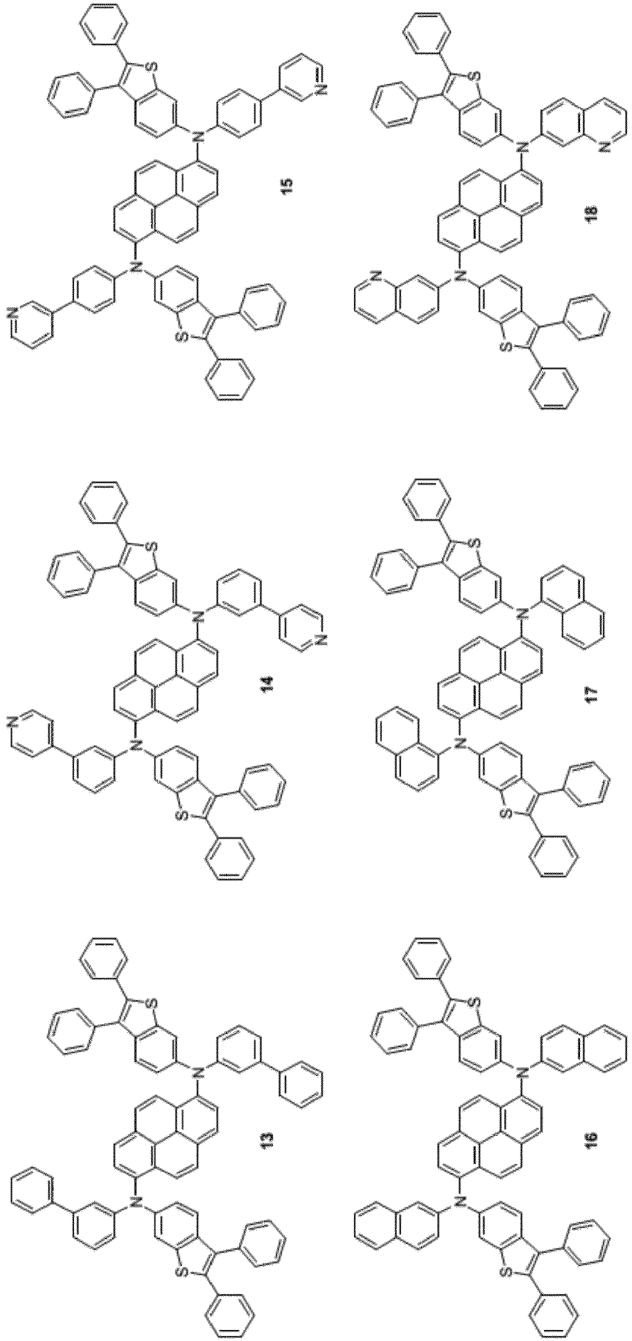

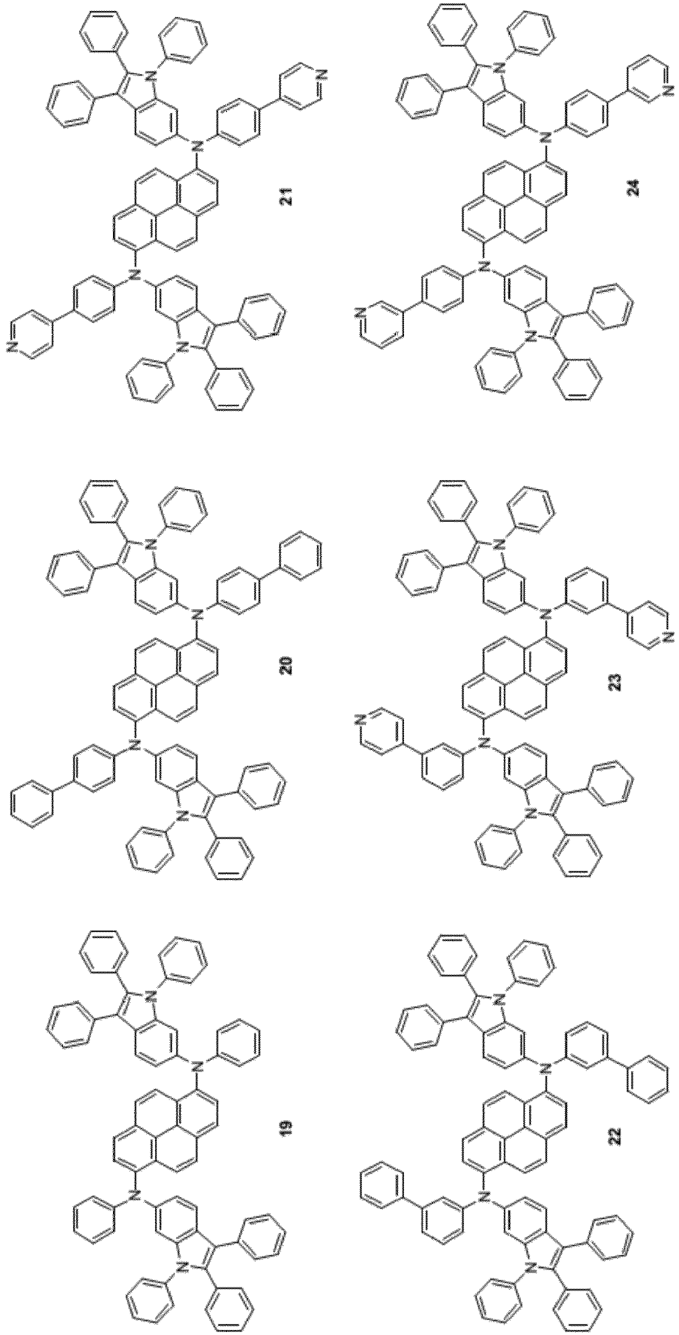

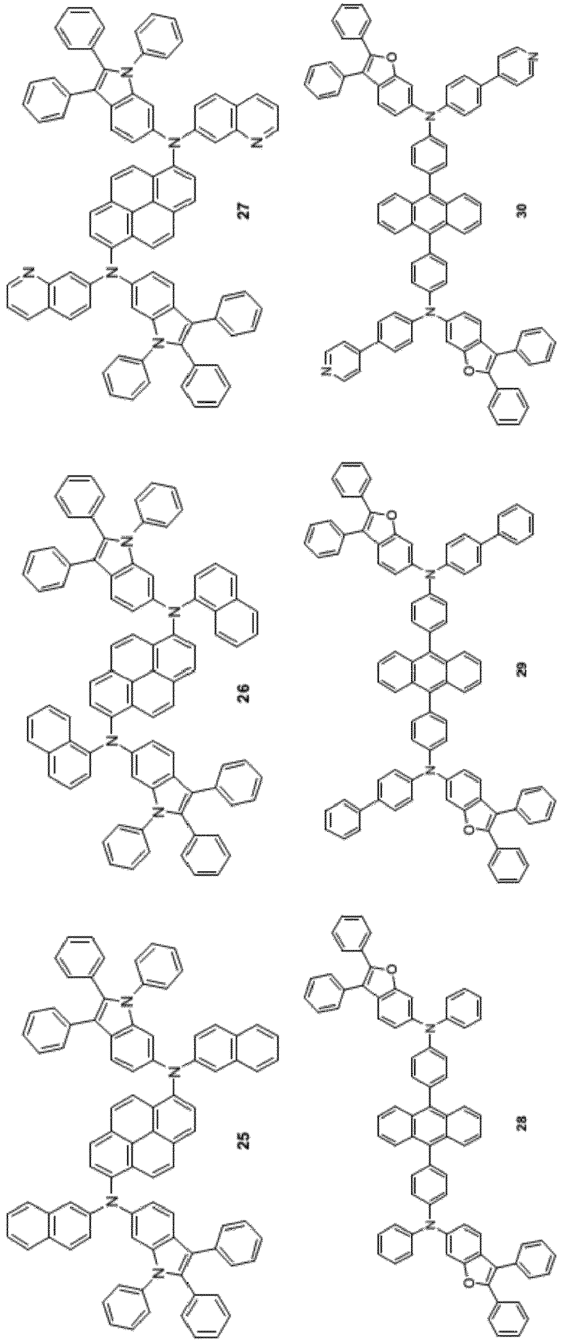

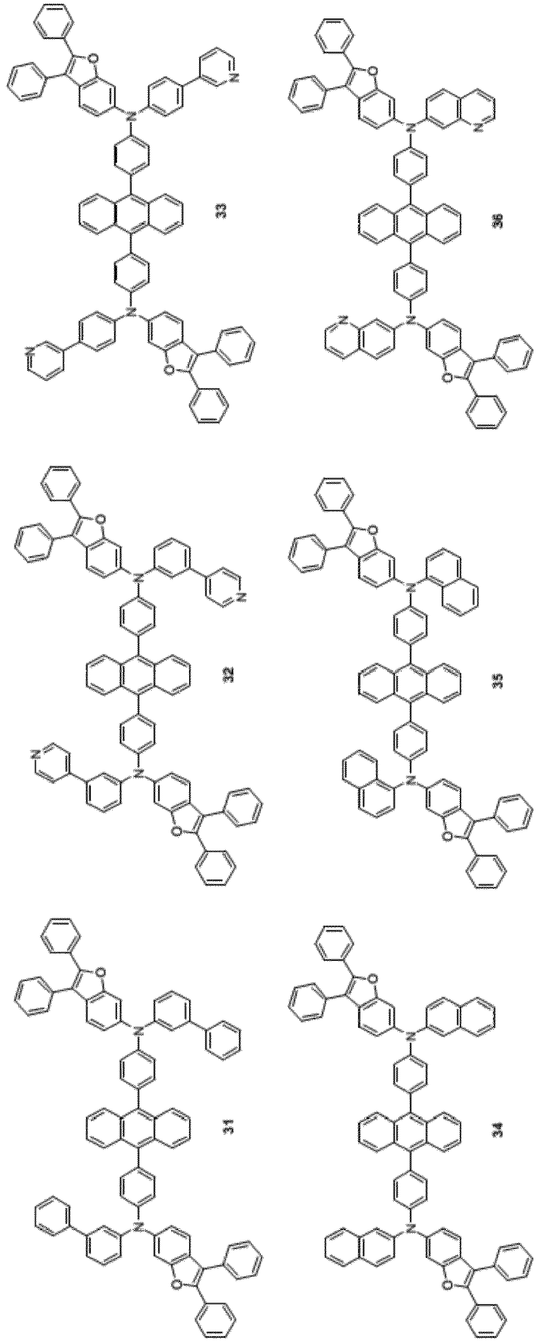

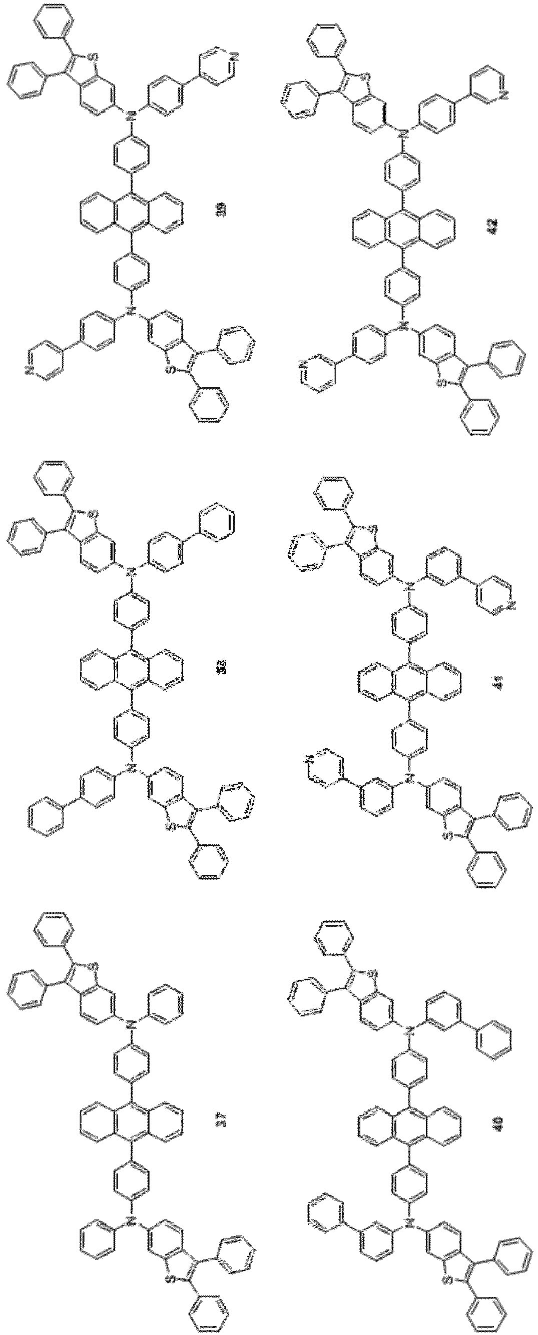

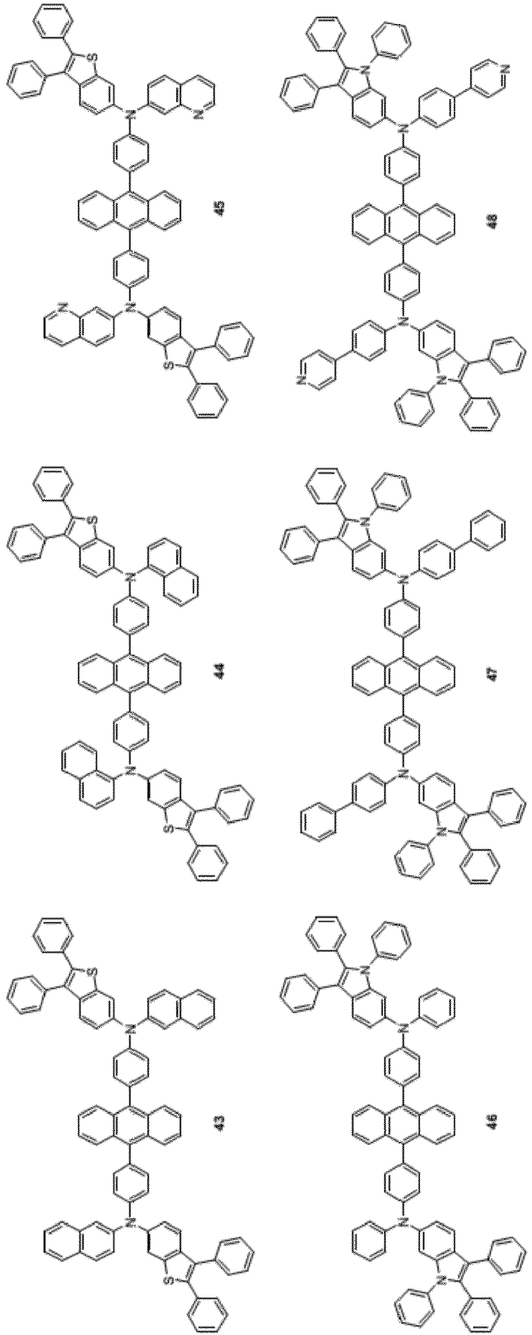

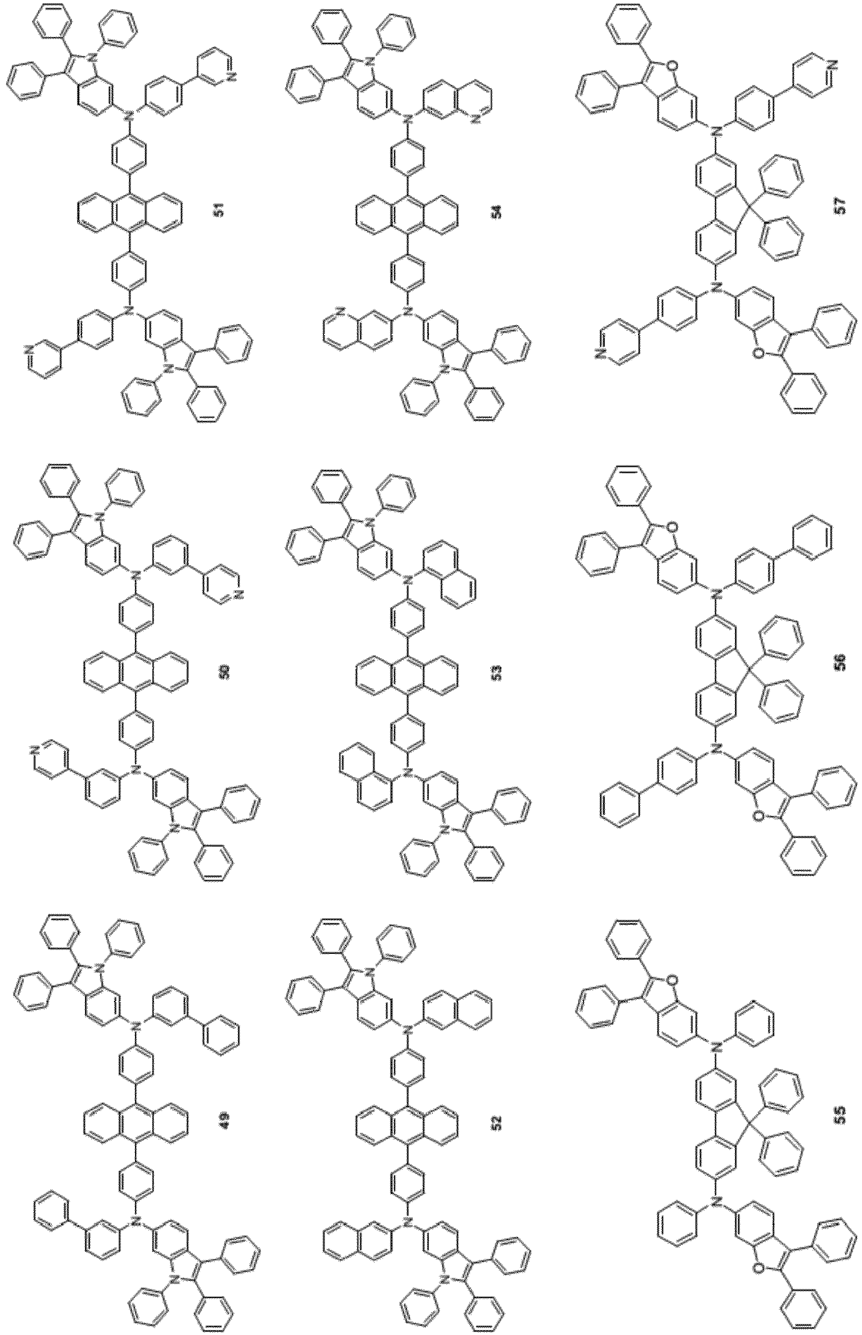

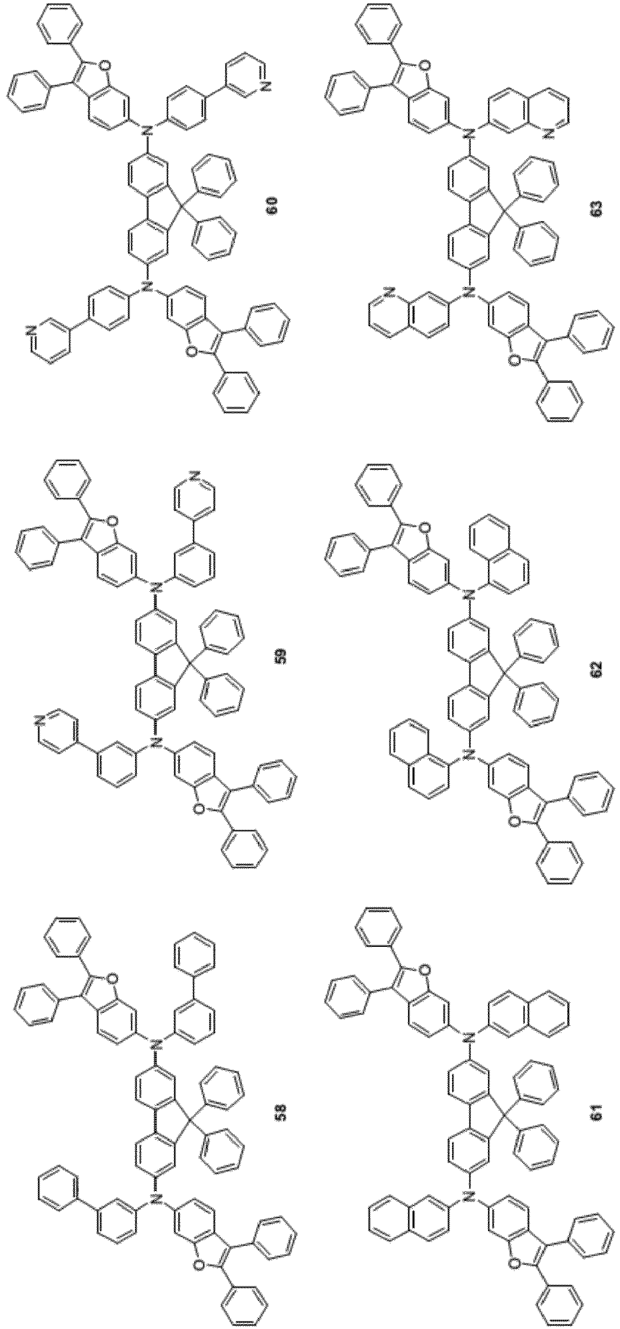

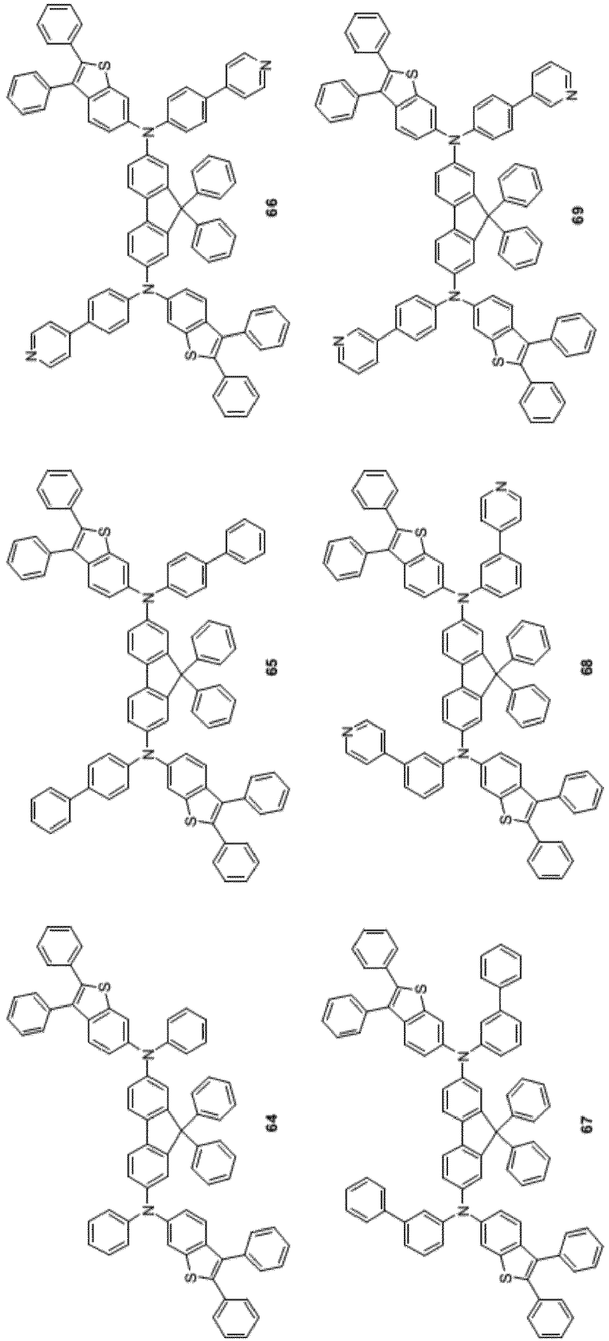

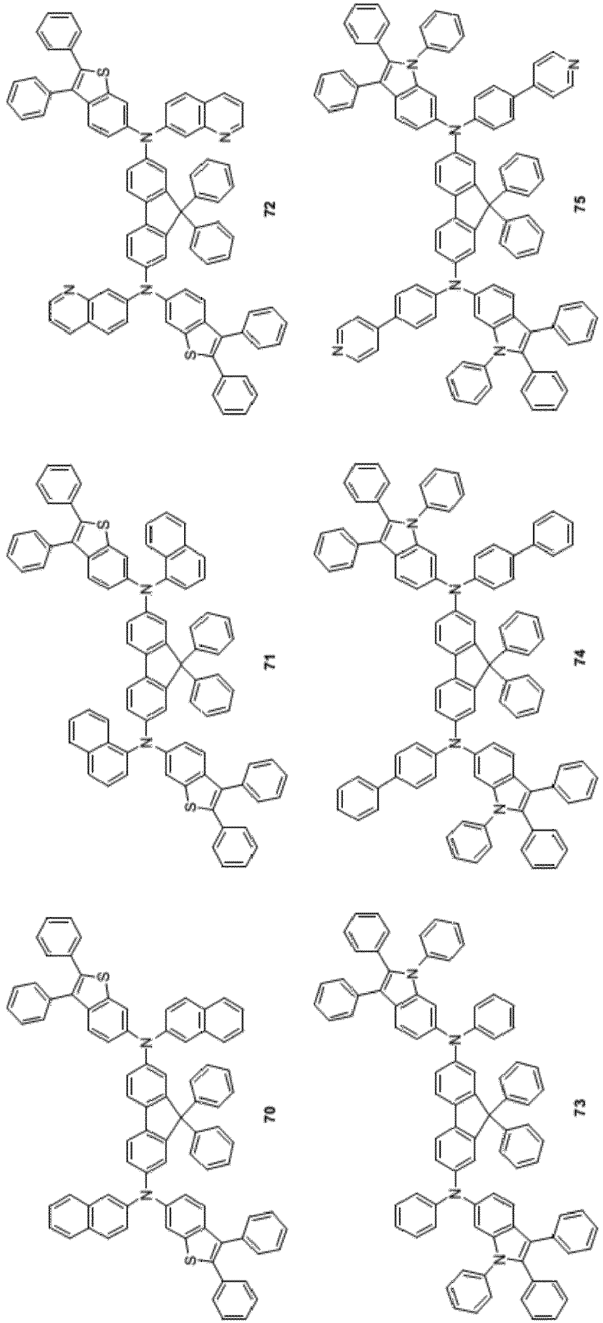

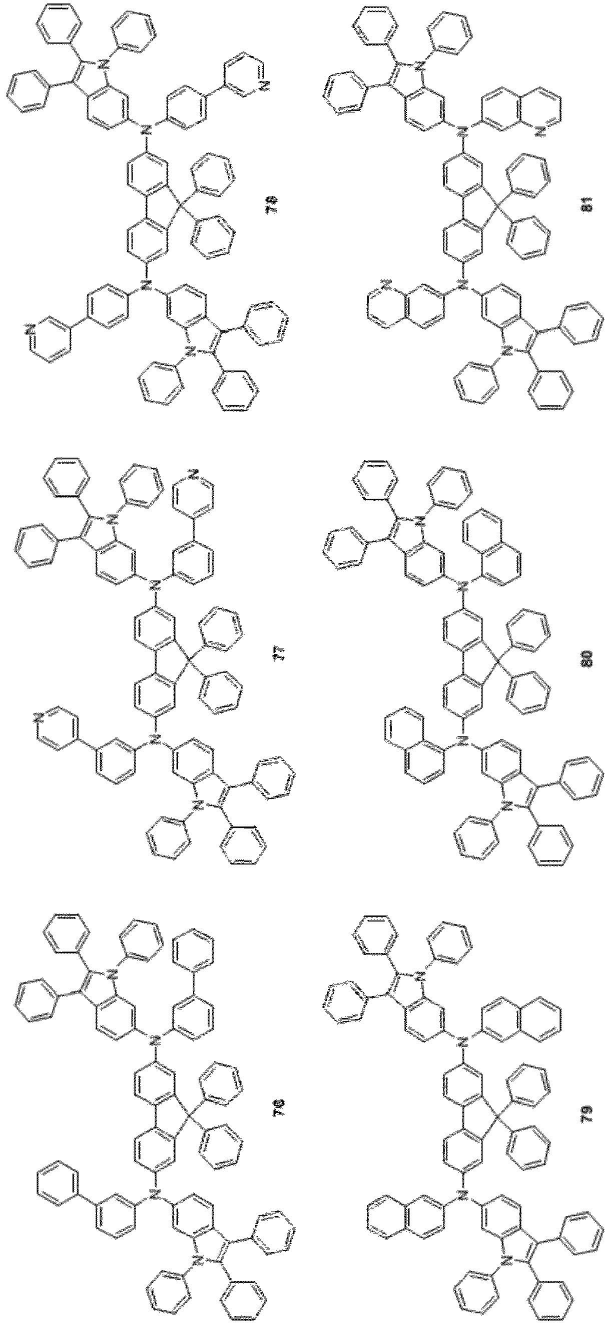

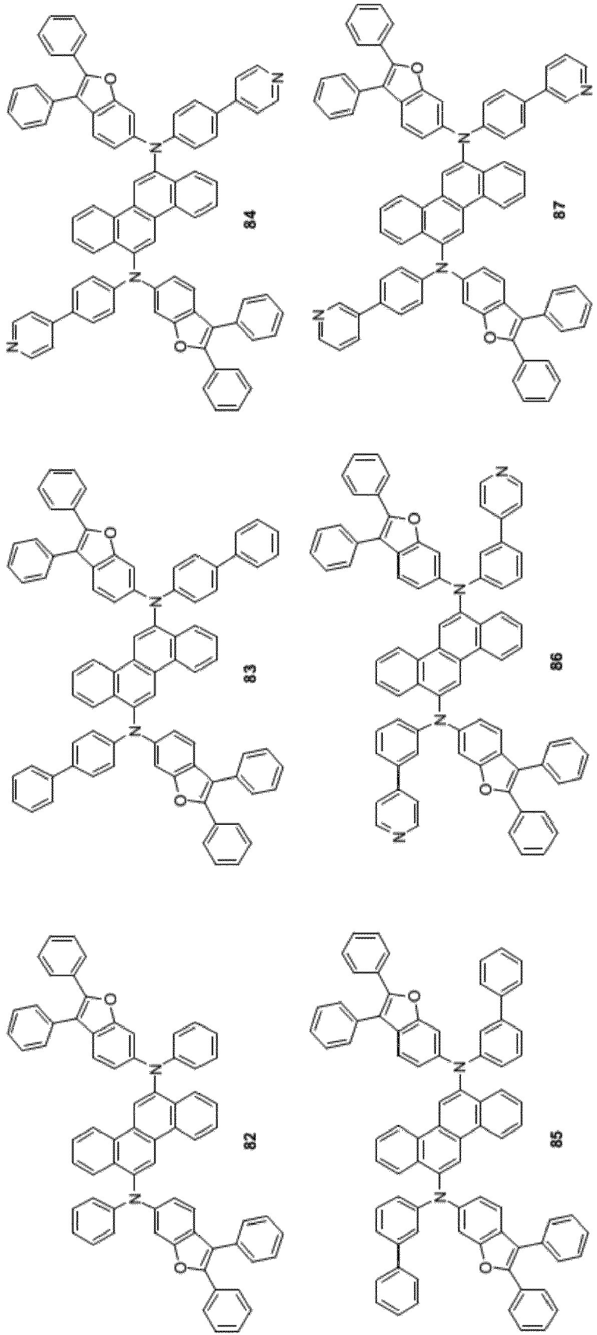

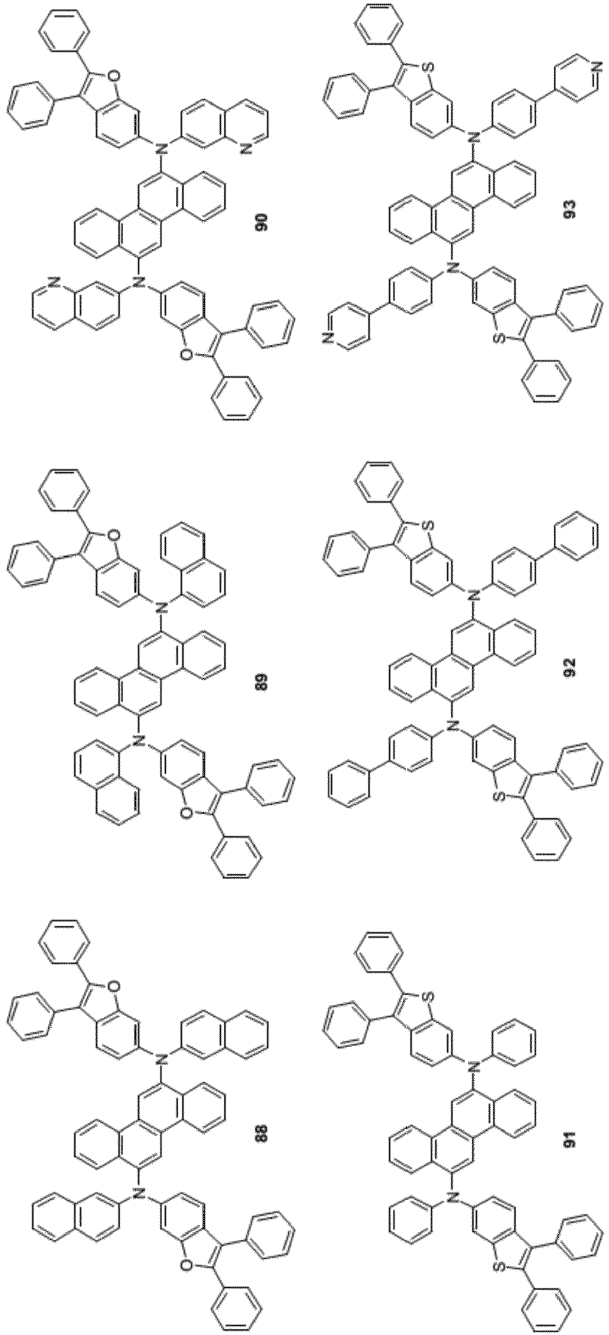

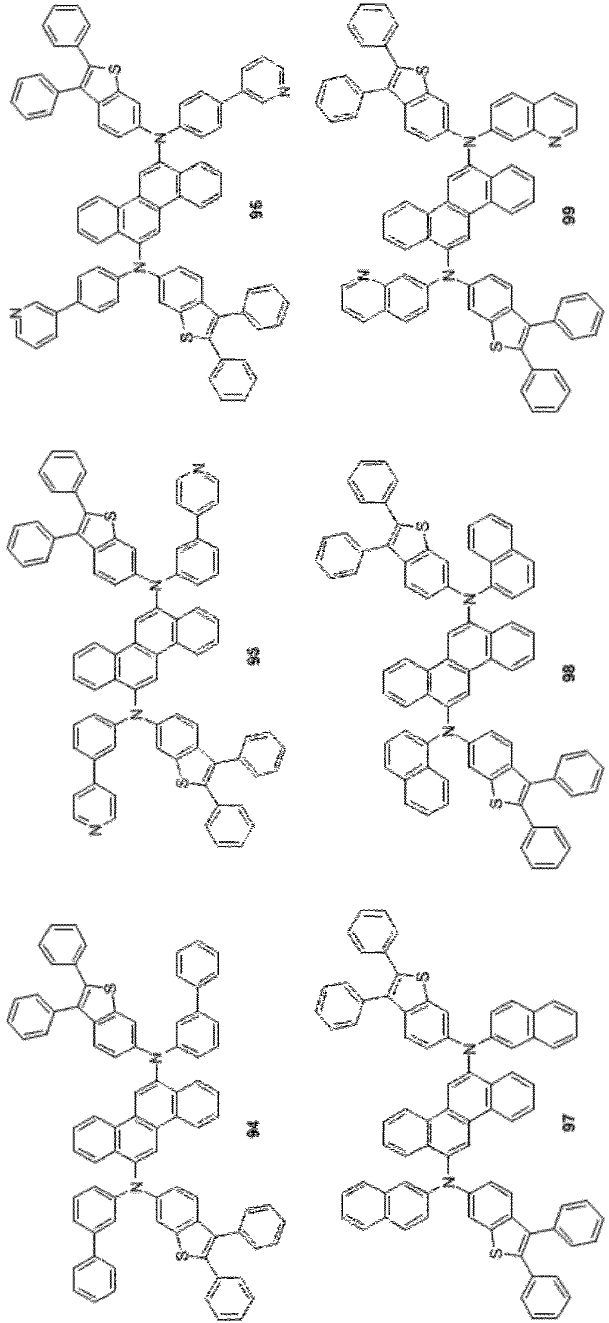

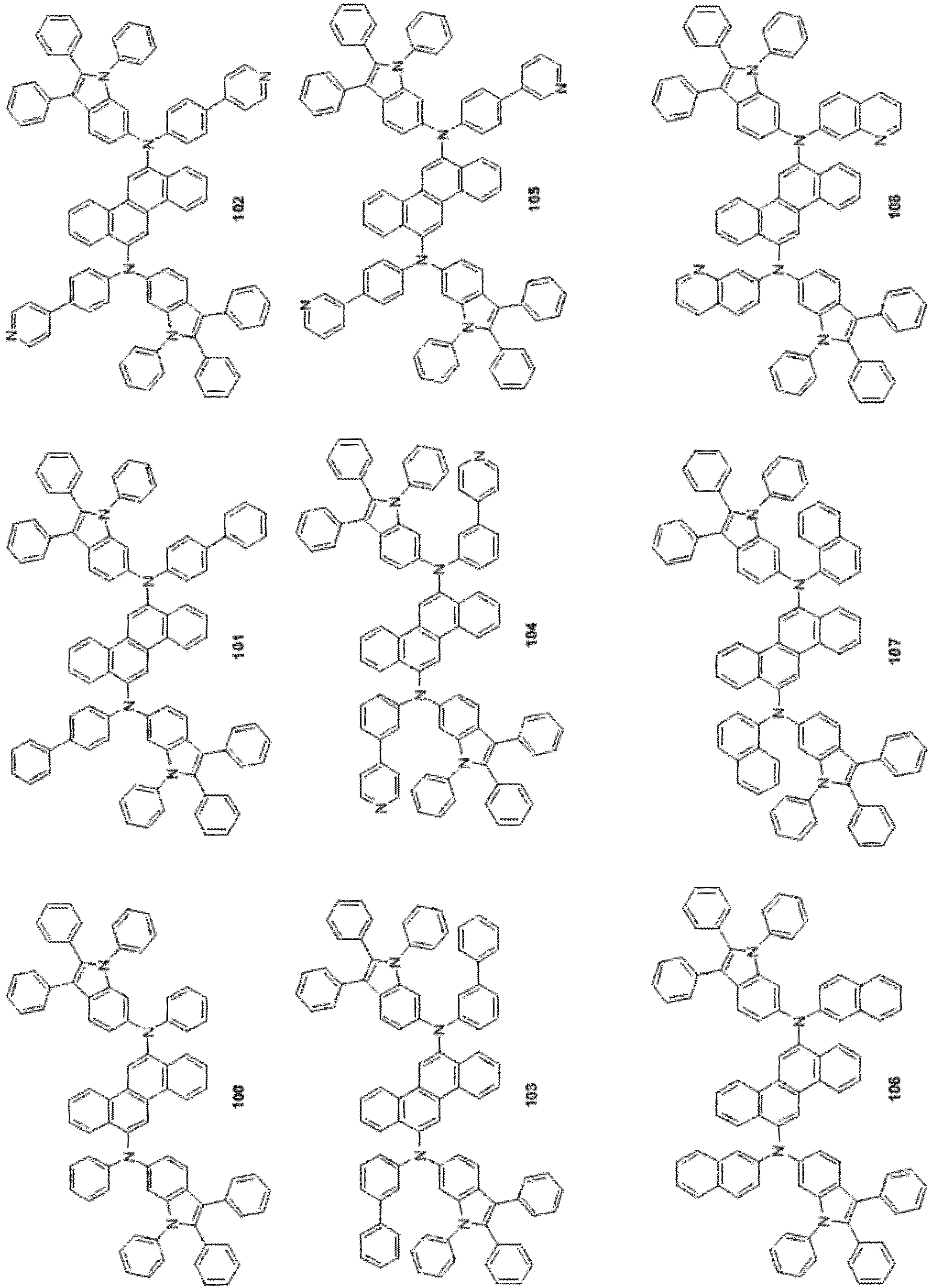

In the amine-based compound represented by Formula 1 above, X₁ is selected from N-[(L₁)ₐ₁-(R₁)_{b1}], S, and O, and X₂ is selected from N-[(L₂)ₐ₂-R₂], S, and O. Accordingly, the amine-based compound of Formula 1 may optimize electron injection balance. Thus, an organic light-emitting device including the amine-based compound of Formula 1 has improved efficiency characteristics.

In the amine-based compound represented by Formula 1 above, "N" is bound to a "benzo group" of an "indole-based moiety" (refer to Formula 1' below), which may provide improved electrical stability, not causing denaturation and/or decomposition of the compound despite of persistent movements of electrons. Accordingly, the amine-based compound of Formula 1 may optimize electron injection balance. Thus, an organic light-emitting device including the amine-based compound of Formula 1 may have improved efficiency characteristics.

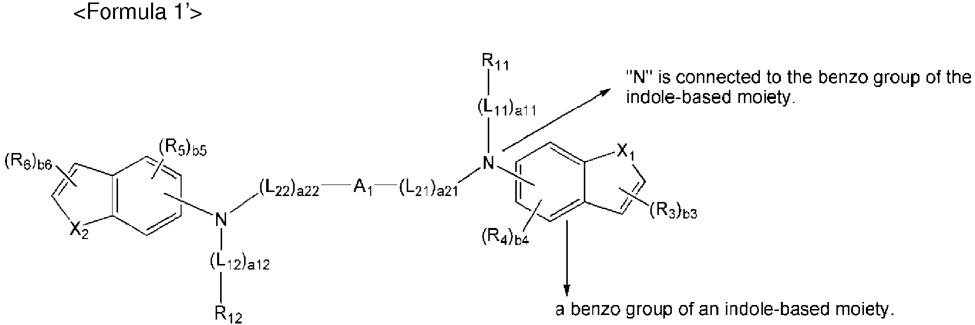

A synthesis method of the amine-based compound of Formula 1 above may be understood by those of ordinary skill in the art from Synthesis Examples 1 to 8 that will be described below.

The amine-based compound of Formula 1 above may be appropriate for use in an organic layer of an organic light-emitting device, for example, as a dopant of an emission layer of the organic layer.

According to another aspect, an organic light-emitting device includes a first electrode, a second electrode, and an organic layer disposed between the first electrode and the second electrode and including at least one of the amine-based compounds of Formula 1 above.

Due to the inclusion of the organic layer including the amine-based compound of Formula 1 above, the organic light-emitting device may have a low driving voltage, a high efficiency, high luminance, long lifetime, and improved color purity.

With regard to the color purity of the organic light-emitting device including the amine-based compound of Formula 1 above, an X coordinate may be from about 0.14 to about 0.16, and a Y coordinate may be about 0.18 or less, and in some embodiments, from about 0.10 to about 0.15, and in some other embodiments, may be from about 0.12 to about 0.14. For example, the amine-based compound of Formula 1 may emit blue light, for example, fluorescent blue light.

The amine-based compound of Formula 1 above may be used between a pair of electrodes of an organic light-emitting device. For example, the amine-based compound of Formula 1 above may be in at least one of an emission layer, a hole transport region between the first electrode and the emission layer (for example, including at least one of a hole injection layer, a hole transport layer, and an electron blocking layer), and an electron transport region between the emission layer and the second electrode (for example, including at least one of a hole blocking layer, an electron transport layer, and an electron injection layer). In some embodiments, the amine-based compound of Formula 1 may be in at least one of the hole transport region, the electron transport region, and the emission layer.

In some other embodiments, the amine-based compound of Formula 1 above may be in the emission layer of the organic light-emitting device. The amine-based compound of Formula 1 above may be a fluorescent dopant emitting, for example, blue light based on fluorescence mechanism.

In some embodiments, the organic light-emitting device may further include a compound represented by Formula 1000 below, in addition to the amine-based compound of Formula 1 above.

<Formula 1000> Y₁-A₂-Y₂

In Formula 1000, A₂ may be selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, aC₆-C₆₀ arylthio group, a C₂-C₆₀ heteroaryl group, -N(Q₁₁)(Q₁₂), and -Si(Q₁₃)(Q₁₄)(Q₁₅).

In Formula 1000, A₂ may be the same as A₁ defined above in conjunction with Formula 1.

In Formula 1000, Y₁ and Y₂ may be each independently selected from
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, and -N(Q₁₀₁)(Q₁₀₂); wherein
Q₁₀₁ and Q₁₀₂ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.

In some embodiments, in Formula 1000, Y₁ and Y₂ may be each independently selected from
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group, and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, a quinazolinyl group, and -N(Q₁₀₁)(Q₁₀₂); wherein
Q₁₀₁ and Q₁₀₂ may be each independently selected from a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group.

The amine-based compound of Formula 1 above and the compound of Formula 1000 above may be both in the emission layer of the organic light-emitting device. An amount of the amine-based compound of Formula 1 in the emission layer may be less than that of the compound of Formula 1000 above. That is, the amine-based compound of Formula 1 above may serve as a dopant, while the compound of Formula 1000 may serve as a host.

As used herein, "(for example, the organic layer) including at least one amine-based compound means that "(the organic layer) including one of the amine-based compounds of Formula 1 above, or at least two different amine-based compounds of Formula 1 above".

In some embodiments, the organic layer may include only Compound 1 above as the amine-based compound. The Compound 1 may be in the EML layer of the organic light-emitting device. In some embodiments, the organic layer may include Compounds 1 and 2 as the amine-based compound. In this regard, Compounds 1 and 2 may be present in the same layer (for example, in the emission layer) or may be present in different layers.

The first electrode may be an anode as a hole injection electrode, while the second electrode may be a cathode as an electron injection electrode. In some embodiments, the first electrode may be a cathode as an electron injection electrode, while the second electrode may be a cathode as a hole injection electrode.

For example, the first electrode may be an anode, the second electrode may be a cathode, and the organic layer may include i) a hole transport region disposed between the first electrode and the emission layer and including at least one of a hole injection layer, a hole transport layer, and an electron blocking layer, and ii) an electron transport region disposed between the emission layer and the second electrode and including at least one of a hole blocking layer, an electron transport layer, and an electron injection layer.

The term "organic layer" as used herein refers to a single layer and/or a plurality of layers disposed between the first and second electrodes of the organic light-emitting device. The "organic layer" may include an organic compound and an organic metal complex including metal.

FIG. 1 illustrates a schematic sectional view of an organic light-emitting device 10 according to an embodiment. Hereinafter, a structure of an organic light-emitting device according to an embodiment and a method of manufacturing the same will now be described with reference to FIG. 1. Referring to FIG. 1, the organic light-emitting device 10 has a structure in which a substrate 11, a first electrode 13, an organic layer 15, and a second electrode 17 are sequentially stacked in this order.

The substrate 11 may be any substrate that is used in existing organic light-emitting devices. In some embodiments the substrate 11 may be a glass substrate or a transparent plastic substrate with strong mechanical strength, thermal stability, transparency, surface smoothness, ease of handling, and water resistance.

The first electrode 13 may be formed by depositing or sputtering a first electrode-forming material on the substrate 11. The first electrode 13 may be an anode. A material for the first electrode 13 may be a material having a high work function to facilitate hole injection. The first electrode 13 may be a reflective electrode or a transmission electrode. The material for the first electrode 13 may be indium tin oxide (ITO), indium zinc oxide (IZO), tin oxide (SnO₂), or zinc oxide (ZnO). The first electrode 13 may be formed as a reflective electrode from magnesium (Mg), aluminum (Al), aluminum-lithium (Al-Li), calcium (Ca), magnesium-indium (Mg-In), magnesium-silver (Mg-Ag), or the like.

The first electrode 13 may have a single-layer structure or a multi-layer structure including at least two layers. For example, the first electrode 13 may have a three-layered structure of ITO/Ag/ITO.

The organic layer 15 may be disposed on the first electrode 13.

The organic layer 15 may include a hole transport region including a hole injection layer (HIL) and a hole transport layer (HTL) that are sequentially stacked upon one another; an emission layer (EML); and an electron transport region including an electron transport layer (ETL) and an electron injection layer (EIL) that are sequentially stacked upon one another.

The HIL may be formed on the first electrode 13 by any of a variety of methods, including vacuum deposition, spin coating, casting, Langmuir-Blodgett (LB) deposition, and the like.

When the HIL is formed using vacuum deposition, vacuum deposition conditions may vary according to the compound that is used to form the HIL, and the desired structure and thermal properties of the HIL to be formed. For example, vacuum deposition may be performed at a temperature of about 100°C to about 500°C, a pressure of about 10⁻⁸ torr to about 10⁻³ torr, and a deposition rate of about 0.01 to about 100 Å/sec. However, the deposition conditions are not limited thereto.

When the HIL is formed using spin coating, the coating conditions may vary according to the compound that is used to form the HIL, and the desired structure and thermal properties of the HIL to be formed. For example, the coating rate may be in the range of about 2000 rpm to about 5000 rpm, and a temperature at which heat treatment is performed to remove a solvent after coating may be in the range of about 80 °C to about 200°C. However, the coating conditions are not limited thereto.

The HIL may be formed of any known hole injecting material. Examples of materials for the HIL are N,N'-diphenyl-N,N'-bis-[4-(phenyl-m-tolyl-amino)-phenyl]-biphenyl-4,4'-diamine, (DNTPD), 4,4',4"-tris (3-methylphenylphenylamino) triphenylamine (m-MTDATA), N,N'-di(1-naphthyl)-N,N'-diphenylbenzidine (NPB), TDATA (refer to a formula below), 2-TNATA (refer to a formula below), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

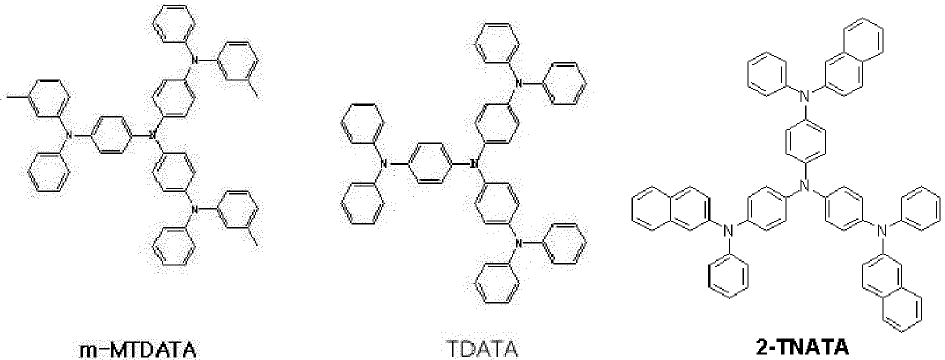

The thickness of the HIL may be about 100 Å to about 10000 Å, and in some embodiments, may be from about 100 Å to about 1000 Å. When the thickness of the HIL is within these ranges, the HIL may have good hole injecting ability without a substantial increase in driving voltage.

Then, a HTL may be formed on the HIL by using vacuum deposition, spin coating, casting, Langmuir-Blodgett (LB) deposition, or the like. When the HTL is formed using vacuum deposition or spin coating, the conditions for deposition and coating may be similar to those for the formation of the HIL, though the conditions for the deposition and coating may vary according to the material that is used to form the HTL.

Examples of suitable known HTL forming materials are carbazole derivatives, such as N-phenylcarbazole or polyvinylcarbazole, N,N'-bis(3-methylphenyl)-N,N'-diphenyl-[1,1-biphenyl]-4,4'-diamine (TPD), 4,4',4"-tris(N-carbazolyl)triphenylamine (TCTA), and N,N'-di(1-naphthyl)-N,N'-diphenylbenzidine) (NPB).

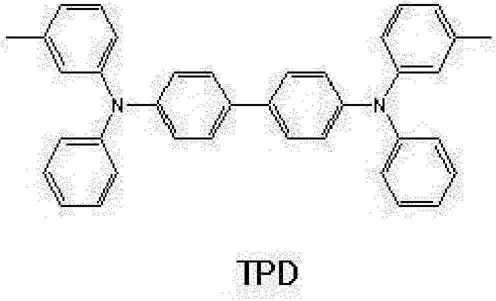

The thickness of the HTL may be from about 50 Å to about 2000 Å, and in some embodiments, may be from about 100 Å to about 1500 Å. When the thickness of the HTL is within these ranges, the HTL may have good hole transporting ability without a substantial increase in driving voltage.

In some embodiments, at least one of the HIL and HTL may include at least one of a compound of Formula 300 below and a compound of Formula 301 below:

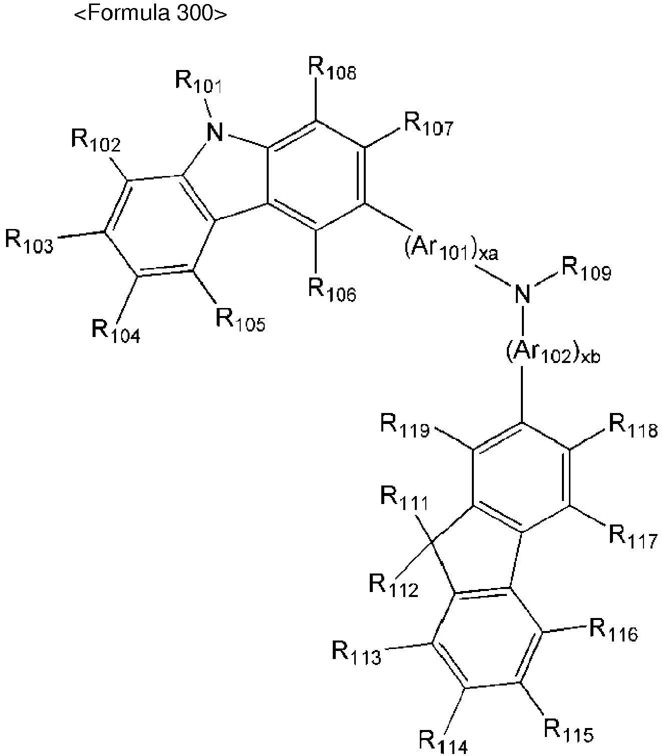

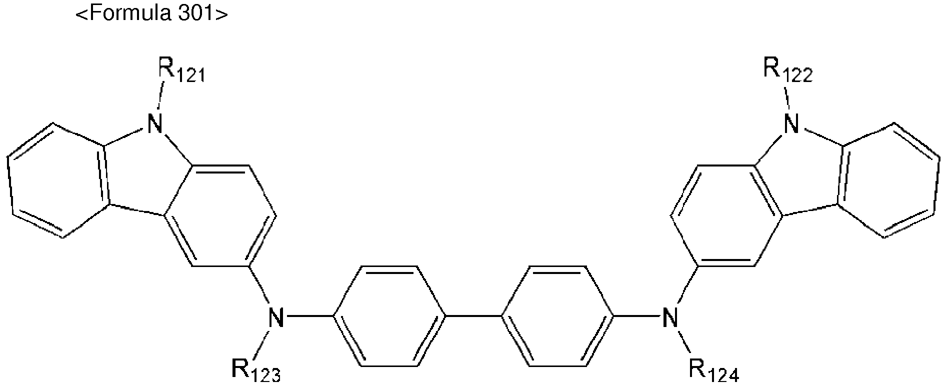

In Formula 300, Ar₁₀₁ and Ar₁₀₂ may be each independently selected from
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group, and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group, each substituted with at least one of a deuterium atom, a halogen atom, a hydroxyl group, a nitro group, a cyano group, an amino group, an amidino group, a hydrazine, a hydrazone, a carboxylic acid group or salt thereof, a sulfuric acid group or salt thereof, a phosphoric acid group or salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₃-C₁₀ heterocycloalkyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.

In Formula 300, xa and xb may be each independently an integer from 0 to 5, for example, may be 0, 1, or 2. For example, xa may be 1, and xb may be 0, but are not limited thereto.

In Formulae 300 and 301, R₁₀₁ to R₁₀₈, R₁₁₁ to R₁₁₉, and R₁₂₁ to R₁₂₄ may be each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, or a hexyl group), and a C₁-C₁₀ alkoxy group (for example, a methoxy group, an ethoxy group, a propoxy group, a butoxy group, or a pentoxy group),
a C₁-C₁₀ alkyl group and a C₁-C₁₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, and a phosphoric acid group or a salt thereof,
a phenyl group, a naphthyl group, an anthracenylene group, a fluorenyl group, and a pyrenyl group, and
a phenyl group, a naphthyl group, an anthracenyl group, a fluorenyl group, and a pyrenyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₁₀ alkyl group, and a C₁-C₁₀ alkoxy group.

In Formula 300, R₁₀₉ may be one of a phenyl group, a naphthyl group, an anthryl group, a biphenyl group, a pyridyl group; and a phenyl group, a naphthyl group, an anthracenyl group, a biphenyl group, and a pyridyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a substituted or unsubstituted C₁-C₂₀ alkyl group, and a substituted or unsubstituted C₁-C₂₀ alkoxy group.

In an embodiment the compound of Formula 300 may be a compound represented by Formula 300A below:

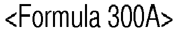

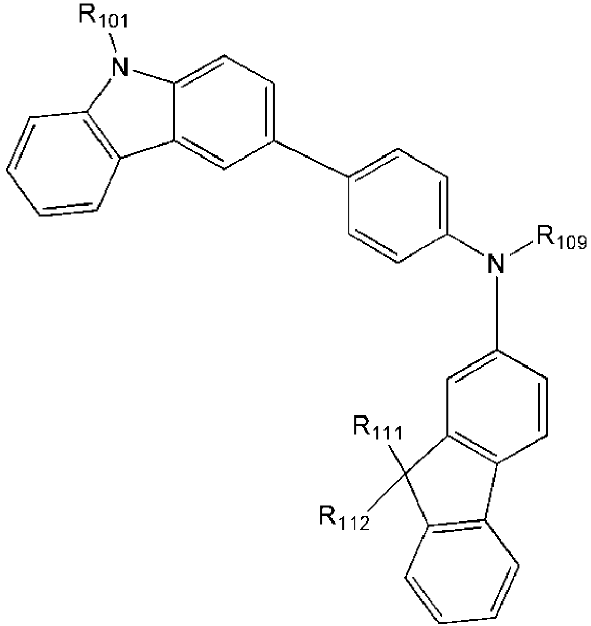

In Formula 300A, R₁₀₁, R₁₁₁, R₁₁₂, and R₁₀₉ may be as defined above.

In some embodiments, at least one of the HIL and HTL may include at least one of compounds represented by Formulae 301 to 320 below:

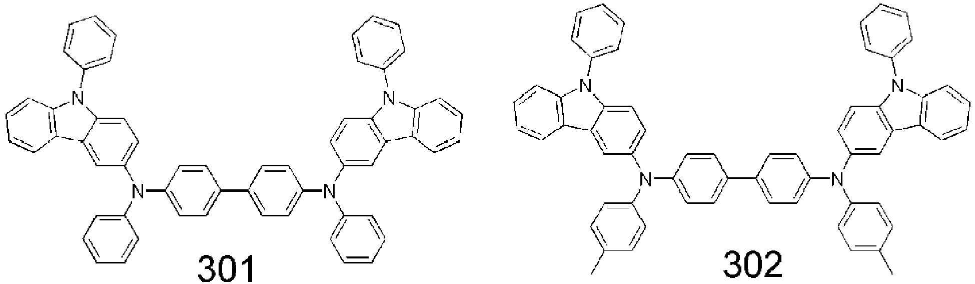

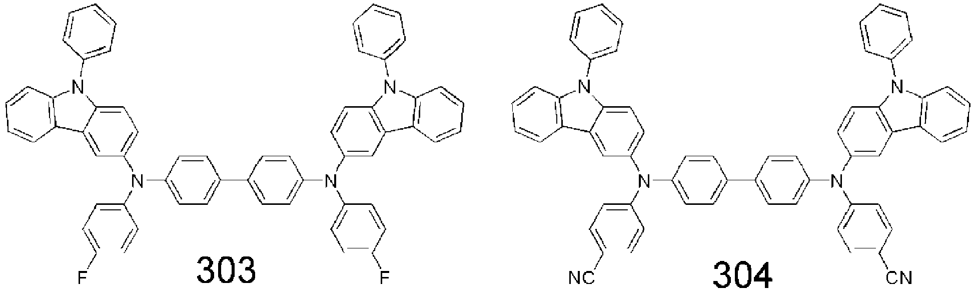

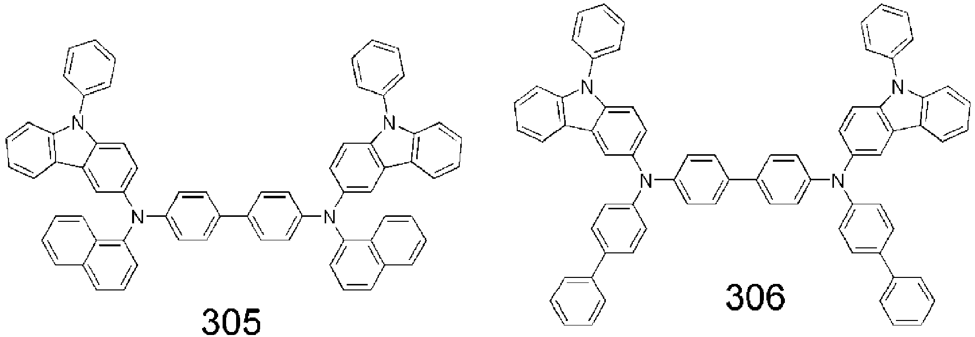

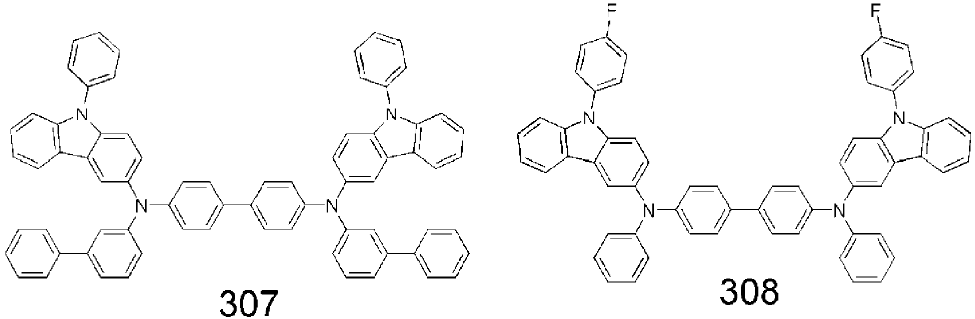

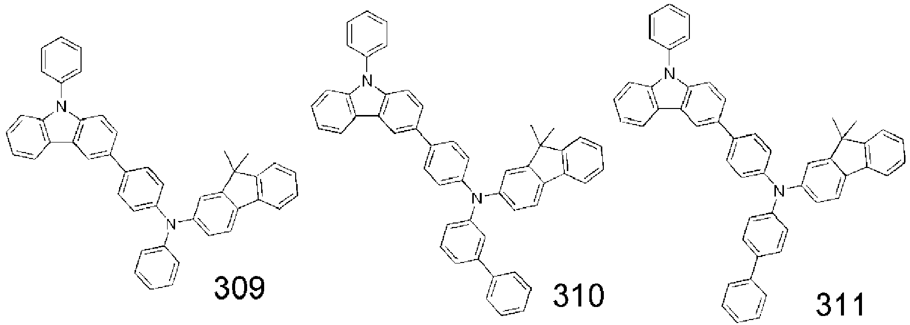

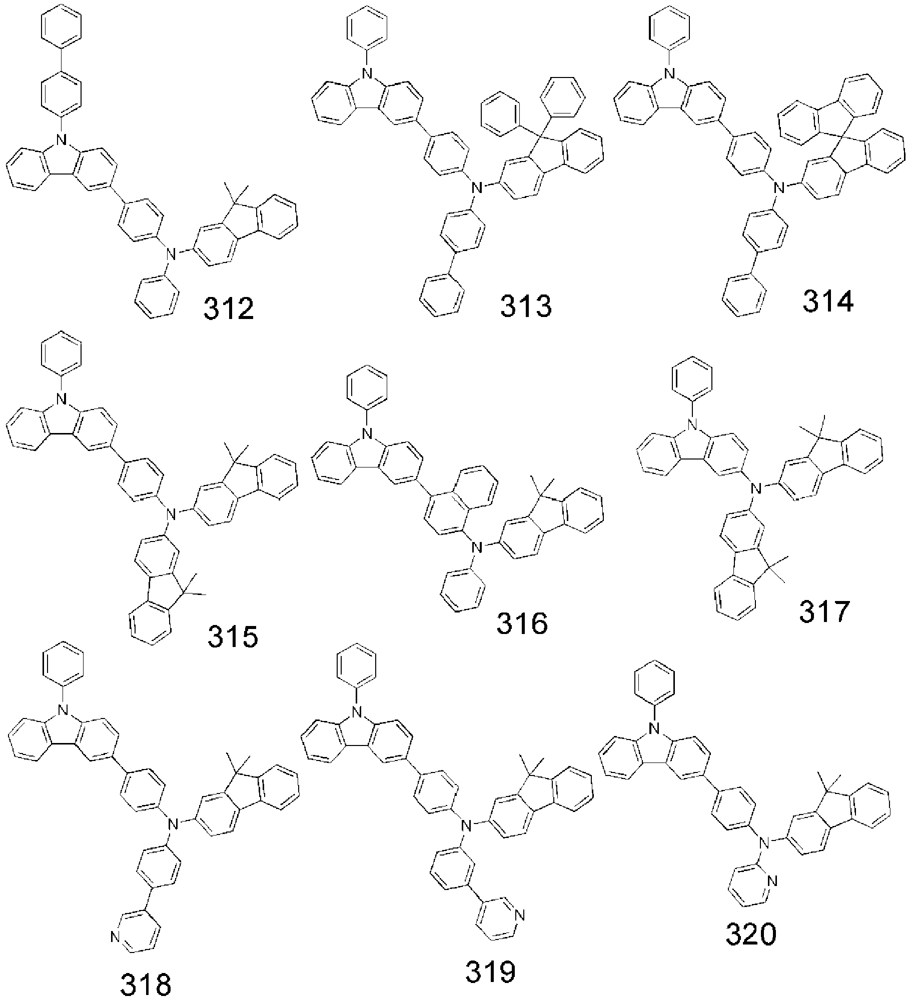

The hole transport region may further include a charge-generating material for improved layer conductivity, in addition to a widely-known hole injection material and a widely-known hole transport material.

The charge-generating material may be, for example, a p-dopant. The p-dopant may be one of quinine derivatives, metal oxides, and compounds with a cyano group, but are not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), and the like; metal oxides such as tungsten oxide, molybdenum oxide, and the like; and cyano-containing compounds such as Compound 200 below.

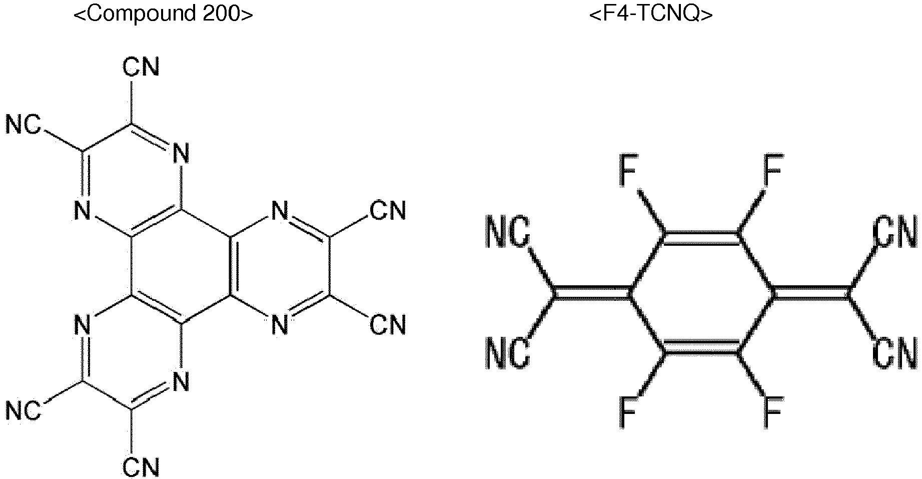

When the hole transport region further includes such a charge-generating material as described above, the charge-generating material may be homogeneously dispersed or inhomogeneously distributed in the hole transport region.

The hole transport region may further include a buffer layer between the HTL and the EML.

The buffer layer may compensate for an optical resonance distance of light according to a wavelength of the light emitted from the EML, and thus may increase efficiency. The butter layer may include any hole injecting material or hole transporting material that are widely known. In some embodiments, the buffer layer may include the same material as one of the materials in the HTL underlying the buffer layer.

Subsequently, the EML may be formed on the hole transport region by using vacuum deposition, spin coating, casting, LB deposition, or the like. When the EML is formed using vacuum deposition or spin coating, the deposition and coating conditions may be similar to those for the formation of the HIL, though the conditions for deposition and coating may vary depending on the material used to form the EML.

The emission layer may include a host, and a dopant,

For example, the host may further include at least one of Alq₃, 4,4'-N,N'- dicarbazole-biphenyl (CBP), 9,10-di(naphthalene-2-yl)anthracene (DNA), TCTA (refer to a formula below), 1,3,5-tris(N-phenylbenzimidazole-2-yl)benzene (TPBI), 3-tert-butyl-9,10-di-2-naphthylanthracene (TBADN), E3 (refer to a formula below), dmCBP (refer to a formula below), and Compounds 501 to 509 below.

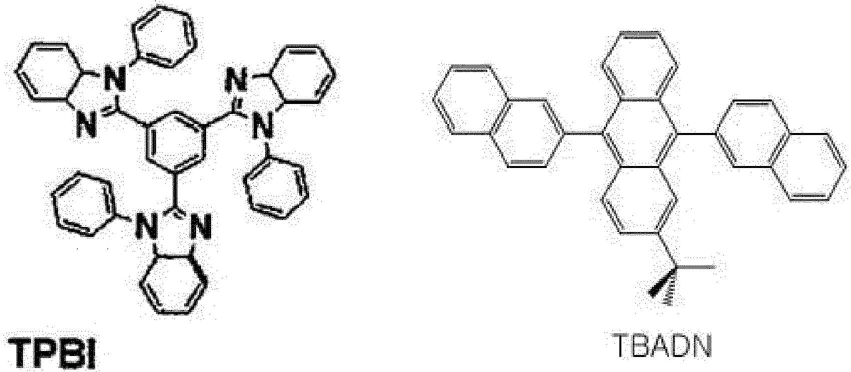

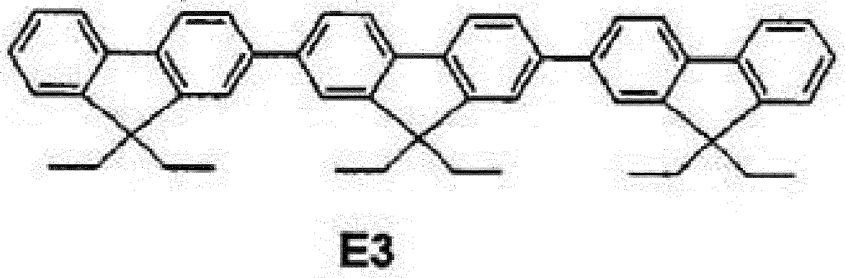

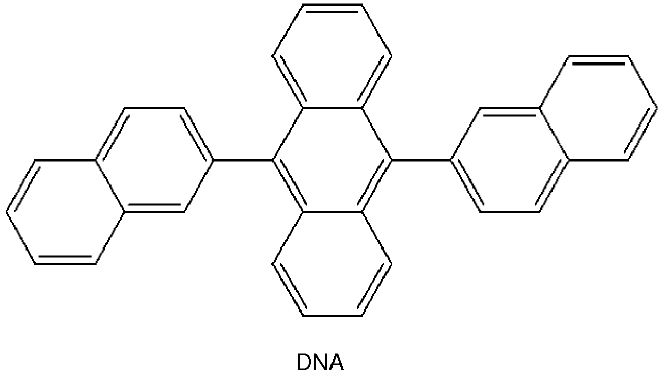

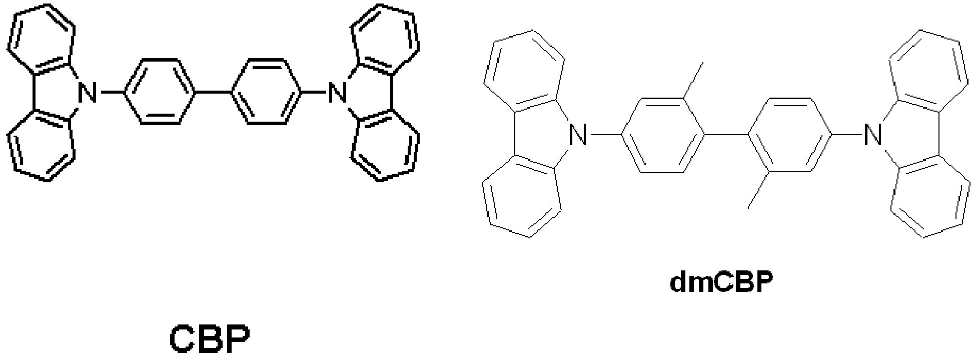

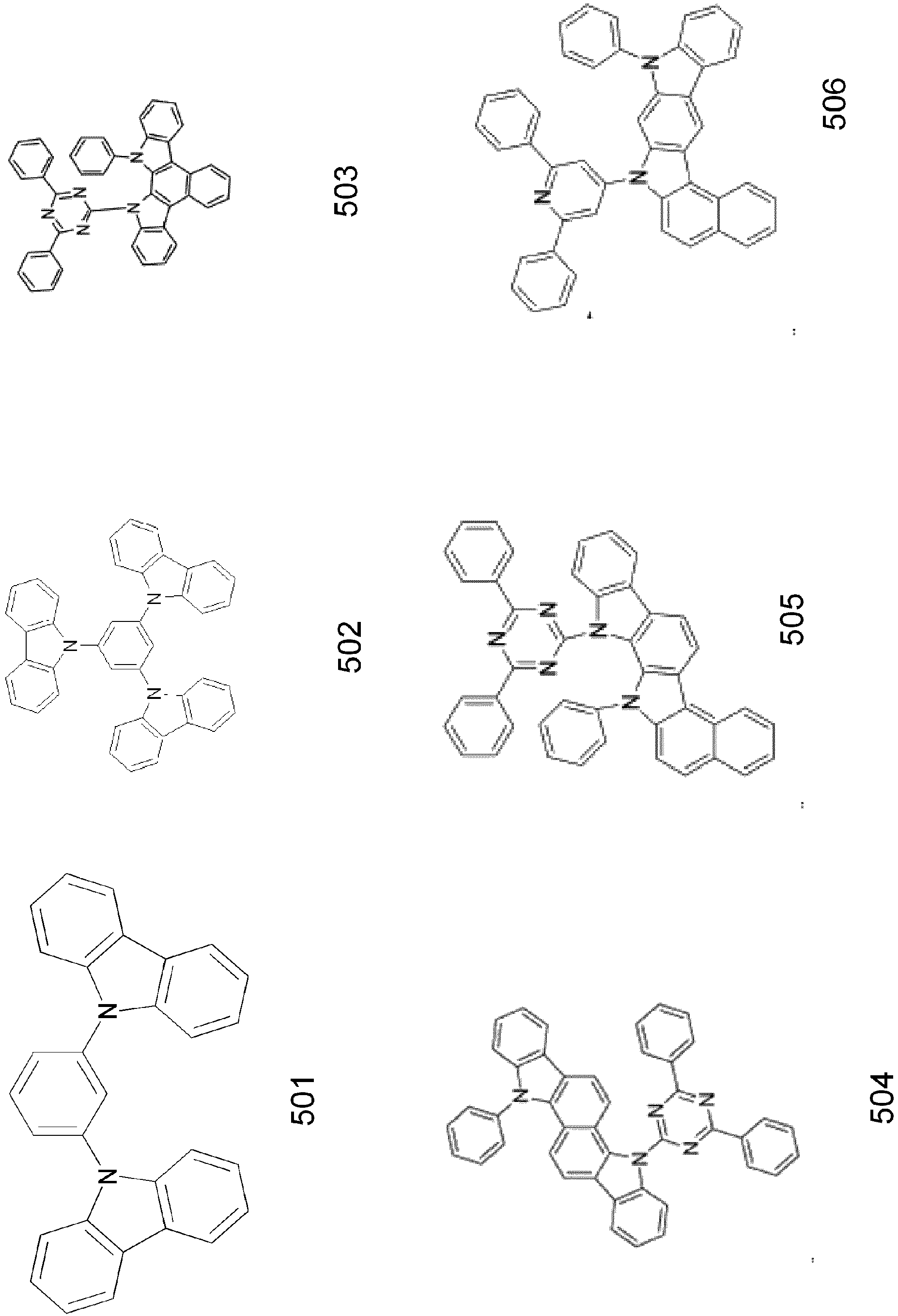

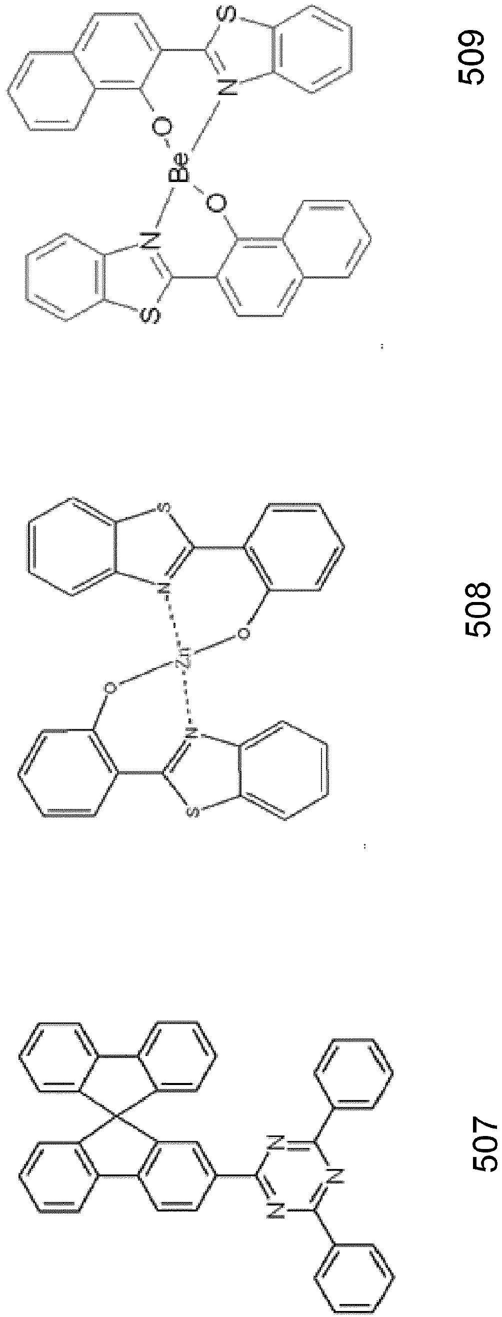

In some embodiments, the host may include an anthracene-based compound represented by Formula 400 below.

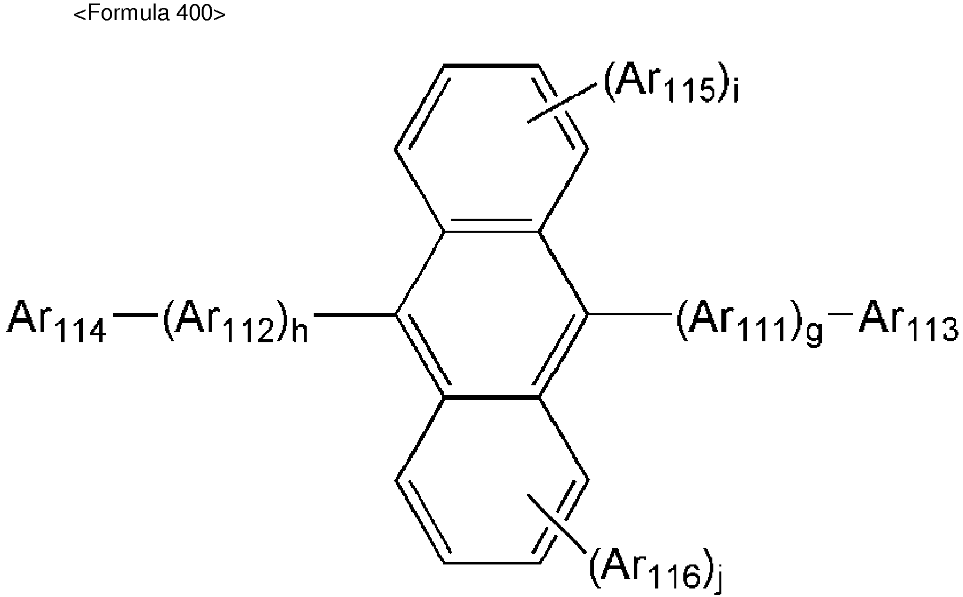

In some embodiments, Ar₁₁₁ and Ar₁₁₂ in Formula 400 may be each independently selected from a phenylene group, a naphthylene group, a phenanthrenylene group, or a pyrenylene group; or a phenylene group, a naphthylene group, a phenanthrenylene group, a fluorenyl group, or a pyrenylene group, each substituted with at least one of a phenyl group, a naphthyl group, and an anthracenyl group.

In Formula 400, Ar₁₁₃ to Ar₁₁₆ may be each independently selected from a C₁-C₁₀alkyl group; a phenyl group, a naphthyl group, a phenanthrenyl group, and a pyrenyl group; and a phenyl group, a naphthyl group, a phenanthrenyl group, a fluorenyl group, and a pyrenyl group, each substituted with at least one of a phenyl group, a naphthyl group, and an anthracenyl group.

In Formula 400, g, h, i, and j may be each independently an integer from 0 to 5, for example, may be 0, 1, or 2.

In Formula 400, Ar₁₁₃ to Ar₁₁₆ may be each independently selected from
a C₁-C₁₀alkyl group substituted with at least one of a phenyl group, a naphthyl group, and an anthracenyl group;
a phenyl group, a naphthyl group, an anthracenyl group, a pyrenyl group, a phenanthrenyl group, and a fluorenyl group;
a phenyl group, a naphthyl group, an anthracenyl group, a pyrenyl group, a phenanthrenyl group, and a fluorenyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a phenyl group, a naphthyl group, an anthracenyl group, a pyrenyl group, a phenanthrenyl group, and a fluorenyl group; and

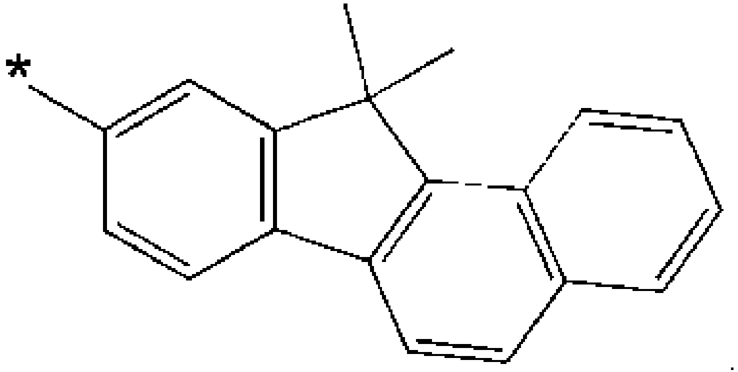

For example, the anthracene-based compound of Formula 400 above may be one of the compounds represented by the following formulae:

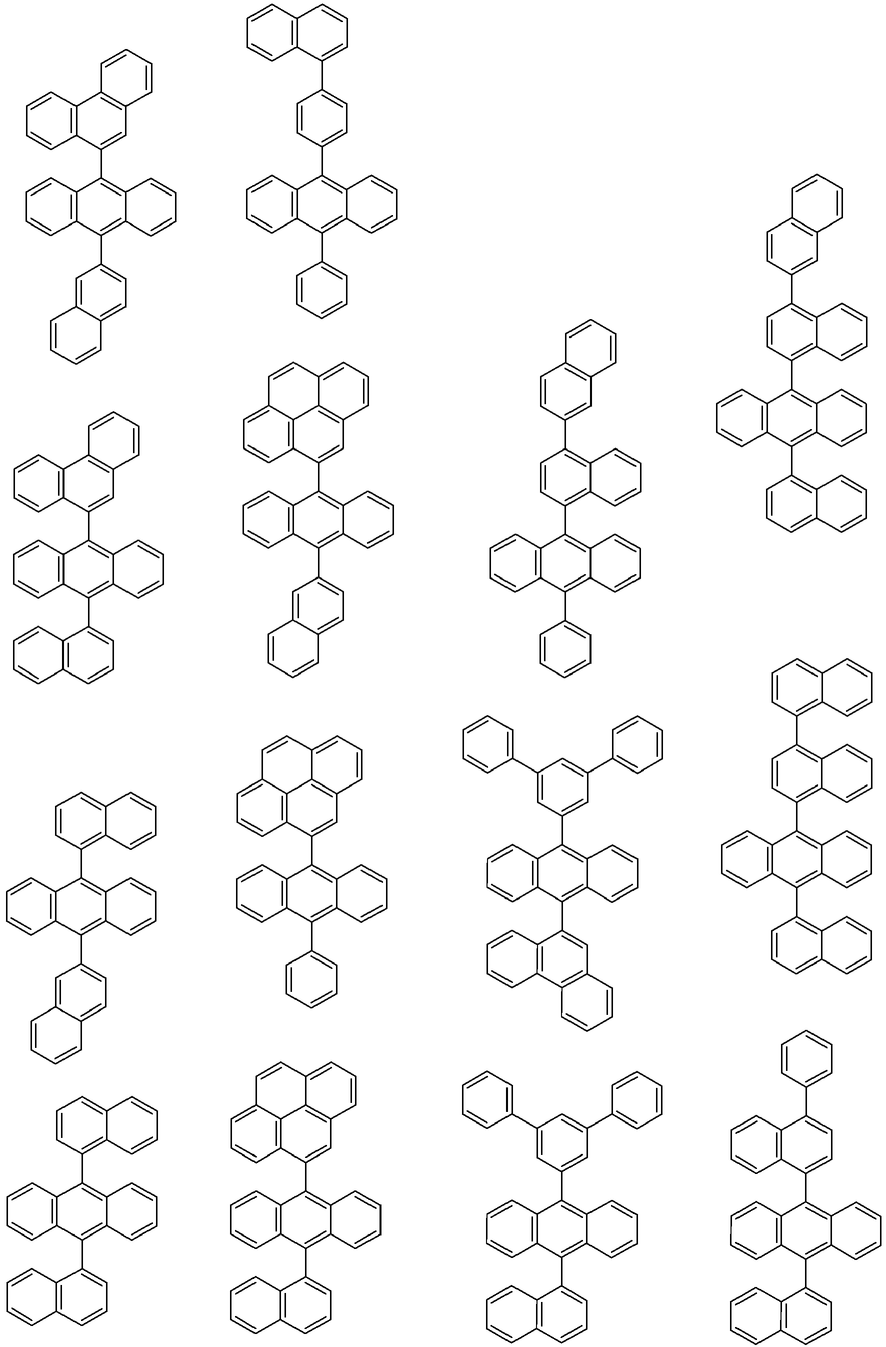

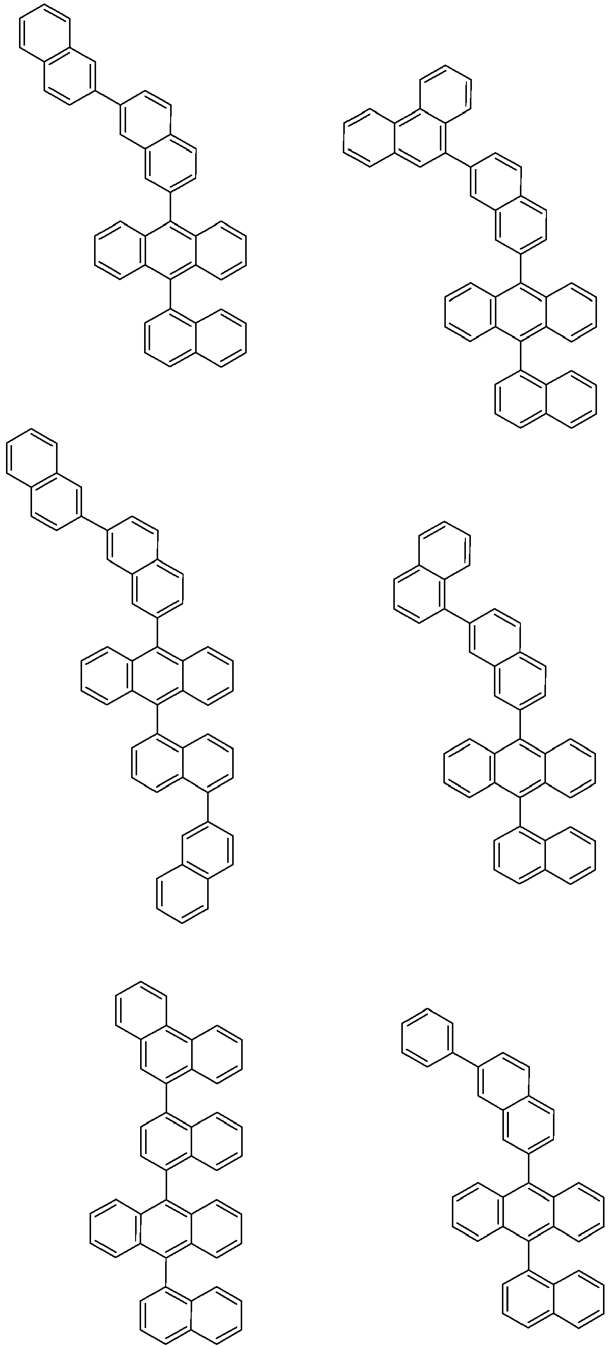

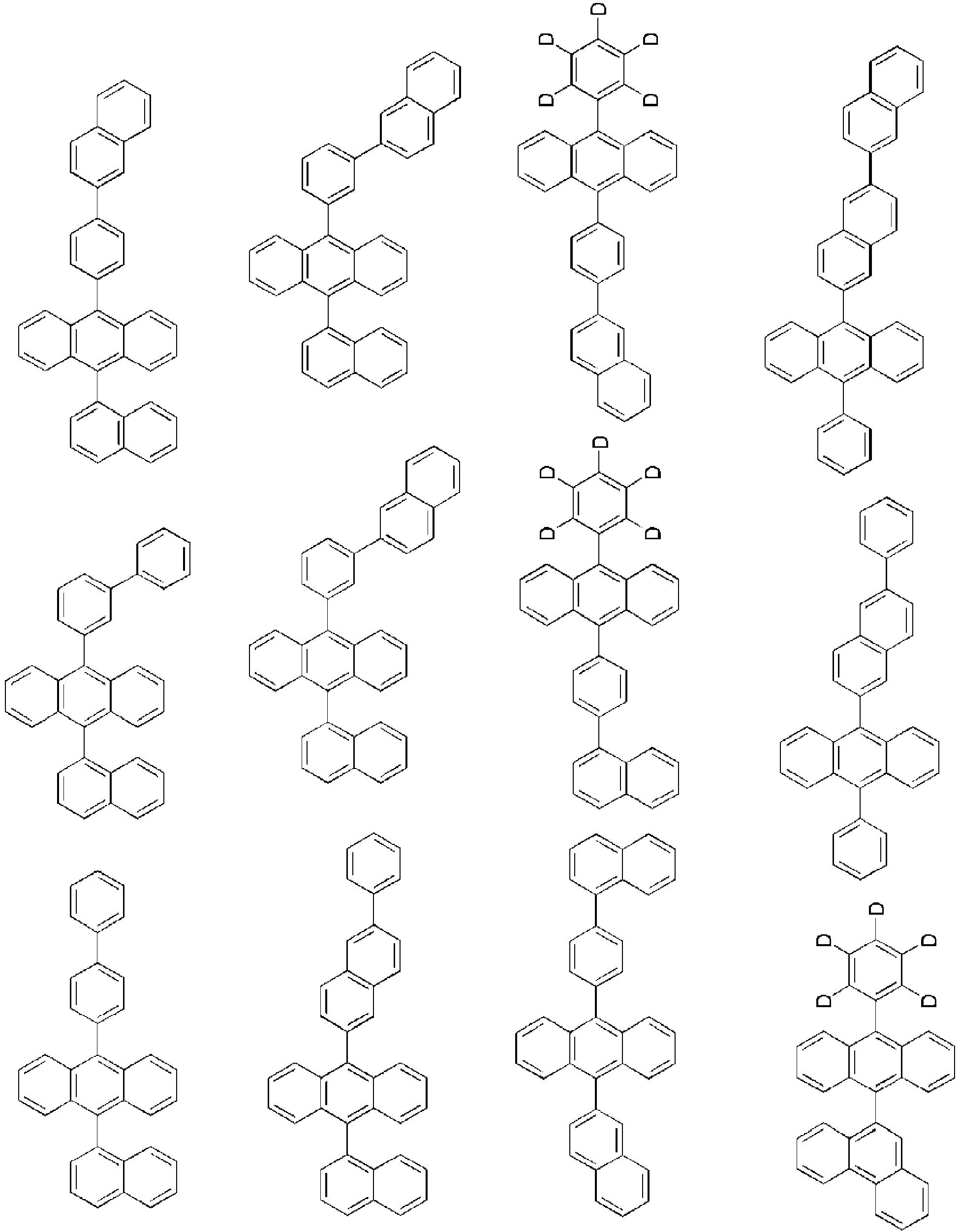

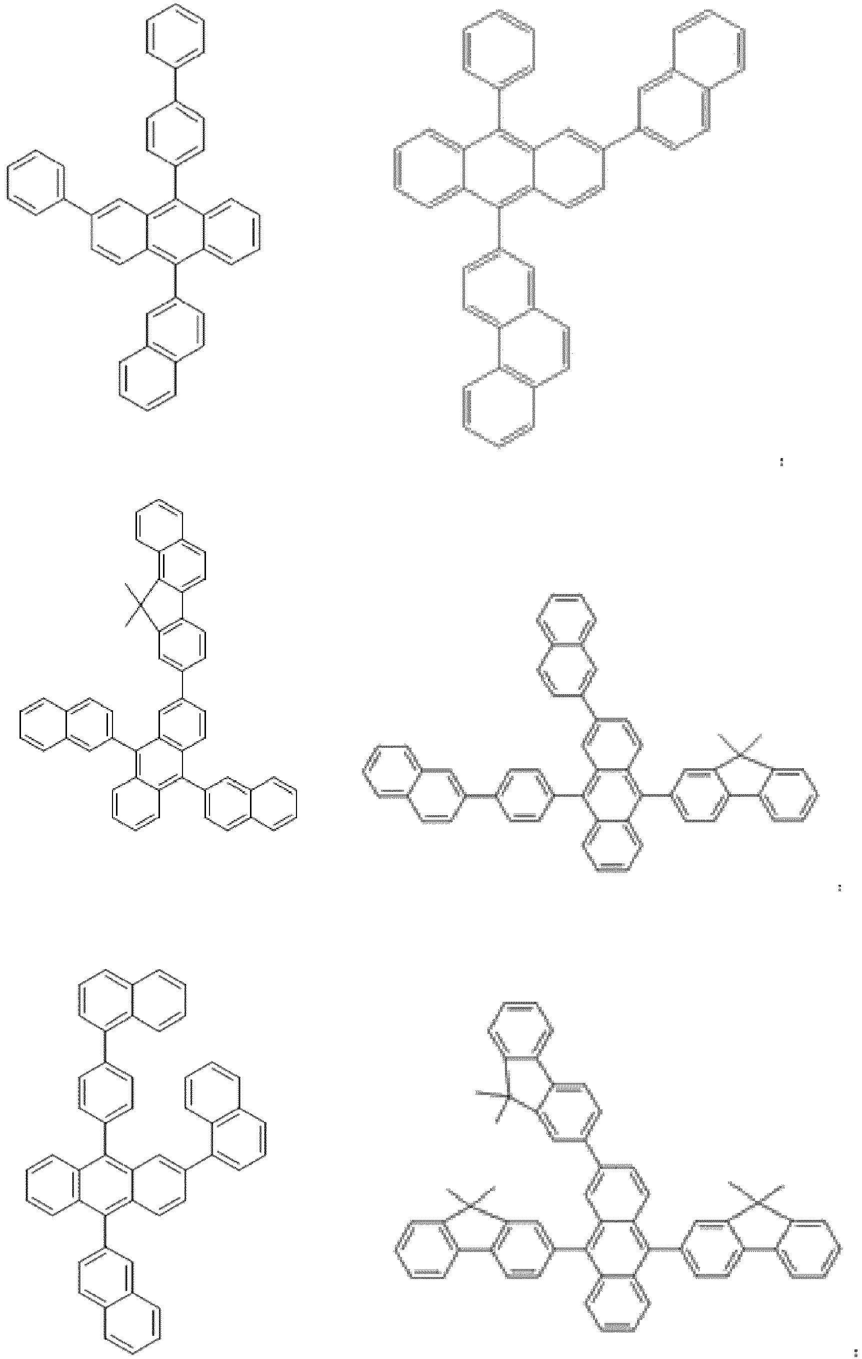

In some embodiments, an anthracene-based compound represented by Formula 401 below may be used as the host.

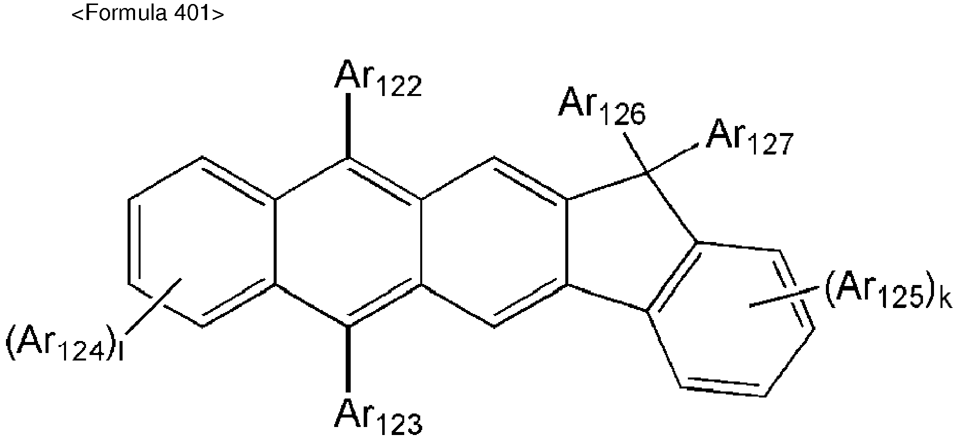

Ar₁₂₂ to Ar₁₂₅ in Formula 401 above may be defined as described above in conjunction with Ar₁₁₃ of Formula 400, and thus detailed descriptions thereof will not be provided here.

Ar₁₂₆ and Ar₁₂₇ in Formula 401 above may be each independently a C₁-C₁₀ alkyl group, for example, a methyl group, an ethyl group, or a propyl group.

In Formula 401, k and I may be each independently an integer from 0 to 4, for example, 0, 1, or 2.

For example, the anthracene compound of Formula 401 above may be one of the compounds represented by the following formulae:

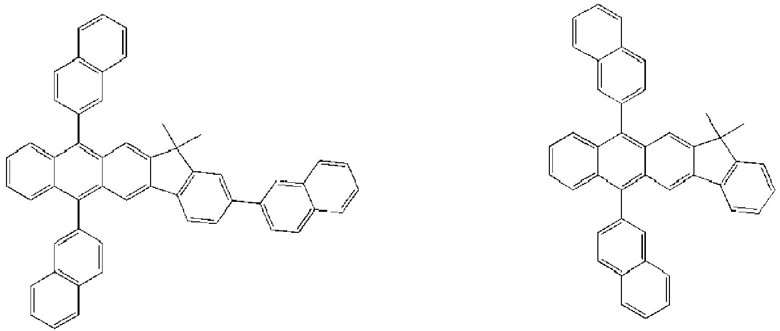

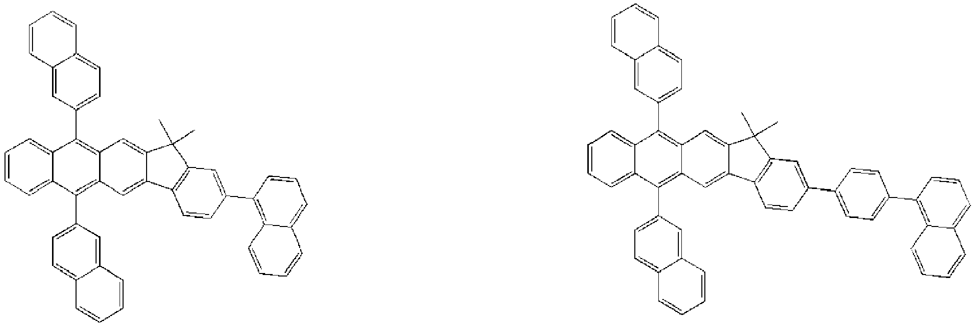

When the organic light-emitting device is a full color organic light-emitting device, the emission layer may be patterned into a red emission layer, a green emission layer, and a blue emission layer. In some embodiments, the EML may have a stack structure including a red emission layer, a green emission layer, and/or a blue emission layer that are stacked upon one another to emit white light.

The EML of the light-emitting device may include a dopant, which may be a fluorescent dopant emitting light based on fluorescence mechanism, or a phosphorescent dopant emitting light based on phosphorescence mechanism. When the EML includes a fluorescent dopant, the fluorescent dopant may include the amine-based compound of Formula 1 above.

For full color emission, the organic light-emitting device may further include a known dopant, in addition to the amine-based compound of Formula 1 above. For example, the blue emission layer of the organic light-emitting device may include the amine-based compound of Formula 1 as a dopant, and the green and red emission layers may include a known phosphorescent dopant, which will be described below.

The known phosphorescent dopant may include an organometallic complex including a transition metal, for example, iridium (Ir), platinum (Pt), osmium (Os), or rhodium (Rh).

Examples of the red dopant are compounds represented by the following formulae.

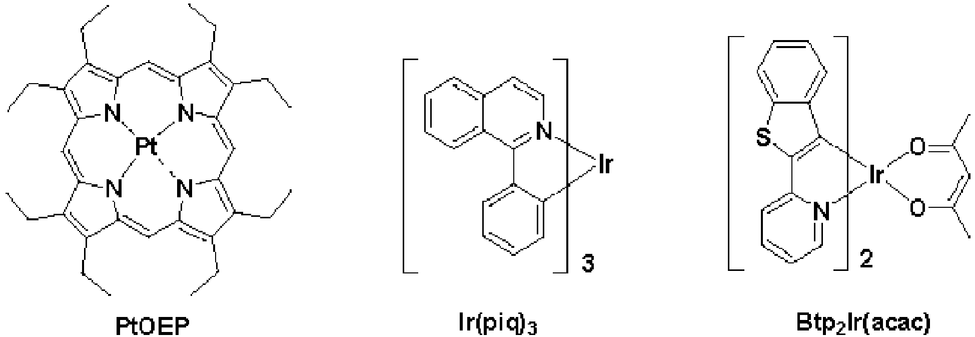

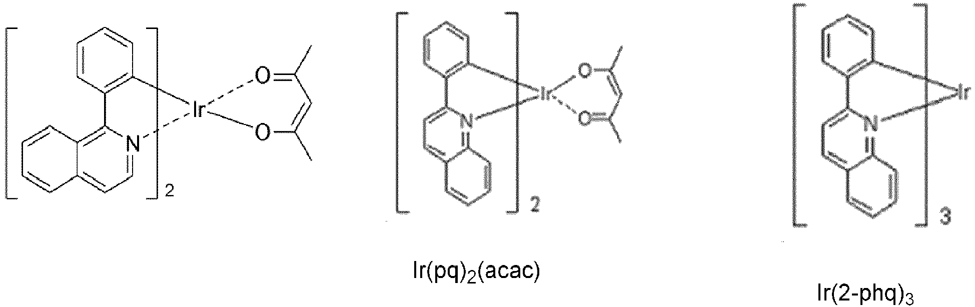

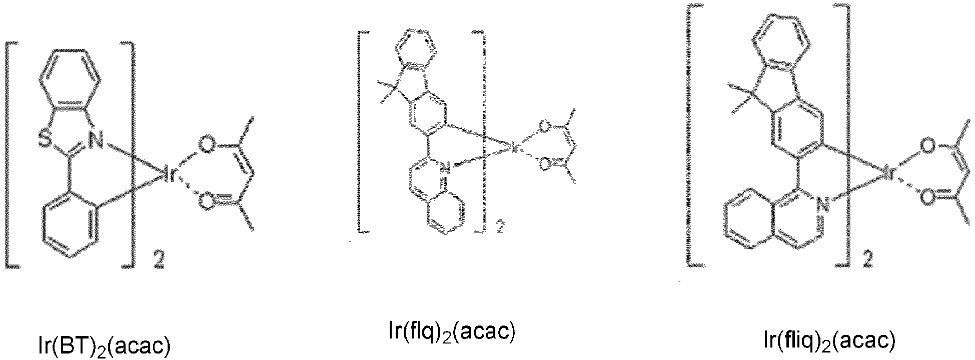

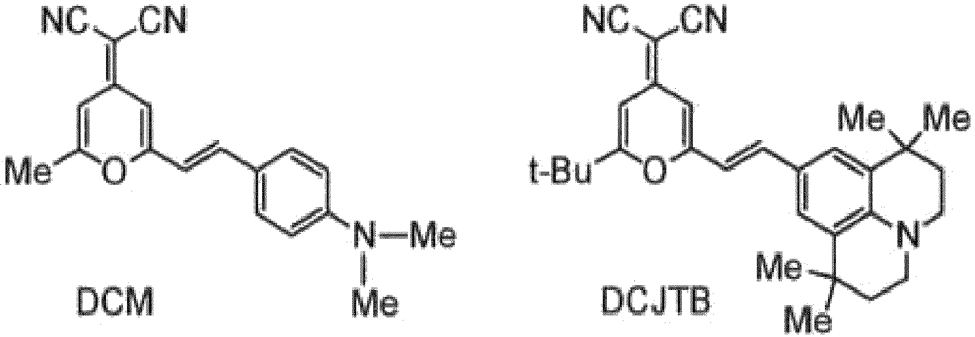

Examples of the green dopant are compounds represented by the following formulae.

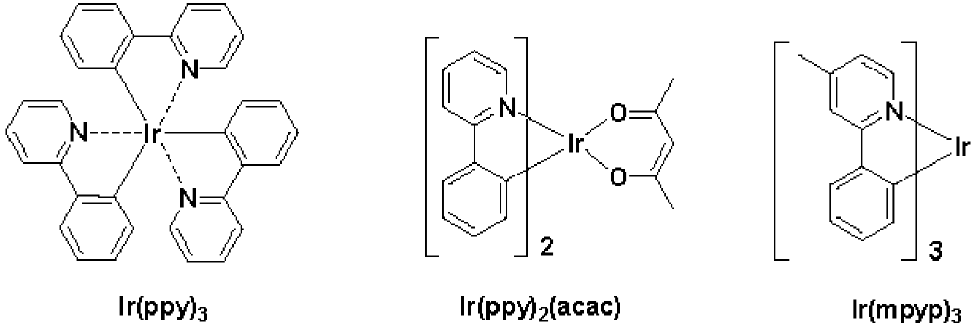

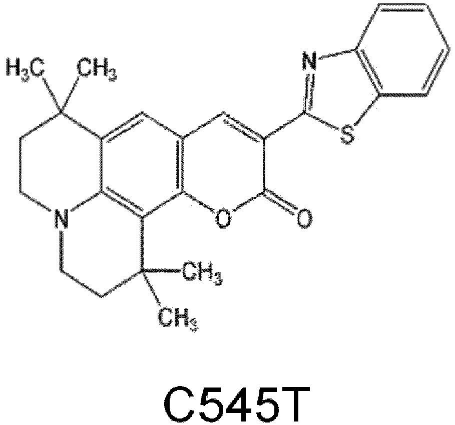

Examples of known dopants that may be used in the EML are organometallic complexes represented by the following formulae.

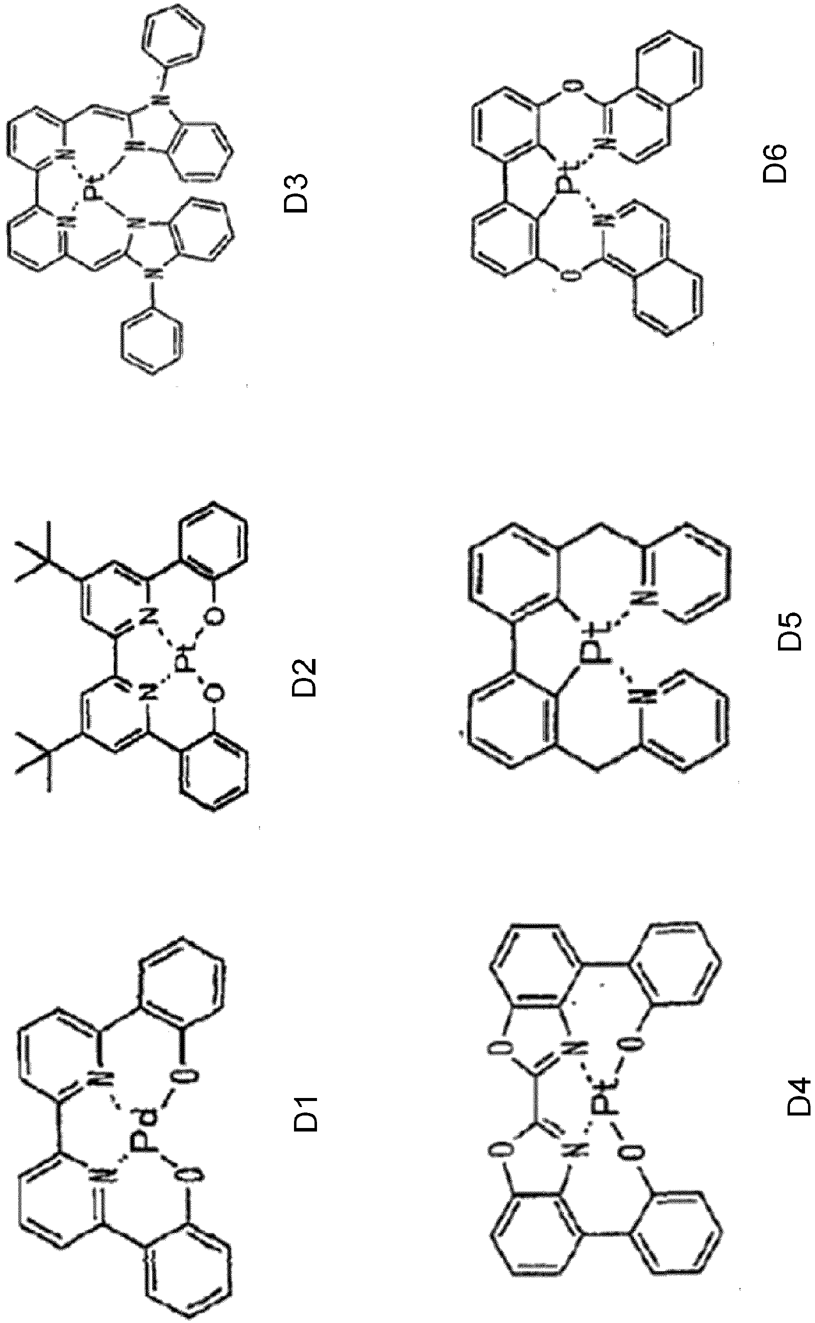

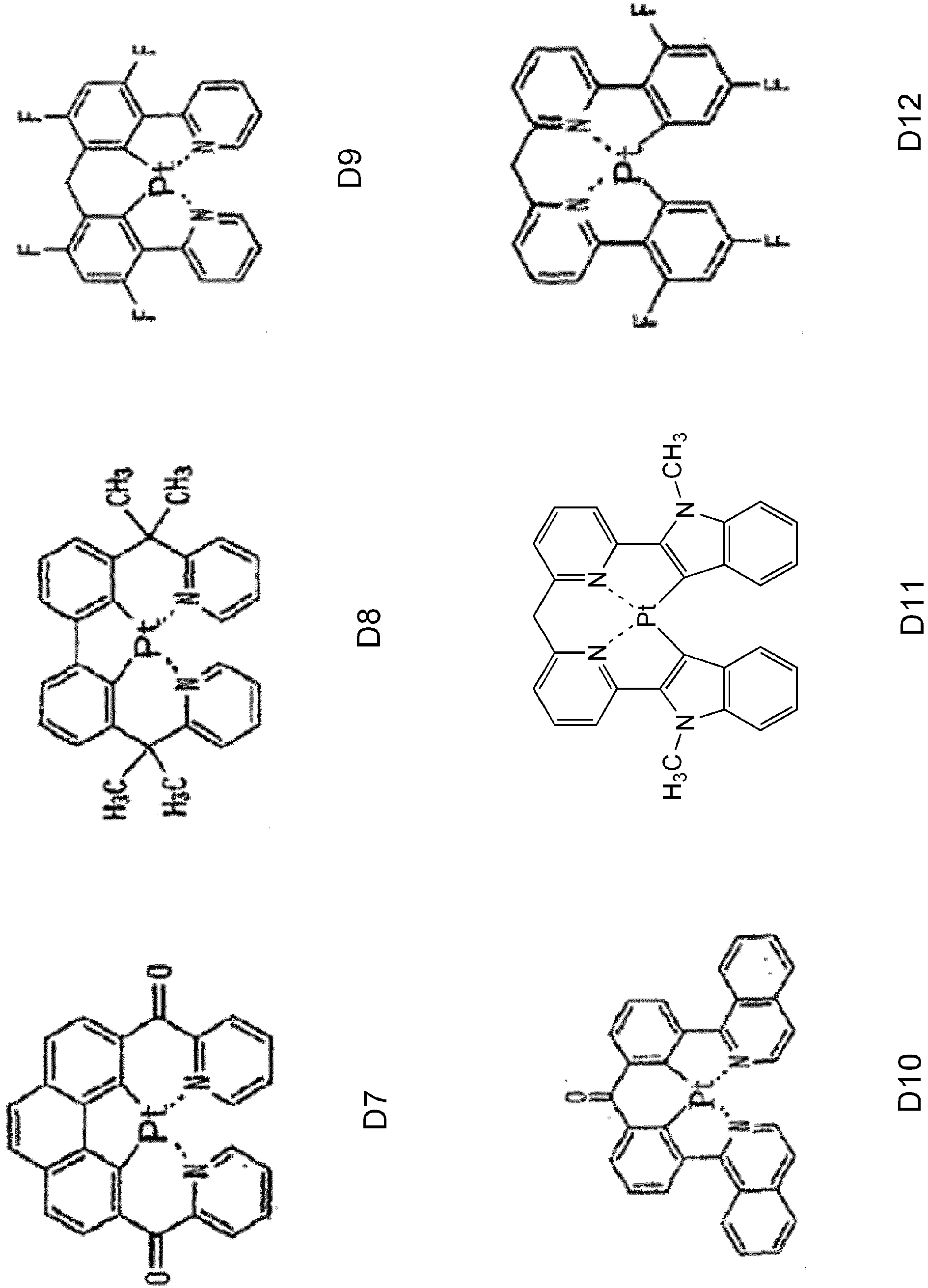

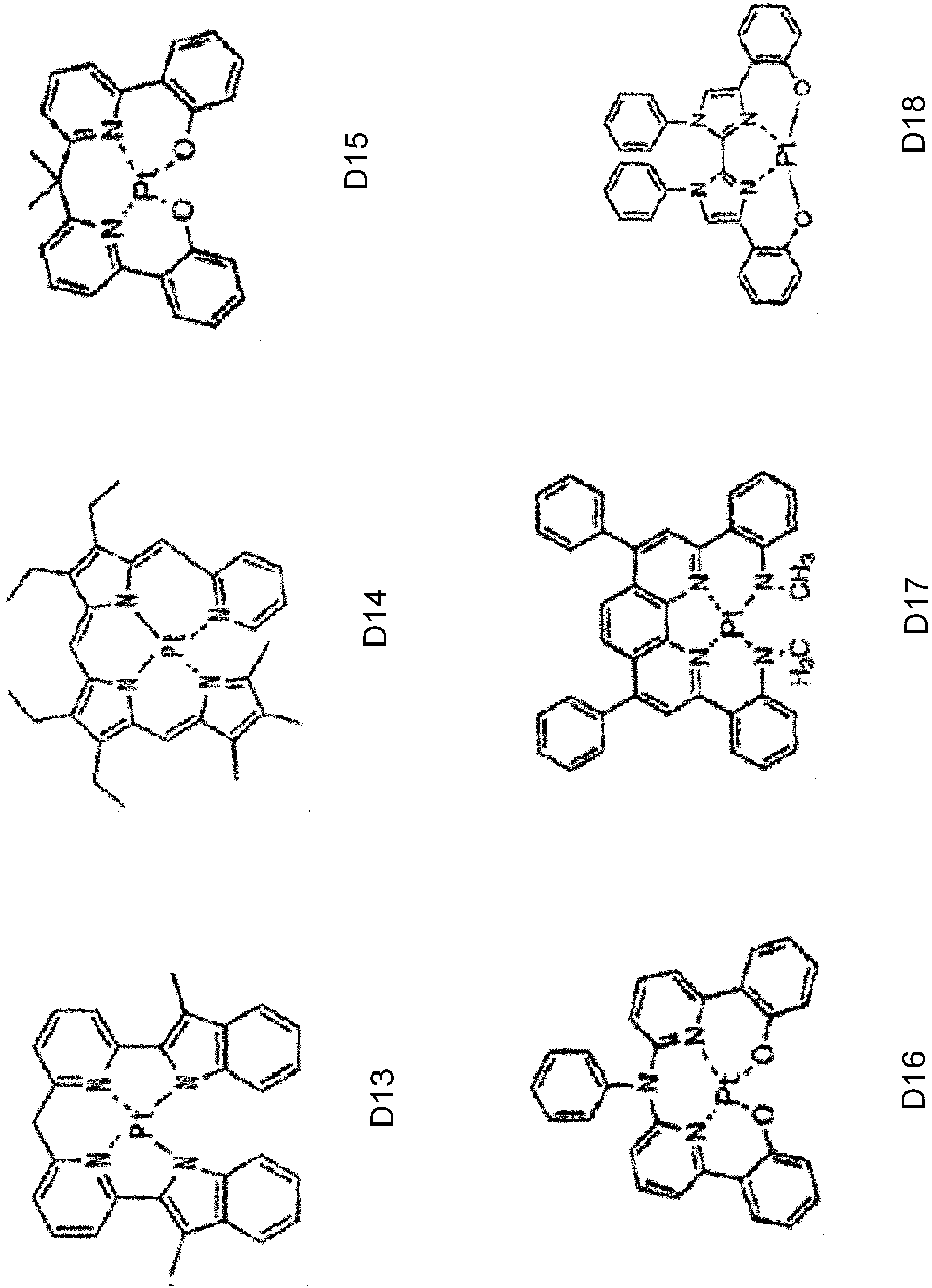

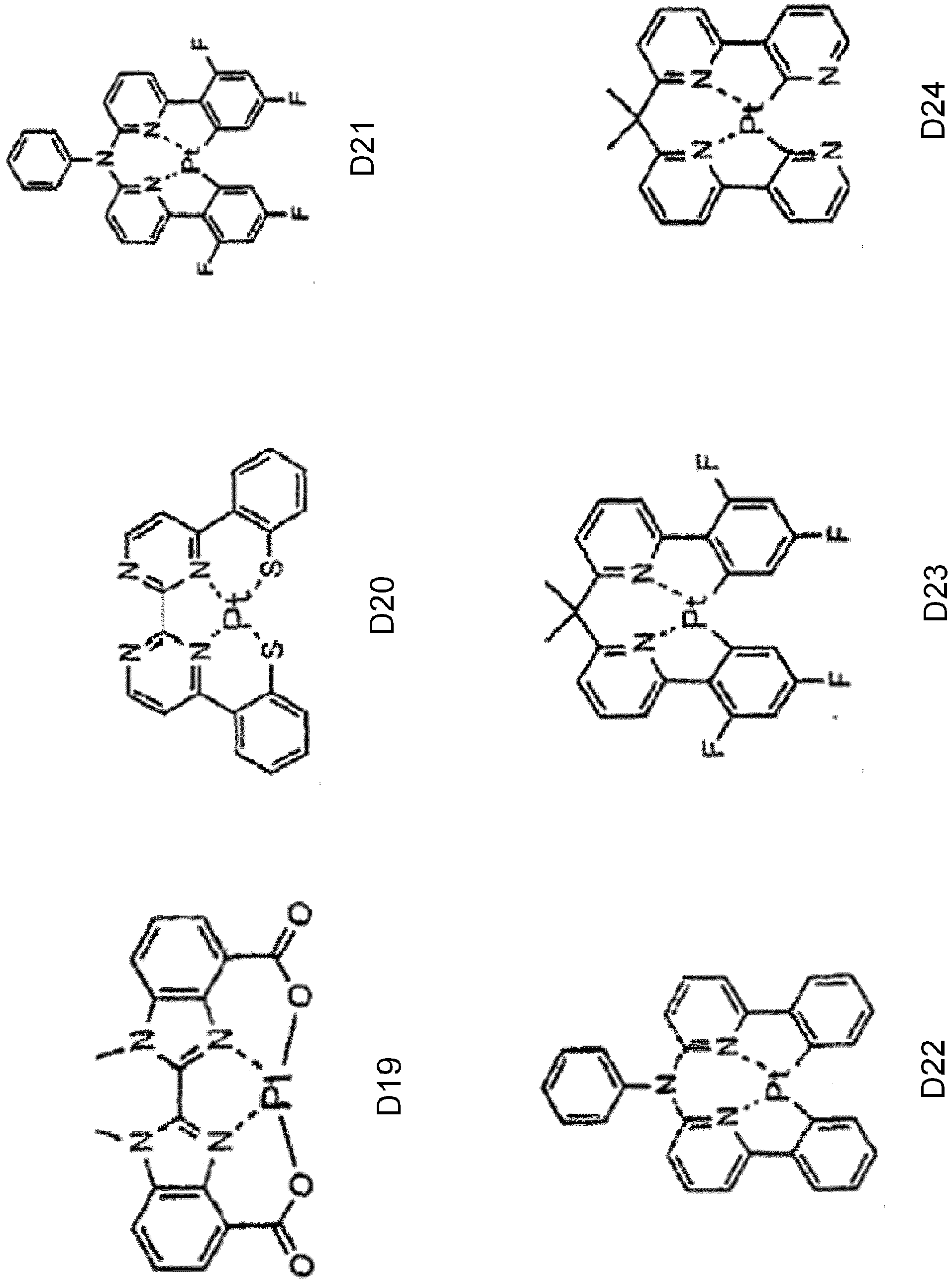

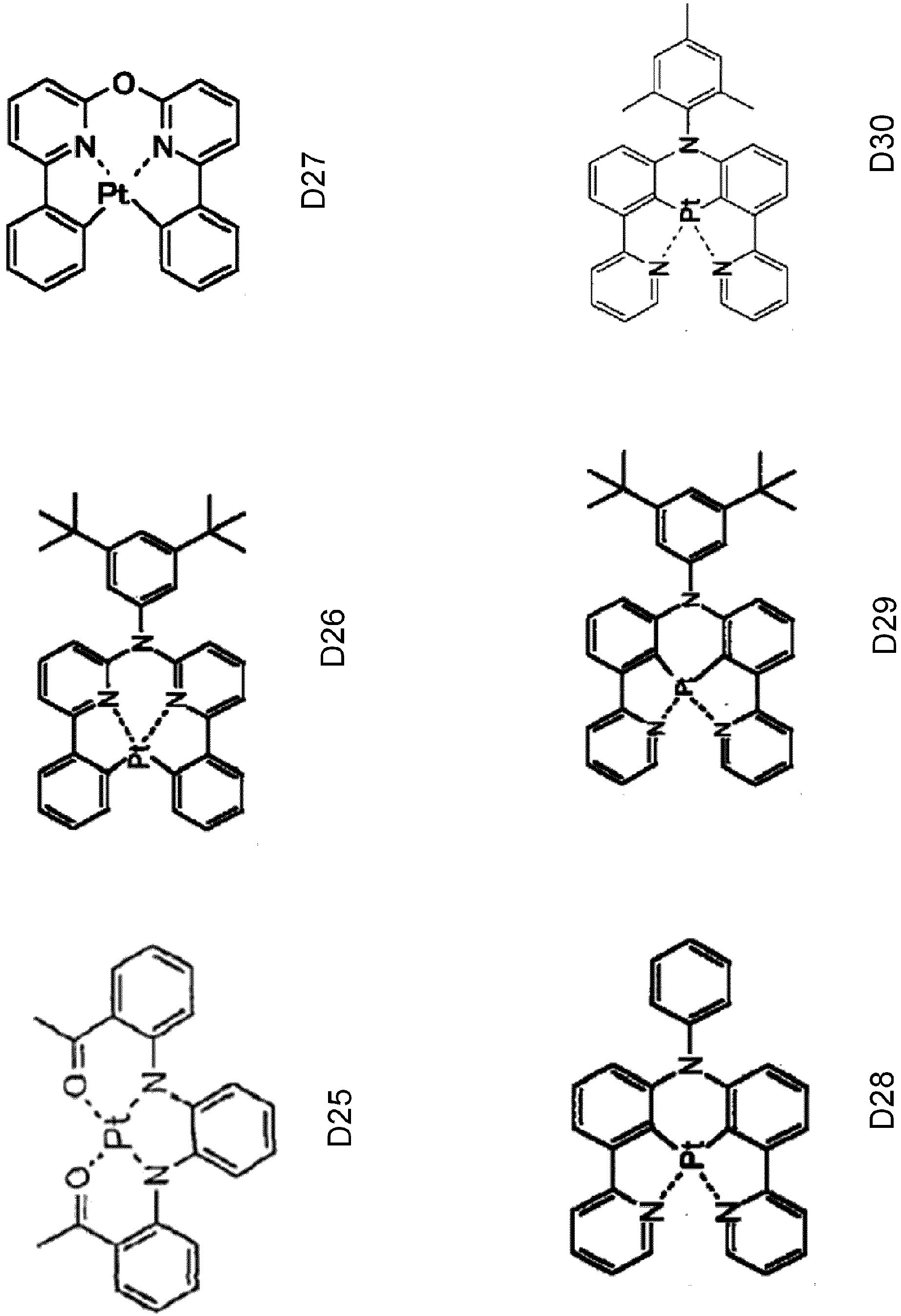

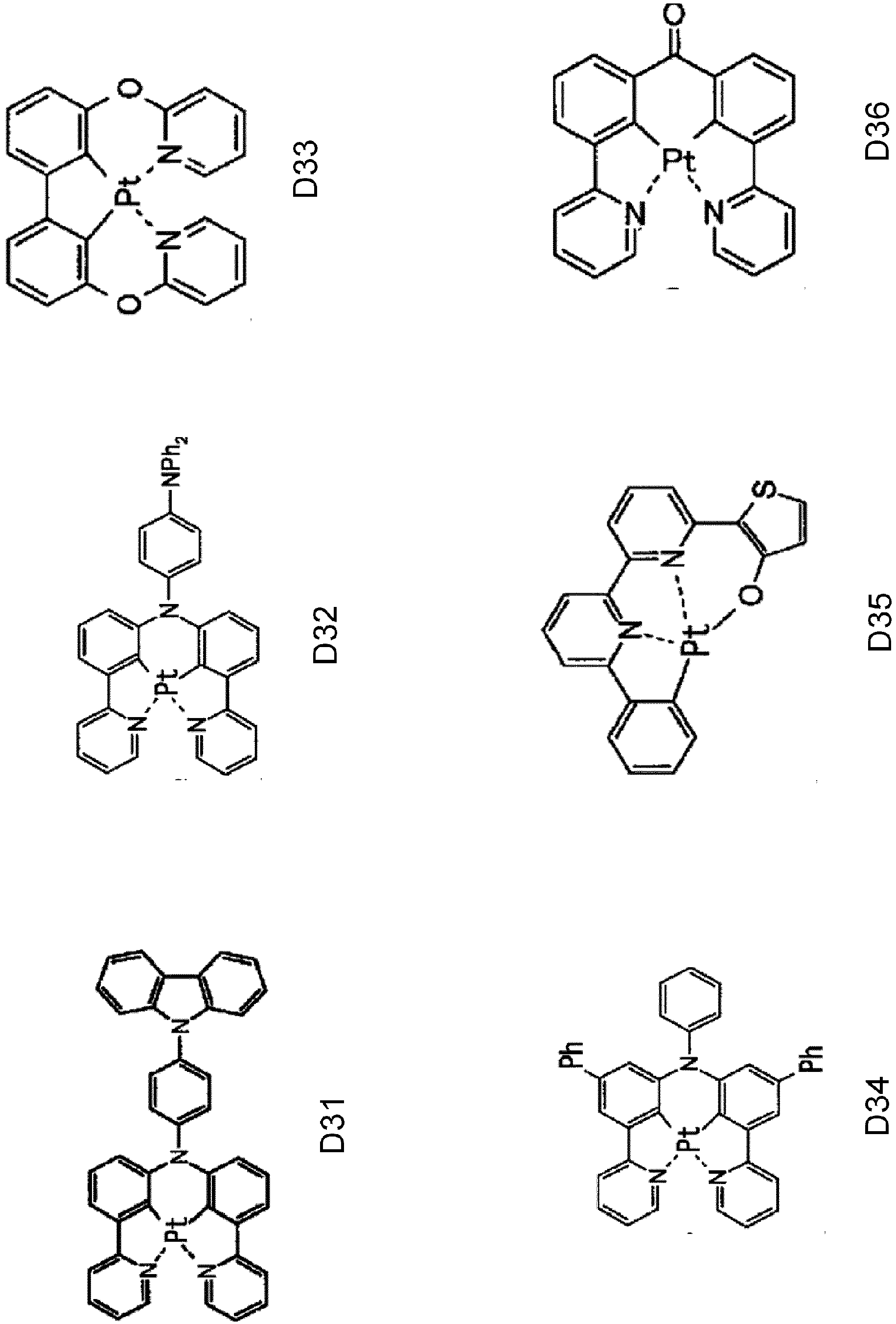

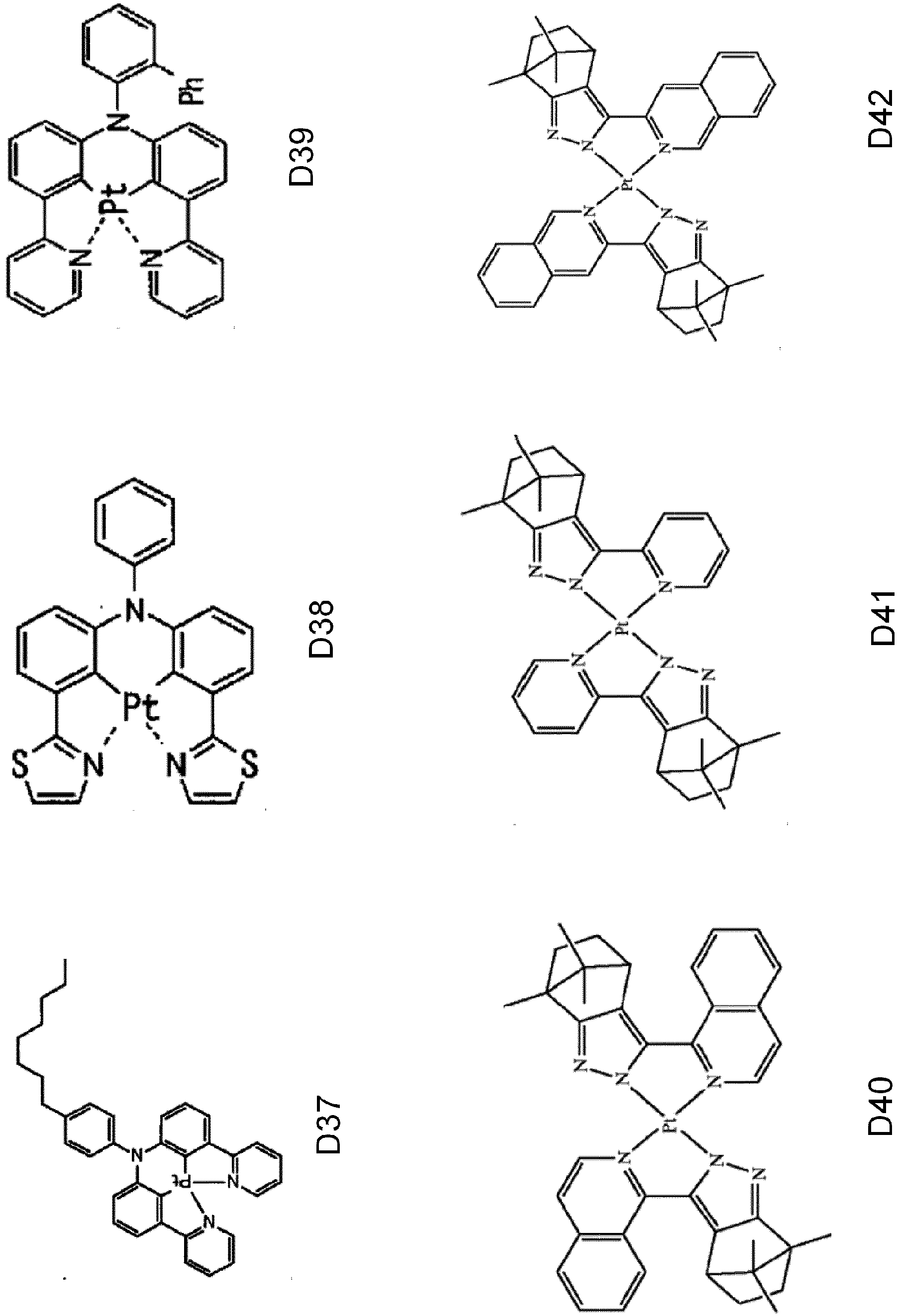

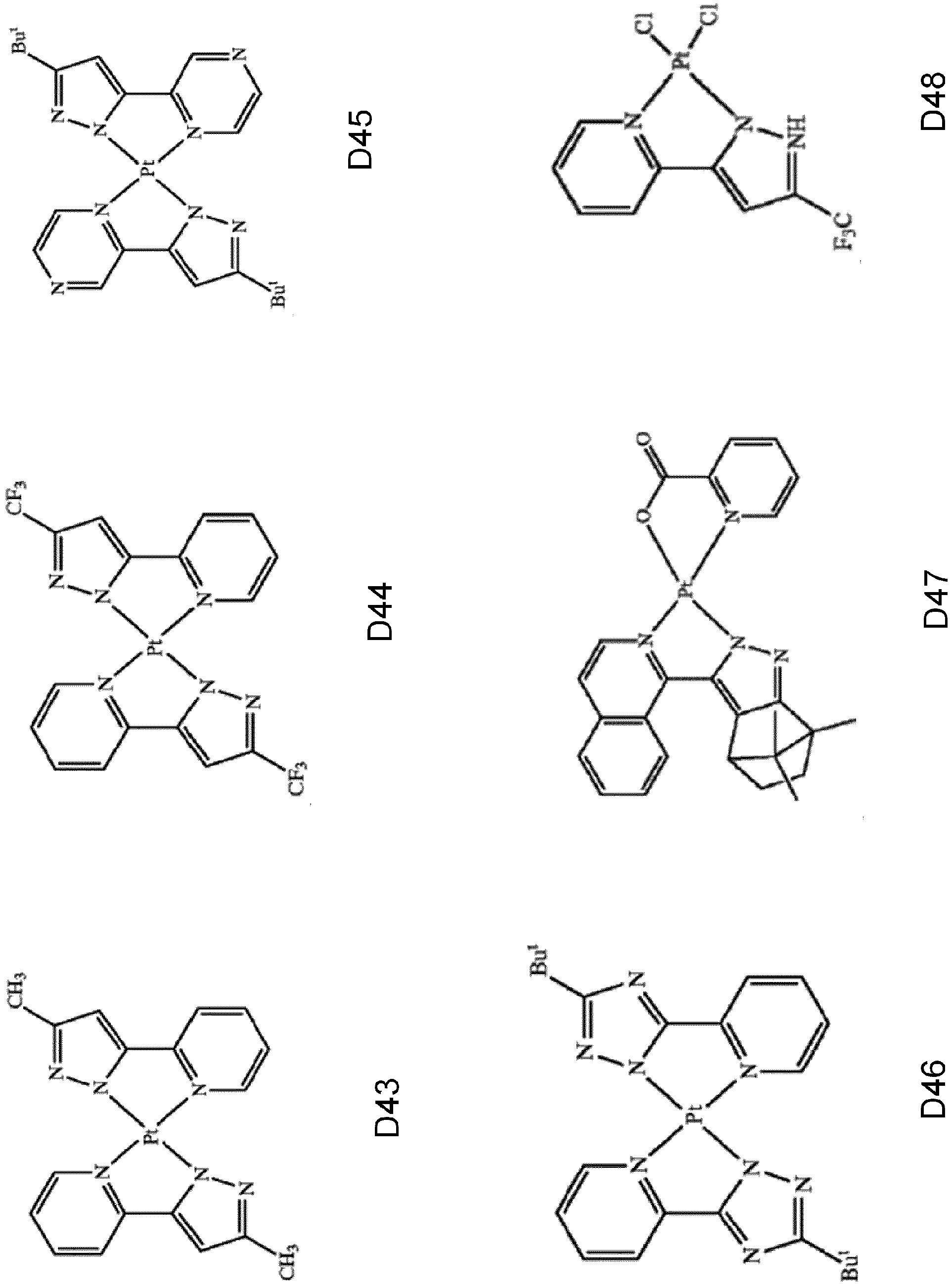

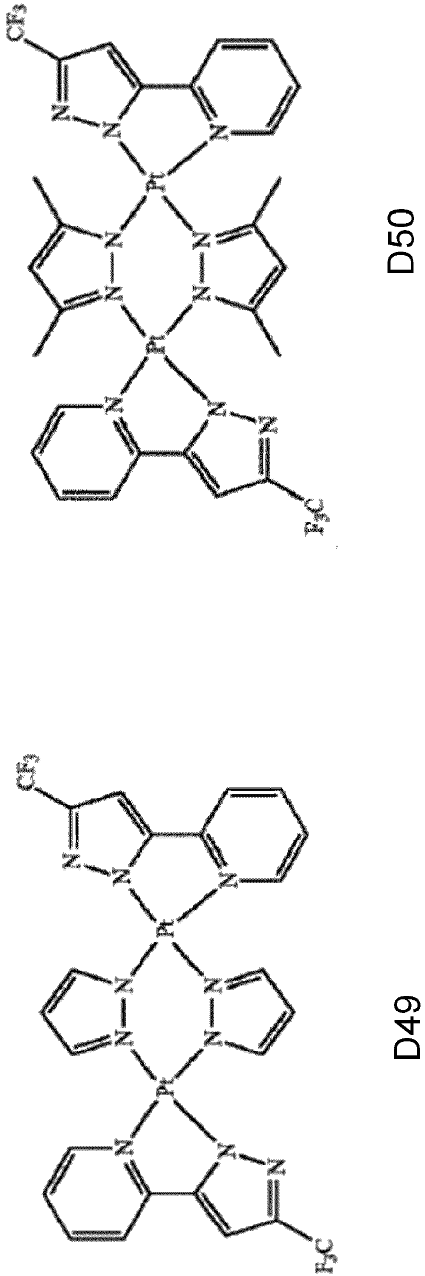

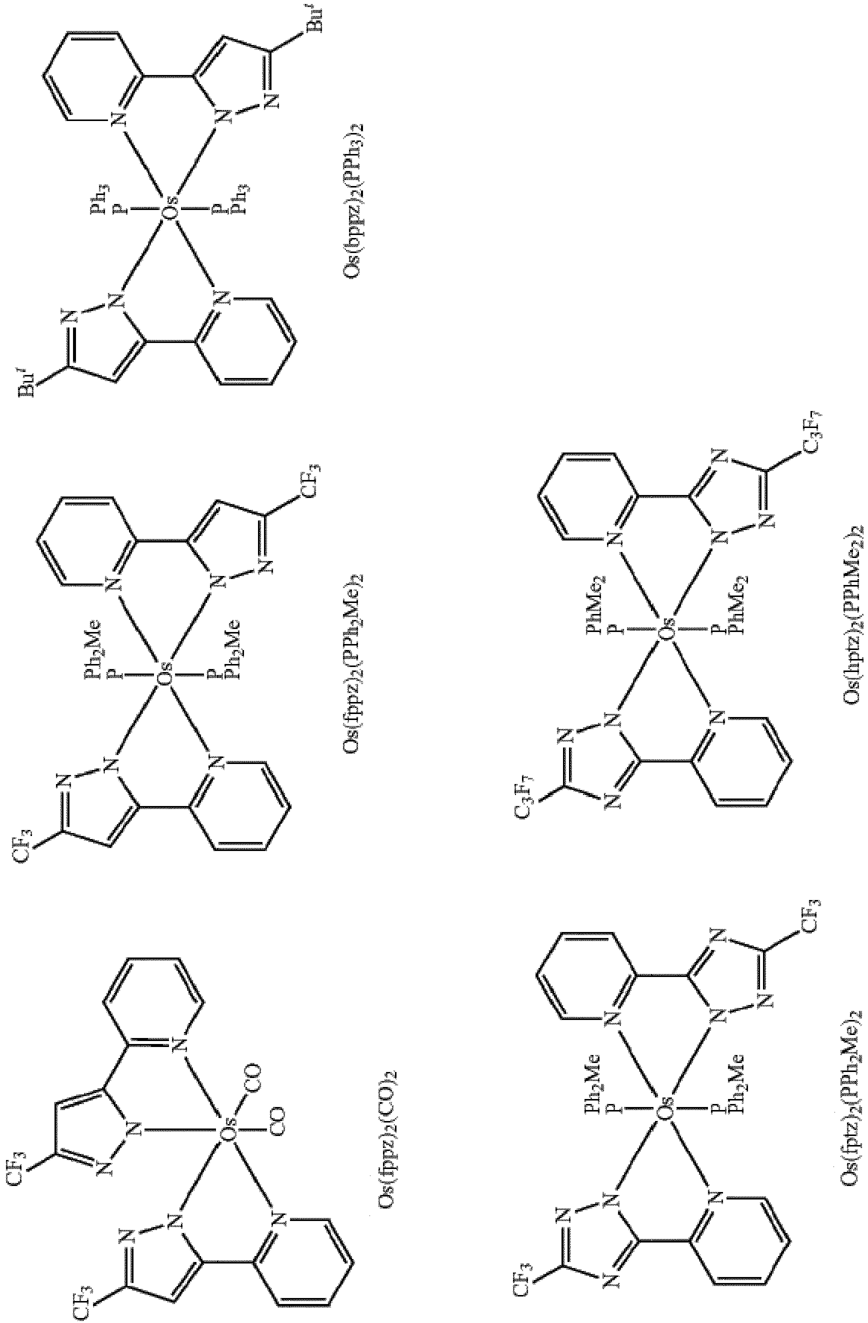

When the EML includes both a host and a dopant, the amount of the dopant may be from about 0.01 to about 15 parts by weight based on 100 parts by weight of the host.

The thickness of the EML may be about 100 Å to about 1000 Å, and in some embodiments, may be from about 200 Å to about 600 Å. When the thickness of the EML is within these ranges, the EML may have good light emitting ability without a substantial increase in driving voltage.

Then, an ETL may be formed on the EML by vacuum deposition, spin coating, casting, or the like. When the ETL is formed using vacuum deposition or spin coating, the deposition and coating conditions may be similar to those for the formation of the HIL, though the deposition and coating conditions may vary according to a compound that is used to form the ETL.

A material for the ETL may be any known material that can stably transport electrons injected from an electron injecting electrode (cathode). Examples of materials for the ETL are a quinoline derivative, such as tris(8-quinolinorate)aluminum (Alq₃), TAZ (refer to a formula below), BAlq (refer to a formula below), beryllium bis(benzoquinolin-10-olate (Bebq₂), 9,10-di(naphthalene-2-yl)anthracene (DNA, refer to a formula below), Compound 201, and Compound 202.

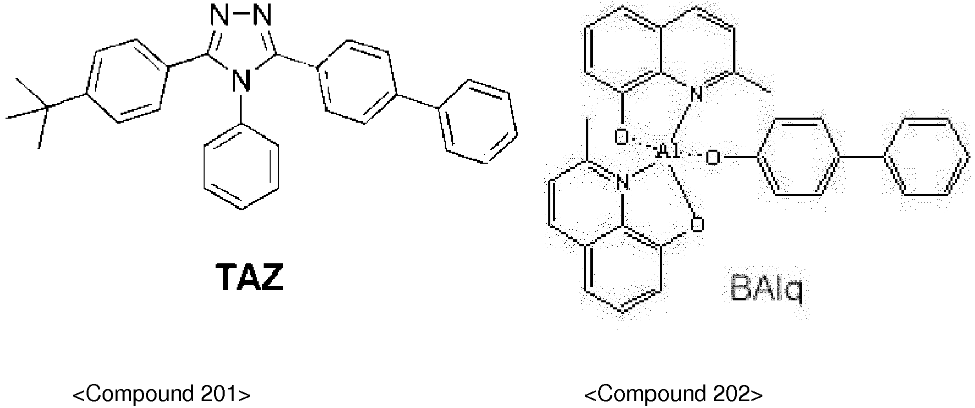

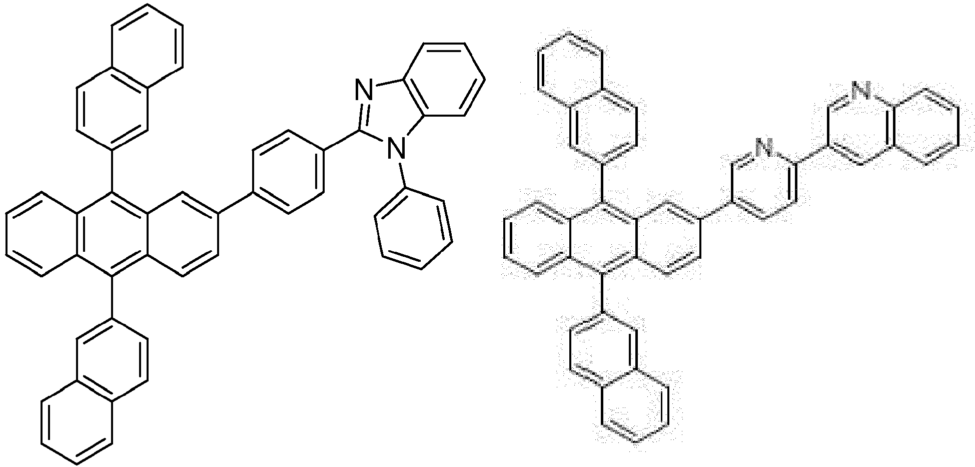

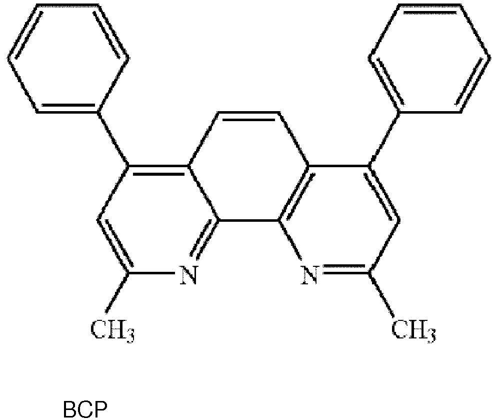

The thickness of the ETL may be from about 100Å to about 1,000 Å, and in some embodiments, may be from about 150 Å to about 500 Å. When the thickness of the ETL is within these ranges, the ETL may have satisfactory electron transporting ability without a substantial increase in driving voltage.

In some embodiments the ETL may further include a metal-containing material, in addition to the above-listed known electron-transporting organic compounds.

The metal-containing material may include a lithium (Li) complex. Examples of the Li complex are lithium quinolate (LiQ) and Compound 203 below:

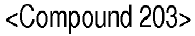

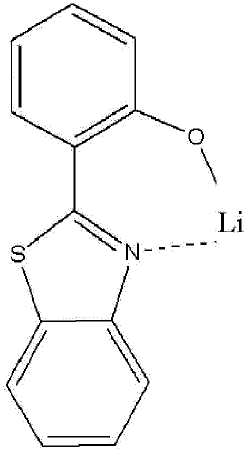

In addition, the EIL, which facilitates injection of electrons from the second electrode (cathode) 17, may be formed on the ETL.

Examples of materials for forming the EIL are LiF, NaCl, CsF, Li₂O, and BaO, which are known in the art. The deposition and coating conditions for forming the EIL may be similar to those for the formation of the HIL, though the deposition and coating conditions may vary depending on the material that is used to form the EIL.

The thickness of the EIL may be from about 1Å to about 100 Å, and in some embodiments, may be from about 3Å to about 90Å. When the thickness of the EIL is within these ranges, the EIL may have satisfactory electron injection ability without a substantial increase in driving voltage.

The second electrode 17 is disposed on the organic layer 15. The second electrode 17 may be a cathode. A material for the second electrode 17 may be a metal, an alloy, or an electrically conductive compound, which have a low work function, or a combination thereof. Examples of the material for the second electrode 17 are lithium (Li), magnesium (Mg), aluminum (Al), aluminum-lithium (Al-Li), calcium (Ca), magnesium-indium (Mg-In), and magnesium-silver (Mg-Ag), or the like. In some embodiments, to manufacture a top-emission light-emitting device, the second electrode 177 may be formed as a transmissive electrode from, for example, indium tin oxide (ITO) or indium zinc oxide (IZO).

Although the organic light-emitting device of FIG. 1 is described above, embodiments are not limited thereto.

For example, in the organic light-emitting device of FIG. 1, the first electrode 13 may be a cathode, and the second electrode 17 may be an anode. The electron transport region may be disposed between the first electrode 13 and the EML and the hole transport region may be disposed between the EML and the second electrode 17.

A hole blocking layer (HBL) may be formed between the EML and the ETL by using vacuum deposition, spin coating, casting, Langmuir-Blodgett (LB) deposition, or the like, in order to prevent diffusion of triplet excitons or holes into the ETL. When the HBL is formed using vacuum deposition or spin coating, the conditions for deposition and coating may be similar to those for the formation of the HIL, although the conditions for deposition and coating may vary according to the material that is used to form the HBL. Any known hole-blocking material may be used. Non-limiting examples of hole-blocking materials are oxadiazole derivatives, triazole derivatives, and phenanthroline derivatives. For example, bathocuproine (BCP) represented by the following formula may be used as a material for forming the HBL.

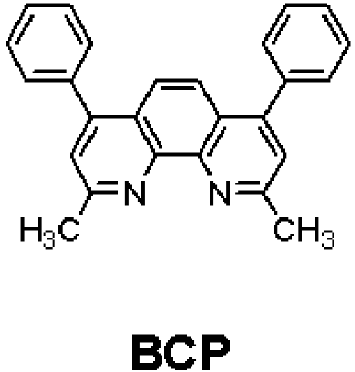

The thickness of the HBL may be from about 20Å to about 1000Å, and in some embodiments, may be from about 30Å to about 300Å. When the thickness of the HBL is within these ranges, the HBL may have improved hole blocking ability without a substantial increase in driving voltage.

As used herein, a C₁-C₆₀ alkyl group may be a linear or branched C1-C60 alkyl group, including, for example, a methyl group, an ethyl group, a propyl group, an isobutyl group, a sec-butyl group, a pentyl group, an iso-amyl group, and a hexyl group. A C₁-C₆₀ alkylene group refers to a divalent group having the same structure as the C₁-C₆₀ alkyl group.

As used herein, a C₁-C₆₀ alkoxy group refer to a moiety represented by -OA (where A is a C₁-C₆₀ alkyl group). Examples of the C₁-C₆₀ alkoxy group are a methoxy group, an ethoxy group, and an isopropyloxy group.

As used herein, a C₂-C₆₀ alkenyl group refers to a moiety with at least one carbon double bond in the middle or at a terminal of thereof. Examples of the C₂-C₆₀ alkenyl group are an ethenyl group, a propenyl group, and a butenyl group. A C₂-C₆₀ alkenylene group refers to a divalent group having the same structure as the C₁-C₆₀ alkenyl group.

As used herein, a C₂-C₆₀ alkynyl group refers to a moiety with at least one carbon-carbon triple bond in the middle or at a terminal thereof. Examples of the C₂-C₆₀ alkynyl group are an ethenyl group and a propynyl group. A C₂-C₆₀ alkynylene group refers to a divalent group having the same structure as the C₁-C₆₀ alkynyl group.

As used herein, a C₃-C₁₀ cycloalkyl group refers to a cyclic alkyl group including 3 to 10 carbon atoms. Examples of the C₃-C₁₀ cycloalkyl group are a cyclopentyl group, a cyclohexyl group, and a cycloheptyl group. A C₃-C₁₀ cycloalkylene group refers to a divalent group having the same structure as the C₃-C₁₀ cycloalkyl group.

As used herein, a C₃-C₁₀ heterocycloalkyl group refers to a C₃-C₁₀ cycloalkyl group including at least one heteroatom of N, S, O, or P. Examples of the C₃-C₁₀ heterocycloalkyl group are a tetrahydrofuranyl group and a tetrahydrothiophenyl group. A C₃-C₁₀ heterocycloalkylene group refers to a divalent group having the same structure as the C₃-C₁₀ heterocycloalkyl group.

As used herein, a C₃-C₁₀ cycloalkenyl group refers to a cyclic alkenyl group including 3 to 10 carbon atoms. Examples of the C₃-C₁₀ cycloalkenyl group are a cyclopentenyl group, a cyclohexenyl group, and a cycloheptenyl group. A C₃-C₁₀ cycloalkenylene group refers to a divalent group having the same structure as the C₃-C₁₀ cycloalkenyl group.

As used herein, a C₃-C₁₀ heterocycloalkenyl group refers to a C₃-C₁₀ cycloalkenyl group with at least one heteroatom of N, S, O, or P. Examples of the C₃-C₁₀ heterocycloalkenyl group are a 2,3-dihydrofuranyl group and a 2,3-dihydrothiophenyl group. A C₃-C₁₀ heterocycloalkenylene group refers to a divalent group having the same structure as the C₃-C₁₀ heterocycloalkenyl group.

As used herein, a C₆-C₆₀ aryl group refers to a monovalent carbocyclic aromatic system including 6 to 60 carbon atoms and at least one aromatic ring. A C₆-C₆₀ arylene group refers to a divalent group having the same structure as the C₆-C₆₀ aryl group. When the C₆-C₆₀ aryl group and the C₆-C₆₀ arylene group have at least two rings, the at least two rings may be fused together.

Examples of the "C₆-C₆₀ aryl group" and "a C₆-C₆₀ aryl group substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, - N(Q₁₁)(Q₁₂), and -Si(Q₁₃)(Q₁₄)(Q₁₅) " are a phenyl group, a C₁-C₁₀ alkylphenyl group (for example, an ethylphenyl group), a C₁-C₁₀ alkylbiphenyl group (for example, an ethylbiphenyl group), a halophenyl group (for example, o-, m- , or p-fluorophenyl group, or a dichlorophenyl group), a dicyanophenyl group, a trifluoromethoxyphenyl group, o-, m-, and p-tolyl group, o-, m- and p-cumenyl group, a mesityl group, a phenoxyphenyl group (α,α-dimethylbenzene), a phenyl group, a (N,N'-dimethyl)aminophenyl group, a N,N'-diphenyl)aminophenyl group, a pentalenyl group, an indenyl group, a naphthyl group, a halonaphthyl group (for example, a fluoronaphthyl group), a C₁-C₁₀ alkylnaphthyl group (for example, a methylnaphthyl group), a C₁-C₁₀ alkoxynaphthyl group (for example, a methoxynaphthyl group), an anthracenyl group, an azulenyl group, a heptalenyl group, an acenaphthyl group, a phenalenyl group, a fluorenyl group, a methylanthracenyl group, a phenanthrenyl group, a triphenylenyl group, a pyrenyl group, a chrysenyl group, an ethyl-chrysenyl group, a picenyl group, a perylenyl group, a chloroperylenyl group, a pentaphenyl group, a pentacenyl group, a tetraphenylenyl group, a hexaphenyl group, a hexacenyl group, a rubicenyl group, a coronenyl group, a trinaphthylenyl group, a heptaphenyl group, a heptacenyl group, a pyranthrenyl group, and an ovalenyl group.

As used herein, a C₂-C₆₀ heteroaryl group refers to a monovalent carbocyclic group having at least one aromatic ring having at least one of the heteroatoms selected from N, O, P, and S. A C₂-C₆₀ heteroarylene group refers to a divalent group having the same structure as the C₂-C₆₀ heteroaryl group. When the C₂-C₆₀ heteroaryl group and the C₂-C₆₀ heteroarylene group have at least two rings, the at least two rings may be fused together.

Examples of the C₂-C₆₀ heteroaryl group are a pyrazolyl group, an imidazolyl group, an oxazolyl group, a thiazolyl group, a triazolyl group, a tetrazolyl group, an oxadiazolyl group, a pyridinyl group, a pyridazinyl group, a pyrimidinyl group, a triazinyl group, a carbazolyl group, an indolyl group, a quinolinyl group, an isoquinolinyl group, a benzoimidazolyl group, an imidazopyridinyl group and an imidazopyrimidinyl group.

As used herein, a C₆-C₆₀ aryloxy group indicates -OA₂ (where A₂ is a C₆-C₆₀ aryl group described above), and a C₆-C₆₀ arylthio group indicates -SA₃ (where A₃ is a C₆-C₆₀ aryl group described above).

Hereinafter, embodiments will be described in detail with reference to the following synthesis examples and other examples.

### [Examples]

### Synthesis Example 1: Synthesis of Compound 1

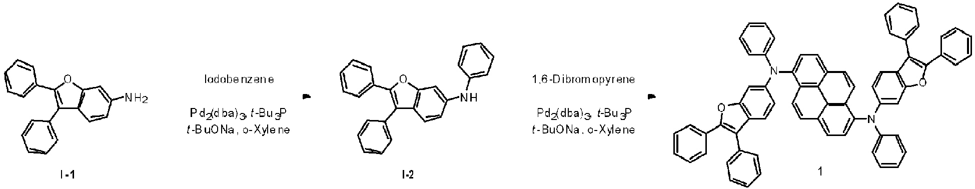

### Synthesis of Intermediate I-2

160.5 mg (0.175 mmol) of Pd₂(dba)₃ and 70.9 mg (0.350 mmol) of t-Bu₃P were dissolved in 50 mL of o-xylene, and then stirred at room temperature for about 10 minutes. 5 g (17.52 mmol) of 2,3-diphenylbenzofuran-6-amine (Intermediate I-1), 3.9 g (19.28 mmol) of iodobenzene, and 1.01 g (10.51 mmol) of t-BuONa were added into the mixture and refluxed at about 160°C for about 48 hours while stirring. After completion of the reaction, 20 mL of cool distilled water was added into the reaction product, followed by extraction with ethylacetate, drying with magnesium sulfate, filtering, and removing the solvent. The resulting product was purified by column chromatography to obtain 4.82 g of Intermediate I-2 (N,2,3-triphenylbenzofuran-6-amine) (Yield: 76%).

### Synthesis of Compound 1

76.3 mg (0.083 mmol) of Pd₂(dba)₃ and 33.7 mg (0.167 mmol) of t-Bu₃P were dissolved in 30 mL of o-xylene, and then stirred at room temperature for about 10 minutes. 3 g (8.33 mmol) of 1,6-dibromopyrene, 3.3 g (9.17 mmol) of Intermediate I-2, and 480 mg (5.00 mmol) of t-BuONa were added into the mixture and refluxed at about 160°C for about 48 hours while stirring. After completion of the reaction, 20 mL of cool distilled water was added into the reaction product, followed by extraction with ethylacetate, drying with magnesium sulfate, filtering, and removing the solvent. The resulting product was purified by column chromatography to obtain 5.64 g of Compound 1 (N1,N6-bis(2,3-diphenylbenzofuran-6-yl)-N1,N6-diphenylpyrene-1,6-diamine) (Yield: 73%).
¹H NMR (300 MHz, a CDCl₃), d (ppm): 8.99-8.87 (4H, m), 8.32-8.26 (2H, d), 8.26-8.20 (2H, m), 8.03-7.97 (2H, d), 7.95-7.85 (6H, m), 7.75-7.68 (2H, m), 7.63-7.48 (10H, m), 7.47-7.36 (6H, m), 7.31-7.23 (4H, t), 7.19-7.10 (4H, m), 7.01-6.93 (2H, m).
EI-MS, m/e, calcd for C₆₈H₄₄N₂O₂ 920.34, found 920.38.

### Synthesis Example 2: Synthesis of Compound 2

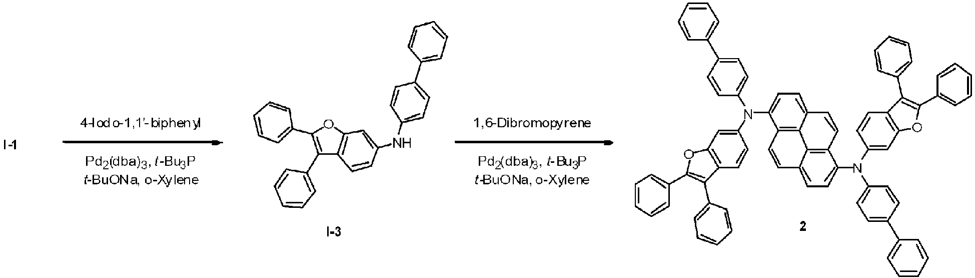

### Synthesis of Intermediate I-3

5.68 g (Yield 74%) of Intermediate I-3 (N-([1,1'-biphenyl]-4-yl)-2,3-diphenylbenzofuran-6-amine) was obtained in the same manner as in the synthesis of Intermediate I-2 in Synthesis Example 1, except that 5.4 g (19.28 mmol) of 4-iodo-1,1'-biphenyl, instead of iodobenzene, was used.

### Synthesis of Compound 2

6.48 g (Yield 72%) of Compound 2 (N1,N6-di([1,1'-biphenyl]-4-yl)-N1,N6-bis(2,3-diphenylbenzofuran-6-yl)pyrene-1,6-diamine) was obtained in the same manner as in the synthesis of Compound 1 in Synthesis Example 1, except that 4.0 g (9.17 mmol) of Intermediate I-3, instead of Intermediate I-2, was used.
¹H NMR (300 MHz, a CDCl₃), d (ppm): 9.00-8.90 (4H, m), 8.69-8.59 (4H, m), 8.44-8.37 (2H, d), 8.26-8.20 (2H, m), 8.14-8.07 (2H, m), 8.00-7.94 (4H, d), 7.93-7.86 (4H, m), 7.65-7.55 (10H, m), 7.55-7.48 (4H, t), 7.48-7.34 (10H, m), 7.32-7.25 (2H, m), 7.22-7.12 (4H, m).
EI-MS, m/e, calcd for C₈₀H₅₂N₂O₂ 1072.40, found 1072.46.

### Synthesis Example 3: Synthesis of Compound 3

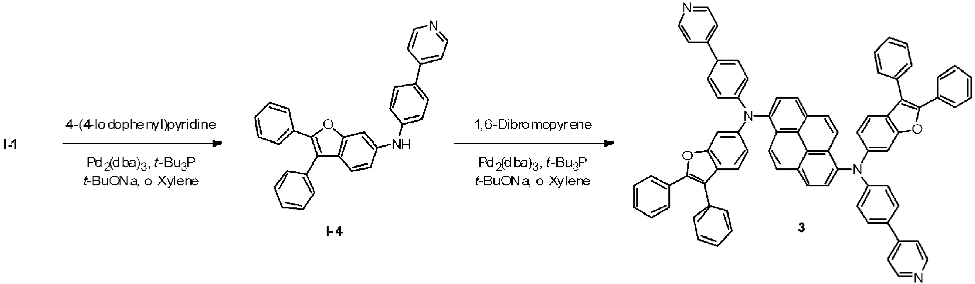

### Synthesis of Intermediate I-4

5.26 g (Yield 68%) of Intermediate I-4 (2,3-diphenyl-N-(4-(pyridin-4-yl)phenyl)benzofuran-6-amine) was obtained in the same manner as in the synthesis of Intermediate I-2 in Synthesis Example 1, except that 5.4 g (19.28 mmol) of 4-(4-iodophenyl)pyridine, instead of iodobenzene, was used.

### Synthesis of Compound 3

6.82 g (Yield 76%) of Compound 3 (N1,N6-bis(2,3-diphenylbenzofuran-6-yl)-N1,N6-bis(4-(pyridin-4-yl)phenyl)pyrene-1,6-diamine) was obtained in the same manner as in the synthesis of Compound 1 in Synthesis Example 1, except that 4.0 g (9.17 mmol) of Intermediate I-4, instead of Intermediate I-2, was used.
¹H NMR (300 MHz, a CDCl₃), d (ppm): 9.00-8.92 (4H, m), 8.89-8.82 (4H, d), 8.53-8.47 (4H, d), 8.43-8.37 (2H, d), 8.22-8.17 (2H, m), 8.12-8.06 (2H, m), 7.96-7.87 (8H, m), 7.64-7.55 (6H, m), 7.54-7.48 (4H, t), 7.48-7.38 (4H, m), 7.38-7.33 (4H, d), 7.31-7.26 (2H, m), 7.22-7.13 (4H, m).
EI-MS, m/e, calcd for C₇₈H₅₀N₄O₂ 1074.39, found 1074.43.

### Synthesis Example 4: Synthesis of Compound 16

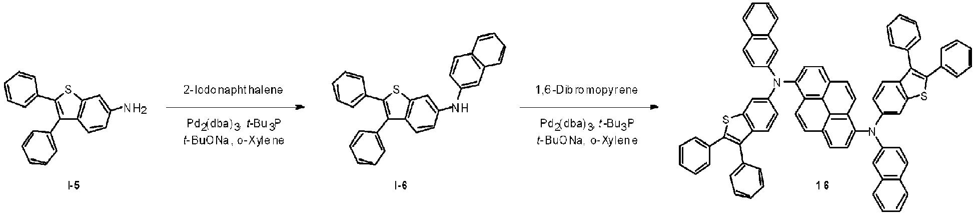

### Synthesis of Intermediate I-6

4.86 g (Yield 69%) of Intermediate I-6 (N-(naphthalen-2-yl)-2,3-diphenylbenzo[b]thiophen-6-amine) was obtained in the same manner as in the synthesis of Intermediate I-2 in Synthesis Example 1, except that 5 g (16.59 mmol) of 2,3-diphenylbenzo[b]thiophen-6-amine (Intermediate I-5) and 4.6 g (18.25 mmol) of 2-iodonaphthalene, instead of 2,3-diphenylbenzofuran-6-amine and iodobenzene, respectively, were used.

### Synthesis of Compound 16

6.97 g (Yield 79%) of Compound 16 (N1,N6-bis(2,3-diphenylbenzo[b]thiophen-6-yl)-N1,N6-di(naphthalen-2-yl)pyrene-1,6-diamine) was obtained in the same manner as in the synthesis of Compound 1 in Synthesis Example 1, except that 3.9 g (9.17 mmol) of Intermediate I-6, instead of Intermediate I-2, was used.
¹H NMR (300 MHz, a CDCl₃), d (ppm): 9.08-9.01 (2H, m), 8.70-8.64 (2H, d), 8.33-8.24 (2H, m), 8.19-8.09 (6H, m), 8.04-7.95 (6H, m), 7.92-7.85 (2H, m), 7.81-7.72 (6H, m), 7.65-7.60 (2H, d), 7.60-7.47 (10H, m), 7.44-7.34 (4H, m), 7.33-7.29 (2H, m), 7.29-7.23 (2H, d), 7.22-7.15 (2H, m).
EI-MS, m/e, calcd for C₇₆H₄₈N₂S₂ 1052.33, found 1052.36.

### Synthesis Example 5: Synthesis of Compound 37

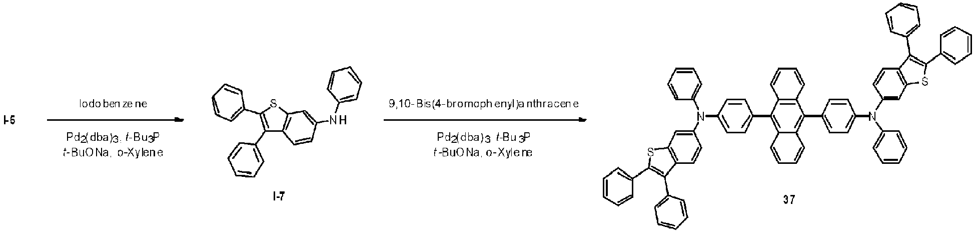

### Synthesis of Intermediate I-7

4.74 g (Yield 76%) of Intermediate I-7 (N,2,3-triphenylbenzo[b]thiophen-6-amine) was obtained in the same manner as in the synthesis of Intermediate I-4 in Synthesis Example 4, except that 3.7 g (18.25 mmol) of iodobenzene, instead of 2-iodonaphthalene, was used.

### Synthesis of Compound 37

5.68 g (Yield 85%) of Compound 37 (N,N'-(anthracene-9,10-diylbis(4,1-phenylene))bis(N,2,3-triphenylbenzo[b]thiophen-6-amine)) was obtained in the same manner as in the synthesis of Compound 16 in Synthesis Example 4, except that 3 g (6.14 mmol) of 9,10-bis(4-bromophenyl)anthracene) and 2.6 g (6.76 mmol) of Intermediate I-7, instead of 1,6-dibromopyrene and Intermediate I-6, respectively, were used.
¹H NMR (300 MHz, a CDCl₃), d (ppm): 9.91-9.79 (4H, m), 8.11-8.02 (2H, d), 7.99-7.92 (4H, m), 7.92-7.86 (2H, d), 7.86-7.79 (4H, m), 7.58-7.53 (4H, t), 7.52-7.48 (4H, d), 7.47-7.37 (14H, m), 7.36-7.29 (2H, m), 7.14-7.07 (4H, t), 7.06-6.99 (4H, m), 6.87-6.79 (2H, m).
EI-MS, m/e, calcd for C₇₈H₅₂N₂S₂ 1080.36, found 1080.42.

### Synthesis Example 6: Synthesis of Compound 64

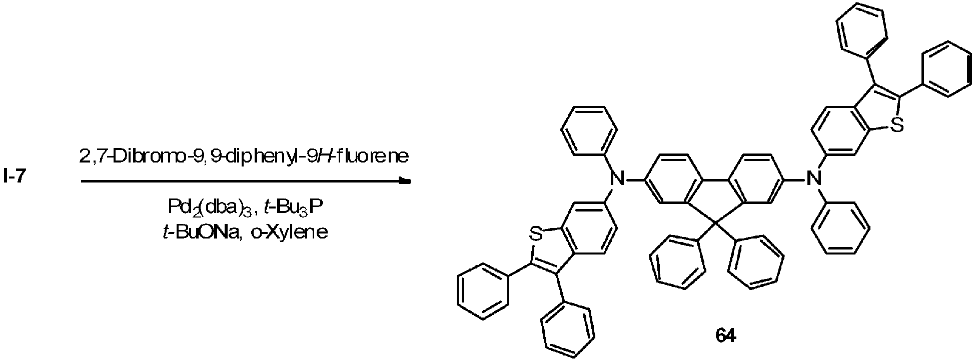

5.82 g (Yield 86%) of Compound 64 (N2,N7-bis(2,3-diphenylbenzo[b]thiophen-6-yl)-N2,N7,9,9-tetraphenyl-9H-fluorene-2,7-diamine) was obtained in the same manner as in the synthesis of Compound 37 in Synthesis Example 5, except that 3 g (6.29 mmol) of 2,7-dibromo-9,9-diphenyl-9H-fluorene, instead of 1,6-dibromopyrene, was used.
¹H NMR (300 MHz, a CDCl₃), d (ppm): 9.89-9.81 (2H, d), 8.14-8.09 (2H, m), 7.82-7.72 (8H, m), 7.71-7.66 (2H, m), 7.59-7.55 (2H, m), 7.50-7.42 (10H, m), 7.40-7.20 (18, m), 7.18-7.12 (4H, m), 7.12-7.07 (2H, m), 6.99-6.93 (2H, m).
EI-MS, m/e, calcd for C₇₇H₅₂N₂S₂ 1068.36, found 1068.41

### Synthesis Example 7: Synthesis of Compound 82

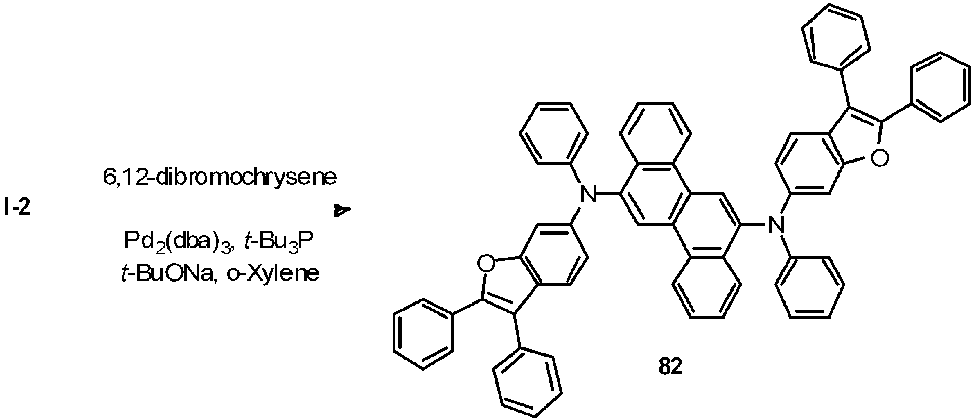

5.42 g (Yield 74%) of Compound 82 (N6,N12-bis(2,3-diphenylbenzofuran-6-yl)-N6,N12-diphenylchrysene-6,12-diamine) was obtained in the same manner as in the synthesis of Compound 1 in Synthesis Example 1, except that 3 g (7.77 mmol) of 6,12-dibromochrysene, instead of 1,6-dibromopyrene, was used, and the amount of Intermediate I-2 was changed to 3.1 g (8.55 mmol).
¹H NMR (300 MHz, a CDCl₃), d (ppm): 8.77-8.70 (2H, m), 8.53-8.44 (4H, m), 8.06-7.99 (4H, m), 7.83-7.78 (2H, m), 7.77-7.59 (14H, m), 7.57-7.50 (4H, m), 7.49-7.41 (2H, m), 7.28-7.19 (4H, t), 7.15-7.06 (4H, m), 6.99-6.89 (4H, m), 6.42-6.34 (2H, m).
EI-MS, m/e, calcd for C₇₀H₄₆N₂O₂ 946.36, found 946.36

### Synthesis Example 8: Synthesis of Compound 91

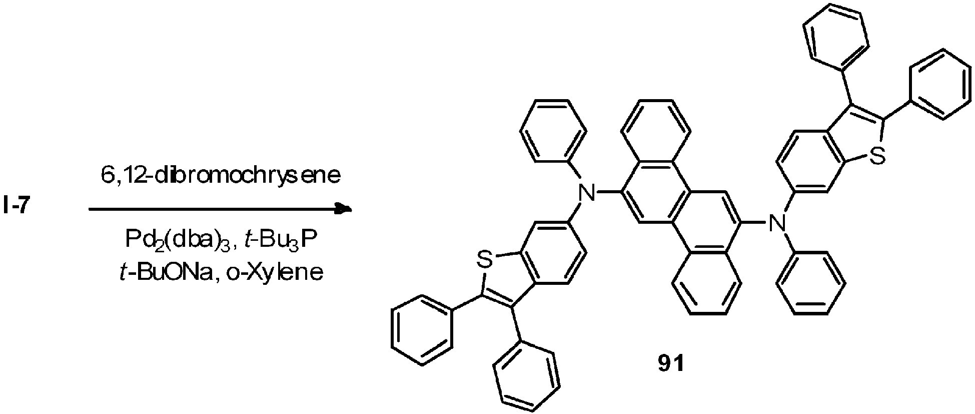

5.84 g (Yield 76%) of Compound 91 (N6,N12-bis(2,3-diphenylbenzofuran-6-yl)-N6,N12-diphenylchrysene-6,12-diamine) was obtained in the same manner as in the synthesis of Compound 37 in Synthesis Example 5, except that 3 g (7.77 mmol) of 6,12-dibromochrysene, instead of 1,6-dibromopyrene, was used, and the amount of Intermediate I-7 was changed to 3.2 g (8.54 mmol).
¹H NMR (300 MHz, a CDCl³), d (ppm): 8.85-8.76 (2H, m), 8.19-8.11 (4H, m), 8.00-7.93 (4H, m), 7.84-7.75 (6H, m), 7.75-7.66 (4H, m), 7.52-7.42 (10H, m), 7.42-7.31 (6H, m), 7.29-7.21 (4H, m), 7.17-7.10 (4H, m), 6.99-6.90 (2H, m).
EI-MS, m/e, calcd for C₇₀H₄₆N₂S₂ 978.31, found 978.38.

### Example 1

To manufacture an anode, a corning 15 Ω/cm² (1200 A) ITO glass substrate was cut to a size of 50 mm x 50 mm x 0.7 mm and then sonicated in isopropyl alcohol and pure water each for five minutes, and then cleaned by irradiation of ultraviolet rays for 30 minutes and exposure to ozone. The resulting glass substrate was loaded into a vacuum deposition device.

2-TNATA was vacuum-deposited on the ITO anode to form an HIL having a thickness of 600Å on the anode, and then 4,4'-bis[N-(1-naphthyl)-N-phenylamino]biphenyl (NPB) was vacuum-deposited on the HIL to form a HTL having a thickness of 300Å.

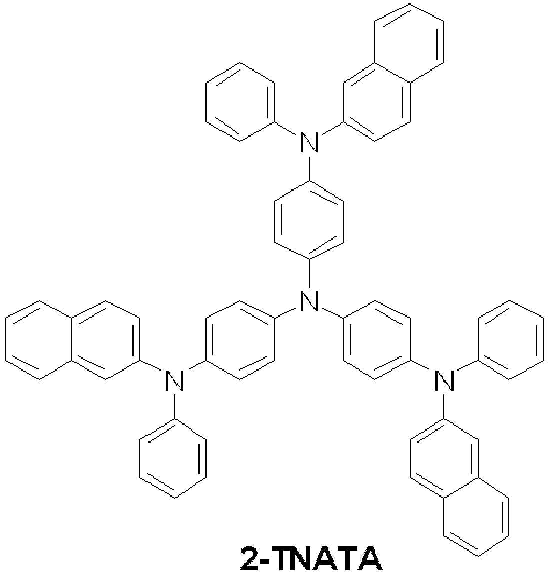

ADN (host) and Compound 1 (dopant) were co-deposited in a weight ratio of about 98:2 on the HTL to form an EML having a thickness of about 400Å, followed by depositing Alq₃ on the EML to form an ETL having a thickness of about 300Å. LiF was deposited on the ETL to form an EIL having a thickness of about 10Å, and Al was deposited on the EIL to form a cathode having a thickness of about 3000Å, thereby completing the manufacture of an organic light-emitting device.

### Example 2

An organic light-emitting device was manufactured in the same manner as in Example 1, except that Compound 2, instead of Compound 1, was used as a dopant in forming the EML.

### Example 3

An organic light-emitting device was manufactured in the same manner as in Example 1, except that Compound 3, instead of Compound 1, was used as a dopant in forming the EML.

### Example 4

An organic light-emitting device was manufactured in the same manner as in Example 1, except that Compound 16, instead of Compound 1, was used as a dopant in forming the EML.

### Example 5

An organic light-emitting device was manufactured in the same manner as in Example 1, except that Compound 37, instead of Compound 1, was used as a dopant in forming the EML.

### Example 6

An organic light-emitting device was manufactured in the same manner as in Example 1, except that Compound 64, instead of Compound 1, was used as a dopant in forming the EML.

### Example 7

An organic light-emitting device was manufactured in the same manner as in Example 1, except that Compound 82, instead of Compound 1, was used as a dopant in forming the EML.

### Example 8

An organic light-emitting device was manufactured in the same manner as in Example 1, except that Compound 91, instead of Compound 1, was used as a dopant in forming the EML.

### Comparative Example 1

An organic light-emitting device was manufactured in the same manner as in Example 1, except that DPVBi, instead of Compound 1, was used as a dopant in forming the EML.

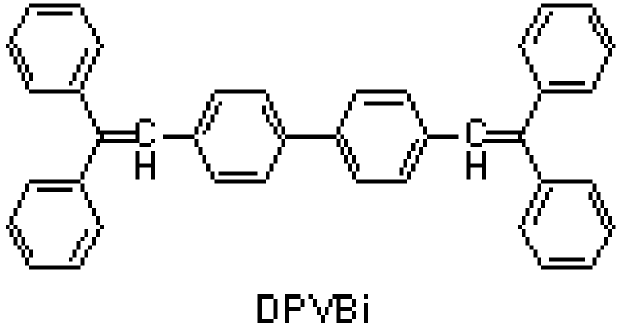

### Comparative Example 2

An organic light-emitting device was manufactured in the same manner as in Example 1, except that Compound A below, instead of Compound 1, was used as a dopant in forming the EML.

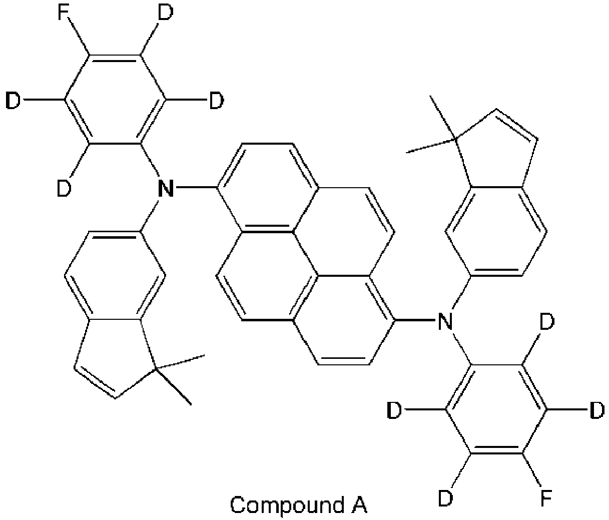

### Comparative Example 3

An organic light-emitting device was manufactured in the same manner as in Example 1, except that Compound B below, instead of Compound 1, was used as a dopant in forming the EML.

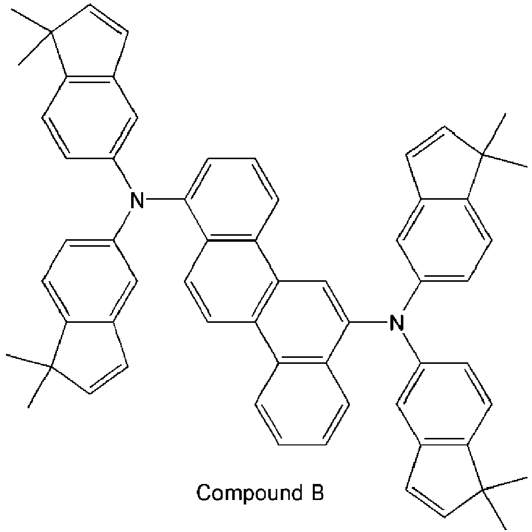

### Comparative Example 4

An organic light-emitting device was manufactured in the same manner as in Example 1, except that Compound C below, instead of Compound 1, was used as a dopant in forming the EML.

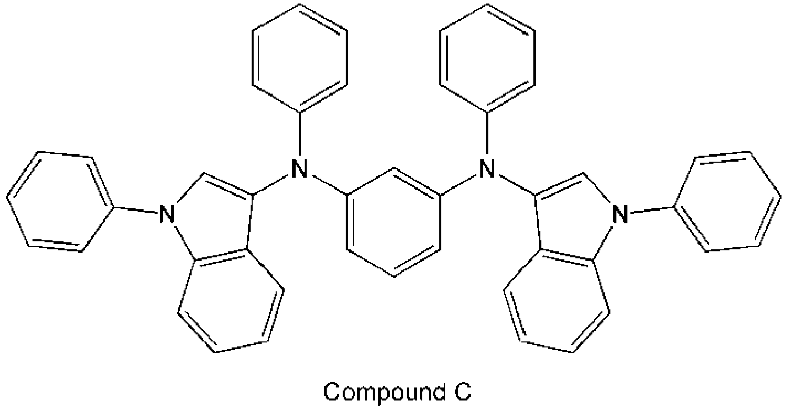

### Comparative Example 5

An organic light-emitting device was manufactured in the same manner as in Example 1, except that Compound D below, instead of Compound 1, was used as a dopant in forming the EML.

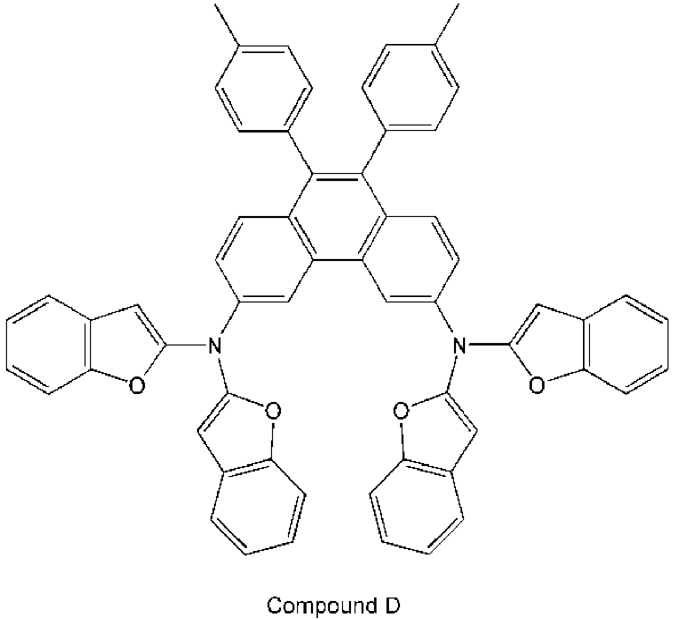

### Comparative Example 6

An organic light-emitting device was manufactured in the same manner as in Example 1, except that Compound E below, instead of Compound 1, was used as a dopant in forming the EML.

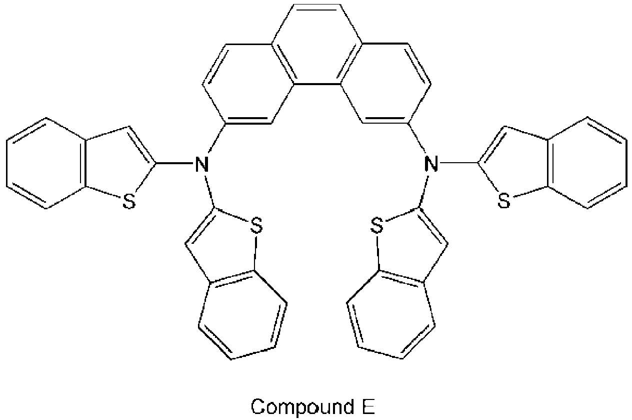

### Evaluation Example 1: Characteristics evaluation of organic light-emitting devices

Efficiencies and color purities of the organic light-emitting devices of Examples 1 to 8 and Comparative Examples 1 to 6 were measured using a voltage source-measure unit (Kethley SMU 236) as a power supply and a PR650 (Spectroscan) Source Measurement Unit (available from Photo Research, Inc.). The results are shown in Table 1.

**[Table 1]**

| Example | Host | Dopant | Current density (mA/cm²) | Emission efficiency (cd/A) | Color coordinates | |
|---|---|---|---|---|---|---|
| | | | | | CIE x | CIE y |
| Example 1 | ADN | Compound 1 | 10 | 7.2 | 0.14 | 0.12 |
| Example 2 | ADN | Compound 2 | 10 | 6.7 | 0.15 | 0.11 |
| Example 3 | ADN | Compound 3 | 10 | 6.9 | 0.14 | 0.12 |
| Example 4 | ADN | Compound 16 | 10 | 7.1 | 0.15 | 0.13 |
| Example 5 | ADN | Compound 37 | 10 | 7.5 | 0.15 | 0.11 |
| Example 6 | ADN | Compound 64 | 10 | 6.8 | 0.15 | 0.10 |
| Example 7 | ADN | Compound 82 | 10 | 6.6 | 0.14 | 0.11 |
| Example 8 | ADN | Compound 91 | 10 | 7.1 | 0.15 | 0.12 |
| Comparative Example 1 | ADN | DPVBi | 10 | 4.3 | 0.15 | 0.20 |
| Comparative Example 2 | ADN | Compound A | 10 | 4.1 | 0.15 | 0.21 |
| Comparative Example 3 | ADN | Compound B | 10 | 4.4 | 0.14 | 0.19 |
| Comparative Example 4 | ADN | Compound C | 10 | 4.3 | 0.15 | 0.22 |
| Comparative Example 5 | ADN | Compound D | 10 | 4.5 | 0.15 | 0.21 |
| Comparative Example 6 | ADN | Compound E | 10 | 4.4 | 0.15 | 0.24 |

Referring to Table 1, the organic light-emitting devices of Examples 1 to 8 were found to have improved emission efficiencies and improved color purities, compared to the organic light-emitting devices of Comparative Examples 1 to 6.

As described above, according to the one or more embodiments, an amine-based compound of Formula 1 above have improved electrical characteristics and improved thermal stability. Accordingly, an organic light-emitting device including the amine-based compound has improved emission efficiency and improved color purity characteristics.

## Claims

1. An amine-based compound represented by Formula 1 below: 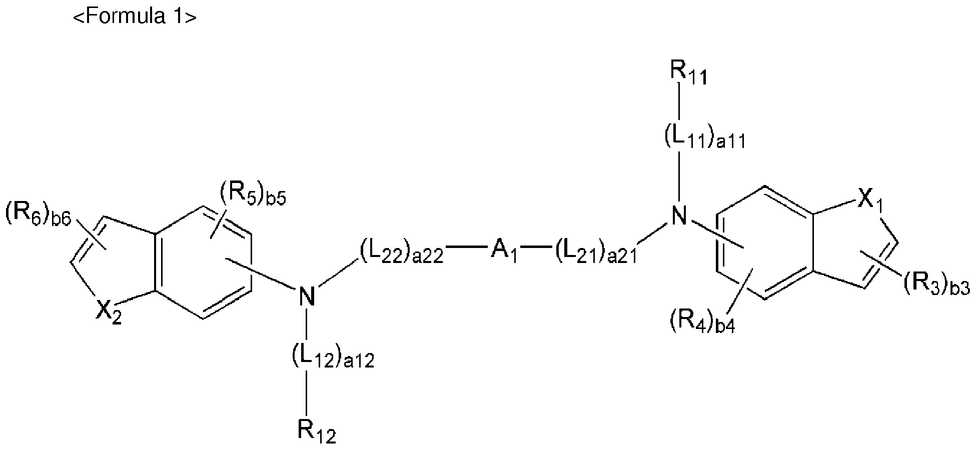 wherein, in Formula 1, A₁ is selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, - N(Q₁₁)(Q₁₂), and -Si(Q₁₃)(Q₁₄)(Q₁₅);
X₁ is selected from N-[(L₁)ₐ₁-(R₁)_{b1}], S, and O;
X₂ is selected from N-[(L₂)ₐ₂-R₂], S, and O;
L₁, L₂, L₁₁, L₁₂, L₂₁, and L₂₂ are each independently selected from
a C₃-C₁₀ cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, and
a C₃-C₁₀cycloalkylene group, a C₃-C₁₀ heterocycloalkylene group, a C₃-C₁₀ cycloalkenylene group, a C₃-C₁₀ heterocycloalkenylene group, a C₆-C₆₀ arylene group, and a C₂-C₆₀ heteroarylene group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, -N(Q₁₁)(Q₁₂), and -Si(Q₁₃)(Q₁₄)(Q₁₅);
a1, a2, a11, a12, a21, and a22 are each independently an integer from 0 to 5;
R₁, R₂, R₁₁, and R₁₂ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, and a C₂-C₆₀ alkynyl group,
a C₁-C₆₀ alkyl group, a C₂-C₆₀ alkenyl group, and a C₂-C₆₀ alkynyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one selected from a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
-N(Q₁)(Q₂), -Si(Q₃)(Q₄)(Q₅), and -B(Q₆)(Q₇);
Q₁₁ to Q₁₅, and Q₁ to Q₇ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group;
b3 and b6 are each independently 1 or 2; and
b1, b4 and b5 are each independently 1, 2, or 3.

2. The amine-based compound as claimed in claim 1, wherein A₁ is selected from
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, and
a naphthylene group, a fluorenylene group, a spiro-fluorenylene group, a phenanthrenylene group, an anthracenylene group, a triphenylenylene group, a pyrenylene group, a chrysenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.

3. The amine-based compound as claimed in claim 1 or 2, wherein X₁ and X₂ are both S, or X₁ and X₂ are both O.

4. The amine-based compound as claimed in any one of proceeding claims, wherein L₁, L₂, L₁₁, L₁₂, L₂₁, and L₂₂ are each independently selected from a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, a pyrrolylene group, an imidazolylene group, a pyrazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzooxazolylene group, a benzoimidazolylene group, a furanylene group, a benzofuranylene group, a thiophenylene group, a benzothiophenylene group, a thiazolylene group, an isothiazolylene group, a benzothiazolylene group, an isoxazolylene group, an oxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substitute with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀alkyl group, a C₂-C₂₀alkenyl group, a C₂-C₂₀alkynyl group, a C₁-C₂₀alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.

5. The amine-based compound as claimed in any one of proceeding claims, wherein L₁, L₂, L₁₁, L₁₂, L₂₁, and L₂₂ are each independently selected from the groups represented by Formulae 2-1 to 2-30 below: 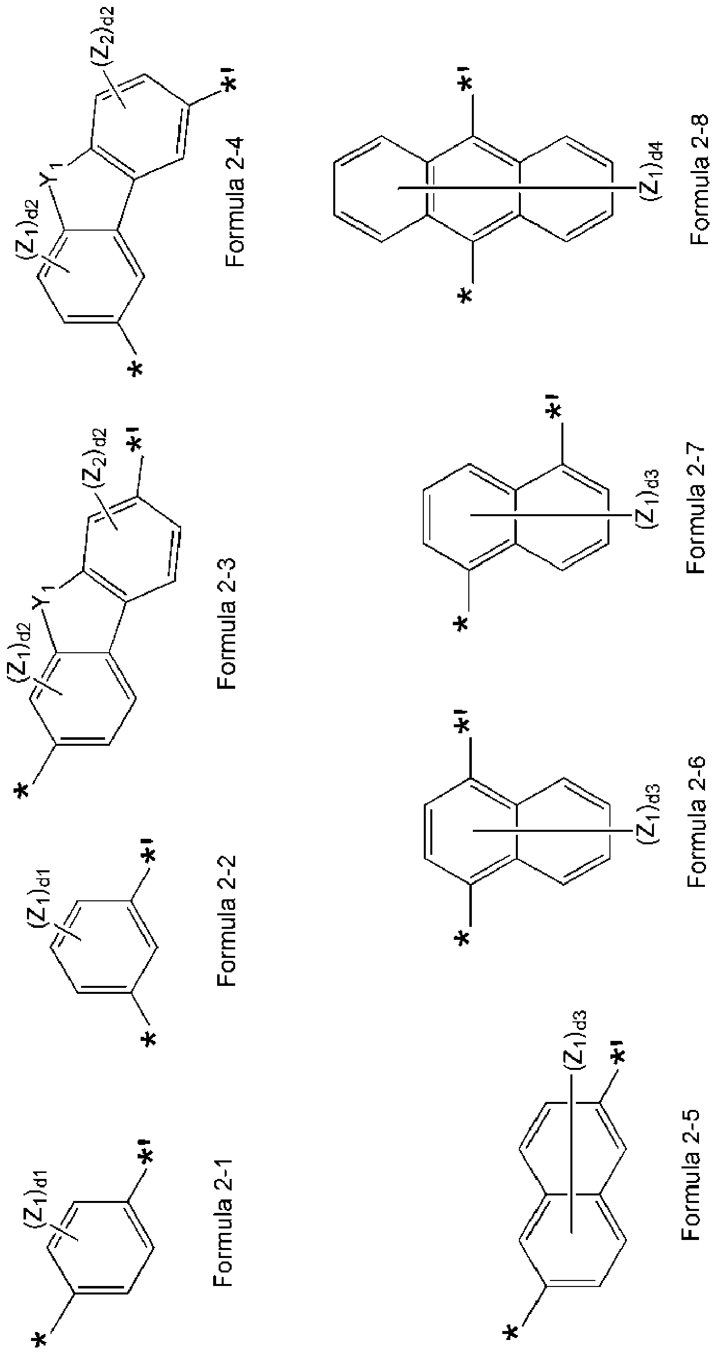 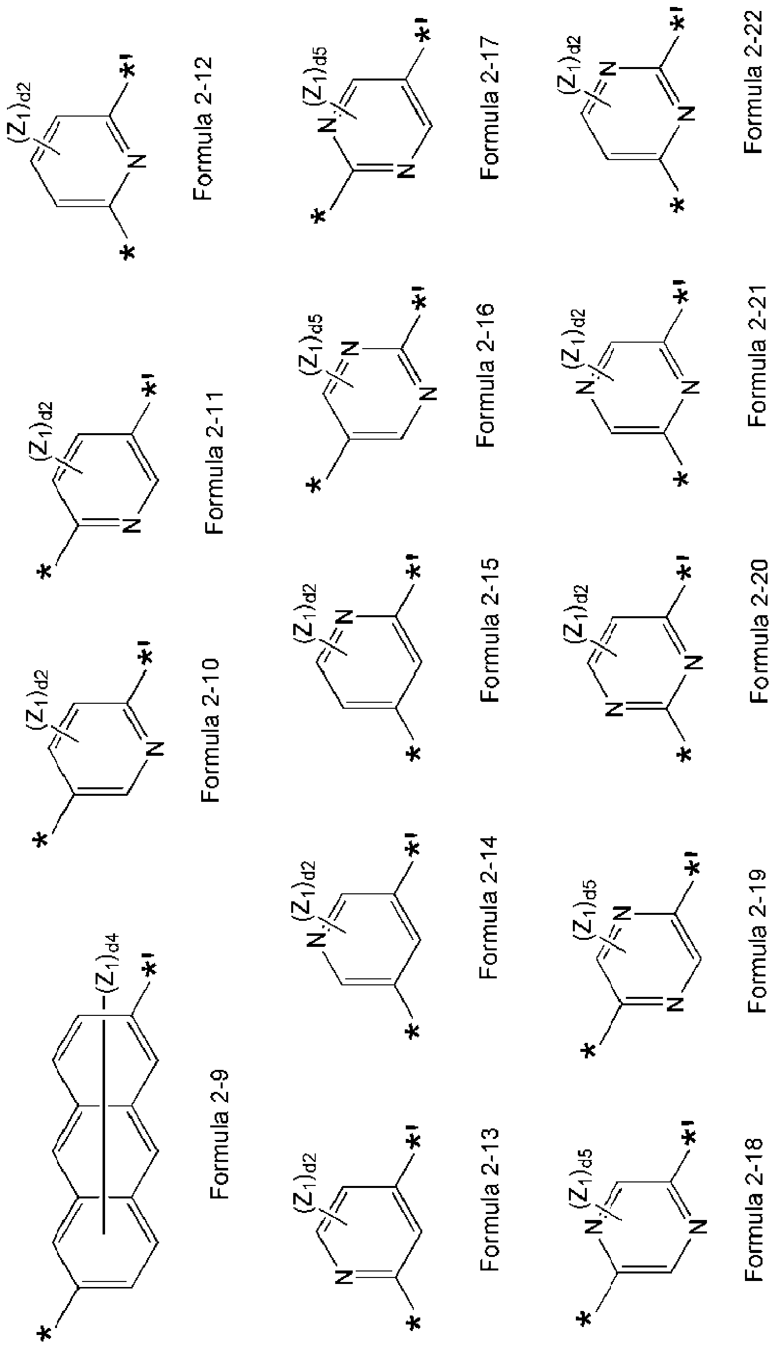 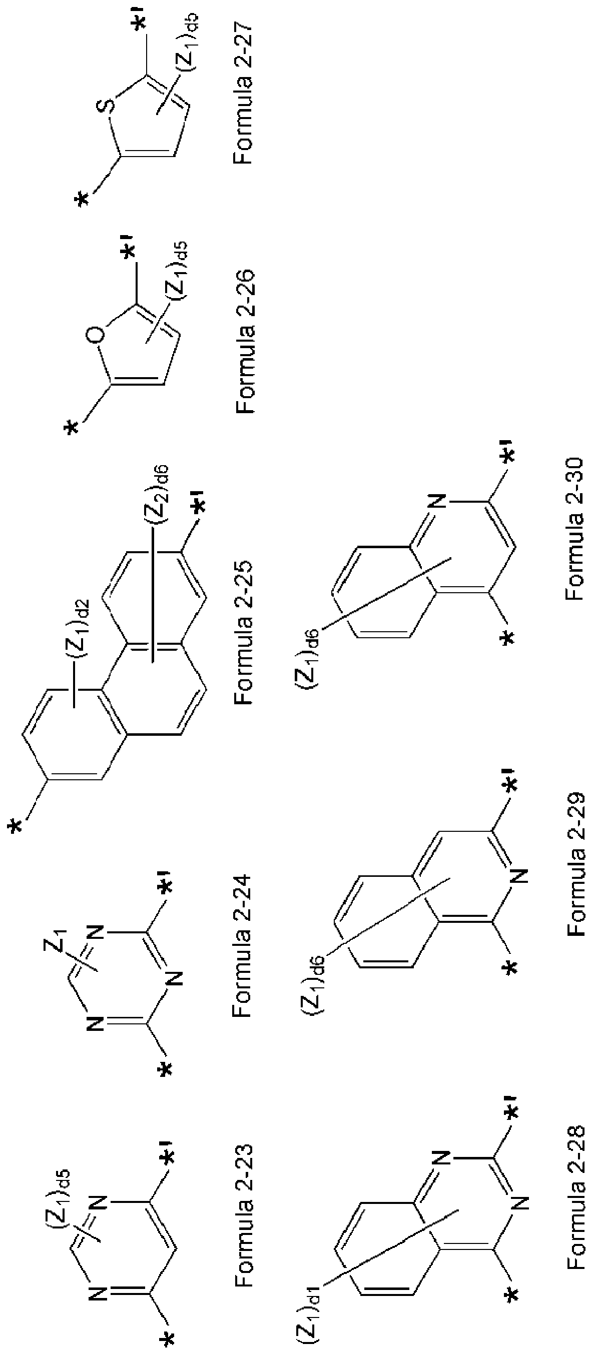
wherein, in Formulae 2-1 to 2-30,
Y₁ is O, S, S(=O), S(=O)₂, C(Z₃)(Z₄), N(Z₅), or Si(Z₆)(Z₇);
Z₁ to Z₇ are each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀alkyl group, a C₂-C₂₀ alkenyl group, a C₂-C₂₀alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
d1 is an integer from 1 to 4;
d2 is an integer from 1 to 3;
d3 is an integer from 1 to 6;
d4 is an integer from 1 to 8;
d5 is 1 or 2;
d6 is an integer from 1 to 5; and
* and *' indicate binding sites of adjacent atoms.

6. The amine-based compound as claimed in any one of proceeding claims, wherein R₁, R₂, R₁₁, and R₁₂ are each independently selected from
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group, and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group.

7. The amine-based compound as claimed in any one of proceeding claims, wherein R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a benzooxazolyl group, a dibenzofuranyl group, a dibenzothiophenyl group, a benzocarbazolyl group, and a dibenzocarbazolyl group;
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, and a benzocarbazolyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group ; and
-N(Q₁)(Q₂), and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group.

8. The amine-based compound as claimed in any one of proceeding claims, wherein R₁, R₂, R₁₁, and R₁₂ are each independently selected from the groups represented by Formulae 4-1 to 4-29 below; and
R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group,
a C₁-C₂₀ alkyl group and a C₁-C₂₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group,
the groups represented by Formulae 4-1 to 4-29 below, and
-N(Q₁)(Q₂) and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group: 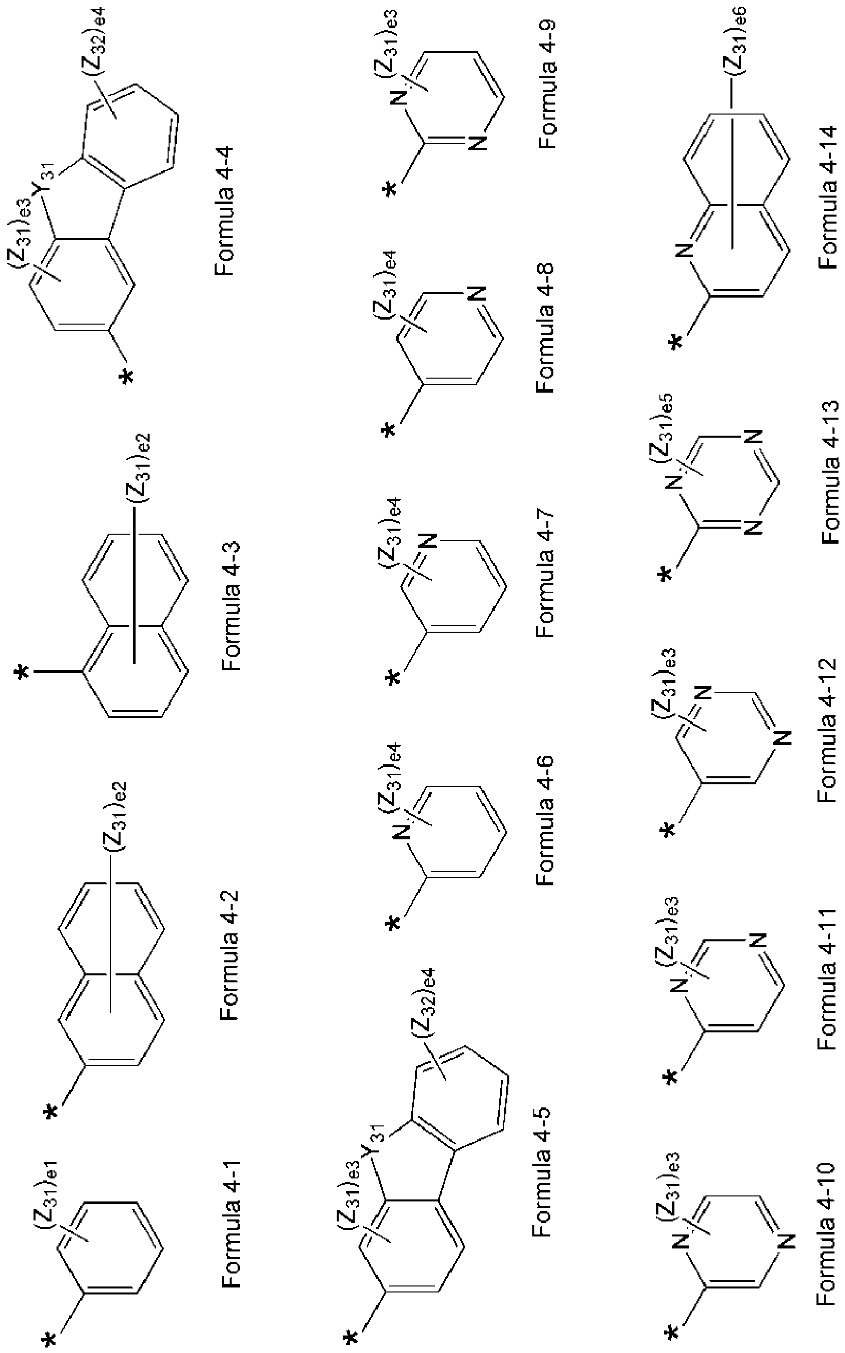 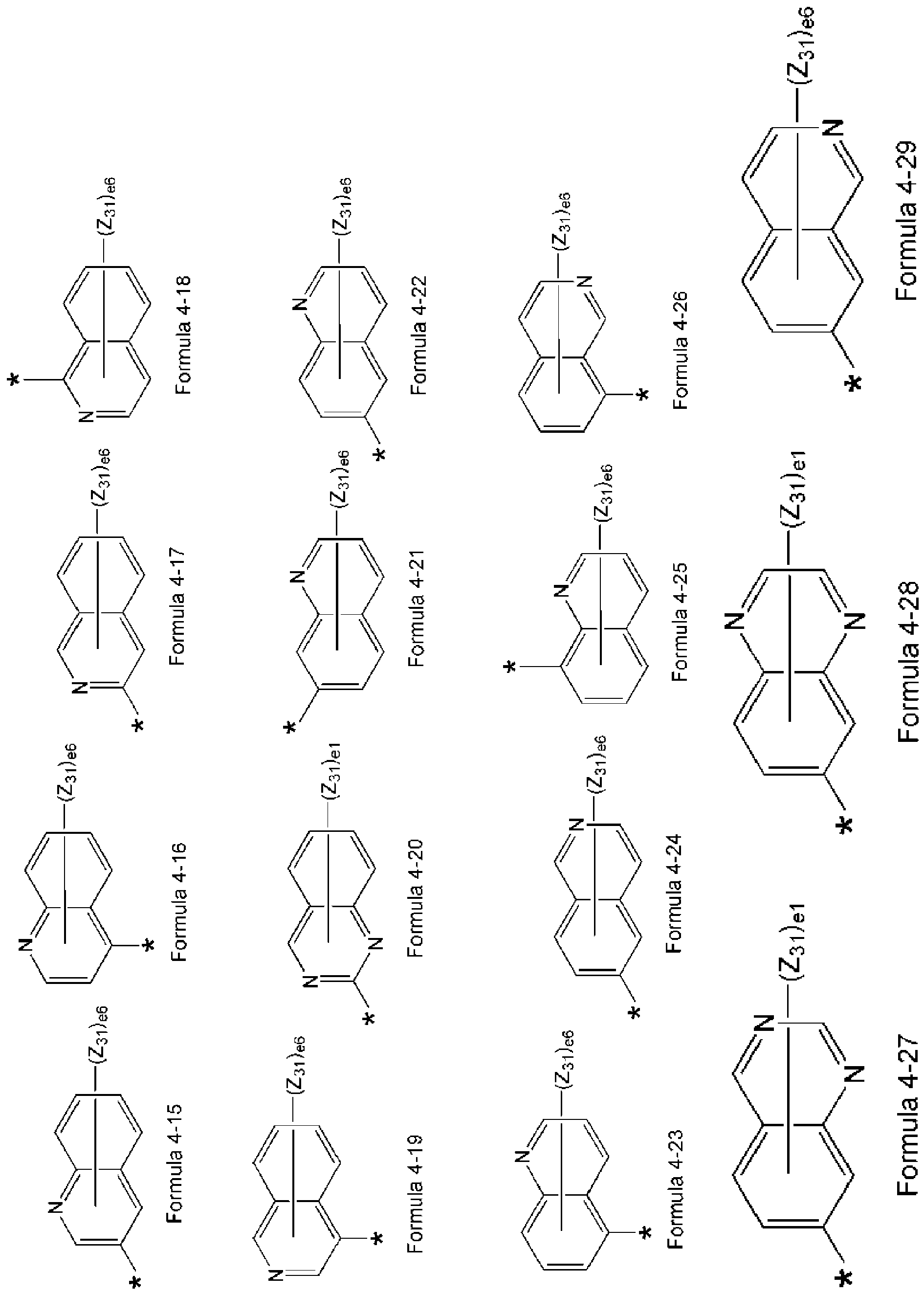
wherein, in Formulae 4-1 to 4-29,
Y₃₁ is O, S, C(Z₃₃)(Z₃₄), N(Z₃₅), or Si(Z₃₆)(Z₃₇);
Z₃₁ to Z₃₇ are each independently selected from a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group;
e1 is an integer from 1 to 5;
e2 is an integer from 1 to 7;
e3 is an integer from 1 to 3;
e4 is an integer from 1 to 4;
e5 is 1 or 2;
e6 is an integer from 1 to 6; and
* indicates a binding site of an adjacent atom.

9. The amine-based compound as claimed in any one of proceeding claims, wherein R₁, R₂, R₁₁, and R₁₂ are each independently selected from the groups represented by Formulae 5-1 to 5-36 below; and
R₃ to R₆ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, and a C₁-C₂₀ alkoxy group,
a C₁-C₂₀ alkyl group and a C₁-C₂₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group,
the groups represented by Formulae 5-1 to 5-36 below, and
-N(Q₁)(Q₂), and -Si(Q₃)(Q₄)(Q₅), wherein Q₁ to Q₅ are each independently selected from
a hydrogen atom, a C₁-C₂₀ alkyl group, a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group; and
a phenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a triazinyl group, a quinolinyl group, an isoquinolinyl group, and a quinazolinyl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a carbazolyl group, a pyridinyl group, a pyrimidinyl group, a triazinyl group, a quinolinyl group, an isoquinolinyl group, and a quinazolinyl group:

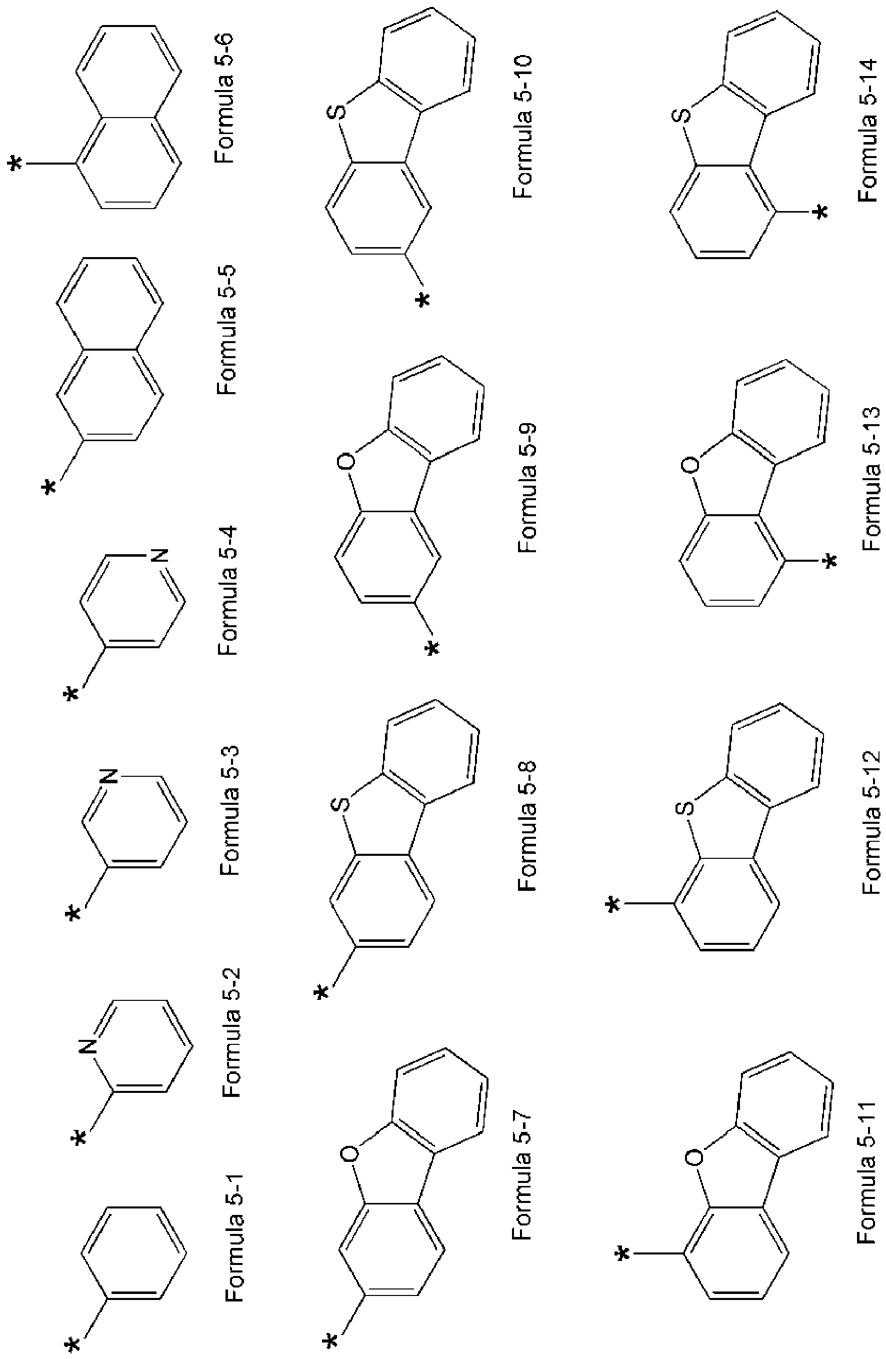

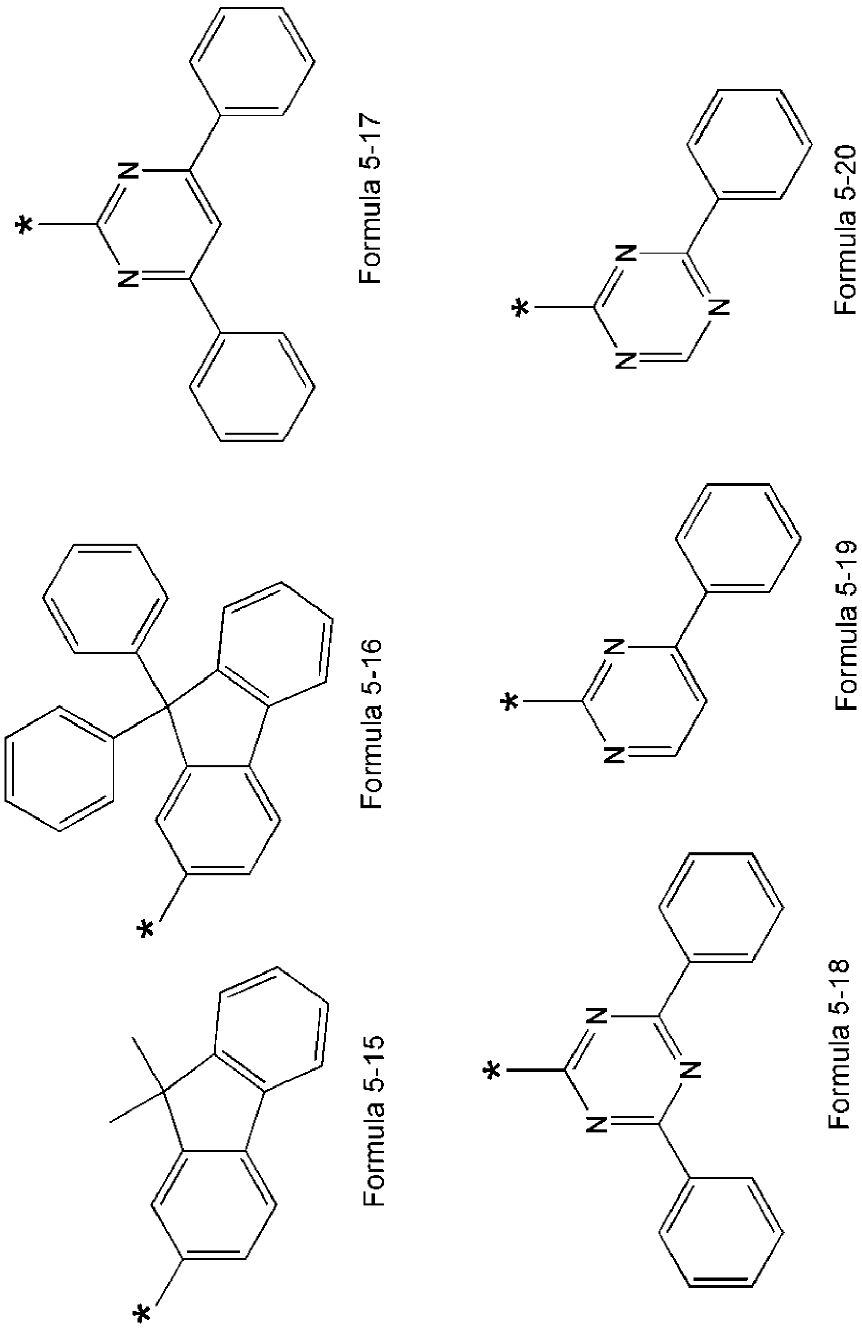

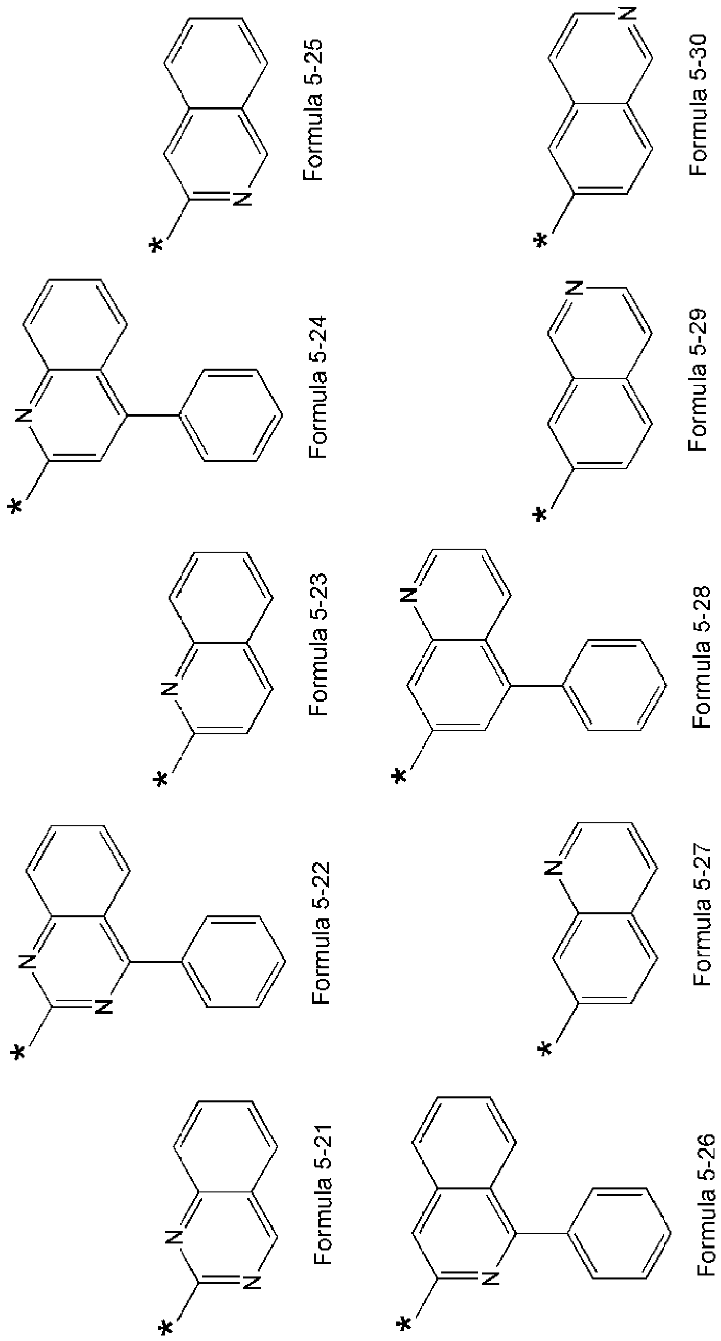

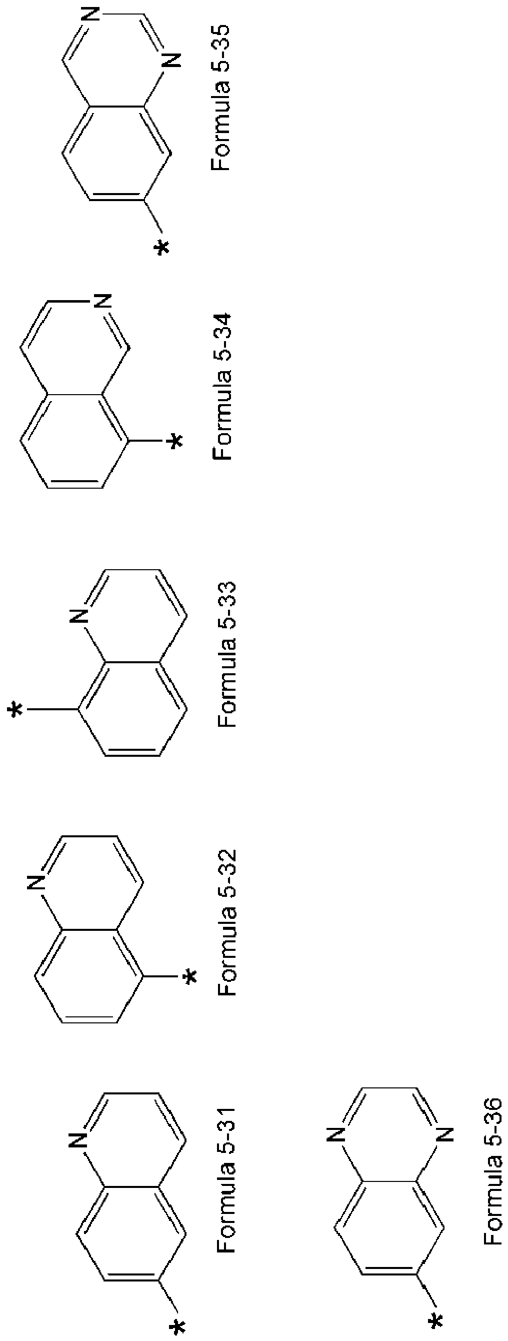

10. The amine-based compound as claimed in any one of proceeding claims, wherein the amine-based compound of Formula 1 is a compound represented by Formula 1A below: 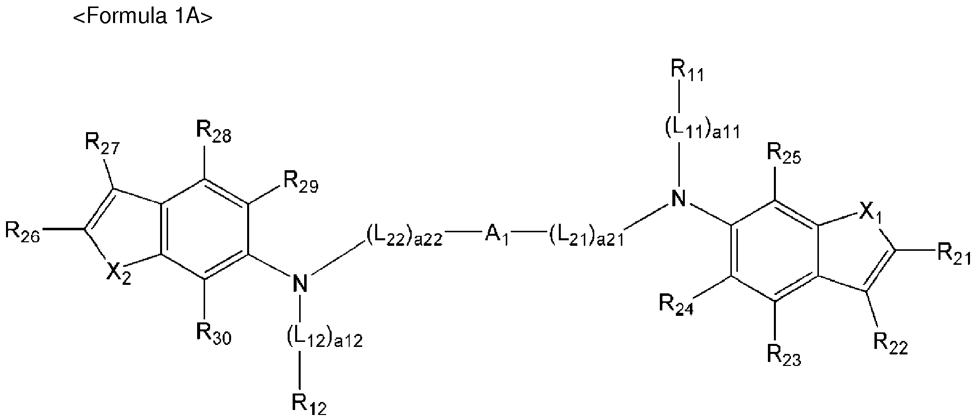 wherein, in Formula 1A, A₁, X₁, X₂, L₁₁, L₁₂, L₂₁, L₂₂, a11, a12, a21, a22, R₁₁, and R₁₂ are the same as those defined in claim 1, and R₂₁ to R₃₀ are the same as R₃ defined in claim 1.

11. The amine-based compound as claimed in any one of proceeding claims, wherein the amine-based compound of Formula 1 is a compound represented by one of Formulae 1A(1) to 1A(4) below: 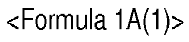 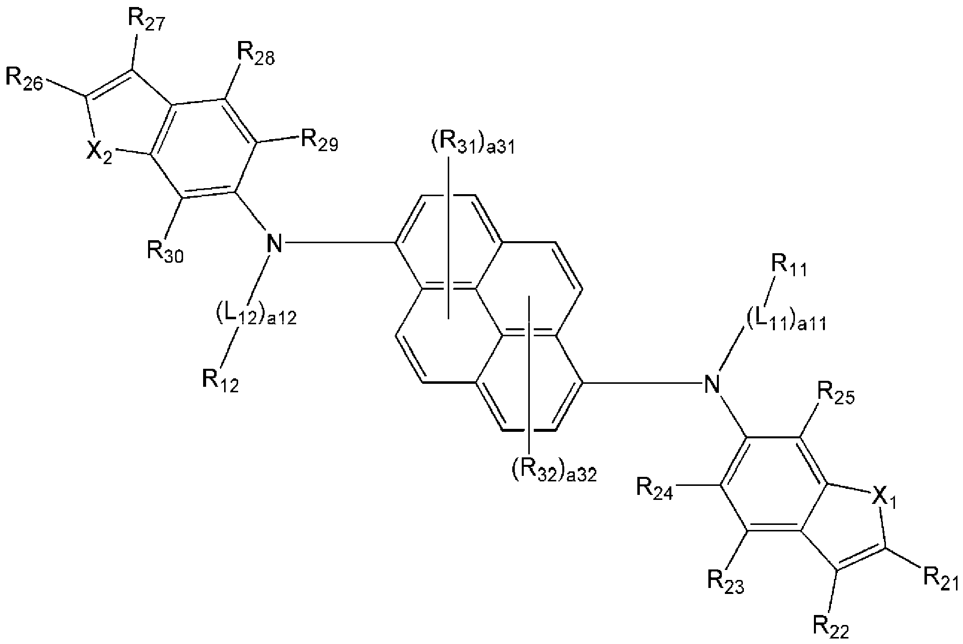 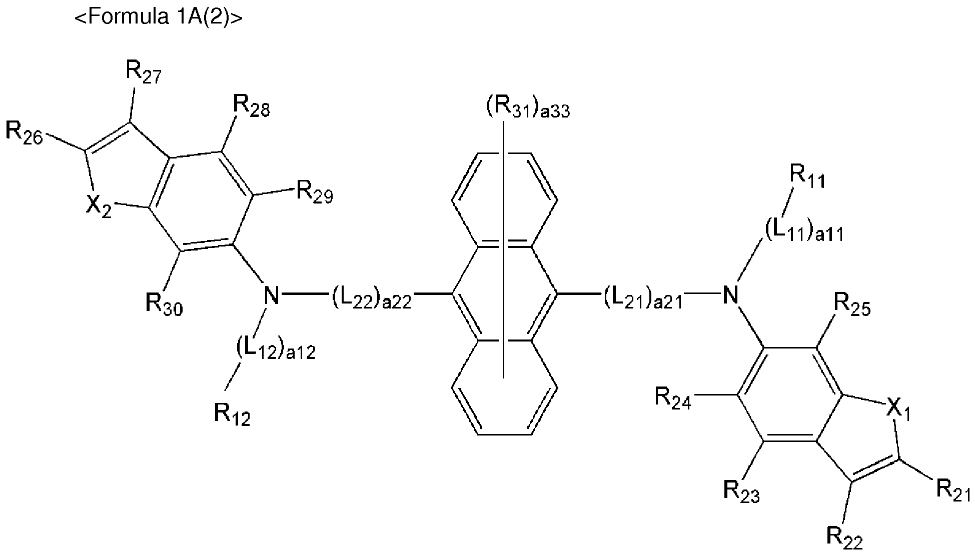 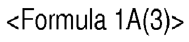 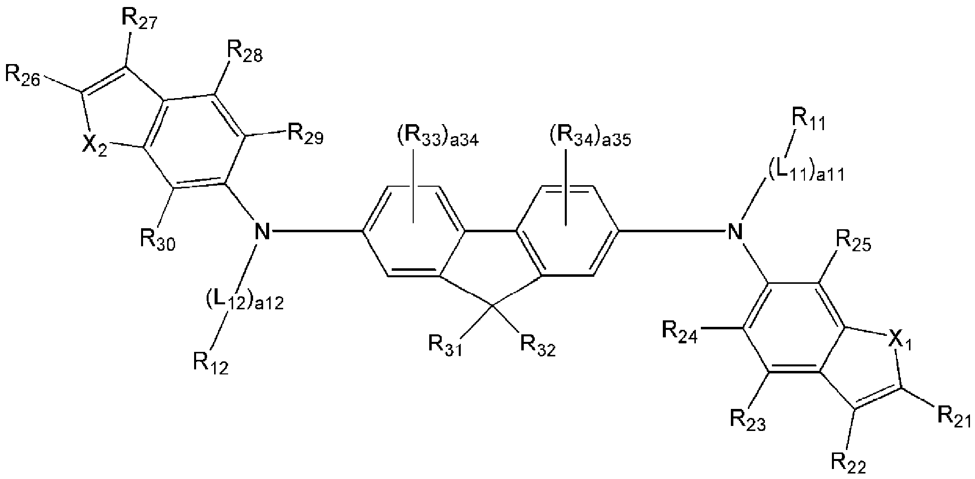 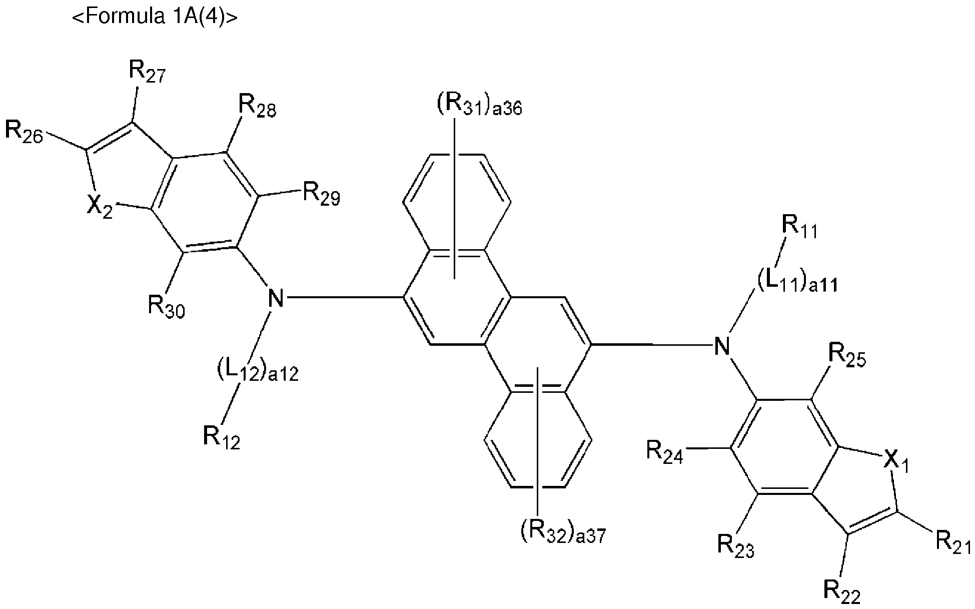 wherein, in Formulae 1A(1) to 1A(4),
X₁, X₂, L₁₁, L₁₂, L₂₁, L₂₂, a11, a12, a21, a22, R₁₁, and R₁₂ are the same as those defined in claim 1;
R₂₁ to R₃₄ are the same as R₃ defined in claim 1;
a31 and a32 are each independently an integer from 1 to 4;
a33 is an integer from 1 to 8;
a34 and a35 are each independently an integer from 1 to 3;
a36 and a37 are each independently an integer from 1 to 5.

12. The amine-based compound as claimed in any one of proceeding claims, wherein the amine-based compound of Formula 1 is one of Compounds 1 to 108 below:

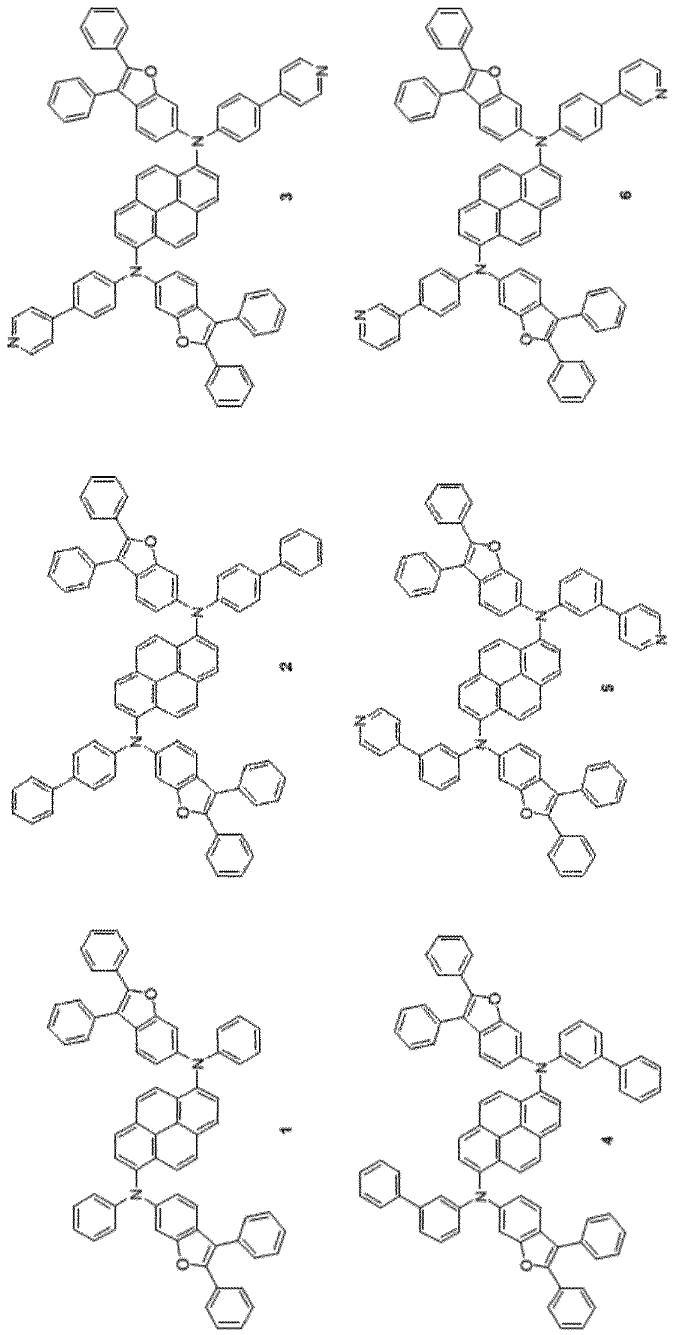

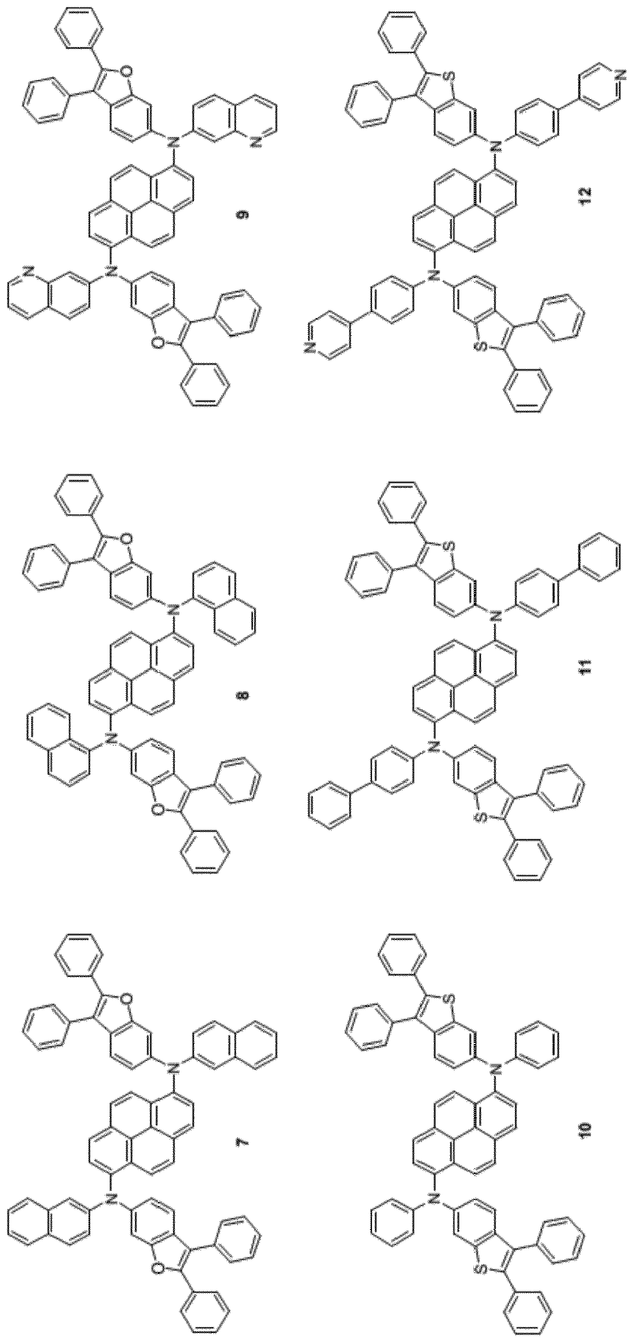

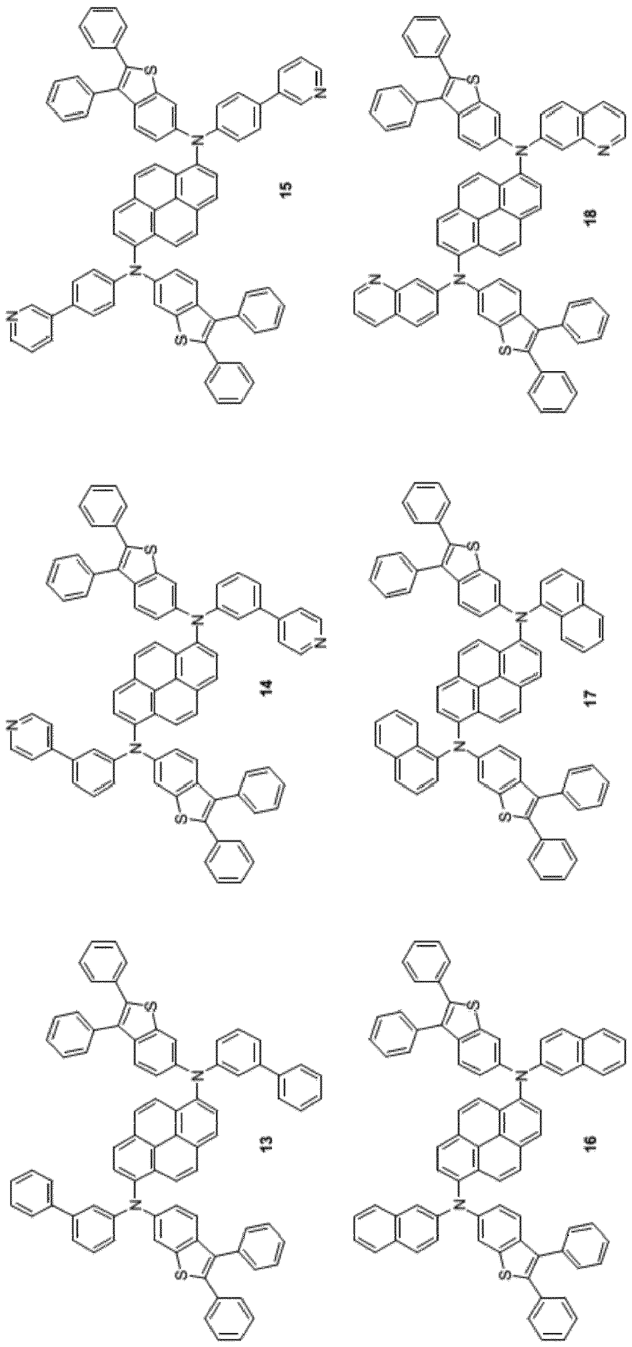

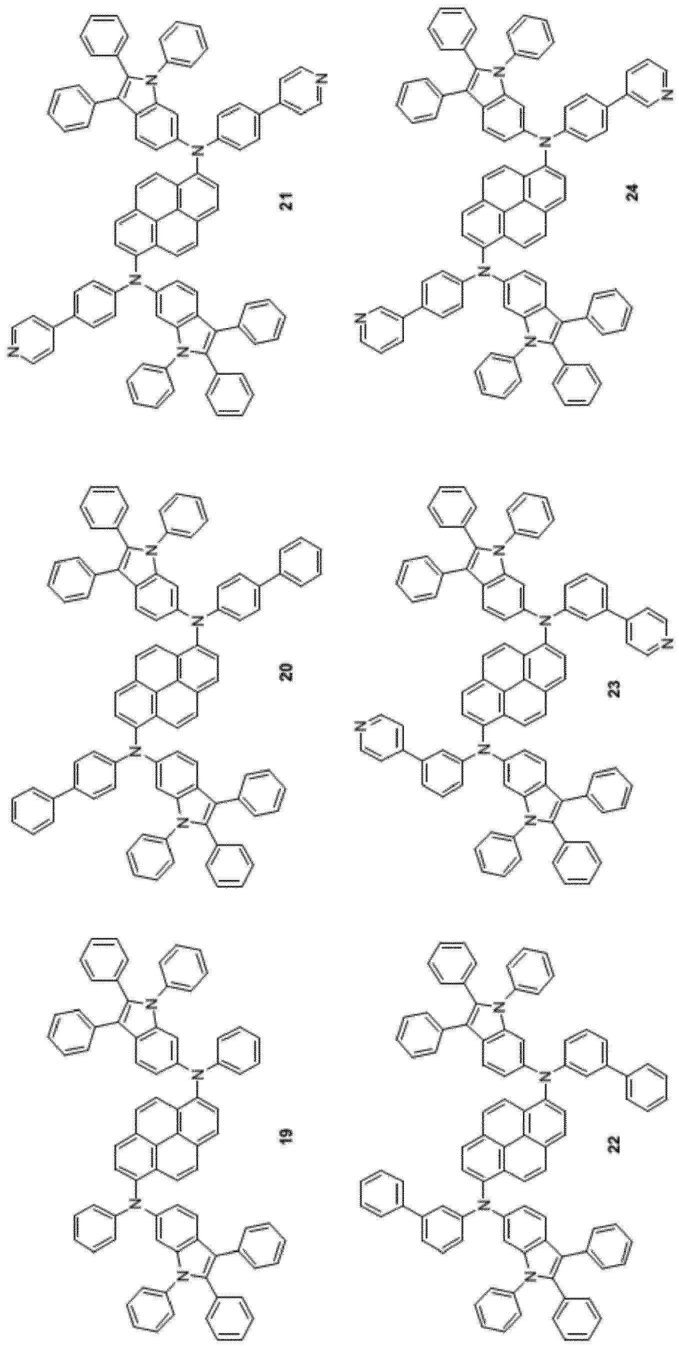

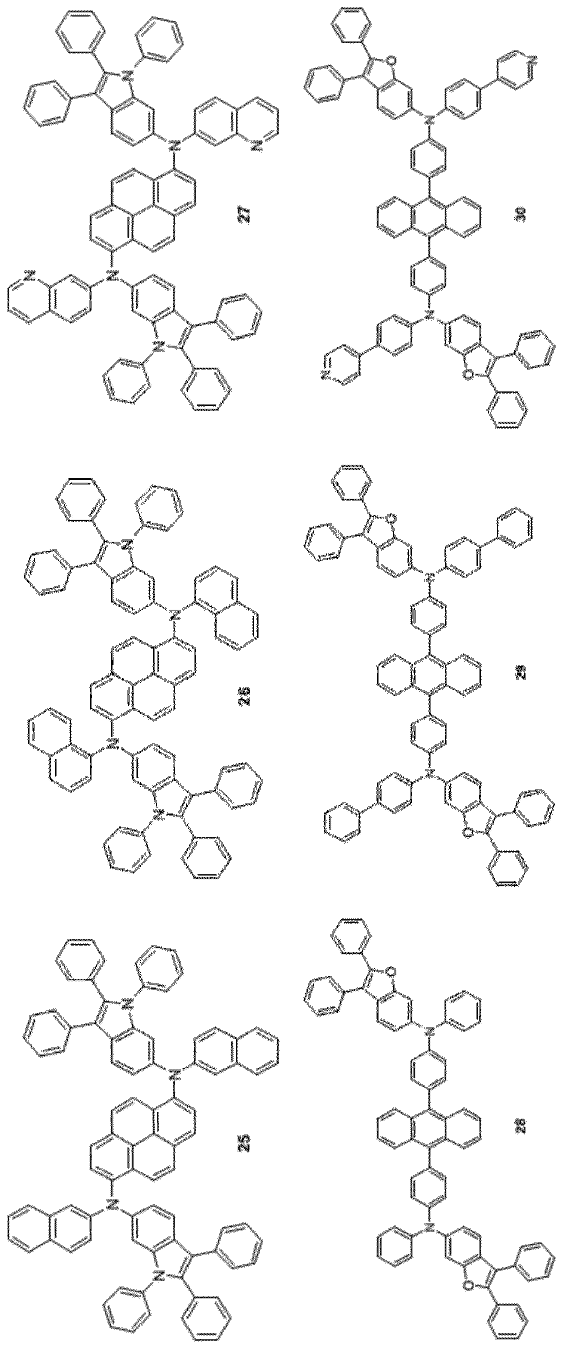

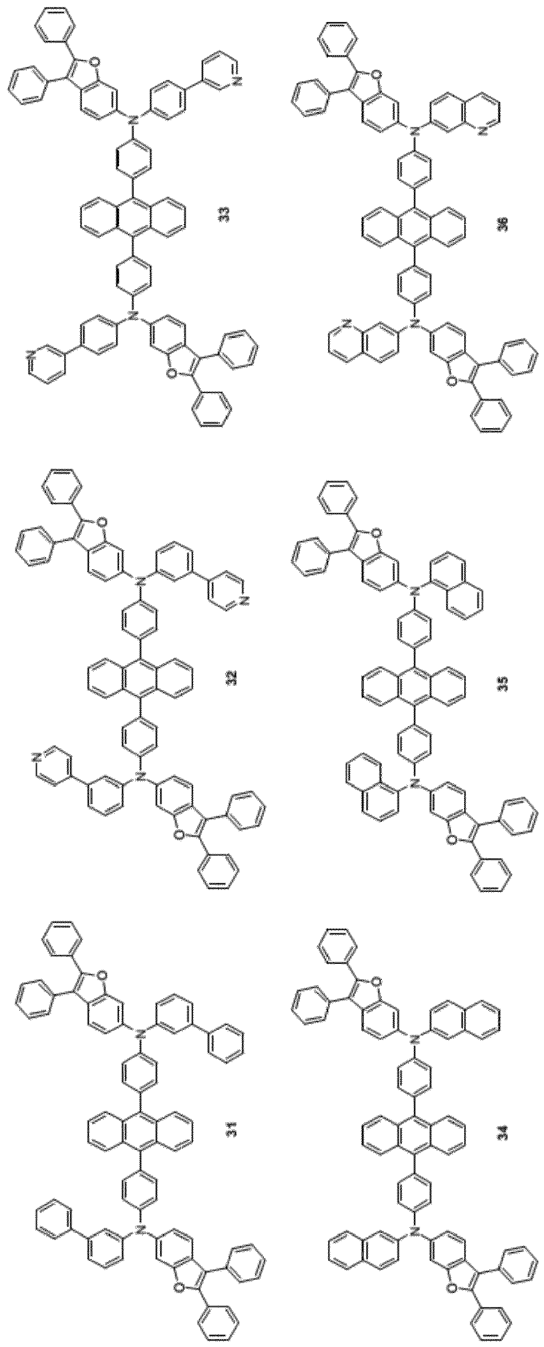

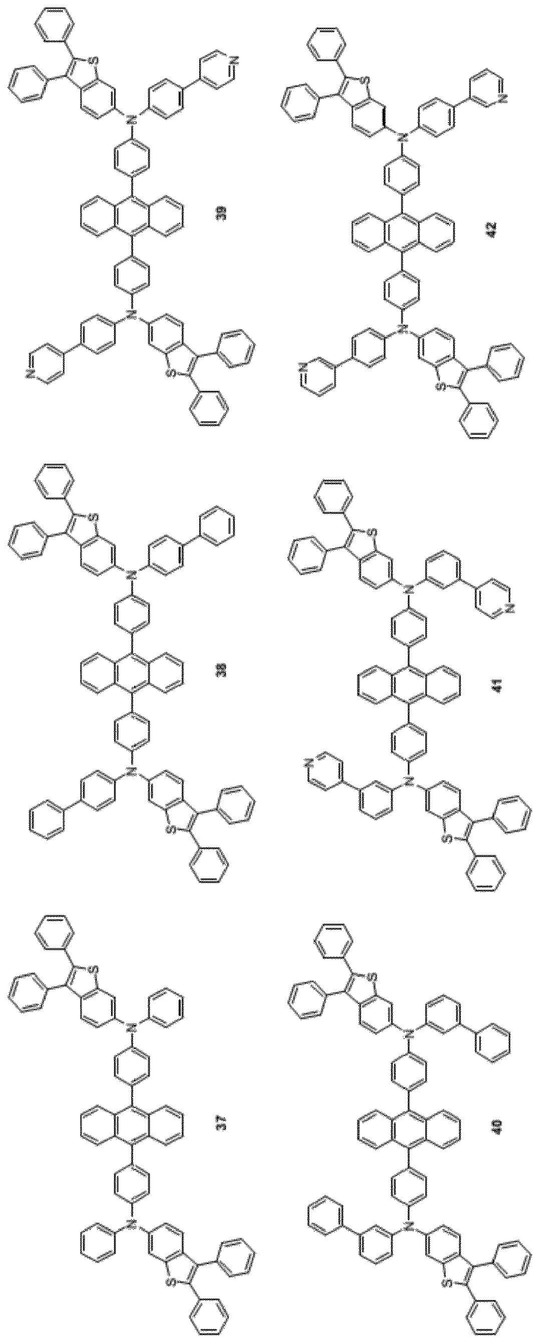

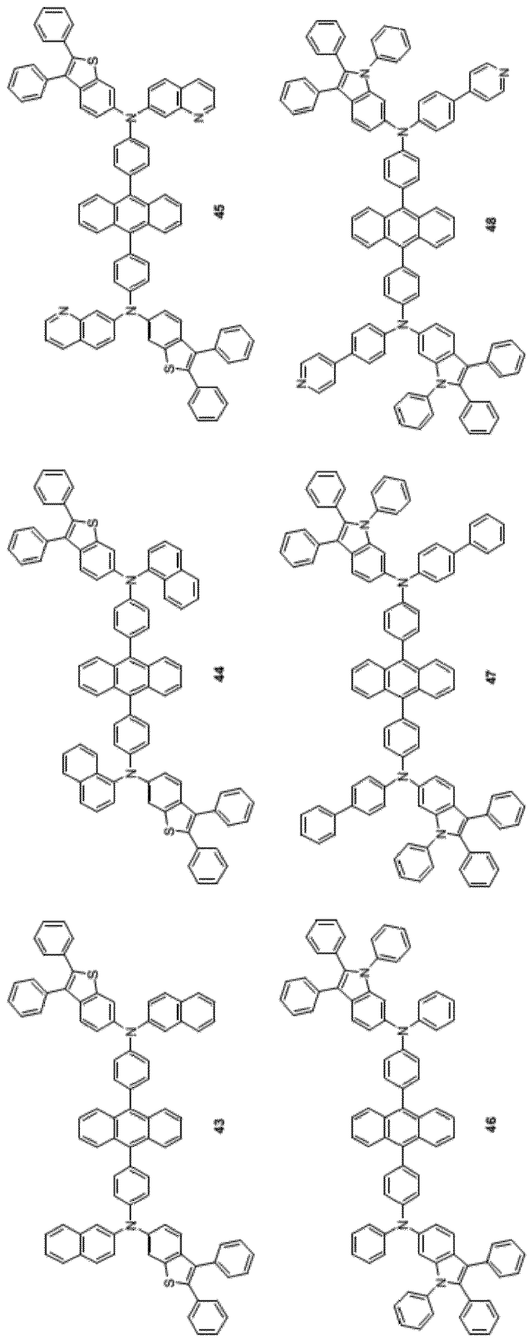

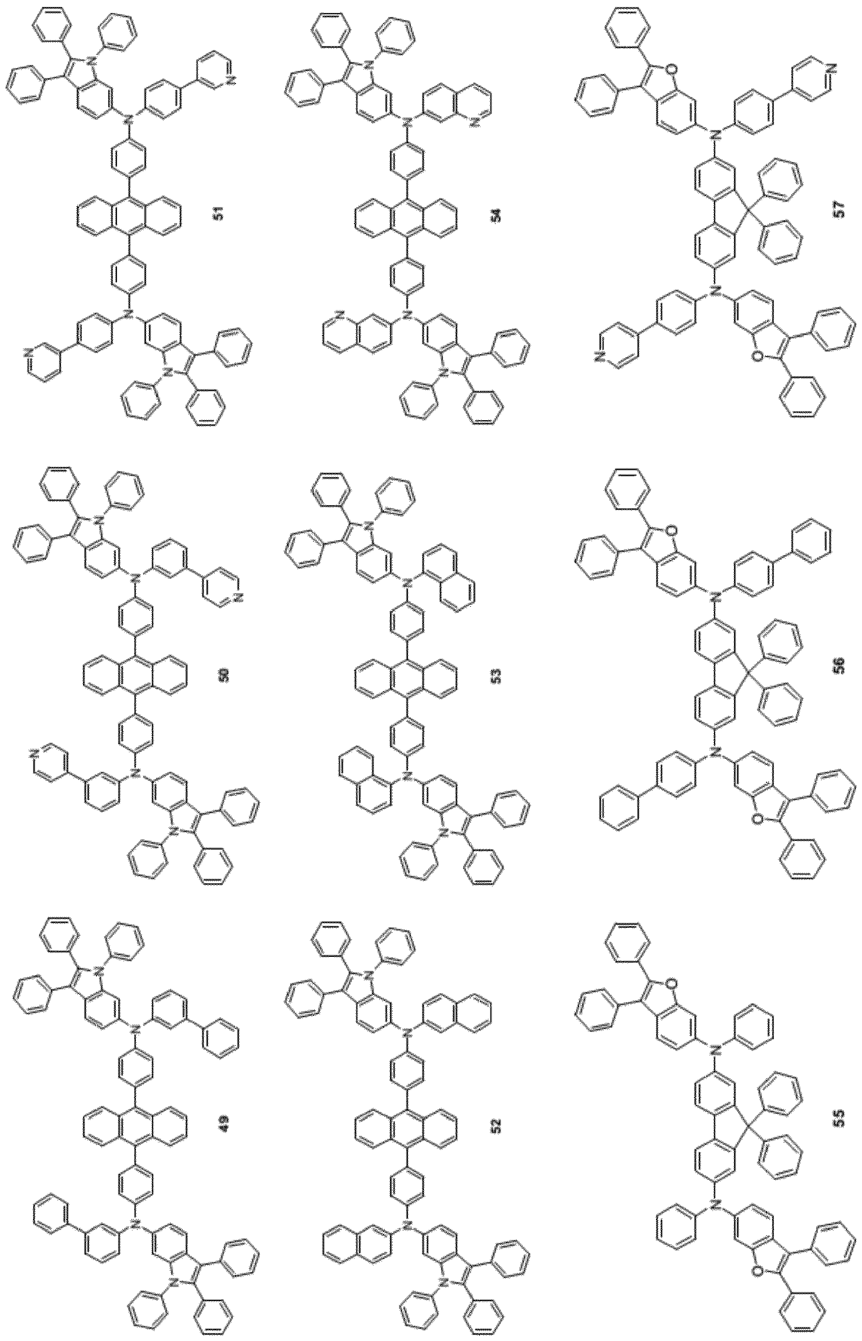

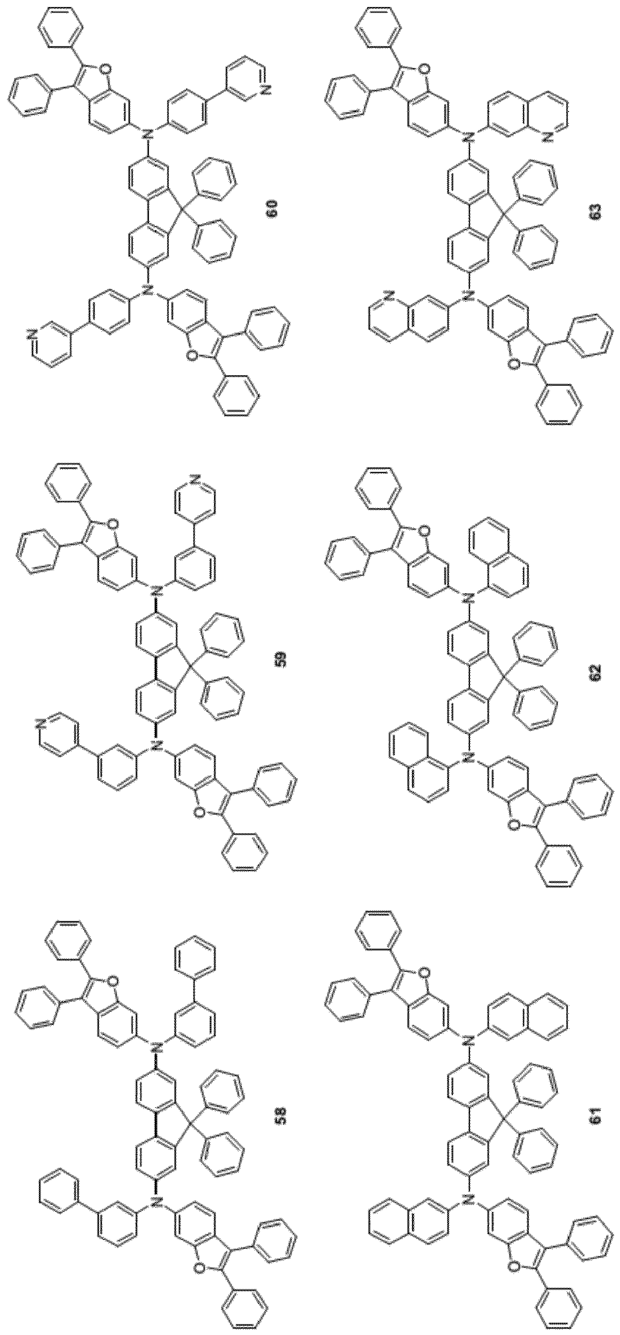

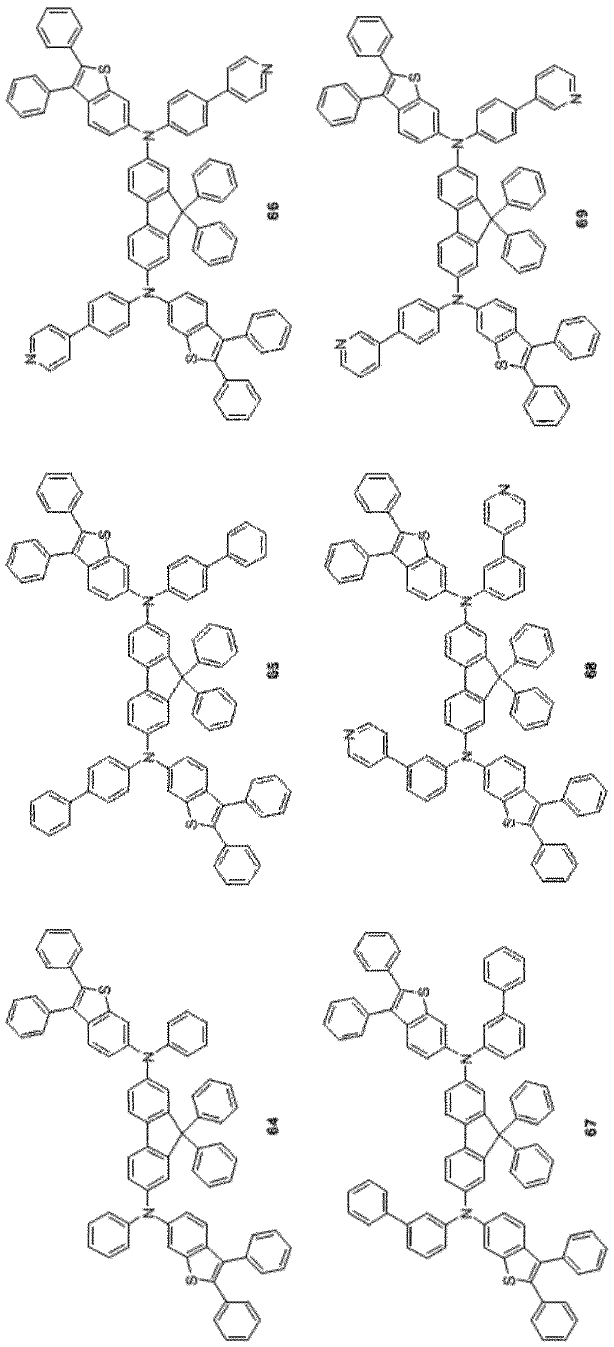

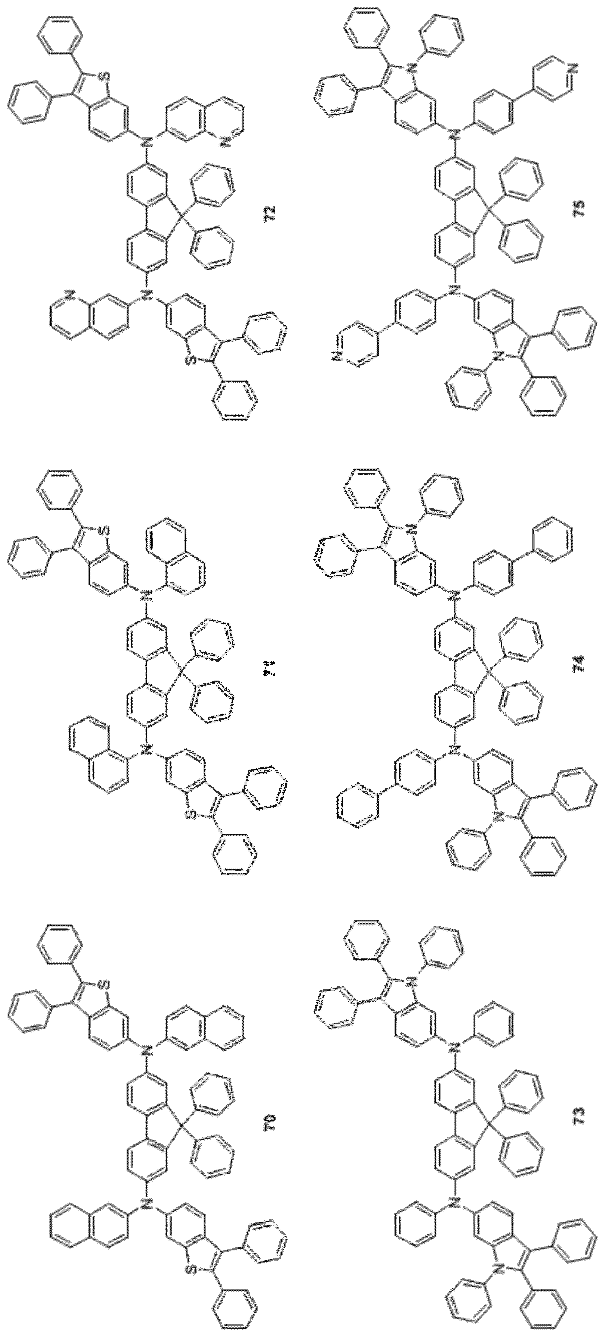

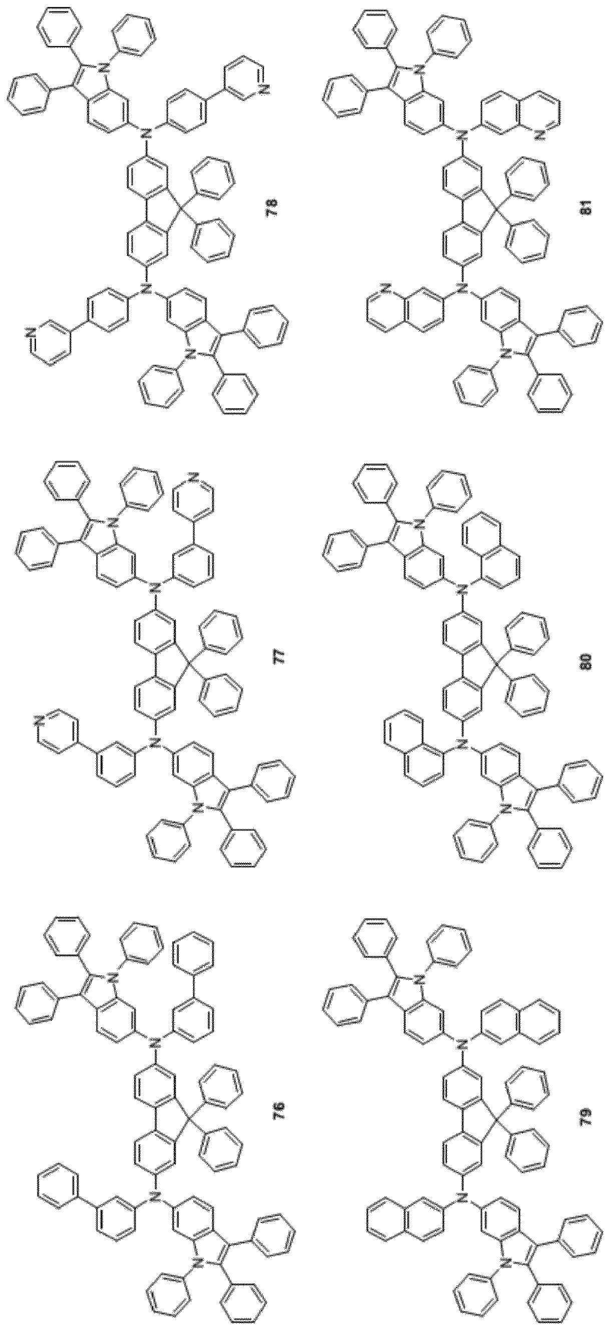

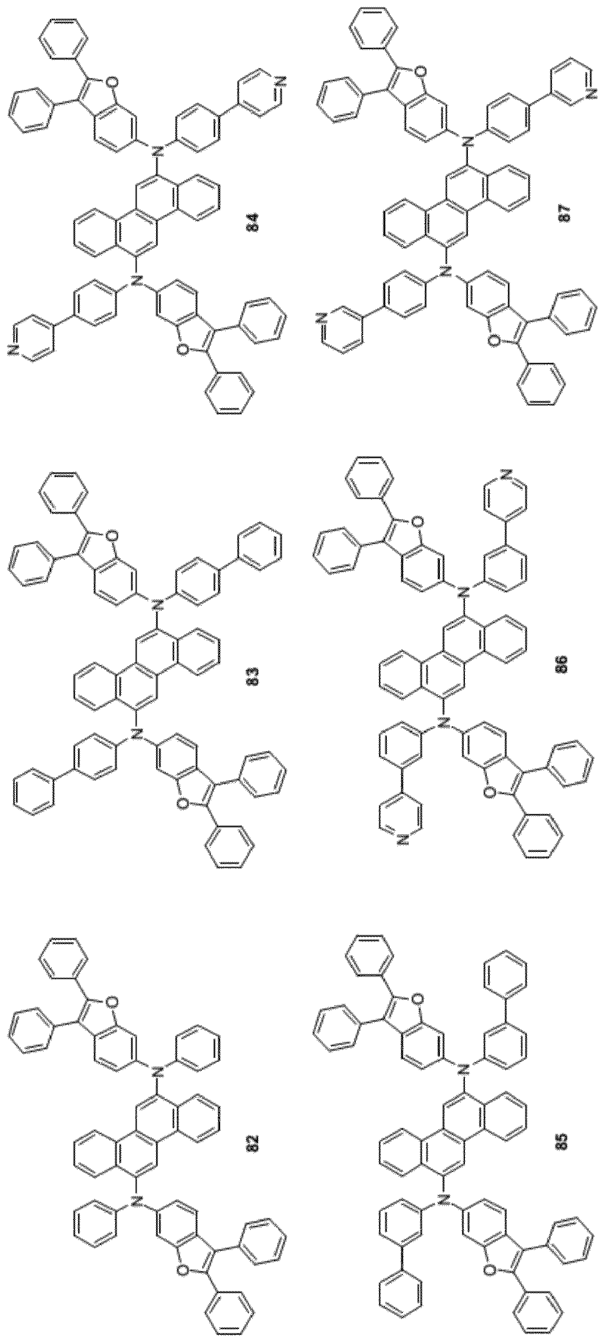

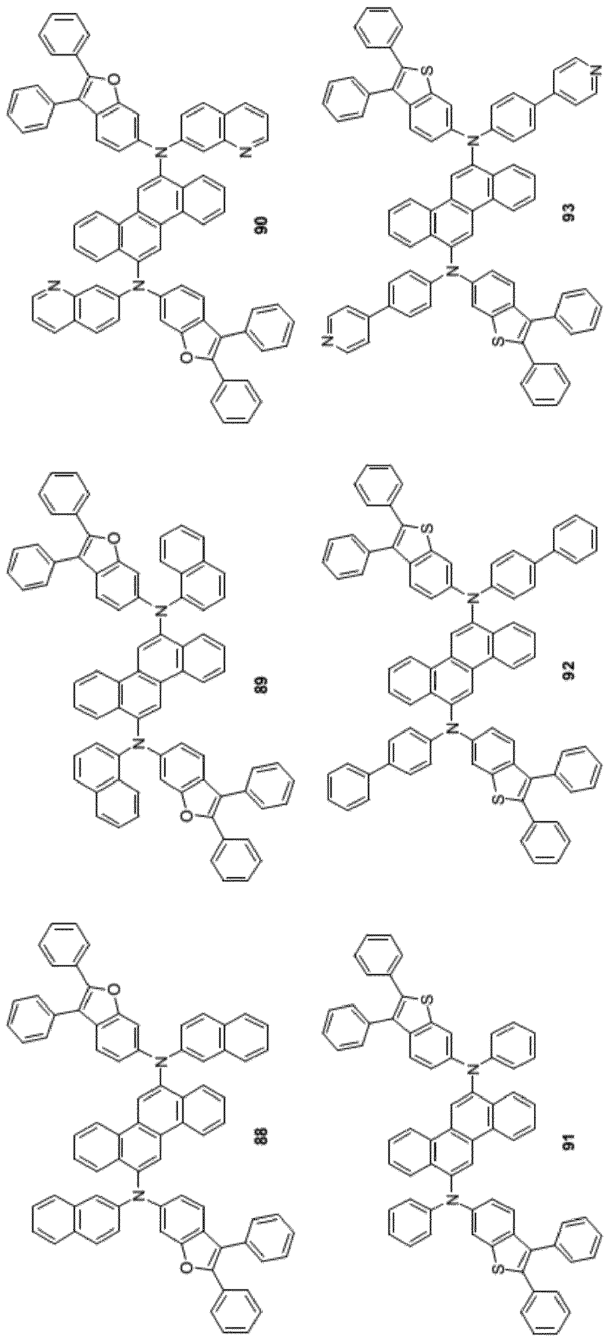

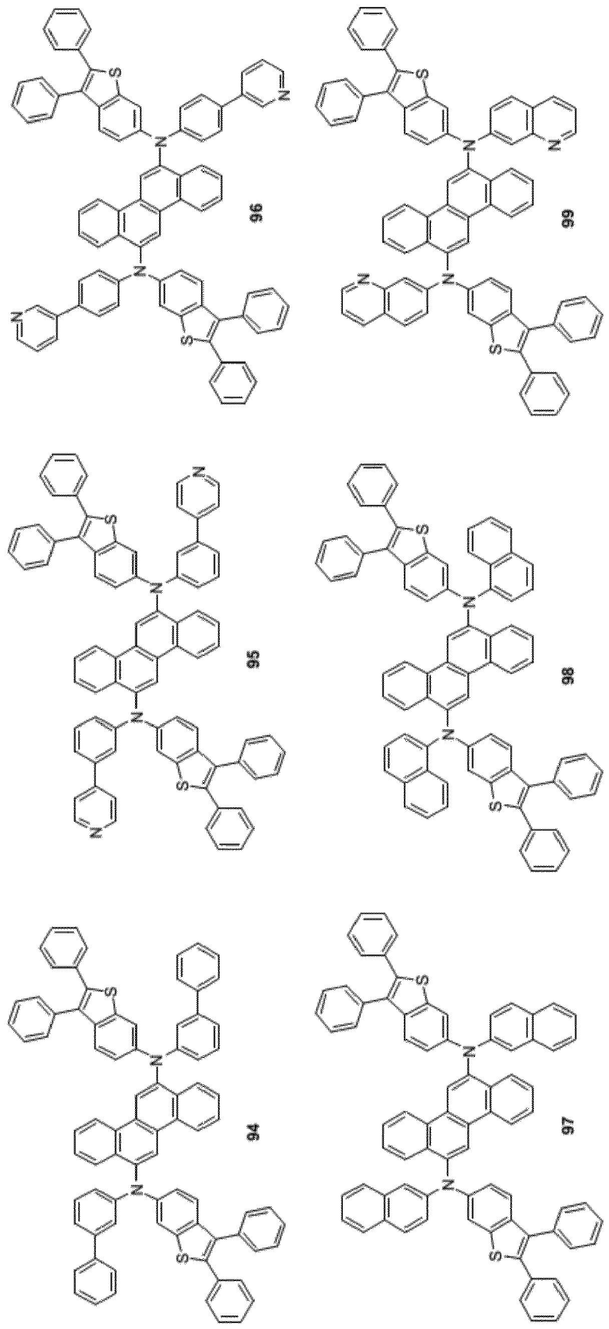

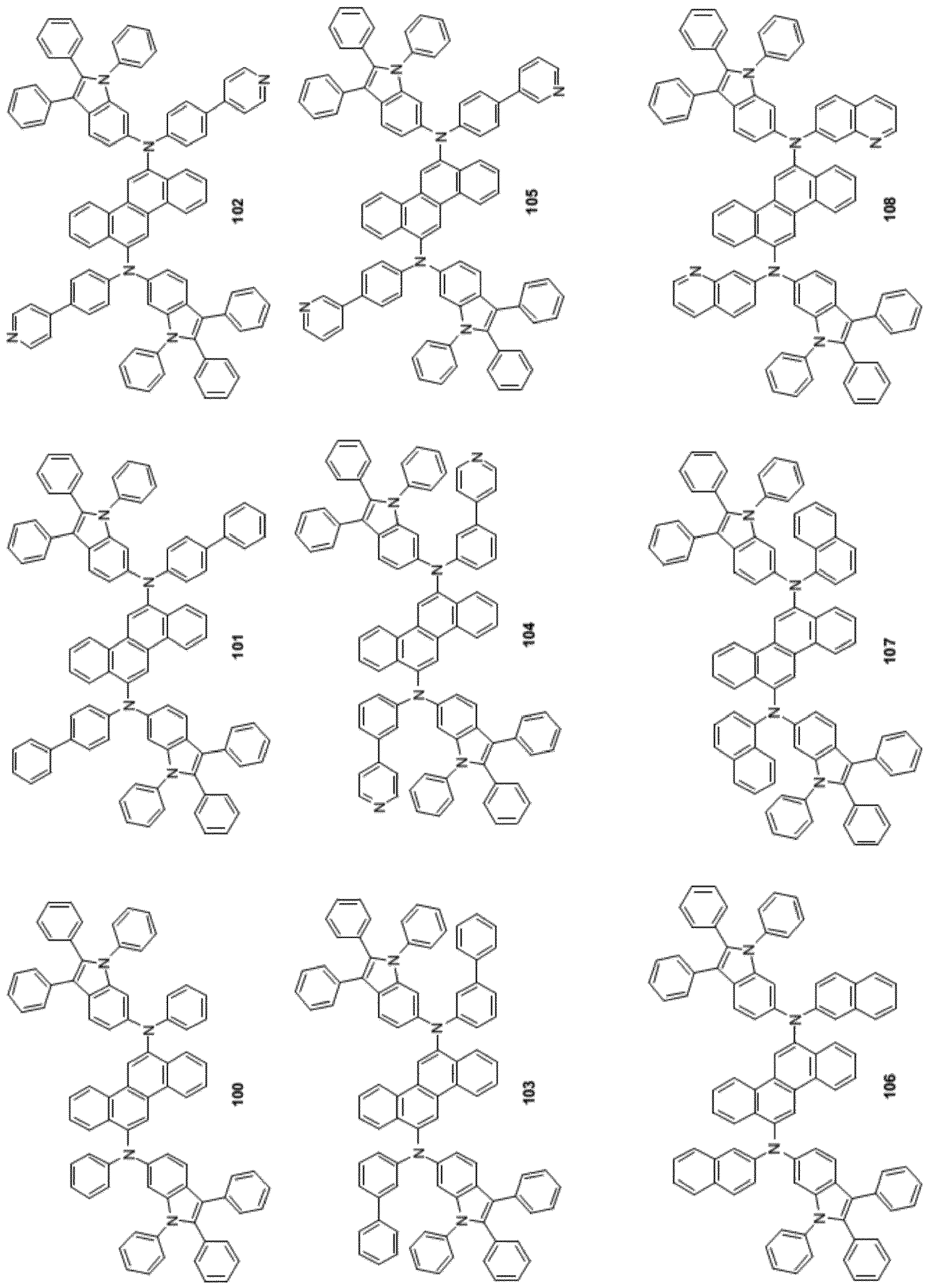

13. An organic light-emitting device, comprising:
a first electrode;
a second electrode; and
an organic layer disposed between the first electrode and the second electrode, and including an emission layer and at least one of the amine-based compounds of any one of claims 1 to 12.

14. The organic light-emitting device as claimed in claim 13, wherein the organic layer further includes a compound represented by Formula 1000 below:
<Formula 1000> Y₁-A₂-Y₂
wherein, in Formula 1000, A₂ is selected from
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, and
a pentalenylene group, an indenylene group, a naphthylene group, an azulenylene group, a heptalenylene group, an indacenylene group, an acenaphthylene group, a fluorenylene group, a spiro-fluorenylene group, a phenalenylene group, a phenanthrenylene group, an anthracenylene group, a fluoranthrenylene group, a triphenylenylene group, a pyrenylene group, a chrysenylene group, a naphthacenylene group, a picenylene group, a perylenylene group, a pentaphenylene group, a hexacenylene group, and a benzofluoranthenylene group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, - N(Q₁₁)(Q₁₂), and -Si(Q₁₃)(Q₁₄)(Q₁₅);
Y₁ and Y₂ are each independently selected from
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, a C₂-C₆₀ heteroaryl group, and -N(Q₁₀₁)(Q₁₀₂); and
Q₁₀₁ and Q₁₀₂ are each independently selected from
a hydrogen atom, a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group, and
a C₁-C₆₀ alkyl group, a C₂-C₆₀ alkenyl group, a C₂-C₆₀ alkynyl group, and a C₁-C₆₀ alkoxy group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group,
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, and
a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group, each substituted with at least one of a deuterium atom, a halogen atom, a hydroxyl group, a cyano group, a nitro group, an amino group, an amidino group, a hydrazine, a hydrazone, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ heterocycloalkyl group, a C₃-C₁₀ cycloalkenyl group, a C₃-C₁₀ heterocycloalkenyl group, a C₆-C₆₀ aryl group, a C₆-C₆₀ aryloxy group, a C₆-C₆₀ arylthio group, and a C₂-C₆₀ heteroaryl group.

15. The organic light-emitting device as claimed in claim 13 or 14, wherein the amine-based compound is in the emission layer.
